# EUROPEAN PATENT APPLICATION

(11) **EP 4 129 402 A1**
(43) Date of publication of application: **08.02.2023**
(21) Application number: 21778705.0
(22) Date of filing: 30.03.2021
(51) Int. Cl.: A61P 3/00, A61P 9/00, A61P 21/00, A61P 25/00, A61P 27/00, A61P 29/00, A61P 35/00, A61P 37/02, C07D 401/14, C07D 403/04, C07D 403/14, C07D 413/14, C07D 417/14, C07D 471/04, C07D 513/04, A61K 31/4178, A61K 31/422, A61K 31/4245, A61K 31/427, A61K 31/437, A61K 31/439

(54) **HYDROXYPYRROLIDINE DERIVATIVE AND MEDICINAL APPLICATION THEREOF**

(30) Priority: 31.03.2020 JP 2020065410
(71) Applicant: Mitsubishi Tanabe Pharma Corporation, Osaka-shi, Osaka 541-8505 (JP)
(72) Inventor: YAMAKOSHI, Shuhei, Fujisawa-shi, Kanagawa 251-0056 (JP); ISHIZAWA, Kouhei, Osaka-shi, Osaka 541-8505 (JP); HAGIHARA, Shuichi, Osaka-shi, Osaka 541-8505 (JP); SAKATA, Komei, Maidenhead, Berkshire SL68DE (GB); NIWA, Yasuki, Osaka-shi, Osaka 541-8505 (JP); TANAKA, Minoru, Osaka-shi, Osaka 541-8505 (JP)
(74) Representative: Hoffmann Eitle
(86) International application number: PCT/JP2021/013694
(87) International publication number: WO 2021/201036

(57) **Abstract**

The present invention provides a target protein degradation-inducing compound that is a bifunctional compound having a portion that binds to VHL, which is a substrate recognition protein of a ubiquitin ligase complex, at one end, and a portion that binds to a target protein at the other end. Specifically, a compound represented by the following structural formula (I): 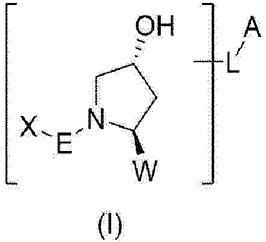 wherein each symbol is as defined in the present specification, or a pharmacologically acceptable salt thereof.

## Description

### [Technical Field]

The present invention relates to a target protein degradation-inducing compound that is a bifunctional compound having a portion that binds to Von-Hippel-Lindau, which is a substrate recognition protein of a ubiquitin ligase complex constituted of low-molecular-weight compounds (hereinafter sometimes to be referred to as VHL binding ligand), at one end, and a portion that is capable of binding or binds to a target protein (hereinafter sometimes to be referred to as target-directed ligand) at the other end, and is useful in the pharmaceutical field.

### [Background Art]

The ubiquitin-proteasome system is a system that degrades and removes proteins no longer needed in cells, through ubiquitination of target proteins by proteasomes in an ATP-dependent manner. The system plays an important role in cell proliferation, survival, maintenance of homeostasis, and the like. The ubiquitin-proteasome system involves multiple enzymes called ubiquitin activating enzyme (E1), ubiquitin conjugating enzyme (E2), and ubiquitin ligase (E3). The ubiquitination of the target protein is achieved by sequential functions of these enzymes. In particular, a target protein that has undergone so-called polyubiquitination, in which a ubiquitin chain is linked via the 48th lysine residue of ubiquitin, is efficiently recognized and degraded by the proteasome. It has been reported that humans have two types of ubiquitin activating enzymes, about 40 types of ubiquitin conjugating enzymes, and about 600 types of ubiquitin ligases. It is known that substrate recognition by ubiquitin ligases is strictly regulated. Ubiquitin ligases include those that function alone and those that form a complex to function. Particularly in the latter, a complex structure in which a substrate recognition protein binds to a scaffold protein via an adapter protein is taken. This substrate recognition protein determines the substrate protein to be ubiquitinated. In recent years, it has been reported that degradation of proteins that are not originally substrates for ubiquitin ligase can be induced by chemically linking a compound that binds to several types of proteins (target-directed ligand) and a compound that binds to a substrate recognition protein. To date, the selective induction of proteolysis (chemical knockdown) by low-molecular-weight compounds via ubiquitin ligase has been studied.

In recent years, a technique in which an artificial complex of E3 [VHL, Cereblon (CRBN), Cellular Inhibitor of Apoptosis Protein1 (cIAP1)] having ubiquitin ligase activity and a target protein is formed in the cell by using a compound in which a ligand for E3 and an inhibitor of the target protein are linked, and degradation of the target protein is induced using the ubiquitin-proteasome system, which is an intracellular protein degradation mechanism has been attracting attention as a new drug discovery technique (Non Patent Literature 1).

Patent Literature 1 discloses a bifunctional compound having, at one end, a VHL ligand that binds to VHL, which is a substrate recognition protein for ubiquitin ligase, and, at the other end, a portion that binds to a target protein.

Patent Literature 2 discloses a bifunctional compound having, at one end, a CRBN ligand that binds to CRBN, which is a substrate recognition protein for ubiquitin ligase, and, at the other end, a portion that binds to a target protein.

Patent Literature 3 discloses a bifunctional compound having a proline compound as a ligand for the substrate recognition protein IAP of the ubiquitin ligase complex.

### [Citation List]

### [Patent Literature]

[PTL 1]
   WO 2013/106643
[PTL 2]
   WO 2015/160845
[PTL 3]
   WO 2016/169989

### [Non Patent Literature]

[NPL 1]
Toure M., Angew Chem Int Ed Engl. 2016, 55: 1966-1973

### [Summary of Invention]

### [Technical Problem]

As described above, while selective induction of the degradation of target protein by bifunctional compounds has been studied, the range of applicable target proteins and the effect of degradation induction have not been necessarily sufficient. Therefore, research and development of bifunctional compounds by using low-molecular-weight compounds with higher effectiveness has been desired.

### [Solution to Problem]

The present inventors have conducted intensive studies in an attempt to solve the aforementioned problems, and completed the present invention. The present invention is as described below.
[1] A compound represented by the following structural formula (I) : 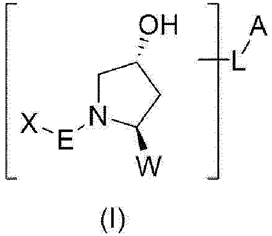
   wherein L is bonded to either W or X;
   E is a bond, -CO-, -SO- or -SO₂-;
   X is an optionally substituted alkyl group,
   an optionally substituted cycloalkyl group,
   an optionally substituted aryl group,
   an optionally substituted heterocyclic group, or
   an optionally substituted heteroaryl group;
   W is an optionally substituted aryl group,
   an optionally substituted fused heterocyclic group containing 1 to 6 same or different atoms selected from a nitrogen atom, an oxygen atom, and a sulfur atom and having 6 to 10 ring-constituting atoms,
   an optionally substituted heteroaryl group containing 1 to 6 same or different atoms selected from a nitrogen atom, an oxygen atom, and a sulfur atom and having 3 to 10 ring-constituting atoms,
   an alkyl group optionally substituted by a group selected from a halogen atom, a hydroxy group, an alkoxy group, and a heterocyclic group,
   a cyano group, or a hydrogen atom;
   L is a bond or a chemical linker; and
   A is a target-directed ligand
   (hereinafter at times referred to as compound (I)), or a pharmacologically acceptable salt thereof.
[2] The compound of the above-mentioned [1], wherein the compound represented by the structural formula (I) is a compound represented by the following structural formula (Ia) or (Ib): 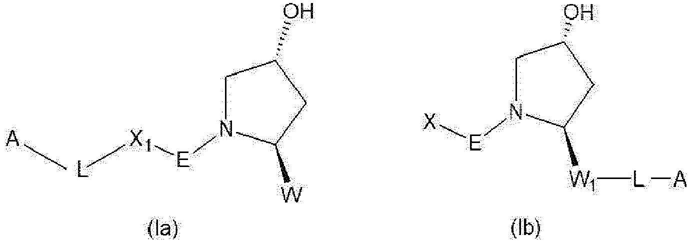 wherein E, X, W, L, and A are as defined above, and X₁ and W₁ are each a divalent group induced from X and W, or a pharmacologically acceptable salt thereof.
[3] The compound of the above-mentioned [1] or [2], wherein W is an optionally substituted fused heterocyclic group containing 1 to 6 same or different atoms selected from a nitrogen atom, an oxygen atom, and a sulfur atom and having 6 to 10 ring-constituting atoms,
   or a pharmacologically acceptable salt thereof.
[4] The compound of the above-mentioned [1] to [3], wherein W is a group represented by the formula: 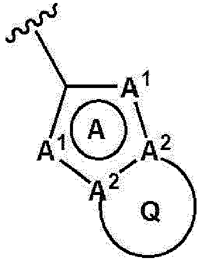
   wherein
   group A is an optionally substituted heteroaryl group having five ring-constituting atoms, two A¹ are each independently a group or atom selected from CR^{Z1}, N, NR^{Z2}, O, and S,
   two A² are each independently C or N; and
   ring Q is
   an aromatic hydrocarbocycle,
   an aromatic heterocycle optionally containing
   1 to 6 same or different atoms selected from a nitrogen atom, an oxygen atom, and a sulfur atom,
   a non-aromatic hydrocarbocycle, or
   a heterocycle optionally containing 1 to 6 same or different atoms selected from a nitrogen atom, an oxygen atom, and a sulfur atom,
   wherein the ring has 5 to 7 ring-constituting atoms and is optionally substituted at a substitutable position by a group selected from
   a halogen atom,
   a hydroxy group,
   a cyano group,
   a hydroxycarbonyl group,
   an oxo group,
   a thioxo group,
   an optionally substituted alkyl group,
   an optionally substituted cycloalkyl group,
   an optionally substituted alkoxy group,
   an optionally substituted cycloalkyloxy group,

      -CO-N(R^{7a})(R^{7b}),

      -N(R^{7a})(R^{7b}),

      -N (R^{7c})-CO-R^{7d}, and

      -CO-R^{7e},

      (in each of the above-mentioned formulas,
      R^{Z1} and R^{Z2} are each independently a hydrogen atom; a halogen atom; a hydroxy group; a cyano group; a hydroxycarbonyl group; an optionally substituted alkyl group; an optionally substituted cycloalkyl group; an optionally substituted alkoxy group; an optionally substituted alkoxycarbonyl group; - CO-N(R^{7a})(R^{7b}); -N(R^{7a})(R^{7b}); -N (R^{7c}) -CO-R^{7d}; an aryl group; or an optionally substituted heteroaryl group containing 1 to 3 same or different atoms selected from a nitrogen atom, an oxygen atom, and a sulfur atom and having 5 or 6 ring-constituting atoms (provided that R^{Z2} is not a halogen atom; hydroxy group; cyano group; hydroxycarbonyl group; -N(R^{7a})(R^{7b}), or - N(R^{7c})-CO-R^{7d});
      R^{7a} and R^{7b} are each independently a hydrogen atom; or an alkyl group optionally substituted by a group selected from a halogen atom, a hydroxy group, an alkoxy group, and a cyano group, or
      R^{7a} and R^{7b} are optionally bonded to each other to form, together with the adjacent nitrogen atom, a heterocycle optionally substituted by a group selected from a halogen atom, a hydroxy group, an alkoxy group, and a cyano group;
      R^{7c} and R^{7d} are each independently a hydrogen atom; or an alkyl group optionally substituted by a group selected from a halogen atom, a hydroxy group, an alkoxy group, and a cyano group; and
      R^{7e} is an optionally substituted alkyl group, an optionally substituted cycloalkyl group, an optionally substituted aryl group, or an optionally substituted heteroaryl group),
      or a pharmacologically acceptable salt thereof.
[5] The compound of the above-mentioned [1] to [4], wherein W is a group represented by the formula: 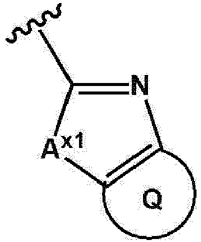
   wherein
   A^{x1} is a group or atom selected from -NR^{Z2}-, -O-, and -S-, ring Q is a non-aromatic hydrocarbocycle having 5 or 6 ring-constituting atoms; or
   a heterocycle optionally containing 1 to 4 same or different atoms selected from a nitrogen atom, an oxygen atom, and a sulfur atom and having 5 or 6 ring-constituting atoms, wherein the ring is optionally substituted at a substitutable position by a group selected from a halogen atom, an alkyl group having 1 to 6 carbon atoms, and -CO-R^{7e},
   wherein R^{z2} and R^{7e} are as defined above,
   or a pharmacologically acceptable salt thereof.
[6] The compound of the above-mentioned [1], wherein W is an optionally substituted heteroaryl group containing 1 to 6 same or different atoms selected from a nitrogen atom, an oxygen atom, and a sulfur atom and having 3 to 10 ring-constituting atoms, or a pharmacologically acceptable salt thereof.
[7] The compound of the above-mentioned [1] or [6], wherein W is a group represented by the formula: 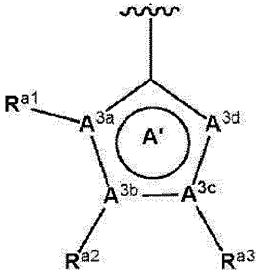 wherein
   group A' is a heteroaryl group having 5 ring-constituting atoms, A^{3a}, A^{3b}, A^{3c}, and A^{3d} are each independently, an atom selected from a nitrogen atom, an oxygen atom, a carbon atom, and a sulfur atom, when A^{3a}, A^{3b}, and A^{3c} have a nitrogen atom or a carbon atom, they respectively have R^{a1}, R^{a2}, and R^{a3},
      wherein R^{a1} is
      (1) a hydrogen atom,
      (2) an optionally substituted alkyl group,
      (3) an optionally substituted cycloalkyl group,
      (4) an optionally substituted aryl group,
      (5) an optionally substituted heteroaryl group, or
      (6) an optionally substituted heterocyclic group, and
   R^{a2} and R^{a3} are each independently
      (1) a hydrogen atom,
      (2) -CN,
      (3) a halogen atom,
      (4) an optionally substituted alkyl group,
      (5) an optionally substituted cycloalkyl group,
      (6) an optionally substituted aryl group,
      (7) an optionally substituted heteroaryl group,
      (8) an optionally substituted heterocyclic group,
      (9) -V^{a3a}- (optionally substituted alkyl),
      (10) -V^{a3a}- (optionally substituted cycloalkyl),
      (11) -V^{a3a}- (optionally substituted aryl),
      (12) -V^{a3a}- (optionally substituted heteroaryl), or
      (13) -V^{a3a}- (optionally substituted heterocyclic group)
         (in the above-mentioned formulas, V^{a3a} is
         1) -CO-,
         2) -NR^{Va3}-
            wherein R^{Va3} is
            (a) a hydrogen atom,
            (b) an optionally substituted alkyl group having 1 to 6 carbon atoms, or
            (c) an optionally substituted cycloalkyl group having 3 to 6 carbon atoms,
         3) -O-,
         4) -S-,
         5) -SO-, or
         6) -SO₂-, or
      (14) -V^{a3b}-NR^{Na3}R^{Na3'}
         wherein V^{a3b} is
         1) -CO-
         2) -SO-, or
         3) -SO₂-, and
   R^{Na3} and R^{Na3'} are each independently
      1) a hydrogen atom,
      2) an optionally substituted alkyl group,
      3) an optionally substituted cycloalkyl group,
      4) an optionally substituted aryl group,
      5) an optionally substituted heteroaryl group, or
      6) an optionally substituted heterocyclic group,
      or a pharmacologically acceptable salt thereof.
[8] The compound of the above-mentioned [7], wherein the group A' is an imidazolyl group, a pyrazolyl group, a thiazolyl group, an isothiazolyl group, a thiadiazolyl group, an isothiadiazolyl group, an oxazolyl group, an isoxazolyl group, an oxadiazolyl group, a triazolyl group, or a tetrazolyl group, or a pharmacologically acceptable salt thereof.
[9] The compound of the above-mentioned [8], wherein the group A' is a group selected from an imidazolyl group, a thiazolyl group, and an oxazolyl group, or a pharmacologically acceptable salt thereof.
[10] The compound of the above-mentioned [1], [6] to [9], wherein
   W is a group represented by the formula: 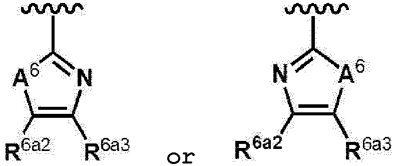 wherein
   A⁶ is -O-, -S-, or -NR^{6a1}-
   wherein R^{6a1} is
      (1) a hydrogen atom,
      (2) an optionally substituted alkyl group,
      (3) an optionally substituted cycloalkyl group,
      (4) an optionally substituted aryl group,
      (5) an optionally substituted heteroaryl group, or
      (6) an optionally substituted heterocyclic group;
   R^{6a2} is
      (1) a hydrogen atom,
      (2) CN,
      (3) a halogen atom,
      (4) an optionally substituted alkyl group having 1 to 4 carbon atoms,
      (5) an optionally substituted cyclopropyl group,
      (6) an optionally substituted oxetanyl group,
      (7) an optionally substituted azetidinyl group,
      (8) an optionally substituted aryl group having 6 to 10 carbon atoms, or
      (9) -CO-NR^{a2N6}R^{a2N6'}
   wherein R^{a2N6} and R^{a2N6'} are each independently
      1) a hydrogen atom,
      2) an optionally substituted alkyl group having 1 to 4 carbon atoms,
      3) an optionally substituted cyclopropyl group,
      4) an optionally substituted oxetanyl group, or
      5) an optionally substituted azetidinyl group; and
   R6a3 is
      (1) a hydrogen atom,
      (2) -CN,
      (3) a halogen atom,
      (4) an optionally substituted alkyl group,
      (5) an optionally substituted cycloalkyl group,
      (6) an optionally substituted aryl group,
      (7) an optionally substituted heteroaryl group,
      (8) an optionally substituted heterocyclic group,
      (9) -CO-(optionally substituted heterocyclic group), or
      (10) -CO-NR^{a3N6}R^{a3N6'}
   wherein R^{a3N6} and R^{a3N6'} are each independently
      1) a hydrogen atom,
      2) an optionally substituted alkyl group,
      3) an optionally substituted cycloalkyl group,
      4) an optionally substituted aryl group,
      5) an optionally substituted heteroaryl group, or
      6) an optionally substituted heterocyclic group,
      or a pharmacologically acceptable salt thereof.
[11] The compound of the above-mentioned [1], [6] to [10], wherein
   W is a group represented by the formula: 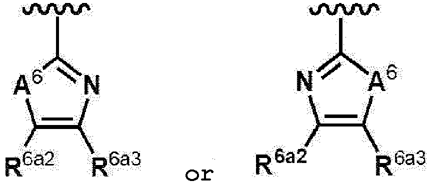 wherein
   A⁶ is -S-, or -NR^{6a1}-
   wherein R^{6a1} is
      (1) a hydrogen atom, or
      (2) an optionally substituted alkyl group;
   R^{6a2} is
      (1) a hydrogen atom,
      (2) an optionally substituted alkyl group having 1 to 4 carbon atoms,
      (3) an optionally substituted aryl group having 6 to 10 carbon atoms, or
      (4) -CO-NR^{a2N6}R^{a2N6'}
   wherein R^{a2N6} and R^{a2N6'} are each independently an optionally substituted alkyl group having 1 to 4 carbon atoms; and
   R6a3 is
      (1) a hydrogen atom,
      (2) an optionally substituted alkyl group,
      (3) an optionally substituted aryl group,
      (4) -CO-(optionally substituted heterocyclic group), or
      (5) -CO-NR^{a3N6}R^{a3N6'}
   wherein R^{a3N6} and R^{a3N6'} are each independently
      1) a hydrogen atom,
      2) an optionally substituted alkyl group,
      3) an optionally substituted cycloalkyl group, or
      4) an optionally substituted heterocyclic group,
      or a pharmacologically acceptable salt thereof.
[12] The compound of [10] or [11], wherein
   R^{6a1} is a hydrogen atom, and
   R^{6a3} is a hydrogen atom or an alkyl group having 1 to 6 carbon atoms,
   or a pharmacologically acceptable salt thereof.
[13] The compound of [12], wherein
   A⁶ is -S- or -NH-, and
   R^{6a2} is hydrogen,
   or a pharmacologically acceptable salt thereof.
[14] The compound of the above-mentioned [10] or [11], wherein
   R^{6a1} is a hydrogen atom, and
   R^{6a3} is
      (1) -Y^{6a}-W^{6a}
      wherein
   Y^{6a} is
      1) a bond,
      2) -(CR^{Y1}R^{Y1'})_{ny6a}-A^{Y6a}-(CR^{Y2}R^{Y2'})_{ny6b}-
      wherein
   R^{Y1}, R^{Y1'}, R^{Y2}, and R^{Y2'} are each independently
      (a) a hydrogen atom,
      (b) a halogen atom,
      (c) -O-(optionally substituted alkyl having 1 to 6 carbon atoms),
      (d) -O-(optionally substituted cycloalkyl having 3 to 6 carbon atoms),
      (e) an optionally substituted alkyl group having 1 to 6 carbon atoms, or
      (f) an optionally substituted cycloalkyl group having 3 to 6 carbon atoms, or
      (g) R^{Y1} and R^{Y1'}, and R^{Y2} and R^{Y2'}, are each independently optionally bonded to each other to form, together with the adjacent carbon atom, an optionally substituted cycloalkyl group having 3 to 10 carbon atoms, or an optionally substituted heterocycle containing 1 to 6 same or different atoms selected from a nitrogen atom, an oxygen atom, and a sulfur atom and having 3 to 10 ring-constituting atoms,
   ny6a is an integer of 1 to 6,
   A^{Y6a} is
      (a) a bond,
      (b) -CO-,
      (c) -O-,
      (d) -SO-,
      (e) -SO₂-,
      (f) -NR^{NY6a}-,
         wherein R^{NY6a} is
         (i) a hydrogen atom,
         (ii) an optionally substituted alkyl group having 1 to 6 carbon atoms,
         (iii) an optionally substituted cycloalkyl group having 3 to 6 carbon atoms,
         (iv) an optionally substituted heterocyclic group having 3 to 6 ring-constituting atoms,
         (v) -CO-(optionally substituted alkyl having 1 to 6 carbon atoms), or
         (vi) -CO-(optionally substituted cycloalkyl having 3 to 6 carbon atoms), or
      (g) -V^{Y6b}-NR^{NY6b}- or -NR^{NY6b}-V^{Y6b}-
         wherein V^{Y6b} is
         (i) -CO-,
         (ii) -SO-, or
         (iii) -SO₂-,
   R^{NY6b} is
      (i) a hydrogen atom,
      (ii) an optionally substituted alkyl group having 1 to 6 carbon atoms,
      (iii) an optionally substituted cycloalkyl group having 3 to 6 carbon atoms, or
      (iv) an optionally substituted heterocyclic group having 3 to 6 ring-constituting atoms,
   ny6b is an integer of 0 to 6, and
   W^{6a} is
      1) a hydrogen atom,
      2) a halogen atom,
      3) an optionally substituted cycloalkyl group having 3 to 10 carbon atoms,
      4) an optionally substituted aryl group having 6 to 10 carbon atoms,
      5) an optionally substituted heteroaryl group containing 1 to 6 same or different atoms selected from a nitrogen atom, an oxygen atom, and a sulfur atom and having 5 to 10 ring-constituting atoms, or
      6) an optionally substituted heterocyclic group containing 1 to 6 same or different atoms selected from a nitrogen atom, an oxygen atom, and a sulfur atom and having 3 to 10 ring-constituting atoms,
      or a pharmacologically acceptable salt thereof.
[15] The compound of the above-mentioned [14], wherein
   A⁶ is -S- or -NR^{6a1}-,
   R^{6a1} is a hydrogen atom, and
   R6a3 is
      (1) -Y^{6a}-W^{6a}
      wherein
   Y^{6a} is
      1) a bond,
      2) -(CR^{Y1}R^{Y1'})_{ny6a}-A^{Y6a}-(CR^{Y2}R^{Y2'})_{ny6b}-
      wherein
   R^{Y1}, R^{Y1'}, R^{Y2}, and R^{Y2'} are each independently
      (a) a hydrogen atom,
   ny6a is an integer of 1 to 6,
   A^{Y6a} is
      (a) a bond,
      (b) -O-,
      (c) -NR^{NY6a}-,
         wherein R^{NY6a} is
         an optionally substituted alkyl group having 1 to 6 carbon atoms, or
      (d) -V^{Y6b}-NR^{NY6b}- or -NR^{NY6b}-V^{Y6b}-
   wherein V^{Y6b} is

      -CO-,
   R^{NY6b} is
   an optionally substituted alkyl group having 1 to 6 carbon atoms,
   ny6b is an integer of 0 to 6, and
   W^{6a} is
      1) a hydrogen atom,
      2) an optionally substituted cycloalkyl group having 3 to 10 carbon atoms,
      3) an optionally substituted aryl group having 6 to 10 carbon atoms,
   or a pharmacologically acceptable salt thereof.
[16] The compound of the above-mentioned [14] or [15], wherein
   A⁶ is -S- or -NR^{6a1}-,
   R^{6a1} is a hydrogen atom, and
   R6a3 is
      (1) -Y^{6a}-W^{6a}
      wherein
   Y^{6a} is
      1) a bond,
      2) -(CR^{Y1}R^{Y1'})_{ny6a}-A^{Y6a}-(CR^{Y2}R^{Y2'})_{ny6b}-
      wherein
   R^{Y1}, R^{Y1'}, R^{Y2}, and R^{Y2'} are each independently
   a hydrogen atom,
   ny6a is an integer of 1 to 6,
   A^{Y6a} is
      (a) a bond
      (b) -O-,
      (c) -NR^{NY6a}-,
         wherein R^{NY6a} is
         an alkyl group having 1 to 6 carbon atoms which is optionally substituted by a substituent selected from a cycloalkyl group having 3 to 6 carbon atoms and an aryl group having 6 to 10 carbon atoms, or
      (d) -V^{Y6b}-NR^{NY6b}- or -NR^{NY6b}-V^{Y6b}-
   wherein V^{Y6b} is

      -CO-,
   R^{NY6b} is
   an alkyl group having 1 to 6 carbon atoms which is optionally substituted by a substituent selected from a cycloalkyl group having 3 to 6 carbon atoms and an aryl group having 6 to 10 carbon atoms,
   ny6b is an integer of 0 to 6, and
   W^{6a} is
      1) a hydrogen atom,
      2) a cycloalkyl group having 3 to 10 carbon atoms, or
      3) an aryl group having 6 to 10 carbon atoms,
   or a pharmacologically acceptable salt thereof.
[17] The compound of the above-mentioned [14] to [16], wherein
   W^{6a} is

      -W^{6a'}-(CR^{Y3}R^{Y3'}) _{ny6c}-A^{Y6b}-(CR^{Y4}R^{Y4'}) _{ny6d}-W^{6b}

      wherein
   W^{6a'} is
      (1) an optionally substituted cycloalkyl divalent group having 3 to 10 carbon atoms,
      (2) an optionally substituted aryl divalent group having 6 to 10 carbon atoms,
      (3) an optionally substituted heteroaryl divalent group containing 1 to 6 same or different atoms selected from a nitrogen atom, an oxygen atom, and a sulfur atom and having 5 to 10 ring-constituting atoms, or
      (4) an optionally substituted heterocyclic divalent group containing 1 to 6 same or different atoms selected from a nitrogen atom, an oxygen atom, and a sulfur atom and having 3 to 10 ring-constituting atoms,
   R^{Y3}, R^{Y3'}, R^{Y4}, and R^{Y4'} are each independently
      (1) a hydrogen atom,
      (2) a halogen atom,
      (3) -O-(optionally substituted alkyl having 1 to 6 carbon atoms),
      (4) -O-(optionally substituted cycloalkyl having 3 to 6 carbon atoms),
      (5) an optionally substituted alkyl group having 1 to 6 carbon atoms,
      (6) an optionally substituted cycloalkyl group having 3 to 6 carbon atoms, or
      (7) R^{Y3} and R^{Y3'}, and R^{Y4} and R^{Y4'}, are each independently optionally bonded to each other to form, together with the adjacent carbon atom, an optionally substituted cycloalkyl group having 3 to 10 carbon atoms, or an optionally substituted heterocycle containing 1 to 6 same or different atoms selected from a nitrogen atom, an oxygen atom, and a sulfur atom and having 3 to 10 ring-constituting atoms,
   A^{Y6b} is
      (1) a bond,
      (2) -O-,
      (3) -SO-,
      (4) -SO₂-,
      (5) -NR^{NY6c}-,
         wherein R^{NY6c} is
         1) a hydrogen atom,
         2) an optionally substituted alkyl group having 1 to 6 carbon atoms,
         3) an optionally substituted cycloalkyl group having 3 to 6 carbon atoms,
         4) an optionally substituted heterocyclic group having 3 to 6 carbon atoms,
         5) -CO-(optionally substituted alkyl having 1 to 6 carbon atoms), or
         6) -CO-(optionally substituted cycloalkyl having 3 to 6 carbon atoms), or
      (6) -V^{Y6d}-NR^{NY6d}- or -NR^{NY6d}-V^{Y6d}-
   wherein V^{Y6d} is
      (a) -CO-,
      (b) -SO-, or
      (c) -SO₂-, and
   R^{NY6d} is
      1) a hydrogen atom,
      2) an optionally substituted alkyl group having 1 to 6 carbon atoms,
      3) an optionally substituted cycloalkyl group having 3 to 6 carbon atoms, or
      4) an optionally substituted heterocyclic group having 3 to 6 carbon atoms,
   ny6c and ny6d are each independently an integer of 0 to 6,
   W^{6b} is
      (1) a hydrogen atom,
      (2) a halogen atom,
      (3) an optionally substituted aryl group having 6 to 10 carbon atoms,
      (4) an optionally substituted heteroaryl group containing 1 to 6 same or different atoms selected from a nitrogen atom, an oxygen atom, and a sulfur atom and having 5 to 10 ring-constituting atoms,
      (5) an optionally substituted cycloalkyl group having 3 to 10 carbon atoms,
      (6) an optionally substituted heterocyclic group containing 1 to 6 same or different atoms selected from a nitrogen atom, an oxygen atom, and a sulfur atom and having 3 to 10 ring-constituting atoms,
      (7) -CO-(optionally substituted heterocyclic group containing 1 to 6 same or different atoms selected from a nitrogen atom, an oxygen atom, and a sulfur atom and having 3 to 10 ring-constituting atoms),
      (8) -CONR^{NW6}R^{NW6'}
         wherein R^{NW6} and R^{NW6'} are each independently
         1) a hydrogen atom,
         2) an optionally substituted alkyl group having 1 to 6 carbon atoms, or
         3) an optionally substituted cycloalkyl group having 3 to 6 carbon atoms, or
      (9) -NR^{NW6"}R^{NW6‴}
         wherein R^{NW6"} and R^{NW6‴} are each independently
         1) a hydrogen atom,
         2) an optionally substituted alkyl group having 1 to 6 carbon atoms,
         3) an optionally substituted cycloalkyl group having 3 to 6 carbon atoms,
         4) an optionally substituted heterocyclic group having 3 to 6 carbon atoms,
         5) -CO-(optionally substituted alkyl having 1 to 6 carbon atoms), or
         6) -CO-(optionally substituted cycloalkyl having 3 to 6 carbon atoms),
   or a pharmacologically acceptable salt thereof.
[18] The compound of the above-mentioned [14] to [17], wherein
   W^{6a} is

      -W^{6a'}-(CR^{Y3}R^{Y3'})_{ny6c}-A^{Y6b}-(CR^{Y4}R^{Y4'})_{ny6d}-W^{6b}

      wherein
   W^{6a'} is
      (1) an optionally substituted cycloalkyl divalent group having 3 to 10 carbon atoms,
      (2) an optionally substituted aryl divalent group having 6 to 10 carbon atoms,
   R^{Y3}, R^{Y3'}, R^{Y4}, and R^{Y4'} are each independently
      (1) a hydrogen atom, or
      (2) a halogen atom,
   A^{Y6b} is
      (1) a bond,
      (2) -O-, or
      (3) -V^{Y6d}-NR^{NY6d}-
   wherein V^{Y6d} is
      (a) -CO-, and
   R^{NY6d} is
   an optionally substituted alkyl group having 1 to 6 carbon atoms,
   ny6c and ny6d are each independently an integer of 0 to 6,
   W^{6b} is
      (1) a hydrogen atom,
      (2) a halogen atom, or
      (3) an optionally substituted heteroaryl group containing 1 to 6 same or different atoms selected from a nitrogen atom, an oxygen atom, and a sulfur atom and having 5 to 10 ring-constituting atoms,
   or a pharmacologically acceptable salt thereof.
[19] The compound of the above-mentioned [14] to [18], wherein
   W^{6a} is

      -W^{6a'}-(CR^{Y3}R^{Y3'})_{ny6c}-A^{Y6b}-(CR^{Y4}R^{Y4'})_{ny6d}-W^{6b}

      wherein
   W^{6a'} is
      (1) a cycloalkyl divalent group having 3 to 10 carbon atoms,
      (2) an aryl divalent group having 6 to 10 carbon atoms,
   R^{Y3}, R^{Y3'}, R^{Y4}, and R^{Y4'} are each independently
      (1) a hydrogen atom, or
      (2) a halogen atom,
   A^{Y6b} is
      (1) a bond,
      (2) -O-, or
      (3) -V^{Y6d}-NR^{NY6d}-
   wherein V^{Y6d} is
   -CO-, and
   R^{NY6d} is
   an alkyl group having 1 to 6 carbon atoms,
   ny6c and ny6d are each independently an integer of 0 to 6, and W^{6b} is
      (1) a hydrogen atom,
      (2) a halogen atom, or
      (3) a heteroaryl group containing 1 to 4 same or different atoms selected from a nitrogen atom and a sulfur atom and having 5 or 6 ring-constituting atoms, which is optionally substituted by an alkyl group having 1 to 6 carbon atoms,
   or a pharmacologically acceptable salt thereof.
[20] The compound of the above-mentioned [17] to [19], wherein
   ny6c and ny6d are both 0, and
   A^{Y6b} is a bond, or a pharmacologically acceptable salt thereof.
[21] The compound of the above-mentioned [10] or [11], wherein
   R^{6a1} is
      (1) a hydrogen atom, or
      (2) an optionally substituted alkyl group,
   R^{6a2} is
      (1) a hydrogen atom,
      (2) CN,
      (3) a halogen atom,
      (4) an optionally substituted alkyl group having 1 to 4 carbon atoms,
      (5) an optionally substituted cyclopropyl group,
      (6) an optionally substituted oxetanyl group,
      (7) an optionally substituted azetidinyl group, or
      (8) an optionally substituted aryl group having 6 to 10 carbon atoms,
   R^{6a3} is a group represented by the formula:

      -CD-R^{9a}-W^{9a}-Y^{9b}-W^{9b}
   wherein
   R^{9a} is
      (1) a group represented by the formula: 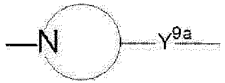
   wherein 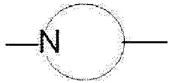
   is
      a heterocyclic group containing a nitrogen atom, optionally further containing 1 to 5 same or different atoms selected from a nitrogen atom, an oxygen atom, and a sulfur atom, and having 3 to 10 ring-constituting atoms, which is optionally further substituted in addition to Y^{9a}, and
   Y^{9a} is
      1) -(CR^{Y5}R^{Y5'})_{ny9a}-A^{Y9a}-(CR^{Y6}R^{Y6'})_{ny9b}-
         wherein R^{Y5}, R^{Y5'}, R^{Y6}, and R^{Y6'} are each independently
            (a) a hydrogen atom,
            (b) a halogen atom,
            (c) -O-(optionally substituted alkyl having 1 to 6 carbon atoms),
            (d) -O-(optionally substituted cycloalkyl having 3 to 6 carbon atoms),
            (e) an optionally substituted alkyl group having 1 to 6 carbon atoms,
            (f) an optionally substituted cycloalkyl group having 3 to 6 carbon atoms, or
            (g) R^{Y5} and R^{Y5'}, and R^{Y6} and R^{Y6'}, are each independently optionally bonded to each other to form, together with the adjacent carbon atom, an optionally substituted cycloalkyl group having 3 to 10 carbon atoms, or an optionally substituted heterocycle containing 1 to 6 same or different atoms selected from a nitrogen atom, an oxygen atom, and a sulfur atom and having 3 to 10 ring-constituting atoms;
         ny9a and ny9b are each independently an integer of 0 to 6;
         A^{Y9a} is
            (a) a bond,
            (b) -O-,
            (c) -SO-,
            (d) -SO₂-,
            (e) -NR^{NY9c}-
               wherein R^{NY9c} is
               (i) a hydrogen atom,
               (ii) an optionally substituted alkyl group having 1 to 6 carbon atoms,
               (iii) an optionally substituted cycloalkyl group having 3 to 6 carbon atoms,
               (iv) an optionally substituted heterocyclic group having 3 to 6 ring-constituting atoms,
               (v) -CO-(optionally substituted alkyl having 1 to 6 carbon atoms), or
               (vi) -CO-(optionally substituted cycloalkyl having 3 to 6 carbon atoms),
            (f) -V^{Y9d}-NR^{NY9d}- or -NR^{NY9d}-V^{Y9d}-
         wherein V^{Y9d} is
            (i) -CO-,
            (ii) -SO-, or
            (iii) -SO₂-, and
         R^{NY9d} is
            (i) a hydrogen atom,
            (ii) an optionally substituted alkyl group having 1 to 6 carbon atoms,
            (iii) an optionally substituted cycloalkyl group having 3 to 6 carbon atoms, or
            (iv) an optionally substituted heterocyclic group having 3 to 6 ring-constituting atoms, or
      2) a group represented by the formula: -NR^{N9}-, -NR^{N9}- (CR^{Y7}R^{Y7'})ₙ₉⁻,

         -NR^{N9}-(CR^{Y7}R^{Y7'})ₙ₉-V⁹-(CR^{Y8}R^{Y8'})ₘ₉-,

         -NR^{N9}-(CR^{Y7}R^{Y7'})ₙ₉-V⁹-NR^{N9'}-(CR^{Y8}R^{Y8'})ₘ₉-,

         -NR^{N9}-(CR^{Y7}R^{Y7'})ₒ₉-NR^{N9'}-V⁹-(CR^{Y8}R^{Y8'})ₘ₉-,

         -NR^{N9}-(CR^{Y7}R^{Y7'})ₒ₉-NR^{N9"}-(CR^{Y8}R^{Y8'})ₘ₉-, -NR^{N9}-(CR^{Y7}R^{Y7'})ₒ₉-O-(CR^{Y8}R^{Y8'})ₘ₉-,

         or

         -NR^{N9}-(CR^{Y7}R^{Y7'})ₒ₉-S-(CR^{Y8}R^{Y8'})ₘ₉-

         in the above-mentioned formulas,
   R^{N9} is
      1) a hydrogen atom,
      2) -(CR^{N9a}R^{N9a'})_{nN9a}-Y^{N9}-W^{N9}
   wherein R^{N9a} and R^{N9a'} are each independently
      (a) a hydrogen atom,
      (b) a halogen atom,
      (c) -O-(optionally substituted alkyl having 1 to 6 carbon atoms),
      (d) -O-(optionally substituted cycloalkyl having 3 to 6 carbon atoms),
      (e) an optionally substituted alkyl group having 1 to 6 carbon atoms,
      (f) an optionally substituted cycloalkyl group having 3 to 6 carbon atoms, or
      (g) R^{N9a} and R^{N9a'} are optionally bonded to each other to form, together with the adjacent carbon atom, an optionally substituted cycloalkyl group having 3 to 10 carbon atoms, or
      (h) an optionally substituted heterocyclic group containing 1 to 6 same or different atoms selected from a nitrogen atom, an oxygen atom, and a sulfur atom and having 3 to 10 ring-constituting atoms;
   nN9a is an integer of 1 to 6;
   Y^{N9} is
      (a) a bond-,
      (b) -O-,
      (c) -SO-,
      (d) -SO₂-,
      (e) -NR^{N9b}-
         wherein R^{N9b} is
         (i) a hydrogen atom,
         (ii) an optionally substituted alkyl group having 1 to 6 carbon atoms,
         (iii) an optionally substituted cycloalkyl group having 3 to 6 carbon atoms,
         (iv) an optionally substituted heterocyclic group having 3 to 6 ring-constituting atoms,
         (v) -CO-(optionally substituted alkyl having 1 to 6 carbon atoms), or
         (vi) -CO-(optionally substituted cycloalkyl having 3 to 6 carbon atoms, or
      (f) -V^{N9c}-NR^{N9c}- or -NR^{N9c}-V^{N9c}-
   wherein V^{N9c} is
      (i) -CO-,
      (ii) -SO-, or
      (iii) -SO₂-,
   RN9c is
      (i) a hydrogen atom,
      (ii) an optionally substituted alkyl group having 1 to 6 carbon atoms, or
      (iii) an optionally substituted cycloalkyl group having 3 to 6 carbon atoms,
   W^{N9} is
      (a) a hydrogen atom,
      (b) an optionally substituted alkyl group having 1 to 6 carbon atoms,
      (c) an optionally substituted cycloalkyl group having 3 to 10 carbon atoms,
      (d) an optionally substituted aryl group having 6 to 10 carbon atoms,
      (e) an optionally substituted heterocyclic group containing 1 to 6 same or different atoms selected from a nitrogen atom, an oxygen atom, and a sulfur atom and having 3 to 10 ring-constituting atoms, or
      (f) a halogen atom;
   R^{Y7}, R^{Y7'}, R^{Y8}, and R^{Y8'} are each independently
      1) a hydrogen atom,
      2) a halogen atom,
      3) -O- (optionally substituted alkyl having 1 to 6 carbon atoms),
      4) -O-(optionally substituted cycloalkyl having 3 to 6 carbon atoms),
      5) an optionally substituted alkyl group having 1 to 6 carbon atoms,
      6) an optionally substituted cycloalkyl group having 3 to 6 carbon atoms, or
      7) R^{Y7} and R^{Y7'}, and R^{Y8} and R^{Y8'}, are each independently optionally bonded to each other to form, together with the adjacent carbon atom, an optionally substituted cycloalkyl group having 3 to 10 carbon atoms, or an optionally substituted heterocycle containing 1 to 6 same or different atoms selected from a nitrogen atom, an oxygen atom, and a sulfur atom and having 3 to 10 ring-constituting atoms;
   n9 is an integer of 1 to 6;
   V⁹ is
      1) -CO-,
      2) -SO-,
      3) -SO₂-, or
      4) an optionally substituted heterocyclic divalent group having 3 to 6 ring-constituting atoms;
   m9 is an integer of 0 to 6;
   R^{N9'} is
      1) a hydrogen atom,
      2) an optionally substituted alkyl group having 1 to 6 carbon atoms,
      3) an optionally substituted cycloalkyl group having 3 to 6 carbon atoms, or
      4) an optionally substituted heterocyclic group having 3 to 6 ring-constituting atoms;
   o9 is an integer of 2 to 6;
   R^{N9"} is
      1) a hydrogen atom,
      2) an optionally substituted alkyl group having 1 to 6 carbon atoms,
      3) an optionally substituted cycloalkyl group having 3 to 6 carbon atoms,
      4) an optionally substituted heterocyclic group having 3 to 6 carbon atoms,
      5) -CO-(optionally substituted alkyl having 1 to 6 carbon atoms), or
      6) -CO-(optionally substituted cycloalkyl having 3 to 6 carbon atoms);
   W^{9a} is
      (1) an optionally substituted alkylene group having 1 to 6 carbon atoms,
      (2) an optionally substituted aryl divalent group having 6 to 10 carbon atoms,
      (3) an optionally substituted heteroaryl divalent group containing 1 to 6 same or different atoms selected from a nitrogen atom, an oxygen atom, and a sulfur atom and having 5 to 10 ring-constituting atoms,
      (4) an optionally substituted cycloalkyl divalent group having 3 to 10 carbon atoms, or
      (5) an optionally substituted heterocyclic divalent group containing 1 to 6 same or different atoms selected from a nitrogen atom, an oxygen atom, and a sulfur atom and having 3 to 10 ring-constituting atoms,
   Y^{9b} is a group represented by
      (1) -(CR^{Y9}R^{Y9'})ny9c-A^{Y9b}-(CR^{Y10}R^{Y10'})ny9d-
   wherein R^{Y9}, R^{Y9'}, R^{Y10}, and R^{Y10'} are each independently
      1) a hydrogen atom,
      2) a halogen atom,
      3) -O-(optionally substituted alkyl having 1 to 6 carbon atoms),
      4) -O-(optionally substituted cycloalkyl having 3 to 6 carbon atoms),
      5) an optionally substituted alkyl having 1 to 6 carbon atoms, or
      6) an optionally substituted cycloalkyl group having 3 to 6 carbon atoms, or
      7) R^{Y9} and R^{Y9'}, and R^{Y10} and R^{Y10'}, are each independently optionally bonded to each other to form, together with the adjacent carbon atom, an optionally substituted cycloalkyl group having 3 to 10 carbon atoms, or an optionally substituted heterocyclic group containing 1 to 6 same or different atoms selected from a nitrogen atom, an oxygen atom, and a sulfur atom and having 3 to 10 ring-constituting atoms,
   ny9c and ny9d are each independently an integer of 0 to 6,
   A^{Y9b} is
      1) a bond,
      2) -O-,
      3) -SO-,
      4) -SO₂-,
      5) -NR^{NY9e}-,
         wherein R^{NY9e} is
         (a) a hydrogen atom,
         (b) an optionally substituted alkyl group having 1 to 6 carbon atoms,
         (c) an optionally substituted cycloalkyl group having 3 to 6 carbon atoms,
         (d) an optionally substituted heterocyclic group having 3 to 6 ring-constituting atoms,
         (e) -CO-(optionally substituted alkyl having 1 to 6 carbon atoms),
         (f) -CO-(optionally substituted cycloalkyl having 3 to 6 carbon atoms), or
      6) -V^{Y9f}-NR^{NY9f}- or -NR^{NY9f}-V^{Y9f}-
   wherein V^{Y9f} is
      (i) -CO-,
      (ii) -SO-, or
      (iii) -SO₂-,
   R^{NY9f} is
      (i) a hydrogen atom,
      (ii) an optionally substituted alkyl group having 1 to 6 carbon atoms,
      (iii) an optionally substituted cycloalkyl group having 3 to 6 carbon atoms, or
      (iv) an optionally substituted heterocyclic group having 3 to 6 ring-constituting atoms,
   W^{9b} is
      (1) a hydrogen atom,
      (2) a halogen atom,
      (3) an optionally substituted aryl group having 6 to 10 carbon atoms,
      (4) an optionally substituted heteroaryl group containing 1 to 6 same or different atoms selected from a nitrogen atom, an oxygen atom, and a sulfur atom and having 5 to 10 ring-constituting atoms,
      (5) an optionally substituted cycloalkyl group having 3 to 10 carbon atoms,
      (6) an optionally substituted heterocyclic group containing 1 to 6 same or different atoms selected from a nitrogen atom, an oxygen atom, and a sulfur atom and having 3 to 10 ring-constituting atoms, or
      (7) -CONR^{NW9}R^{NW9'}
   wherein R^{NW9} and R^{NW9'} are each independently
      1) a hydrogen atom,
      2) an optionally substituted alkyl group having 1 to 6 carbon atoms, or
      3) an optionally substituted cycloalkyl group having 3 to 6 carbon atoms, or
      4) -NR^{NW9"}R^{NW9‴}
   wherein R^{NW9"} and R^{NW9‴} are each independently
      (a) a hydrogen atom,
      (b) an optionally substituted alkyl group having 1 to 6 carbon atoms,
      (c) an optionally substituted cycloalkyl group having 3 to 6 carbon atoms,
      (d) an optionally substituted heterocyclic group having 3 to 6 ring-constituting atoms,
      (e) -CO-(optionally substituted alkyl having 1 to 6 carbon atoms), or
      (f) -CO-(optionally substituted cycloalkyl having 3 to 6 carbon atoms),
   or a pharmacologically acceptable salt thereof.
[22] The compound of the above-mentioned [21], wherein
   R6a1 is
      (1) a hydrogen atom, or
      (2) an optionally substituted alkyl group,
   R6a2 is
      (1) a hydrogen atom,
      (2) an optionally substituted alkyl group having 1 to 4 carbon atoms, or
      (3) an optionally substituted aryl group having 6 to 10 carbon atoms,
   R^{6a3} is a group represented by the formula:

      -CD-R^{9a}-W^{9a}-Y^{9b}-W^{9b}

      wherein
   R^{9a} is
      (1) a group represented by the formula: 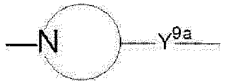 wherein 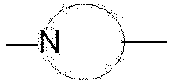 is
         a heterocyclic group containing a nitrogen atom, optionally further containing 1 to 5 same or different atoms selected from a nitrogen atom, an oxygen atom, and a sulfur atom, and having 3 to 10 ring-constituting atoms, which is optionally further substituted in addition to Y^{9a}, and
         Y^{9a} is
            1) -(CR^{Y5}R^{Y5'})_{ny9a}-A^{Y9a}-(CR^{Y6}R^{Y6'})_{ny9b}-
            wherein
         R^{Y5}, R^{Y5'}, R^{Y6}, and R^{Y6'} are each independently a hydrogen atom,
         ny9a and ny9b are each independently an integer of 0 to 6,
         A^{Y9a} is
            (a) a bond,
            (b) -O-,
            (c) -NR^{NY9c}-
               wherein R^{NY9c} is
               (i) a hydrogen atom, or
            (d) -V^{Y9d}-NR^{NY9d}- or -NR^{NY9d}-V^{Y9d}-
         wherein V^{Y9d} is
            (i) -CO-, and
         R^{NY9d} is
            (i) a hydrogen atom, or
            (ii) an optionally substituted alkyl group having 1 to 6 carbon atoms, or
      (2) a group represented by the formula: -NR^{N9}-, -NR^{N9}-

         (CR^{Y7}R^{Y7'})ₙ₉-,

         -NR^{N9}-(CR^{Y7}R^{Y7'})ₙ₉-V⁹-(CR^{Y8}R^{Y8'})ₘ₉-,

         -NR^{N9}-(CR^{Y7}R^{Y7'})ₙ₉-V⁹-NR^{N9'}-(CR^{Y8}R^{Y8'})ₘ₉-,

         -NR^{N9}-(CR^{Y7}R^{Y7'})ₒ₉-NR^{N9'}-V⁹-(CR^{Y8}R^{Y8'})ₘ₉-,

         -NR^{N9}-(CR^{Y7}R^{Y7'})ₒ₉-NR^{N9"}-(CR^{Y8}R^{Y8'})ₘ₉-, -NR^{N9}-(CR^{Y7}R^{Y7'})ₒ₉-O-(CR^{Y8}R^{Y8'})ₘ₉-,

         or
         in the above-mentioned formulas,
   R^{N9} is
      1) a hydrogen atom,
      2) -(CR^{N9a}R^{N9a'})_{nN9a}-Y^{N9}-W^{N9}
   wherein R^{N9a} and R^{N9a'} are each independently
      (a) a hydrogen atom,
      (b) a halogen atom,
      (c) an optionally substituted alkyl group having 1 to 6 carbon atoms, or
      (d) R^{N9a} and R^{N9a'} are optionally bonded to each other to form, together with the adjacent carbon atom, an optionally substituted cycloalkyl group having 3 to 10 carbon atoms, or an optionally substituted heterocyclic group containing 1 to 6 same or different atoms selected from a nitrogen atom, an oxygen atom, and a sulfur atom and having 3 to 10 ring-constituting atoms;
   nN9a is an integer of 1 to 6;
   Y^{N9} is
      (a) a bond,
      (b) -O-, or
      (c) -NR^{N9c}-V^{N9c}-
   wherein V^{N9c} is
      (i) -CO-, and
   RN9c is
      (i) a hydrogen atom, or
      (ii) an optionally substituted alkyl group having 1 to 6 carbon atoms,
   W^{N9} is
      (a) a hydrogen atom,
      (b) an optionally substituted alkyl group having 1 to 6 carbon atoms,
      (c) an optionally substituted cycloalkyl group having 3 to 10 carbon atoms,
      (d) an optionally substituted aryl group having 6 to 10 carbon atoms, or
      (e) a halogen atom;
   R^{Y7}, R^{Y7'}, R^{Y8} and R^{Y8'} are each independently
      1) a hydrogen atom, or
      2) an optionally substituted alkyl group having 1 to 6 carbon atoms;
   n9 is an integer of 1 to 6;
   V⁹ is
      1) -CO-, or
      2) an optionally substituted heterocyclic divalent group having 3 to 6 ring-constituting atoms;
   m9 is an integer of 0 to 6;
   R^{N9'} is
      1) a hydrogen atom, or
      2) an optionally substituted alkyl group having 1 to 6 carbon atoms;
   o9 is an integer of 2 to 6;
   R^{N9"} is
      1) a hydrogen atom;
   [0042] W^{9a} is
      (1) an optionally substituted alkylene group having 1 to 6 carbon atoms,
      (2) an optionally substituted aryl divalent group having 6 to 10 carbon atoms,
      (3) an optionally substituted heteroaryl divalent group containing 1 to 6 same or different atoms selected from a nitrogen atom, an oxygen atom, and a sulfur atom and having 5 to 10 ring-constituting atoms, or
      (4) an optionally substituted cycloalkyl divalent group having 3 to 10 carbon atoms, or
   an optionally substituted heterocyclic divalent group containing 1 to 6 same or different atoms selected from a nitrogen atom, an oxygen atom, and a sulfur atom and having 3 to 10 ring-constituting atoms,
   [0043] Y^{9b} is a group represented by:
      (1) -(CR^{Y9}R^{Y9'})ny9c-A^{Y9b}-(CR^{Y10}R^{Y10'})ny9d-
   wherein R^{Y9}, R^{Y9'}, R^{Y10}, and R^{Y10'} are each independently
      1) a hydrogen atom, or
      2) a halogen atom,
   ny9c and ny9d are each independently an integer of 0 to 6,
   A^{Y9b} is
      1) a bond, or
      2) -O-, and
   W^{9b} is
      (1) a hydrogen atom,
      (2) a halogen atom,
      (3) an aryl group having 6 to 10 carbon atoms,
      (4) an optionally substituted heteroaryl group containing 1 to 6 same or different atoms selected from a nitrogen atom, an oxygen atom, and a sulfur atom and having 5 to 10 ring-constituting atoms,
      (5) -CONR^{NW9}R^{NW9'}
   wherein R^{NW9} and R^{NW9'} are each independently
      1) a hydrogen atom, or
      2) an optionally substituted alkyl group having 1 to 6 carbon atoms,
   or a pharmacologically acceptable salt thereof.
[23] The compound of the above-mentioned [21] or [22], wherein
   R^{6a1} is (1) a hydrogen atom, or
   (2) an alkyl group optionally substituted by an optionally substituted aryl group having 6 to 10 carbon atoms,
   R6a2 is
      (1) a hydrogen atom,
      (2) an alkyl group having 1 to 4 carbon atoms, or
      (3) an aryl group having 6 to 10 carbon atoms and optionally substituted by a group selected from an alkyl group having 1 to 4 carbon atoms, an alkoxy group having 1 to 4 carbon atoms, and halogen,
   R^{6a3} is a group represented by the formula:

      -CD-R^{9a}-W^{9a}-Y^{9b}-W^{9b}

      wherein
   R^{9a} is a group represented by:
      (1) a group represented by the formula: 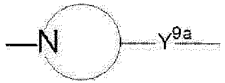 wherein 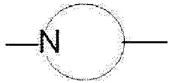 is
         a heterocyclic group containing a nitrogen atom, optionally further containing 1 to 5 same or different atoms selected from a nitrogen atom, an oxygen atom, and a sulfur atom, and having 3 to 10 ring-constituting atoms, which is optionally further substituted in addition to Y^{9a}, and
         Y^{9a} is
            1) -(CR^{Y5}R^{Y5'})_{ny9a}-A^{Y9a}-(CR^{Y6}R^{Y6'})_{ny9b}-
         wherein R^{Y5}, R^{Y5'}, R^{Y6}, and R^{Y6'} are each independently
            (a) a hydrogen atom,
         ny9a and ny9b are each independently an integer of 0 to 6;
         AY9a is
            (a) a bond,
            (b) -O-,
            (c) -NR^{NY9c}-
               wherein R^{NY9c} is
               (i) a hydrogen atom, or
            (d) -V^{Y9d}-NR^{NY9d}- or -NR^{NY9d}-V^{Y9d}-
               wherein V^{Y9d} is
               (i) -CO-, and
         R^{NY9d} is
            (i) a hydrogen atom, or
            (ii) an alkyl group having 1 to 6 carbon atoms, or
      (2) a group represented by the formula: -NR^{N9}-, -NR^{N9}-(CR^{Y7}R^{Y7'})ₙ₉-,

         -NR^{N9}-(CR^{Y7}R^{Y7'})ₙ₉-V⁹-(CR^{Y8}R^{Y8'})ₘ₉-,

         -NR^{N9}-(CR^{Y7}R^{Y7'})ₙ₉-V⁹-NR^{N9'}-(CR^{Y8}R^{Y8'})ₘ₉-,

         -NR^{N9}-(CR^{Y7}R^{Y7'})ₒ₉-NR^{N9'}-V⁹-(CR^{Y8}R^{Y8'})ₘ₉-,

         -NR^{N9}-(CR^{Y7}R^{Y7'})ₒ₉-NR^{N9"}-(CR^{Y8}R^{Y8'})ₘ₉-, -NR^{N9}-(CR^{Y7}R^{Y7'})ₒ₉-O-(CR^{Y8}R^{Y8'})ₘ₉-,

         or

         -NR^{N9}-(CR^{Y7}R^{Y7'})ₒ₉-S-(CR^{Y8}R^{Y8'})ₘ₉-

         in the above-mentioned formulas,
         R^{N9} is
            1) a hydrogen atom, or
            2) -(CR^{N9a}R^{N9a'})_{nN9a}-Y^{N9}-W^{N9}
         wherein R^{N9a} and R^{N9a'} are each independently
            (a) a hydrogen atom,
            (b) a halogen atom,
            (c) an optionally substituted alkyl group having 1 to 6 carbon atoms, or
            (d) R^{N9a} and R^{N9a'} are optionally bonded to each other to form, together with the adjacent carbon atom, a cycloalkyl group having 3 to 6 carbon atoms or a heterocyclic group containing 1 or 2 oxygen atoms, and having 5 or 6 ring-constituting atoms;
         nN9a is an integer of 1 to 6;
         Y^{N9} is
            (a) a bond,
            (b) -O-, or
            (c) -NR^{N9c}-V^{N9c}-
         wherein V^{N9c} is
            (i) -CO-, and
         RN9c is
            (i) a hydrogen atom, or
            (ii) an optionally substituted alkyl group having 1 to 6 carbon atoms,
         W^{N9} is
            (a) a hydrogen atom,
            (b) an optionally substituted alkyl group having 1 to 6 carbon atoms,
            (c) a cycloalkyl group having 3 to 6 carbon atoms,
            (d) an aryl group having 6 to 10 carbon atoms, or
            (e) a halogen atom;
         R^{Y7}, R^{Y7'}, R^{Y5}, and R^{Y8'} are each independently
            1) a hydrogen atom, or
            2) an optionally substituted alkyl group having 1 to 6 carbon atoms,
         n9 is an integer of 1 to 6;
         V⁹ is
            1) -CO-,
            2) an optionally substituted heterocyclic divalent group having 3 to 6 ring-constituting atoms;
         m9 is an integer of 0 to 6;
         R^{N9'} is
            1) a hydrogen atom, or
            2) an alkyl group having 1 to 6 carbon atoms;
         o9 is an integer of 2 to 6;
         R^{N9"} is
            1) a hydrogen atom;
         W^{9a} is
            (1) an optionally substituted alkylene group having 1 to 6 carbon atoms,
            (2) an optionally substituted aryl divalent group having 6 to 10 carbon atoms,
            (3) an optionally substituted heteroaryl divalent group containing 1 to 4 same or different atoms selected from a nitrogen atom and a sulfur atom, and having 5 or 6 ring-constituting atoms, or
            (4) an optionally substituted heterocyclic divalent group containing 1 to 4 nitrogen atoms, and having 5 or 6 ring-constituting atoms,
         Y^{9b} is
            (1) - (CR^{Y9}R^{Y9'})ny9c-A^{Y9b}-(CR^{Y10}R^{Y10'})ny9d-
         wherein R^{Y9}, R^{Y9}'^{,} R^{Y10}, and R^{Y10'} are each independently
            1) a hydrogen atom, or
            2) a halogen atom,
         ny9c and ny9d are each independently an integer of 0 to 6,
         A^{Y9b} is
            1) a bond, or
            2) -O-,
         W^{9b} is
            (1) a hydrogen atom,
            (2) a halogen atom,
            (3) an aryl group having 6 to 10 carbon atoms and optionally substituted by halogen,
            (4) a heteroaryl group containing 1 to 4 same or different atoms selected from a nitrogen atom and a sulfur atom, and having 5 or 6 ring-constituting atoms, which is optionally substituted by an alkyl group having 1 to 6 carbon atoms, (8) -CONR^{NW9}R^{NW9'}
         wherein R^{NW9} and R^{NW9'} are each independently
            1) a hydrogen atom, or
            2) an alkyl group having 1 to 6 carbon atoms,
   or a pharmacologically acceptable salt thereof.
[24] The compound of the above-mentioned [21], wherein
   R^{9a} is a group represented by the formula:
      (1)

         -NR^{N9}-,
      (2)

         -NR^{N9}-(CR^{Y7}R^{Y7'})ₙ₉-,
      (3)

         -NR^{N9}-(CR^{Y7}R^{Y7'})ₙ₉-V⁹-(CR^{Y8}R^{Y8'})ₘ₉-,
      (4)

         -NR^{N9}-(CR^{Y7}R^{Y7'})ₙ₉-V⁹-NR^{N9'}-(CR^{Y8}R^{Y8'})ₘ₉-,
      (5)

         -NR^{N9}-(CR^{Y7}R^{Y7'})ₒ₉-NR^{N9'}-V⁹-(CR^{Y8}R^{Y8'})ₘ₉-,
      (6)

         -NR^{N9}-(CR^{Y7}R^{Y7'})ₒ₉-NR^{N9"}-(CR^{Y8}R^{Y8'})ₘ₉-,
      (7)

         -NR^{N9}-(CR^{Y7}R^{Y7'})ₒ₉-O-(CR^{Y8}R^{Y8'})ₘ₉-

         or
      (8)

         -NR^{N9}-(CR^{Y7}R^{Y7'})ₒ₉-S-(CR^{Y8}R^{Y8'})ₘ₉-
      (in the above-mentioned formulas, each symbol is as defined above),
   or a pharmacologically acceptable salt thereof.
[25] The compound of the above-mentioned [24], wherein
   W^{9a} is
      (1) an optionally substituted alkylene group having 1 to 6 carbon atoms,
      (2) an optionally substituted aryl divalent group having 6 to 10 carbon atoms,
      (3) an optionally substituted heteroaryl divalent group containing 1 to 6 same or different atoms selected from a nitrogen atom, an oxygen atom, and a sulfur atom and having 5 to 10 ring-constituting atoms, or
      (4) an optionally substituted heterocyclic divalent group containing 1 to 4 nitrogen atoms, and having 5 or 6 ring-constituting atoms,
   or a pharmacologically acceptable salt thereof.
[26] The compound of the above-mentioned [25], wherein
   W^{9a} is
      (1) an optionally substituted alkylene group having 1 to 6 carbon atoms,
      (2) an optionally substituted aryl divalent group having 6 to 10 carbon atoms,
      (3) an optionally substituted heteroaryl divalent group containing 1 to 4 same or different atoms selected from a nitrogen atom and a sulfur atom, and having 5 or 6 ring-constituting atoms, or
      (4) an optionally substituted heterocyclic divalent group containing 1 to 4 nitrogen atoms, and having 5 or 6 ring-constituting atoms,
   or a pharmacologically acceptable salt thereof.
[27] The compound of the above-mentioned [25] or [26], wherein ny9c and ny9d are both 0, and A^{Y9b} is a bond,
   or a pharmacologically acceptable salt thereof.
[28] The compound of the above-mentioned [25] to [27], wherein
   W^{9a} is
      (1) an optionally substituted alkylene group having 1 to 6 carbon atoms,
      (2) an optionally substituted phenylene group,
      (3) an optionally substituted pyridine-diyl group,
      (4) an optionally substituted thiazole-diyl group, or
      (5) an optionally substituted piperidine-diyl group,
   or a pharmacologically acceptable salt thereof.
[29] The compound of the above-mentioned [21], wherein
   R^{9a} is a group represented by the formula: 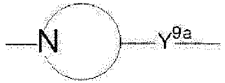 wherein 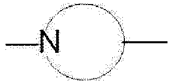
   and Y^{9a} are as defined above,
   or a pharmacologically acceptable salt thereof.
[30] The compound of the above-mentioned [29], wherein
   ny9c and ny9d are both 0, and A^{Y9b} is a bond,
   or a pharmacologically acceptable salt thereof.
[31] The compound of the above-mentioned [29] or [30], wherein
   W^{9b} is a hydrogen atom,
   or a pharmacologically acceptable salt thereof.
[32] The compound of the above-mentioned [21], [22], [23], [29], [30], or [31], wherein
   a group represented by the formula: 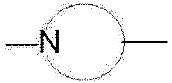
   is an azetidinyl group, a pyrrolidinyl group, a piperidyl group,
   a piperazinyl group, a morpholinyl group, a
   tetrahydronaphthyridinyl group, a tetrahydrothiazolopyridyl group, or a tetrahydroisoquinolyl group, each of which is optionally further substituted by an oxo group or a halogen atom in addition to Y^{9a},
   or a pharmacologically acceptable salt thereof.
[33] The compound of the above-mentioned [10] or [11], wherein
   A⁶ is -NR^{6a1}-,
   R6a1 is
      (1)

         -Y^{10a}-W^{10a}-Y^{10b}-W^{10b}
   wherein Y^{10a} is
      1) a bond,
      2)

         - (CR^{Y11}R^{Y11'})ₙ₁₀-,
      3)

         - (CR^{Y11}R^{Y11'})ₘ₁₀-V¹⁰-(CR^{Y12}R^{Y12'})ₘ₁₀-,
      4)

         - (CR^{Y11}R^{Y11'})ₙ₁₀-V¹⁰-NR^{N10'}-(CR^{Y12}R^{Y12'})ₘ₁₀-,
      5)

         - (CR^{Y11}R^{Y11'})ₒ₁₀-NR^{N10'}-V^{1D}-(CR^{Y12}R^{Y12'})ₘ₁₀-,
      6)

         - (CR^{Y11}R^{Y11'})ₒ₁₀-NR^{N10"}-(CR^{Y12}R^{Y12'})ₘ₁₀-,
      7)

         - (CR^{Y11}R^{Y11'})ₒ₁₀-O-(CR^{Y12}R^{Y12'})ₘ₁₀-, or
      8)

         - (CR^{Y11}R^{Y11'})ₒ₁₀-S-(CR^{Y12}R^{Y12'})ₘ₁₀-
   wherein R^{Y11}, R^{Y11'}, R^{Y12}, and R^{Y12'} are each independently
      (a) a hydrogen atom,
      (b) a halogen atom,
      (c) -O-(optionally substituted alkyl having 1 to 6 carbon atoms),
      (d) -O-(optionally substituted cycloalkyl having 3 to 6 carbon atoms),
      (e) an optionally substituted alkyl group having 1 to 6 carbon atoms,
      (f) an optionally substituted cycloalkyl group having 3 to 6 carbon atoms, or
      (g) R^{Y11} and R^{Y11'}, and R^{Y12} and R^{Y12'}, are each independently optionally bonded to form an optionally substituted cycloalkyl having 3 to 10 carbon atoms, or an optionally substituted heterocycle containing 1 to 6 atoms selected from a nitrogen atom, an oxygen atom, and a sulfur atom and having 3 to 10 ring-constituting atoms,
   n10 is an integer of 1 to 6,
   V¹⁰ is
      (a) -CO-,
      (b) -SO-, or
      (c) -SO₂-,
   m10 is an integer of 0 to 6,
   R^{N10'} is
      (a) a hydrogen atom,
      (b) an optionally substituted alkyl group having 1 to 6 carbon atoms,
      (c) an optionally substituted cycloalkyl group having 3 to 6 carbon atoms, or
      (d) an optionally substituted heterocyclic group having 3 to 6 ring-constituting atoms,
   o10 is an integer of 2 to 6,
   R^{N10"} is
      (a) a hydrogen atom,
      (b) an optionally substituted alkyl group having 1 to 6 carbon atoms,
      (c) an optionally substituted cycloalkyl group having 3 to 6 carbon atoms,
      (d) an optionally substituted heterocyclic group having 3 to 6 carbon atoms,
      (e) -CO-(optionally substituted alkyl having 1 to 6 carbon atoms), or
      (f) -CO-(optionally substituted cycloalkyl having 3 to 6 carbon atoms),
   W^{10a} is
      1) an optionally substituted alkylene group having 1 to 6 carbon atoms,
      2) an optionally substituted aryl divalent group having 6 to 10 carbon atoms,
      3) an optionally substituted heteroaryl divalent group containing 1 to 6 atoms selected from a nitrogen atom, an oxygen atom, and a sulfur atom and having 5 to 10 ring-constituting atoms,
      4) an optionally substituted cycloalkyl divalent group having 3 to 10 carbon atoms, or
      5) an optionally substituted heterocycle divalent group containing 1 to 6 atoms selected from a nitrogen atom, an oxygen atom, and a sulfur atom and having 3 to 10 ring-constituting atoms,
   Y^{10b} is
      1) - (CR^{Y13}R^{Y13'})_{ny10c}-A^{Y1b}-(CR^{Y14}R^{Y14'}) _{ny10d}-
   wherein R^{Y13}, R^{Y13'}, R^{Y14}, and R^{Y14'} are each independently
      (a) a hydrogen atom,
      (b) a halogen atom,
      (c) -O-(optionally substituted alkyl having 1 to 6 carbon atoms),
      (d) -O-(optionally substituted cycloalkyl having 3 to 6 carbon atoms),
      (e) an optionally substituted alkyl group having 1 to 6 carbon atoms,
      (f) an optionally substituted cycloalkyl group having 3 to 6 carbon atoms, or
      (g) R^{Y13} and R^{Y13'}, and R^{Y14} and R^{Y14'}, are each independently optionally bonded to each other to form, together with the adjacent carbon atom, an optionally substituted monocyclic or condensed cycloalkane having 3 to 10 carbon atoms, or an optionally substituted heterocycle containing 1 to 6 same or different atoms selected from a nitrogen atom, an oxygen atom, and a sulfur atom and having 3 to 10 ring-constituting atoms, ny10c and ny10d are each independently an integer of 0 to 6,
   A^{Y10b} is
      (a) a bond,
      (b) -O-,
      (c) -SO-,
      (d) -SO₂-,
      (e) -NRN^{Y10e}-
         wherein R^{NY10e} is
         (i) a hydrogen atom,
         (ii) an optionally substituted alkyl group having 1 to 6 carbon atoms,
         (iii) an optionally substituted cycloalkyl group having 3 to 6 carbon atoms,
         (iv) an optionally substituted heterocyclic group having 3 to 6 carbon atoms,
         (v) -CO-(optionally substituted alkyl having 1 to 6 carbon atoms),
         (vi) -CO-(optionally substituted cycloalkyl having 3 to 6 carbon atoms), or
      (f) -V^{Y10f}-NR^{NY10f}- or NR^{NY10f}-V^{Y10f}-
   wherein V^{Y10f} is

      -CO-,

      -SO-

      or

      -SO₂-,
   R^{NY10f} is
      a hydrogen atom,
      an optionally substituted alkyl group having 1 to 6 carbon atoms,
      an optionally substituted cycloalkyl group having 3 to 6 carbon atoms, or
      an optionally substituted heterocyclic group having 3 to 6 carbon atoms,
   W^{10b} is
      1) a hydrogen atom,
      2) a halogen atom,
      3) an optionally substituted aryl group having 6 to 10 carbon atoms,
      4) an optionally substituted heteroaryl group containing 1 to 6 same or different atoms selected from a nitrogen atom, an oxygen atom, and a sulfur atom and having 5 to 10 ring-constituting atoms,
      5) an optionally substituted monocyclic or condensed cycloalkyl having 3 to 10 carbon atoms,
      6) an optionally substituted heterocyclic group containing 1 to 6 atoms selected from a nitrogen atom, an oxygen atom, and a sulfur atom and having 3 to 10 ring-constituting atoms,
      7) -CONR^{NW10}R^{NW10'}
         wherein R^{NW10} and R^{NW10'} may be the same or different and are each independently
         (a) a hydrogen atom,
         (b) an optionally substituted alkyl group having 1 to 6 carbon atoms, or
         (c) an optionally substituted cycloalkyl group having 3 to 6 carbon atoms, or
      8) -NR^{NW10"}R^{NW10‴}
   wherein R^{NW10"} and R^{NW10‴} may be the same or different and are each independently
      (a) a hydrogen atom,
      (b) substituted or unsubstituted alkyl having 1 to 6 carbon atoms,
      (c) an optionally substituted cycloalkyl having 3 to 6 carbon atoms,
      (d) an optionally substituted heterocyclic group having 3 to 6 ring-constituting atoms,
      (e) -CO-(optionally substituted alkyl having 1 to 6 carbon atoms), or
      (f) -CO-(optionally substituted cycloalkyl having 3 to 6 carbon atoms),
   or a pharmacologically acceptable salt thereof.
[34] The compound of the above-mentioned [33], wherein
   A⁶ is -NR^{6a1}-,
   R6a1 is
      (1)

         -Y^{10a}-W^{10a}-Y^{10b}-W^{10b}
   wherein Y^{10a} is
      1)

         - (CR^{Y11}R^{Y11'})ₙ₁₀-,

         or
      2)

         - (CR^{Y11}R^{Y11'})ₙ₁₀-V¹⁰-NR^{N10'}-(CR^{Y12}R^{Y12'})ₘ₁₀-,
   wherein R^{Y11}, R^{Y11'}, R^{Y12}, and R^{Y12'} are each independently
      (a) a hydrogen atom,
   n10 is an integer of 1 to 6,
   V¹⁰ is
      (a) -CO-,
   m10 is an integer of 0 to 6,
   R^{N10'} is
      (a) a hydrogen atom,
   W^{10a} is
      1) an aryl divalent group having 6 to 10 carbon atoms, or
      2) a heteroaryl divalent group containing 1 to 4 same or different atoms selected from a nitrogen atom and an oxygen atom, and having 5 or 6 ring-constituting atoms,
   Y^{10b} is
      1) - (CR^{Y13}R^{Y13'})_{ny10c}-A^{Y10b}-(CR^{Y14}R^{Y14'})_{ny10d}-
   wherein R^{Y13}, R^{Y13'}, R^{Y14}, and R^{Y14'} are each independently
      (a) a hydrogen atom, or
      (b) a halogen atom,
   ny10c and ny10d are each independently an integer of 0 to 6,
   A^{Y10b} is
      (a) a bond, or
      (b) -O-,
   W^{10b} is
      1) a hydrogen atom,
      2) an aryl group having 6 to 10 carbon atoms and optionally substituted by a group selected from an alkyl group having 1 to 6 carbon atoms and optionally substituted by halogen and halogen, or
      3) a heteroaryl group containing 1 to 4 same or different atoms selected from a nitrogen atom and a sulfur atom, and having 5 or 6 ring-constituting atoms, which is optionally substituted by an alkyl group having 1 to 6 carbon atoms,
   or a pharmacologically acceptable salt thereof.
[35] The compound of the above-mentioned [33] to [34], wherein
   Y^{10a} is
      (1) a bond,
      (2) - (CR^{Y11}R^{Y11'})ₙ₁₀- or
      (3) - (CR^{Y11}R^{Y11'})ₒ₁₀-O-(CR^{Y12}R^{Y12'})ₘ₁₀-
   wherein each symbol is as defined above,
   or a pharmacologically acceptable salt thereof.
[36] The compound of the above-mentioned [33] to [35], wherein
   W^{10a} is
      (1) an optionally substituted aryl divalent group having 6 to 10 carbon atoms,
      (2) an optionally substituted heteroaryl divalent group containing 1 to 6 same or different atoms selected from a nitrogen atom, an oxygen atom, and a sulfur atom and having 5 to 10 ring-constituting atoms,
      (3) an optionally substituted heterocyclic divalent group containing 1 to 6 same or different atoms selected from a nitrogen atom, an oxygen atom, and a sulfur atom and having 3 to 10 ring-constituting atoms, or
      (4) an optionally substituted alkylene group having 1 to 6 carbon atoms,
   or a pharmacologically acceptable salt thereof.
[37] The compound of the above-mentioned [33] to [36], wherein
   ny10c and ny10d are both 0, and
   A^{Y10b} is a bond,
   or a pharmacologically acceptable salt thereof.
[38] The compound of the above-mentioned [33] to [37], wherein
   W^{10a} is
      (1) a phenylene group,
      (2) a pyridine-diyl group,
      (3) an optionally substituted monocyclic heterocyclic divalent group containing 1 or 2 same or different atoms selected from a nitrogen atom, an oxygen atom, and a sulfur atom, having 4 to 7 ring-constituting atoms, or
      (4) an optionally substituted alkylene group having 1 to 6 carbon atoms,
   or a pharmacologically acceptable salt thereof.
[39] The compound of the above-mentioned [33] to [38], wherein
   W^{10b} is
      (1) an optionally substituted aryl group having 6 to 10 carbon atoms, or
      (2) an optionally substituted heteroaryl containing 1 to 6 atoms selected from a nitrogen atom, an oxygen atom, and a sulfur atom, and having 5 to 10 ring-constituting atoms,
   or a pharmacologically acceptable salt thereof.
[40] The compound of the above-mentioned [1] to [39], wherein
   E is
      (1) a bond
      (2) -CO-,
      (3) -SO-, or
      (4) -SO₂-,
   X is
      (1) an optionally substituted alkyl group having 1 to 6 carbon atoms,
      (2) an optionally substituted cycloalkyl group having 3 to 10 carbon atoms,
      (3) an optionally substituted aryl group having 6 to 10 carbon atoms,
      (4) an optionally substituted heterocyclic group containing 1 to 6 same or different atoms selected from a nitrogen atom, an oxygen atom, and a sulfur atom and having 3 to 10 ring-constituting atoms, or
      (5) an optionally substituted heteroaryl group containing 1 to 6 same or different atoms selected from a nitrogen atom, an oxygen atom, and a sulfur atom and having 5 to 10 ring-constituting atoms,
   or a pharmacologically acceptable salt thereof.
[41] The compound of the above-mentioned [1] to [40], wherein
   E is -CO-,
   or a pharmacologically acceptable salt thereof.
[42] The compound of the above-mentioned [1] to [41], wherein
   X is

      -CHR^{m}-X¹-X²

      wherein
   R^{m} is
      (1) an optionally substituted alkyl group having 1 to 10 carbon atoms,
      (2) an optionally substituted aryl group,
      (3) an optionally substituted heteroaryl group,
      (4) an optionally substituted heterocyclic group, or
      (5) an optionally substituted cycloalkyl group having 3 to 10 carbon atoms,
   X¹ is
      (1) a bond,
      (2) an optionally substituted aryl divalent group having 6 to 10 carbon atoms,
      (3) an optionally substituted heteroaryl divalent group containing 1 to 6 same or different atoms selected from a nitrogen atom, an oxygen atom, and a sulfur atom and having 5 to 10 ring-constituting atoms,
      (4) an optionally substituted cycloalkyl divalent group having 3 to 10 carbon atoms,
      (5) an optionally substituted heterocyclic divalent group containing 1 to 6 same or different atoms selected from a nitrogen atom, an oxygen atom, and a sulfur atom and having 3 to 10 ring-constituting atoms, or
      (6) -NR^{1X}-
         wherein
   R^{1X} is
      1) a hydrogen atom,
      2) an optionally substituted alkyl group having 1 to 6 carbon atoms, or
      3) an optionally substituted cycloalkyl group having 3 to 6 carbon atoms,
   X² is
      (1) a hydrogen atom,
      (2) an optionally substituted alkyl group,
      (3) an optionally substituted aryl group having 6 to 10 carbon atoms,
      (4) optionally substituted heteroaryl group containing 1 to 6 same or different atoms selected from a nitrogen atom, an oxygen atom, and a sulfur atom and having 5 to 10 ring-constituting atoms,
      (5) an optionally substituted cycloalkyl group having 3 to 10 carbon atoms,
      (6) optionally substituted heterocyclic group containing 1 to 6 same or different atoms selected from a nitrogen atom, an oxygen atom, and a sulfur atom and having 3 to 10 ring-constituting atoms,
      (7) -CO-,
      (8) -CO-(optionally substituted alkyl),
      (9) -CO-(optionally substituted aryl having 6 to 10 carbon atoms),
      (10) -CO-(optionally substituted heteroaryl containing 1 to 6 same or different atoms selected from a nitrogen atom, an oxygen atom, and a sulfur atom and having 5 to 10 ring-constituting atoms),
      (11) -CO-(optionally substituted cycloalkyl having 3 to 10 carbon atoms),
      (12) -CO-(optionally substituted heterocyclic group containing 1 to 6 same or different atoms selected from a nitrogen atom, an oxygen atom, and a sulfur atom and having 3 to 10 ring-constituting atoms),
      (13) -CO-(optionally substituted alkylene having 1 to 6 carbon atoms)-(O- optionally substituted alkylene having 1 to 6 carbon atoms)ᵣ-
         wherein r is an integer of 1 to 6,
      (14) -CO-(optionally substituted alkylene having 1 to 6 carbon atoms)-O-(optionally substituted alkylene having 1 to 6 carbon atoms)-CONH-(optionally substituted alkylene having 1 to 6 carbon atoms)-,
      (15) -CO-(optionally substituted alkylene having 1 to 6 carbon atoms)-CONH-(optionally substituted alkylene having 1 to 6 carbon atoms)-, or
      (16) -OR^{2X} or -NR^{2X}R^{2X'}
         wherein
   R^{2X} and R^{2X'} are each independently
      1) a hydrogen atom,
      2) an optionally substituted alkyl group having 1 to 6 carbon atoms, or
      3) an optionally substituted cycloalkyl group having 3 to 6 carbon atoms,
   (provided when X¹ is NR^{1x}, then X² is not -OR^{2X} or -NR^{2X}R^{2X'},
   or a pharmacologically acceptable salt thereof.
[43] The compound of the above-mentioned [42], wherein
   X¹ is

      -NR^{1X}-
   wherein R^{1X} is as defined above,
   or a pharmacologically acceptable salt thereof.
[44] The compound of the above-mentioned [43], wherein
   X² is
      (1) -CO-(optionally substituted alkyl having 1 to 6 carbon atoms),
      (2) -CO-(optionally substituted cycloalkyl having 3 to 6 carbon atoms),
      (3) -CO-(optionally substituted alkylene having 1 to 6 carbon atoms)-(O- optionally substituted alkylene having 1 to 6 carbon atoms)ᵣ-
         wherein r is an integer of 1 to 6,
      (4) -CO-(optionally substituted alkylene having 1 to 6 carbon atoms)-O-(optionally substituted alkylene having 1 to 6 carbon atoms)-CONH-(optionally substituted alkylene having 1 to 6 carbon atoms)-, or
      (5) -CO-(optionally substituted alkylene having 1 to 6 carbon atoms)-CONH-(optionally substituted alkylene having 1 to 6 carbon atoms)-,
   or a pharmacologically acceptable salt thereof.
[45] The compound of the above-mentioned [42], wherein
   X¹ is
      (1) an optionally substituted aryl divalent group having 6 to 10 carbon atoms, or
      (2) an optionally substituted heteroaryl divalent group containing 1 to 6 same or different atoms selected from a nitrogen atom, an oxygen atom, and a sulfur atom and having 5 to 10 ring-constituting atoms,
   or a pharmacologically acceptable salt thereof.
[46] The compound of the above-mentioned [45], wherein
   X¹ is
      (1) an optionally substituted heteroaryl divalent group containing 1 to 4 same or different atoms selected from a nitrogen atom, an oxygen atom, and a sulfur atom, and having 5 or 6 ring-constituting atoms,
   or a pharmacologically acceptable salt thereof.
[47] The compound of the above-mentioned [46], wherein
   X¹ is
      (1) an optionally substituted heteroaryl divalent group containing 1 to 4 same or different atoms selected from a nitrogen atom, an oxygen atom, and a sulfur atom, and having 5 ring-constituting atoms,
   or a pharmacologically acceptable salt thereof.
[48] The compound of the above-mentioned [1] to [47], wherein
   L is

      - (Lₐ)_{q}-

      wherein
   q is an integer of 1 to 100, and
   Lₐ in the number of q are each independently
      (1) a bond,
      (2) CR^{L1}R^{L1'},
      (3) O,
      (4) S,
      (5) CO,
      (6) SO,
      (7) SO₂,
      (8) NR^{L1},
      (9) CONR^{L1}
      (10) NR^{L1}CO,
      (11) NR^{L1}CONR^{L1'},
      (12) SONR^{L1},
      (13) NR^{L1}SO,
      (14) SO₂NR^{L1},
      (15) NR^{L1}SO₂,
      (16) NR^{L1}SO₂NR^{L1'}
      (17) CR^{L1}=CR^{L1'},
      (18) C ≡ C,
      (19) SiR^{L1}R^{L1'},
      (20) P(O)R^{L1},
      (21) P(O)OR^{L1},
      (22) NR^{L1}C(=NCN)NR^{L1'},
      (23) -NR^{L1}C(=NCN) -,
      (24) an optionally substituted cycloalkyl divalent group,
      (25) an optionally substituted heterocyclic divalent group,
      (26) an optionally substituted aryl divalent group, or
      (27) an optionally substituted heteroaryl divalent group (in the above-mentioned formulas, R^{L1} and R^{L1'} are each independently a group selected from
         1) a hydrogen atom
         2) a halogen atom,
         3) -CN,
         4) -NO₂,
         5) -SF₅,
         6) -CO₂H,
         7) -N(R^{L2}R^{L2'}),
         8) -A^{L2}R^{L2}
         9) an optionally substituted alkyl group,
         10) an optionally substituted cycloalkyl group,
         11) an optionally substituted heterocyclic group,
         12) an optionally substituted aryl group,
         13) an optionally substituted heteroaryl group,
         14)

            -SO₂R^{L2},
         15)

            -P(O)(OR^{L2})OR^{L2'},
         16)

            -C≡CR^{L2},
         17)

            -C(R^{L2})=C(R^{L2'}R^{L2"}),
         18)

            -COR^{L2},
         19)

            -CON(R^{L2}R^{L2'}),
         20)

            -SO₂N(R^{L2}R^{L2'}),
         21)

            -N(R^{L2})CON(R^{L2'}R^{L2"}),

            and
         22)

            -N(R^{L2})SO₂N(R^{L2'}R^{L2"})
   (in the above-mentioned formulas, A^{L2} is an oxygen atom or a sulfur atom,
   R^{L2}, R^{L2'}, and R^{L2"} are each independently
      a) a hydrogen atom,
      b) an optionally substituted C₁-C₈ alkyl group, or
      c) an optionally substituted C₃-C₈ cycloalkyl group,
   or a pharmacologically acceptable salt thereof.
[49] The compound of the above-mentioned [48], wherein
   q is an integer of 1 to 50,
   or a pharmacologically acceptable salt thereof.
[50] The compound of the above-mentioned [48] or [49], wherein
   L is -L₁-L₂-L₃-
      [wherein
   L₁ and L₃ are each independently
      (1) a bond,
      (2) CR^{L1} R^{L1'},
      (3) O,
      (4) S,
      (5) SO,
      (6) SO₂,
      (7) NR^{L1},
      (8) SO₂NR^{L1},
      (9) NR^{L1}SO₂
      (10) SONR^{L1},
      (11) NR^{L1}SO,
      (12) CONR^{L1},
      (13) NR^{L1}CO,
      (14) NR^{L1}CONR^{L1'},
      (15) NR^{L1}SO₂NR^{L1'}, or
      (16) CO, and
   L₂ is

      (CH₂)ₚ₁ₐ-O-(CH₂-CH₂-O)ₚ₂ₐ-(CH₂)ₚ₃ₐ

      wherein
   p1a and p3a are each an integer of 0 to 10 when an atom that directly binds of the adjacent L₁ or L₃ is a carbon atom or bond, and an integer of 2 to 10 in other cases, and
   p2a is an integer of 0 to 10,
   or a pharmacologically acceptable salt thereof.
[51] The compound of the above-mentioned [48] or [49], wherein
   L is -L_{b1}-L_{b2}-L_{b3}-
      wherein
   L_{b1} and L_{b3} are each independently
      (1) a bond,
      (2) CR^{L1}R^{L1'},
      (3) O,
      (4) S,
      (5) SO,
      (6) SO₂,
      (7) NR^{L1},
      (8) SO₂NR^{L1},
      (9) NR^{L1}SO₂,
      (10) SONR^{L1},
      (11) NR^{L1}SO,
      (12) CONR^{L1},
      (13) NR^{L1}CO,
      (14) NR^{L1}CONR^{L1'},
      (15) NR^{L1}SO₂NR^{L1'}, or
      (16) CO, and
   L_{b2} is
      (1) a bond,
      (2)

         (CH₂)₁₋₁₀,
      (3)

         (CH₂)₀₋₆-O-(CH₂)₀₋₆,
      (4)

         (CH₂)₀₋₆-CONH-(CH₂)₀₋₆,
      (5)

         (CH₂)₀₋₆-NHCO-(CH₂)₀₋₆,
      (6)

         (CH₂)₀₋₆-NH-(CH₂)₀₋₆,
      (7)

         (CH₂)₀₋₆-NHSO₂-(CH₂)₀₋₆,

         or
      (8)

         (CH₂)₀₋₆-SO₂NH-(CH₂)₀₋₆,
   wherein R^{L1} and R^{L1'} are as defined above,
   or a pharmacologically acceptable salt thereof.
[52] The compound of any of the above-mentioned [1] to [51], wherein
   A is a group having a moiety capable of binding to a target protein or a moiety that binds to the target protein, or a pharmacologically acceptable salt thereof.
[53] The compound of any of the above-mentioned [1] to [52], wherein
   the target protein to which A binds is a protein having a biological function selected from the group consisting of structure, regulation, hormone, enzyme, gene regulation, immunity, contraction, storage, transport, and signal transduction, or a pharmacologically acceptable salt thereof.
[54] The compound of any of the above-mentioned [1] to [52], wherein
   the target protein to which A binds is selected from the group consisting of structural protein, receptor, enzyme, cell surface protein, and proteins related to integrated cellular function, including proteins involved in catalytic activity, aromatase activity, motor activity, helicase activity, metabolic process (anabolism and catabolism), antioxidant activity, proteolysis, biosynthesis, kinase activity, oxidoreductase activity, transferase activity, hydrolase activity, lyase activity, isomerase activity, ligase activity, enzyme regulatory factor activity, signal transducer activity, structural molecular activity, binding activity (protein, lipid carbohydrates), receptor activity, cellular motility, membrane fusion, intercellular signal transduction, control of biological processes, development, cell differentiation, stimulus response, cell adhesion, cell death, transport (protein transporter activity, nuclear transport, transporter activity, channel transporter activity, carrier activity, permease activity, secretion activity, electron transporter activity), pathogenesis, virus outer shell, chaperon regulatory factor activity, nucleic acid binding activity, transcriptional regulator activity, epigenetics control, aggregation, extracellular organization, biogenetic activity, or translation regulatory factor activity, or a pharmacologically acceptable salt thereof.
[55] The compound of any of the above-mentioned [1] to [52], wherein
   the target protein to which A binds is selected from the group consisting of proteins related to cancer related protein, autoimmune disease related protein, inflammatory disease related protein, neurodegenerative disease related protein, muscular disease related protein, sensory system disease related protein, circulatory disease related protein, metabolic disease related protein, and genetic disease related protein, or a pharmacologically acceptable salt thereof.
[56] A medicament comprising the compound of the above-mentioned [1] or a pharmacologically acceptable salt thereof as an active ingredient.
[57] The medicament of the above-mentioned [56] which is a prophylactic or therapeutic agent for diseases caused by dysregulation of protein activity.
[58] A pharmaceutical composition comprising the compound of the above-mentioned [1] or a pharmacologically acceptable salt thereof as an active ingredient together with a pharmaceutically acceptable carrier.
[59] Use of the compound of the above-mentioned [1] or a pharmacologically acceptable salt thereof for the production of a prophylactic or therapeutic agent for a disease caused by dysregulation of protein activity.
[60] A method for regulating protein activity of a target protein in a mammal, comprising administering an effective amount of the compound of the above-mentioned [1] or a pharmacologically acceptable salt thereof to the mammal.
[61] A method for preventing or treating a disease caused by dysregulation of protein activity, comprising administering an effective amount of the compound of the above-mentioned [1] or a pharmacologically acceptable salt thereof to a mammal in need of the medication.
[62] The compound of the above-mentioned [1] or a pharmacologically acceptable salt thereof for use in the prophylaxis or treatment of a disease caused by dysregulation of protein activity.

### [Advantageous Effects of Invention]

The present invention relates to a target protein degradation-inducing compound that is a bifunctional compound having a portion that binds to VHL, which is a substrate recognition protein of a ubiquitin ligase complex, at one end and a portion that binds to a target protein at the other end. The target protein degradation-inducing compound of the present invention can exert a wide range of pharmacological activities by regulating the ubiquitination of the target protein and inducing the degradation of the target protein.

### [Brief Description of Drawings]

[Fig. 1]

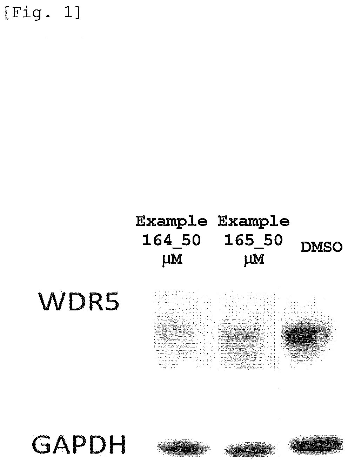

   Fig. 1 shows the results of the test on protein degradation-inducing action in cancer cells in Experimental Example 2 described later.
[Fig. 2]

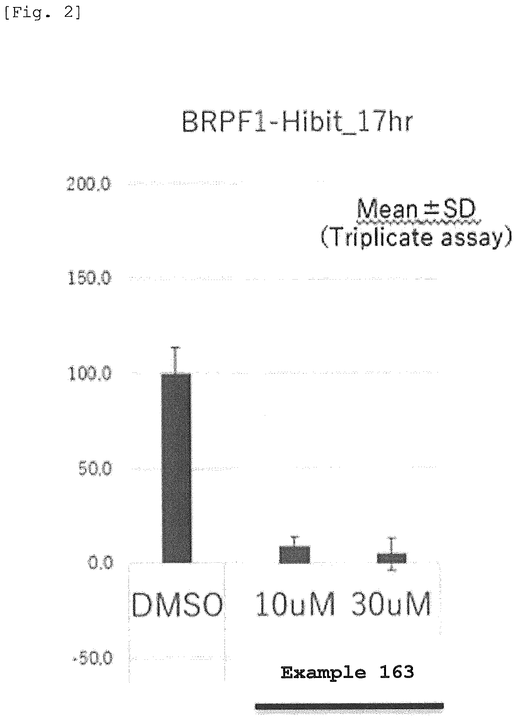

   Fig. 2 shows the results of the Hibit assay in Experimental Example 3 described later.

### [Description of Embodiments]

### [The target protein degradation-inducing compound of the present invention]

The present invention is explained in detail in the following.

### (Definition of each group used in the present specification)

The definition of each group used in the present specification is described in detail in the following. Unless particularly noted, each group has the following definition.

In the present specification, when the number of carbon atoms constituting a certain group is indicated, it is indicated at times as "C₁-C₆ " instead of "1 to 6 carbon atoms". When the number of atoms constituting a certain ring is indicated, it may be indicated as "3- to 10-membered" instead of "3 to 10 atoms constituting the ring".

When compound (I) has an acidic functional group and/or a basic functional group within the compound, it can form a salt. Examples of such salt include metal salts, ammonium salts, salts with organic bases, salts with inorganic acids, salts with organic acids, salts with basic or acidic amino acids, and the like.

In the present specification, examples of the "halogen atom" include fluorine, chlorine, bromine, and iodine.

In the present specification, as the "alkyl group" (including the "alkyl" moiety in the definition), an alkyl group having 1 to 10 carbon atoms can be mentioned. Examples thereof include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, 3-methyloctyl, nonyl, and decyl.

A more preferred embodiment is an alkyl group having 1 to 8 carbon atoms. Another preferred embodiment is an alkyl group having 1 to 6 carbon atoms. A still another embodiment is an alkyl group having 1 to 4 carbon atoms.

In the present specification, as the "alkenyl group" (including the "alkenyl" moiety in the definition), an alkenyl group having 2 - 6 carbon atoms can be mentioned. Examples thereof include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, and 5-hexenyl.

In the present specification, as the "alkynyl group" (including the "alkynyl" moiety in the definition), an alkynyl group having 2 - 6 carbon atoms can be mentioned. Examples thereof include ethynyl, 1-propynyl, 2-propynyl, 1-butynyl, 2-butynyl, 3-butynyl, 1-pentynyl, 2-pentynyl, 3-pentynyl, 4-pentynyl-1Hexynyl, 2-hexynyl, 3-hexynyl, 4-hexynyl, 5-hexynyl, and 4-methyl-2-pentynyl.

In the present specification, as the "alkylene group" (including the "alkylene" moiety in the definition), an alkylene group having 1 to 6 carbon atoms can be mentioned. Examples thereof include -CH₂-, -(CH₂)₂-, -(CH₂)₃-, -(CH₂)₄-, - (CH₂)₅-, -(CH₂)₆-, -CH(CH₃)-, -C(CH₃)₂-, -CH(C₂H₅)-, -CH(C₃H₇)-, - CH(CH(CH₃)₂)-, - (CH(CH₃))₂-, -CH₂-CH(CH₃)-, -CH (CH₃)-CH₂-, -CH₂-CH₂-C(CH₃)₂-, -C(CH₃)₂-CH₂-CH₂-, -CH₂-CH₂-CH₂-C(CH₃)₂-, and - C(CH₃)₂-CH₂-CH₂-CH₂-.

In the present specification, as the "alkenylene group" (including the "alkenylene" moiety in the definition), an alkenylene group having 2 - 6 carbon atoms can be mentioned. Examples thereof include -CH=CH-, -CH₂-CH=CH-, -CH=CH-CH₂-, - C(CH₃)₂-CH=CH-, -CH=CH-C(CH₃)₂-, -CH₂-CH=CH-CH₂-, -CH₂-CH₂-CH=CH-, -CH=CH-CH₂-CH₂-, -CH=CH-CH=CH-, -CH=CH-CH₂-CH₂-CH₂-, and -CH₂-CH₂-CH₂-CH=CH-.

In the present specification, as the "alkynylene group" (including the "alkynylene" moiety in the definition), an alkynylene group having 2 - 6 carbon atoms can be mentioned. Examples thereof include -C≡C-, -CH₂-C≡C-, -C≡C-CH₂-, - C(CH₃)₂-C≡C-, -C≡C-C(CH₃)₂-, -CH₂-C≡C-CH₂-, -CH₂-CH₂-C≡C-, -C≡ C-CH₂-CH₂-, -C≡C-C≡C-, -C≡C-CH₂-CH₂-CH₂-, and -CH₂-CH₂-CH₂-C≡C-.

In the present specification, as the "alkoxy group" (including the "alkoxy" moiety in the definition), an alkoxy group having 1 to 6 carbon atoms can be mentioned. Examples thereof include methoxy, ethoxy, propoxy, isopropoxy, butoxy, isobutoxy, sec-butoxy, tert-butoxy, pentyloxy, and hexyloxy.

A more preferred embodiment is an alkoxy group having 1 to 4 carbon atoms.

In the present specification, as the "alkoxycarbonyl group ", an (alkoxy having 1 to 6 carbon atoms)-carbonyl group can be mentioned. Examples thereof include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, and hexyloxycarbonyl.

In the present specification, as the "cycloalkyl group" (including the "cycloalkyl" moiety in the definition), a monocycle or condensed cycloalkyl group having 3 to 10 (preferably, 3 to 8) carbon atoms can be mentioned. Examples thereof include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, and adamantyl.

A more preferred embodiment is a cycloalkyl group having 3 to 6 carbon atoms.

In the present specification, as the "cycloalkyl divalent group" (at times referred to as "cycloalkane-diyl group") (including the " cycloalkyl divalent group "moiety in the definition), a divalent group formed because the "cycloalkyl group having 3 to 10 carbon atoms" explained above has another bond can be mentioned, for example, 1,3-cyclopropane-diyl.

A more preferred embodiment is a cycloalkyl divalent group having 3 to 6 carbon atoms.

In the present specification, as the "cycloalkyloxy group", a (cycloalkyl having 3 to 10 carbon atoms)-oxy group can be mentioned. Examples thereof include cyclopropyloxy, cyclobutyloxy, cyclopentyloxy, cyclohexyloxy, and cycloheptyloxy, cyclooctyloxy.

In the present specification, as the "aryl group" (including the "aryl" moiety in the definition), a monocyclic or condensed aryl group having 6 to 14 carbon atoms can be mentioned. Examples thereof include phenyl, 1-naphthyl, 2-naphthyl, 1-anthryl, 2-anthryl, and 9-anthryl.

A more preferred embodiment is an aryl group having 6 to 10 carbon atoms.

In the present specification, examples of the "C₇₋₁₆ aralkyl group" (including the "C₇₋₁₆ aralkyl" moiety in the definition) include benzyl, phenethyl, naphthylmethyl, and phenylpropyl.

In the present specification, as the "aryl divalent group" (including the "aryl divalent group "moiety in the definition) (at times referred to as "arylene group"), a divalent group formed because the "aryl group" explained above has another bond can be mentioned. Examples thereof include an aryl divalent group having 6 to 14 carbon atoms (preferably, aryl divalent group having 6 to 10 carbon atoms), for example, phenylene.

In the present specification, as the "heterocyclic group" (including the "heterocycle" moiety in the definition) (at times referred to as "non-aromatic heterocyclic group"), a monocyclic or condensed heterocyclic group (non-aromatic heterocyclic group) containing 1 to 6 (preferably 1 to 4) same or different hetero atoms selected from a nitrogen atom, an oxygen atom, and a sulfur atom as ring-constituting atoms besides carbon atom, and 3 to 14 (preferably 3 to 10) ring-constituting atoms, can be mentioned.

Preferred examples of the "heterocyclic group" ("non-aromatic heterocyclic group") include a monocyclic heterocyclic group having 3 to 8 ring-constituting atoms (non-aromatic heterocyclic group) such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolylpiperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl, and the like; and fused polycyclic (preferably bi or tricyclic)heterocyclic group (non-aromatic heterocyclic group) having 9 to 14 ring-constituting atoms such as dihydrobenzofuranyl, dihydrobenzoimidazolyl, dihydrobenzooxazolyl, dihydrobenzothiazolyl, dihydrobenzoisothiazolyl, dihydronaphto[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzoazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl, and the like.

In the present specification, the "heterocyclic group containing 1 to 6 same or different atoms selected from a nitrogen atom, an oxygen atom, and a sulfur atom and having 3 to 10 ring-constituting atoms" refers to the "heterocyclic group" explained above and containing 1 to 6 hetero atoms as ring-constituting atoms and having 3 to 10 ring-constituting atoms.

In the present specification, the "fused heterocyclic group containing 1 to 6 same or different atoms selected from a nitrogen atom, an oxygen atom, and a sulfur atom and having 6 to 10 ring-constituting atoms" refers to the "heterocyclic group" explained above which is a fused polycyclic group containing 1 to 6 hetero atoms as ring-constituting atoms and having 6 to 10 ring-constituting atoms.

In the present specification, the "heterocyclic group having 3 to 6 ring-constituting atoms" refers to the "heterocyclic group" explained above and having 3 to 6 ring-constituting atoms.

In the present specification, the "heterocyclic group containing a nitrogen atom and 1 to 5 same or different atoms selected from a nitrogen atom, an oxygen atom, and a sulfur atom and having 3 to 10 ring-constituting atoms" refers to the "heterocyclic group" explained above and containing 1 to 6 hetero atoms as ring-constituting atoms and at least one nitrogen atom as a ring-constituting atom, and having 3 to 10 ring-constituting atoms.

In the present specification, the "heterocyclic group containing 1 or 2 oxygen atoms, and having 5 or 6 ring-constituting atoms" refers to the "heterocyclic group" explained above and containing 1 or 2 oxygen atoms, and having 5 or 6 ring-constituting atoms.

In the present specification, as the "heterocyclic divalent group" (including the "heterocyclic divalent group "moiety in the definition), a divalent group formed because the "heterocyclic group" explained above has another bond can be mentioned, for example, piperazine-diyl.

In the present specification, the "heterocyclic divalent group having 3 to 6 ring-constituting atoms" refers to the "heterocyclic divalent group" explained above and having 3 to 6 ring-constituting atoms.

In the present specification, the "heterocyclic divalent group containing 1 to 6 same or different atoms selected from a nitrogen atom, an oxygen atom, and a sulfur atom and having 3 to 10 ring-constituting atoms" refers to the "heterocyclic divalent group" explained above and containing 1 to 6 hetero atoms as ring-constituting atoms and having 3 to 10 ring-constituting atoms.

In the present specification, the "heterocyclic divalent group containing 1 to 4 nitrogen atoms, and having 5 or 6 ring-constituting atoms" refers to the "heterocyclic divalent group" explained above and containing 1 to 4 nitrogen atoms, and having 5 or 6 ring-constituting atoms.

In the present specification, the "monocyclic heterocyclic divalent group containing 1 or 2 same or different atoms selected from a nitrogen atom, an oxygen atom, and a sulfur atom, and having 4 to 7 ring-constituting atoms" refers to the "heterocyclic divalent group" explained above and containing 1 or 2 hetero atoms and having 4 to 7 ring-constituting atoms.

In the present specification, as the "heteroaryl group" (including the "heteroaryl" moiety in the definition) (at times referred to as "aromatic heterocyclic group"), a monocyclic or condensed heteroaryl group (aromatic heterocyclic group) containing 1 to 6 (preferably 1 to 4) same or different hetero atom selected from a nitrogen atom, an oxygen atom, and a sulfur atom as ring-constituting atoms besides carbon atom, and having 5 to 14 (preferably 5 to 10) ring-constituting atoms can be mentioned.

Preferred examples of the "heteroaryl group" ("aromatic heterocyclic group") include a monocyclic heteroaryl group (aromatic heterocyclic group) having 5 or 6 ring-constituting atoms, such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl, and the like; and a fused polycyclic (preferably bi or tricyclic)heteroaryl group (aromatic heterocyclic group) having 8 to 14 ring-constituting atoms, such as benzothiophenyl, benzofuranyl, benzoimidazolyl, benzooxazolyl, benzoisoxazolyl, benzothiazolyl, benzoisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acrydinyl, phenazinyl, phenothiazinyl, phenoxazinyl, and the like.

In the present specification, the "heteroaryl group containing 1 to 6 same or different atoms selected from a nitrogen atom, an oxygen atom, and a sulfur atom and having 5 to 10 ring-constituting atoms" refers to the "heteroaryl group" explained above and containing 1 to 6 hetero atoms as ring-constituting atoms and having 5 to 10 ring-constituting atoms.

In the present specification, the "heteroaryl group having 5 ring-constituting atoms" refers to the "heteroaryl group" explained above and having 5 ring-constituting atoms.

In the present specification, the "heteroaryl group containing 1 to 4 same or different atoms selected from a nitrogen atom, an oxygen atom, and a sulfur atom, and having 5 or 6 ring-constituting atoms" refers to the "heteroaryl group" explained above and containing 1 to 4 hetero atoms as ring-constituting atoms and having 5 or 6 ring-constituting atoms.

In the present specification, as the "heteroaryl divalent group" (including the "heteroaryl divalent group "moiety in the definition), a divalent group formed because the "heteroaryl group" explained above has another bond can be mentioned, for example, pyridine-diyl.

In the present specification, the "heteroaryl divalent group containing 1 to 6 same or different atoms selected from a nitrogen atom, an oxygen atom, and a sulfur atom and having 5 to 10 ring-constituting atoms" refers to the "heteroaryl divalent group" explained above and containing 1 to 6 hetero atoms as ring-constituting atoms and having 5 to 10 ring-constituting atoms.

In the present specification, the "heteroaryl divalent group containing 1 to 4 same or different atoms selected from a nitrogen atom, an oxygen atom, and a sulfur atom, and having 5 or 6 ring-constituting atoms" refers to the "heteroaryl divalent group" explained above and containing 1 to 4 hetero atoms and having 5 or 6 ring-constituting atoms.

In the present specification, as the "aromatic hydrocarbocycle", an aromatic hydrocarbocycle having 6 to 14 (preferably, 6 to 10) carbon atoms can be mentioned. Examples thereof include benzene and naphthalene.

In the present specification, as the "non-aromatic hydrocarbocycle", a saturated or partially unsaturated cyclic hydrocarbocycle can be mentioned. Examples thereof include cycloalkane and cycloalkene.

In the present specification, as the "cycloalkane", "C₃₋₁₀ cycloalkane" can be mentioned. Examples thereof include cyclopropane, cyclobutane, cyclopentane, cyclohexane, cycloheptane, cyclooctane, cyclononane, and cyclodecane.

In the present specification, as the "cycloalkene", "C₃₋₁₀ cycloalkene" can be mentioned. Examples thereof include cyclopropene, cyclobutene, cyclopentene, cyclohexene, cycloheptene, cyclooctene, cyclononene, and cyclodecene.

In the present specification, the "non-aromatic hydrocarbocycle having 5 or 6 ring-constituting atoms" refers to the "non-aromatic hydrocarbocycle" explained above and having 5 or 6 ring-constituting atoms.

In the present specification, as the "aromatic heterocycle", an aromatic heterocycle containing 1 to 6 (preferably 1 to 4) hetero atoms selected from a nitrogen atom, an oxygen atom, and a sulfur atom as ring-constituting atoms besides carbon atom, and having 5 to 14 (preferably 5 to 10) ring-constituting atoms can be mentioned. Preferred examples of the "aromatic heterocycle" include monocyclic aromatic heterocycle having 5 or 6 (5- or 6- membered) ring-constituting atoms such as thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, triazine and the like;
(8- to 14-membered) fused polycyclic (preferably bi or tricyclic) aromatic heterocycle having 8 to 14 ring-constituting atoms such as benzothiophene, benzofuran, benzoimidazole, benzoxazole, benzoisoxazole, benzothiazole, benzoisothiazole, benzotriazole, imidazopyridine, thienopyridine, furopyridine, pyrrolopyridine, pyrazolopyridine, oxazolopyridine, thiazolopyridine, imidazopyrazine, imidazopyrimidine, thienopyrimidine, furopyrimidine, pyrrolopyrimidine, pyrazolopyrimidine, oxazolopyrimidine, thiazolopyrimidine, pyrazolopyrimidine, pyrazolotriazine, naphtho[2,3-b]thiophene, phenoxathiine, indole, isoindole, 1H-indazole, purine, isoquinoline, quinoline, phthalazine, naphthyridine, quinoxaline, quinazoline, cinnoline, carbazole, β-carboline, phenanthridine, acridine, phenazine, phenothiazine, phenoxathiine and the like.

In the present specification, the "aromatic heterocycle containing a nitrogen atom, and further optionally containing 1 to 5 same or different atoms selected from a nitrogen atom, an oxygen atom, and a sulfur atom" refers to the "aromatic heterocycle" explained above and containing 1 to 6 hetero atoms as ring-constituting atoms and having at least one nitrogen atom as the ring-constituting atom.

In the present specification, as the "heterocycle", for example, a non-aromatic heterocycle containing 1 to 6(preferably, 1 to 4) hetero atoms selected from a nitrogen atom, an oxygen atom, and a sulfur atom as ring-constituting atoms besides carbon atom, and having 3 to 14 (preferably 3 to 10) ring-constituting atoms can be mentioned. Preferred examples of the "heterocycle" ("non-aromatic heterocycle") include a monocyclic non-aromatic heterocycle having 3 to 8 (3-to 8-membered) ring-constituting atoms such as aziridine, oxirane, thiirane, azetidine, oxetane, thietane, tetrahydrothiophene, tetrahydrofuran, pyrroline, pyrrolidine, imidazoline, imidazolidine, oxazoline, oxazolidine, pyrazoline, pyrazolidine, thiazoline, thiazolidine, tetrahydroisothiazole, tetrahydrooxazole, tetrahydroisoxazole, piperidine, piperazine, tetrahydropyridine, dihydropyridine, dihydrothiopyran, tetrahydropyrimidine, tetrahydropyridazine, dihydropyran, tetrahydropyran, tetrahydrothiopyran, morpholine, thiomorpholine, azepane, diazepane, azepine, azocane, diazocane, oxepane and the like; and
a fused polycyclic (preferably bi or tricyclic) non-aromatic heterocycle having 9 to 14 (9- to 14-membered) ring-constituting atoms such as dihydrobenzofuran, dihydrobenzoimidazole, dihydrobenzooxazole, dihydrobenzothiazole, dihydrobenzoisothiazole, dihydronaphto[2,3-b]thiophene, tetrahydroisoquinoline, tetrahydroquinoline, 4H-quinolizine, indoline, isoindoline, tetrahydrothieno[2,3-c]pyridine, tetrahydrobenzoazepine, tetrahydroquinoxaline, tetrahydrophenanthridine, hexahydrophenothiazine, hexahydrophenoxathiine, tetrahydrophthalazine, tetrahydronaphthyridine, tetrahydroquinazoline, tetrahydrocinnoline, tetrahydrocarbazole, tetrahydro-β-carboline, tetrahydroacridine, tetrahydrophenazine, tetrahydrothioxanthene, octahydroisoquinoline and the like.

In the present specification, the "heterocycle containing a nitrogen atom, and further optionally containing 1 to 5 same or different atoms selected from a nitrogen atom, an oxygen atom, and a sulfur atom" refers to the "heterocycle" explained above and containing 1 to 6 hetero atoms as the ring-constituting atoms and having at least one nitrogen atom as the ring-constituting atom.

In the present specification, the "heterocycle containing a nitrogen atom, and further optionally containing 1 to 3 same or different atoms selected from a nitrogen atom, an oxygen atom, and a sulfur atom, and having 5 or 6 ring-constituting atoms" refers to the "heterocycle" explained above and containing 1 to 4 hetero atoms as the ring-constituting atoms and containing at least one nitrogen atom as the ring-constituting atom and having 5 or 6 ring-constituting atoms.

In the present specification, the "heterocycle containing 1 to 6 same or different atoms selected from a nitrogen atom, an oxygen atom, and a sulfur atom, and having 3 to 10 ring-constituting atoms" refers to the "heterocycle" explained above and containing 1 to 6 hetero atoms as the ring-constituting atoms and having 3 to 10 ring-constituting atoms.

In the present specification, as the "substituent" of the "optionally substituted group and ring" in the "optionally substituted alkyl group", "optionally substituted cycloalkyl group", "optionally substituted aryl group", "optionally substituted heterocyclic group", "optionally substituted heteroaryl group", "optionally substituted fused heterocyclic group containing 1 to 6 same or different atoms selected from a nitrogen atom, an oxygen atom, and a sulfur atom and having 6 to 10 ring-constituting atoms", "optionally substituted heteroaryl group containing 1 to 6 same or different atoms selected from a nitrogen atom, an oxygen atom, and a sulfur atom and having 5 to 10 ring-constituting atoms", and other definitions relating to compound (I), the substituents selected from the following [Substituent Group] can be mentioned.

1 to 5 (preferably 1 to 3) "substituents" may be present at substitutable positions. When the number of the substituents is two or more, each substituent may be the same or different.

When specific explanations on the "substituent" of each group, ring, and the like are given, such explanations are to be followed.

### [Substituent Group]

(1) halogen atom,
(2) nitro group,
(3) cyano group,
(4) oxo group,
(5) hydroxy group,
(6) optionally halogenated C₁-C₆ alkoxy group (e.g., methoxy, chloromethoxy, trifluoroethoxy),
(7) C₆-C₁₄ aryloxy group (e.g., phenoxy, naphthoxy),
(8) C₇-C₁₆ aralkyloxy group (e.g., benzyloxy),
(9) C₁-C₆ alkyl-carbonyloxy group (e.g., acetoxy, propanoyloxy),
(10) C₆-C₁₄ aryl-carbonyloxy group (e.g., benzoyloxy, 1-naphthoyloxy, 2-naphthoyloxy),
(11) C₁-C₆ alkoxy-carbonyloxy group (e.g., methoxycarbonyloxy, ethoxycarbonyloxy, propoxycarbonyloxy, butoxycarbonyloxy),
(12) 5- to 14-membered aromatic heterocyclic group,
(13) 3- to 14-membered non-aromatic heterocyclic group,
(14) formyl group,
(15) carboxy group,
(16) optionally halogenated C₁-C₆ alkyl-carbonyl group (e.g., acetyl, chloroacetyl, trifluoroacetyl),
(17) C₆-C₁₄ aryl-carbonyl group (e.g., benzoyl, 1-naphthoyl, 2-naphthoyl),
(18) C₁-C₆ alkoxy-carbonyl group (e.g., methoxycarbonyl, ethoxycarbonyl),
(19) carbamoyl group,
(20) amino group,
(21) mono- or di-C₁-C₆ alkylamino group (e.g., methylamino, ethylamino, propylamino, isopropylamino, butylamino, dimethylamino, diethylamino, dipropylamino, dibutylamino, N-ethyl-N-methylamino),
(22) mono- or di-C₆-C₁₄ arylamino group (e.g., phenylamino),
(23) formylamino group,
(24) C₁-C₆ alkyl-carbonylamino group (e.g., acetylamino, propanoylamino, butanoylamino),
(25) C₁-C₆ alkoxy-carbonylamino group (e.g., methoxycarbonylamino, ethoxycarbonylamino, propoxycarbonylamino, butoxycarbonyl amino, tert-butoxycarbonyl amino),
(26) optionally halogenated C₁-C₆ alkyl group (e.g., methyl, chloromethyl, difluoromethyl, trifluoromethyl, ethyl, 2-bromoethyl, 2,2,2-trifluoroethyl),
(27) C₂-C₆ alkenyl group (e.g., ethenyl, 1-propenyl, 2-propenyl, 2-methyl-1-propenyl, 1-butenyl),
(28) C₂-C₆ alkynyl group (e.g., ethynyl, 1-propynyl, 2-propynyl, 1-butynyl, 2-butynyl, 3-butynyl),
(29) C₃-C₁₀ cycloalkyl group (e.g., cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, adamantyl),
(30) C₃-C₁₀ cycloalkenyl group (e.g., cyclopropenyl, cyclobutenyl, cyclopentenyl, cyclohexenyl, cycloheptenyl, cyclooctenyl), and
(31) C₆-C₁₄ aryl group (e.g., phenyl, naphthyl).

In the present specification, the "chemical linker" can be determined appropriately by those of ordinary skill in the art according to the selection of the target protein. The detail is described later.

In the present specification, the "target-directed ligand" is, for example, a group having a portion capable of binding to the "target protein" or a portion that binds to the "target protein". The detail is described later.

The compound of the present invention is a compound (I) represented by the following structural formula (I) or a pharmacologically acceptable salt thereof. 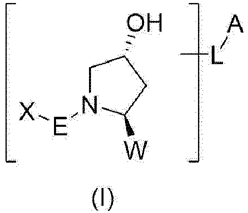
wherein L is bonded to either W or X;
E is a bond, -CO-, -SO-, or -SO₂-;
X is an optionally substituted alkyl group,
an optionally substituted cycloalkyl group,
an optionally substituted aryl group,
an optionally substituted heterocyclic group, or
an optionally substituted heteroaryl group;
W is an optionally substituted aryl group,
an optionally substituted fused heterocyclic group containing 1 to 6 same or different atoms selected from a nitrogen atom, an oxygen atom, and a sulfur atom and having 6 to 10 ring-constituting atoms,
an optionally substituted heteroaryl group containing 1 to 6 same or different atoms selected from a nitrogen atom, an oxygen atom, and a sulfur atom and having 3 to 10 ring-constituting atoms,
an alkyl group optionally substituted by a group selected from a halogen atom, a hydroxy group, an alkoxy group, and a heterocyclic group,
a cyano group, or
a hydrogen atom;
L is a bond or a chemical linker; and
A is a target-directed ligand.)

In compound (I), L is bonded to either W or X. Therefore, compound (I) may structurally take the following two embodiments. These embodiments are all encompassed in the scope of the present invention. 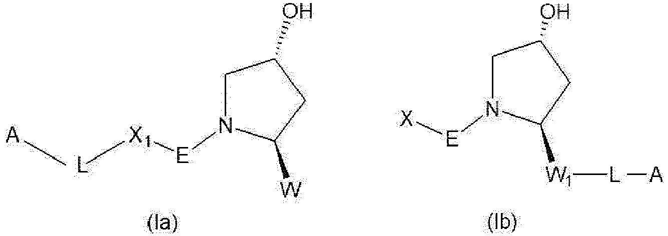 wherein E, X, W, L, and A are as defined above, and X₁ and W₁ are divalent groups derived from X and W, respectively.

Each symbol of compound (I) is described in detail below.

### [E]

E is a bond, -CO-, -SO-, or -SO₂-, preferably, a bond or -CO-, more preferably -CO-.

### [X]

X is
an optionally substituted alkyl group,
an optionally substituted cycloalkyl group,
an optionally substituted aryl group,
an optionally substituted heterocyclic group, or
an optionally substituted heteroaryl group,
preferably,
an optionally substituted alkyl group having 1 to 6 carbon atoms,
an optionally substituted cycloalkyl group having 3 to 10 carbon atoms,
an optionally substituted aryl group having 6 to 10 carbon atoms,
an optionally substituted heterocyclic group containing 1 to 6 same or different atoms selected from a nitrogen atom, an oxygen atom, and a sulfur atom and having 3 to 10 ring-constituting atoms, (more preferably, an optionally substituted heterocyclic group containing 1 to 4 same or different atoms selected from nitrogen atom, oxygen atom, and sulfur atom, and having 5 or 6 ring-constituting atoms), or
an optionally substituted heteroaryl group containing 1 to 6 same or different atoms selected from a nitrogen atom, an oxygen atom, and a sulfur atom and having 5 to 10 ring-constituting atoms (more preferably, an optionally substituted heteroaryl group containing 1 to 4 same or different atoms selected from a nitrogen atom, an oxygen atom, and a sulfur atom, and having 5 or 6 ring-constituting atoms).

In another preferred embodiment, X is a group represented by -CHR^{m}-X¹-X², wherein R^{m} is
(1) an optionally substituted alkyl group having 1 to 10 carbon atoms (preferably, an optionally substituted alkyl group having 1 to 6 carbon atoms),
(2) an optionally substituted aryl group (preferably, an optionally substituted aryl group having 6 to 10 carbon atoms),
(3) an optionally substituted heteroaryl group (preferably, an optionally substituted heteroaryl group containing 1 to 6 same or different atoms selected from a nitrogen atom, an oxygen atom, and a sulfur atom and having 5 to 10 ring-constituting atoms),
(4) an optionally substituted heterocyclic group (preferably, an optionally substituted heterocyclic group containing 1 to 6 same or different atoms selected from a nitrogen atom, an oxygen atom, and a sulfur atom and having 3 to 10 ring-constituting atoms)
(5) an optionally substituted cycloalkyl group having 3 to 10 carbon atoms,

X¹ is
   (1) a bond,
   (2) an optionally substituted aryl divalent group having 6 to 10 carbon atoms,
   (3) an optionally substituted heteroaryl divalent group containing 1 to 6 same or different atoms selected from a nitrogen atom, an oxygen atom, and a sulfur atom and having 5 to 10 ring-constituting atoms,
      preferably, a heteroaryl divalent group containing 1 to 4 same or different atoms selected from a nitrogen atom, an oxygen atom, and a sulfur atom, and having 5 or 6 ring-constituting atoms (e.g., pyridine-diyl, pyrimidine-diyl, pyrazole-diyl, imidazole-diyl, triazole-diyl, tetrazole-diyl, isoxazole-diyl, oxadiazole-diyl, etc.), which is optionally substituted by an alkyl group having 1 to 6 carbon atoms,
   (4) an optionally substituted cycloalkyl divalent group having 3 to 10 carbon atoms,
   (5) an optionally substituted heterocyclic divalent group containing 1 to 6 same or different atoms selected from a nitrogen atom, an oxygen atom, and a sulfur atom and having 3 to 10 ring-constituting atoms, or
   (6) -NR^{1X}-
      wherein
R^{1X} is
   1) a hydrogen atom,
   2) an optionally substituted alkyl group having 1 to 6 carbon atoms, or
   3) an optionally substituted cycloalkyl group having 3 to 6 carbon atoms,
X² is
   (1) a hydrogen atom,
   (2) an optionally substituted alkyl group (preferably, an optionally substituted alkyl group having 1 to 6 carbon atoms, more preferably, an alkyl group having 1 to 6 carbon atoms),
   (3) an optionally substituted aryl group having 6 to 10 carbon atoms,
   (4) an optionally substituted heteroaryl group containing 1 to 6 same or different atoms selected from a nitrogen atom, an oxygen atom, and a sulfur atom and having 5 to 10 ring-constituting atoms,
      preferably, a heteroaryl group containing 1 to 4 same or different atoms selected from a nitrogen atom, an oxygen atom, and a sulfur atom, and having 5 or 6 ring-constituting atoms (e.g., pyridyl, isoxazolyl, etc.), which is optionally substituted by 1 to 3 substituents selected from an alkyl group having 1 to 6 carbon atoms and an alkoxy group having 1 to 6 carbon atoms,
   (5) an optionally substituted cycloalkyl group having 3 to 10 carbon atoms,
   (6) an optionally substituted heterocyclic group containing 1 to 6 same or different atoms selected from a nitrogen atom, an oxygen atom, and a sulfur atom and having 3 to 10 ring-constituting atoms,
   (7) -CO-,
   (8) -CO-(optionally substituted alkyl),
      preferably, -CO-(optionally substituted alkyl having 1 to 6 carbon atoms),
      more preferably, -CO-(alkyl having 1 to 6 carbon atoms which is optionally substituted by an optionally substituted heteroaryl group containing 1 to 6 same or different atoms selected from a nitrogen atom, an oxygen atom, and a sulfur atom and having 5 to 10 ring-constituting atoms),
      further preferably, -CO-(alkyl having 1 to 6 carbon atoms optionally substituted by a heteroaryl group containing 1 to 4 same or different atoms selected from a nitrogen atom, an oxygen atom, and a sulfur atom, and having 5 or 6 ring-constituting atoms (e.g., isoxazolyl, etc.), which is optionally substituted by an alkyl group having 1 to 6 carbon atoms),
   (9) -CO-(optionally substituted aryl having 6 to 10 carbon atoms),
   (10) -CO-(optionally substituted heteroaryl containing 1 to 6 same or different atoms selected from a nitrogen atom, an oxygen atom, and a sulfur atom and having 5 to 10 ring-constituting atoms),
   (11) -CO-(optionally substituted cycloalkyl having 3 to 10 carbon atoms),
      preferably, -CO-(cycloalkyl having 3 to 6 carbon atoms (e.g., cyclopropyl) optionally substituted by halogen),
   (12) -CO-(a optionally substituted heterocyclic group containing 1 to 6 same or different atoms selected from a nitrogen atom, an oxygen atom, and a sulfur atom and having 3 to 10 ring-constituting atoms),
   (13) -CO-(optionally substituted alkylene having 1 to 6 carbon atoms)-(O- optionally substituted alkylene having 1 to 6 carbon atoms)ᵣ-
      wherein r is an integer of 1 to 6,
      preferably, -CO-(alkylene having 1 to 6 carbon atoms)-(O-alkylene having 1 to 6 carbon atoms)ᵣ-
      wherein r is an integer of 1 to 6,
   (14) -CO-(optionally substituted alkylene having 1 to 6 carbon atoms)-O-(optionally substituted alkylene having 1 to 6 carbon atoms)-CONH-(optionally substituted alkylene having 1 to 6 carbon atoms)-,
      preferably, -CO-(alkylene having 1 to 6 carbon atoms)-O-(alkylene having 1 to 6 carbon atoms)-CONH-(alkylene having 1 to 6 carbon atoms)-,
      more preferably, -CO-(alkylene having 1 to 6 carbon atoms)-O-(alkylene having 1 to 6 carbon atoms)-CONH-(alkylene having 1 to 6 carbon atoms)-,
   (15) -CO-(optionally substituted alkylene having 1 to 6 carbon atoms)-CONH-(optionally substituted alkylene having 1 to 6 carbon atoms)-,
      preferably, -CO-(alkylene having 1 to 6 carbon atoms)-CONH-(alkylene having 1 to 6 carbon atoms)-, or
   (16) -OR^{2X} or -NR^{2X}R^{2X'}
      wherein
R^{2X} and R^{2X'} are each independently
   1) a hydrogen atom,
   2) an optionally substituted alkyl group having 1 to 6 carbon atoms (preferably, an alkyl group having 1 to 6 carbon atoms), or
   3) an optionally substituted cycloalkyl group having 3 to 6 carbon atoms.

### [W]

W is
(W1) an optionally substituted aryl group,
(W2) an optionally substituted fused heterocyclic group containing 1 to 6 same or different atoms selected from a nitrogen atom, an oxygen atom, and a sulfur atom and having 6 to 10 ring-constituting atoms,
(W3) an optionally substituted heteroaryl group containing 1 to 6 same or different atoms selected from a nitrogen atom, an oxygen atom, and a sulfur atom and having 3 to 10 ring-constituting atoms,
(W4) an alkyl group optionally substituted by a group selected from a halogen atom, a hydroxy group, an alkoxy group, and a heterocyclic group,
(W5) a cyano group, or
(W6) a hydrogen atom.

The groups (W1) to (W4) for W are described in detail below.

### group (W1): an optionally substituted aryl group

The "aryl group" of the "optionally substituted aryl group" is preferably an aryl group having 6 to 14 carbon atoms, more preferably, an aryl group having 6 to 10 carbon atoms.

The aryl group is optionally substituted at substitutable position(s) by 1 to 3 same or different substituents selected from the "Substituent Group" explained in the aforementioned "Definition of each group used in the present specification".

### group (W2): an optionally substituted fused heterocyclic group containing 1 to 6 same or different atom selected from nitrogen atom, oxygen atom, and sulfur atom, and having 6 to 10 ring-constituting atoms

The aforementioned "Definition of each group used in the present specification" can be referred to for the meaning of the "fused heterocyclic group" of the "optionally substituted fused heterocyclic group containing 1 to 6 same or different atoms selected from a nitrogen atom, an oxygen atom, and a sulfur atom and having 6 to 10 ring-constituting atoms. Preferably, both an embodiment in which two 6-membered rings are condensed, and an embodiment in which a 5-membered ring and a 6-membered ring are condensed are encompassed.

The "fused heterocyclic group" is optionally substituted at substitutable position(s) by 1 to 3 same or different substituents selected from the "Substituent Group" explained in the aforementioned "Definition of each group used in the present specification".

The former encompasses, for example, tetrahydroquinoline, tetrahydroquinazoline, and the like.

The latter is more specifically explained below. The latter is preferably a group represented by the formula (W2a): 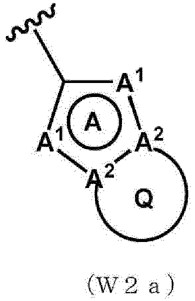 wherein
group A is an optionally substituted heteroaryl group having five ring-constituting atoms, and two A¹ are each independently a group or atom selected from CR^{Z1}, N, NR^{Z2}, O, and S,
two A² are each independently C or N; and
ring Q is
   an aromatic hydrocarbocycle,
   an aromatic heterocycle optionally containing 1 to 6 same or different atoms selected from a nitrogen atom, an oxygen atom, and a sulfur atom,
   a non-aromatic hydrocarbocycle, or
   a heterocycle optionally containing 1 to 6 same or different atoms selected from a nitrogen atom, an oxygen atom, and a
   sulfur atom,
wherein the ring has 5 to 7 ring-constituting atoms and is optionally substituted at a substitutable position by a group selected from
   halogen atom,
   hydroxy group,
   cyano group,
   hydroxycarbonyl group,
   oxo group,
   thioxo group,
   optionally substituted alkyl group,
   optionally substituted cycloalkyl group,
   optionally substituted alkoxy group,
   optionally substituted cycloalkyloxy group,

      -CO-N(R^{7a})(R^{7b}),

      -N(R^{7a})(R^{7b}),

      -N(R^{7c})-CO-R^{7d}, and

      -CO-R^{7e},

      (in each of the above-mentioned formulas,
R^{Z1} and R^{Z2} are each independently a group selected from a hydrogen atom; a halogen atom; a hydroxy group; a cyano group; a hydroxycarbonyl group; an optionally substituted alkyl group; an optionally substituted cycloalkyl group; an optionally substituted alkoxy group; an optionally substituted alkoxycarbonyl group; -CO-N (R^{7a})(R^{7b}); -N (R^{7a})(R^{7b}); - N(R^{7c})-CO-R^{7d}; an aryl group; an optionally substituted heteroaryl group containing 1 to 3 same or different atoms selected from a nitrogen atom, an oxygen atom, and a sulfur atom and having 5 or 6 ring-constituting atoms; (provided that R^{Z2} is not a halogen atom; a hydroxy group; a cyano group; a hydroxycarbonyl group; -N (R^{7a})(R^{7b}), or -N (R^{7c})-CO-R^{7d});
R^{7a} and R^{7b} are each independently a hydrogen atom; or an alkyl group optionally substituted by a group selected from a halogen atom, a hydroxy group, an alkoxy group, and a cyano group, or
R^{7a} and R^{7b} are optionally bonded to each other to form, together with the adjacent nitrogen atom, a heterocycle optionally substituted by a group selected from a halogen atom, a hydroxy group, an alkoxy group, and a cyano group;
R^{7c} and R^{7d} are each independently a hydrogen atom; or an alkyl group optionally substituted by a group selected from a halogen atom, a hydroxy group, an alkoxy group, and a cyano group; and
R^{7e} is an optionally substituted alkyl group, an optionally substituted cycloalkyl group, an optionally substituted aryl group, or an optionally substituted heteroaryl group.

The "optionally substituted heteroaryl group having five ring-constituting atoms, and two A¹ are each independently a group or atom selected from =CR^{Z1}-, =N-, -NR^{Z2}-, -O-, and -S-, two A² are each independently a carbon atom or a nitrogen atom" for group A is, for example, pyrrolyl, imidazolyl, thienyl, furyl, oxazolyl, thiazolyl, triazolyl, tetrazolyl, or the like.

The aforementioned "Definition of each group used in the present specification" can be referred to for the meaning of each group of the "aromatic hydrocarbocycle", "aromatic heterocycle optionally containing 1 to 6 same or different atoms selected from a nitrogen atom, an oxygen atom, and a sulfur atom", "non-aromatic hydrocarbocycle", and "heterocycle optionally containing 1 to 6 same or different atoms selected from a nitrogen atom, an oxygen atom, and a sulfur atom" in ring Q.

The aforementioned "Definition of each group used in the present specification" can be also referred to for the meaning of each group of the "halogen atom", "optionally substituted alkyl group", "optionally substituted cycloalkyl group", "optionally substituted alkoxy group", "optionally substituted cycloalkyloxy group", "-CO-N(R^{7a})(R^{7b})", "-N(R^{7a})(R^{7b})", "-N(R^{7c})-CO-R^{7d}", and "-CO-R^{7e}", which are the substituents used for substituting the above-mentioned "aromatic hydrocarbocycle" and the like in ring Q.

Group (W2) is more preferably a group represented by the formula (W2b):

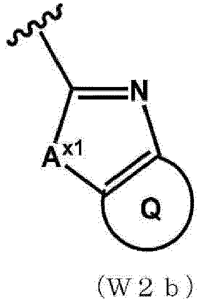

wherein
A^{x1} is a group or atom selected from -NR^{Z2}-, -O-, and -S-(more preferably, a group or atom selected from -NR^{Z2}- and -S-);
ring Q is a non-aromatic hydrocarbocycle having 5 or 6 ring-constituting atoms (e.g., tetrahydrobenzene); or heterocycle optionally containing 1 to 3 same or different atoms selected from a nitrogen atom, an oxygen atom, and a sulfur atom, and having 5 or 6 ring-constituting atoms (more preferably, heterocycle optionally containing 1 to 3 same or different atoms selected from a nitrogen atom and an oxygen atom, and having 6 ring-constituting atoms), wherein the ring is optionally substituted at a substitutable position by a substituent selected from -CO-R^{7e}, halogen atom, optionally substituted alkyl group having 1 to 4 carbon atoms, and cycloalkyl group having 3 or 4 carbon atoms,
wherein R^{z2} and R^{7e} are as defined above.

group (W3): an optionally substituted heteroaryl group containing 1 to 6 same or different atoms selected from a nitrogen atom, an oxygen atom, and a sulfur atom and having 3 to 10 ring-constituting atoms

The aforementioned "Definition of each group used in the present specification" can be referred to for the meaning of the "heteroaryl group containing 1 to 6 same or different atoms selected from a nitrogen atom, an oxygen atom, and a sulfur atom and having 3 to 10 ring-constituting atoms" and the "substituent" used to optionally substitute the heteroaryl group.

The group (W3) is preferably a group represented by the following formula (W3a): 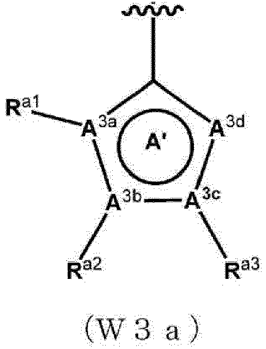 wherein
group A' is a heteroaryl group having 5 ring-constituting atoms, A^{3a}, A^{3b}, A^{3c}, and A^{3d} are each independently an atom selected from a nitrogen atom, an oxygen atom, a carbon atom, and a sulfur atom, and when A^{3a}, A^{3b}, and A^{3c} have a nitrogen atom or a carbon atom, they respectively have R^{a1}, R^{a2}, and R^{a3}.
   wherein R^{a1} is
   (1) a hydrogen atom,
   (2) an optionally substituted alkyl group,
   (3) an optionally substituted cycloalkyl group,
   (4) an optionally substituted aryl group,
   (5) an optionally substituted heteroaryl group, or
   (6) an optionally substituted heterocyclic group, and
R^{a2} and R^{a3} are each independently
   (1) a hydrogen atom,
   (2) CN,
   (3) a halogen atom,
   (4) an optionally substituted alkyl group,
   (5) an optionally substituted cycloalkyl group,
   (6) an optionally substituted aryl group,
   (7) an optionally substituted heteroaryl group,
   (8) an optionally substituted heterocyclic group,
   (9) -V^{a3a}-(optionally substituted alkyl),
   (10) -V^{a3a}-(optionally substituted cycloalkyl),
   (11) -V^{a3a}-(optionally substituted aryl),
   (12) -V^{a3a}-(optionally substituted heteroaryl), or
   (13) -V^{a3a}-(optionally substituted heterocyclic group)
      (in the above-mentioned formulas, V^{a3a} is
      1) -CO-,
      2) -NR^{Va3}-
         wherein R^{Va3} is
         (a) a hydrogen atom,
         (b) an optionally substituted alkyl group having 1 to 6 carbon atoms, or
         (c) an optionally substituted cycloalkyl group having 3 to 6 carbon atoms,
      3) -O-,
      4) -S-,
      5) -SO-, or
      6) -SO₂-, or
   (14) -V^{a3b}-NR^{Na3}R^{Na3'}
      wherein V^{a3b} is
         1) -CO-
         2) -SO-, or
         3) -SO₂-,
      R^{Na3} and R^{Na3'} are each independently
         1) a hydrogen atom,
         2) an optionally substituted alkyl group,
         3) an optionally substituted cycloalkyl group,
         4) an optionally substituted aryl group,
         5) an optionally substituted heteroaryl group, or
         6) an optionally substituted heterocyclic group,
         more preferably,
      R^{a1} is
         (1) a hydrogen atom,
         (2) an optionally substituted alkyl group, and
      R^{a2} and R^{a3} are each independently
         (1) a hydrogen atom,
         (4) an optionally substituted alkyl group,
         (6) an optionally substituted aryl group,
   (13) -V^{a3a}-(optionally substituted heterocyclic group)
      (in the above-mentioned formulas, V^{a3a} is
      1) -CO-, or
   (14) -V^{a3b}-NR^{Na3}R^{Na3'}
      wherein V^{a3b} is
      1) -CO-,
R^{Na3} and R^{Na3'} are each independently
   1) a hydrogen atom,
   2) an optionally substituted alkyl group,
   3) an optionally substituted cycloalkyl group, or
   6) an optionally substituted heterocyclic group.

The "group A'" in the group represented by the formula (W3a) is preferably a group selected from imidazolyl group, pyrazolyl group, thiazolyl group (e.g., 1,2-thiazolyl, 1,3-thiazolyl), isothiazolyl group, thiadiazolyl group, isothiadiazolyl group, oxazolyl group, isoxazolyl group, oxadiazolyl group, triazolyl group (1,2,3-triazolyl, 1,2,4-triazolyl), and tetrazolyl group, further preferably, a group selected from imidazolyl group, thiazolyl group, and oxazolyl group.

The aforementioned "Definition of each group used in the present specification" can be referred to for the meanings of R^{a1}, R^{a2} and R^{a3} in the group represented by the formula (W3a) .

The group (W3) is more preferably a group represented by the following formula (W3b) or (W3c): 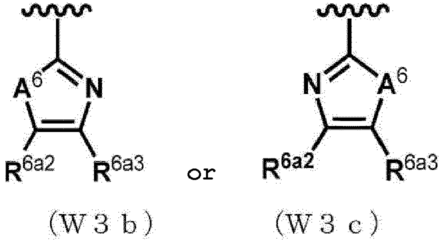 wherein
A⁶ is -O-, -S-, or -NR^{6a1}-
wherein R^{6a1} is
   (1) a hydrogen atom,
   (2) an optionally substituted alkyl group,
   (3) an optionally substituted cycloalkyl group,
   (4) an optionally substituted aryl group,
   (5) an optionally substituted heteroaryl group, or
   (6) an optionally substituted heterocyclic group;
R6a2 is
   (1) a hydrogen atom,
   (2) CN,
   (3) a halogen atom,
   (4) an optionally substituted alkyl group having 1 to 4 carbon atoms,
   (5) an optionally substituted cyclopropyl group,
   (6) an optionally substituted oxetanyl group,
   (7) an optionally substituted azetidinyl group,
   (8) an optionally substituted aryl group having 6 to 10 carbon atoms, or
   (9) -CO-NR^{a2N6}R^{a2N6'}
wherein R^{a2N6} and R^{a2N6'} are each independently
   1) a hydrogen atom,
   2) an optionally substituted alkyl group having 1 to 4 carbon atoms,
   3) an optionally substituted cyclopropyl group,
   4) an optionally substituted oxetanyl group, or
   5) an optionally substituted azetidinyl group; and
R6a3 is
   (1) a hydrogen atom,
   (2) CN,
   (3) a halogen atom,
   (4) an optionally substituted alkyl group,
   (5) an optionally substituted cycloalkyl group,
   (6) an optionally substituted aryl group,
   (7) an optionally substituted heteroaryl group,
   (8) an optionally substituted heterocyclic group,
   (9) -CO-(optionally substituted heterocyclic group), or
   (10) -CO-NR^{a3N6}R^{a3N6'}
wherein R^{a3N6} and R^{a3N6'} are each independently
   1) a hydrogen atom,
   2) an optionally substituted alkyl group,
   3) an optionally substituted cycloalkyl group,
   4) an optionally substituted aryl group,
   5) an optionally substituted heteroaryl group, or
   6) an optionally substituted heterocyclic group.

R^{6a1}, R^{6a2}, and R^{6a3} in the above-mentioned formula are explained in detail below.

R^{6a1} is
(1) a hydrogen atom,
(2) an optionally substituted alkyl group,
(3) an optionally substituted cycloalkyl group,
(4) an optionally substituted aryl group,
(5) an optionally substituted heteroaryl group, or
(6) an optionally substituted heterocyclic group,
   preferably,
   (1) a hydrogen atom,
   (2) an optionally substituted alkyl group having 1 to 6 carbon atoms,
   (3) an optionally substituted cycloalkyl group having 3 to 10 carbon atoms,
   (4) an optionally substituted aryl group having 6 to 14 carbon atoms,
   (5) an optionally substituted heteroaryl group containing 1 to 6 same or different atoms selected from a nitrogen atom, an oxygen atom, and a sulfur atom and having 5 to 10 ring-constituting atoms, or
   (6) an optionally substituted heterocyclic group containing 1 to 6 same or different atoms selected from a nitrogen atom, an oxygen atom, and a sulfur atom and having 3 to 10 ring-constituting atoms.

More preferably, R^{6a1} is
(1) hydrogen,
(2) an alkyl group having 1 to 6 carbon atoms, or
(3)

   -Y^{10a}-W^{10a}-Y^{10b}-W^{10b}

   wherein Y^{10a} is
      1) a bond,
      2)

         - (CR^{Y11}R^{Y11'})ₙ₁₀-,
      3)

         - (CR^{Y11}R^{Y11'})ₙ₁₀-V^{1D}-(CR^{Y12}R^{Y12'})ₘ₁₀-,
      4)

         - (CR^{Y11}R^{Y11'})ₙ₁₀-V¹⁰-NR^{N10'}- (CR^{Y12}R^{Y12'})ₘ₁₀-,
      5)

         - (CR^{Y11}R^{Y11'})ₒ₁₀-NR^{N10'}-V¹⁰-(CR^{Y12}R^{Y12'})ₘ₁₀-,
      6)

         - (CR^{Y11}R^{Y11'})ₒ₁₀-NR^{N10"}-(CR^{Y12}R^{Y12'})ₘ₁₀-,
      7)

         - (CR^{Y11}R^{Y11'})ₒ₁₀-O-(CR^{Y12}R^{Y12'})ₘ₁₀-

         or
      8)

         - (CR^{Y11}R^{Y11'})ₒ₁₀-S-(CR^{Y12}R^{Y12'})ₘ₁₀-
   wherein R^{Y11}, R^{Y11'}, R^{Y12}, and R^{Y12'} are each independently
      (a) a hydrogen atom,
      (b) a halogen atom,
      (c) -O-(optionally substituted alkyl having 1 to 6 carbon atoms),
      (d) -O-(optionally substituted cycloalkyl having 3 to 6 carbon atoms),
      (e) an optionally substituted alkyl group having 1 to 6 carbon atoms, or
      (f) an optionally substituted cycloalkyl group having 3 to 6 carbon atoms, or
      (g) R^{Y11} and R^{Y11'}, and R^{Y12} and R^{Y12'}, are each independently optionally bonded to form an optionally substituted cycloalkyl having 3 to 10 carbon atoms, or an optionally substituted heterocycle containing 1 to 6 atoms selected from a nitrogen atom, an oxygen atom, and a sulfur atom and having 3 to 10 ring-constituting atoms,
   n10 is an integer of 1 to 6,
   V¹⁰ is
      (a) -CO-,
      (b) -SO- or
      (c) -SO2-,
   m10 is an integer of 0 to 6,
   R^{N10'} is
      (a) a hydrogen atom,
      (b) an optionally substituted alkyl group having 1 to 6 carbon atoms,
      (c) an optionally substituted cycloalkyl group having 3 to 6 carbon atoms, or
      (d) an optionally substituted heterocyclic group having 3 to 6 ring-constituting atoms,
   o10 is an integer of 2 to 6,
   R^{N10"} is
      (a) a hydrogen atom,
      (b) an optionally substituted alkyl group having 1 to 6 carbon atoms,
      (c) an optionally substituted cycloalkyl group having 3 to 6 carbon atoms,
      (d) an optionally substituted heterocyclic group having 3 to 6 carbon atoms,
      (e) -CO-(optionally substituted alkyl having 1 to 6 carbon atoms), or
      (f) -CO-(optionally substituted cycloalkyl having 3 to 6 carbon atoms),
   W^{10a} is
      1) an optionally substituted alkylene group having 1 to 6 carbon atoms,
      2) an optionally substituted aryl divalent group having 6 to 10 carbon atoms,
      3) an optionally substituted heteroaryl divalent group containing 1 to 6 atoms selected from a nitrogen atom, an oxygen atom, and a sulfur atom and having 3 to 10 ring-constituting atoms,
      4) an optionally substituted cycloalkyl divalent group having 3 to 10 carbon atoms, or
      5) an optionally substituted heterocyclic divalent group containing 1 to 6 atoms selected from a nitrogen atom, an oxygen atom, and a sulfur atom and having 3 to 10 ring-constituting atoms,
   Y^{10b} is
      1) - (CR^{Y13}R^{Y13'})_{ny10c}-A^{Y10b}-(CR^{Y14}R^{Y14'})_{ny10d}-
         wherein R^{Y13}, R^{Y13'}, R^{Y14}, and R^{Y14'} are each independently
         (a) a hydrogen atom,
         (b) a halogen atom,
         (c) -O-(optionally substituted alkyl having 1 to 6 carbon atoms),
         (d) -O-(optionally substituted cycloalkyl having 3 to 6 carbon atoms),
         (e) an optionally substituted alkyl group having 1 to 6 carbon atoms, or
         (f) an optionally substituted cycloalkyl group having 3 to 6 carbon atoms, or
         (g) R^{Y13} and R^{Y13'}, and R^{Y14} and R^{Y14'}, are each independently
      optionally bonded to each other to form, together with the adjacent carbon atom, an optionally substituted monocyclic or condensed cycloalkane having 3 to 10 carbon atoms, or an optionally substituted heterocycle containing 1 to 6 same or different atoms selected from a nitrogen atom, an oxygen atom, and a sulfur atom and having 3 to 10 ring-constituting atoms, ny10c and ny10d are each independently an integer of 0 to 6,
   A^{Y10b} is
      (a) a bond,
      (b) -O-,
      (c) -SO-,
      (d) -SO2-,
      (e) -NR^{NY10e}-
         wherein R^{NY10e} is
         (i) a hydrogen atom,
         (ii) an optionally substituted alkyl group having 1 to 6 carbon atoms,
         (iii) an optionally substituted cycloalkyl group having 3 to 6 carbon atoms,
         (iv) an optionally substituted heterocyclic group having 3 to 6 carbon atoms,
         (v) -CO-(optionally substituted alkyl having 1 to 6 carbon atoms), or
         (vi) -CO-(optionally substituted cycloalkyl having 3 to 6 carbon atoms), or
      (f) -VY^{10f}-NR^{NY10f}- or NR^{NY10f}-V^{Y10f}-
   wherein V^{Y10f} is

      -CO-,

      -SO- or

      -SO₂-,
   R^{NY10f} is
      a hydrogen atom,
      an optionally substituted alkyl group having 1 to 6 carbon atoms,
      an optionally substituted cycloalkyl group having 3 to 6 carbon atoms or
      an optionally substituted heterocyclic group having 3 to 6 carbon atoms,
   W^{10b} is
      1) a hydrogen atom,
      2) a halogen atom,
      3) an optionally substituted aryl group having 6 to 10 carbon atoms,
      4) an optionally substituted heteroaryl group containing 1 to 6 same or different atoms selected from a nitrogen atom, an oxygen atom, and a sulfur atom and having 5 to 10 ring-constituting atoms,
      5) an optionally substituted monocyclic or condensed cycloalkyl having 3 to 10 carbon atoms,
      6) an optionally substituted heterocyclic group containing 1 to 6 atoms selected from a nitrogen atom, an oxygen atom, and a sulfur atom and having 3 to 10 ring-constituting atoms,
      7) -CONR^{NW10}R^{NW10'}
         wherein R^{NW10} and R^{NW10'} may be the same or different, are each independently
         (a) a hydrogen atom,
         (b) an optionally substituted alkyl group having 1 to 6 carbon atoms, or
         (c) an optionally substituted cycloalkyl group having 3 to 6 carbon atoms,
      8) -NR^{NW10"}R^{NW10‴}
         wherein R^{NW10"} and R^{NW10‴} may be the same or different, are each independently
         (a) a hydrogen atom,
         (b) unsubstituted or substituted alkyl having 1 to 6 carbon atoms,
         (c) an optionally substituted cycloalkyl having 3 to 6 carbon atoms,
         (d) an optionally substituted heterocyclic group having 3 to 6 ring-constituting atoms,
         (e) -CO-(optionally substituted alkyl having 1 to 6 carbon atoms), or
         (f) -CO-(optionally substituted cycloalkyl having 3 to 6 carbon atoms),
(4) an optionally substituted cycloalkyl group having 3 to 10 carbon atoms,
(5) an optionally substituted aryl group having 6 to 14 carbon atoms,
(6) an optionally substituted heteroaryl group containing 1 to 6 same or different atoms selected from a nitrogen atom, an oxygen atom, and a sulfur atom and having 3 to 10 ring-constituting atoms, or
(7) an optionally substituted heterocyclic group containing 1 to 6 same or different atoms selected from a nitrogen atom, an oxygen atom, and a sulfur atom and having 3 to 10 ring-constituting atoms.

Further preferably, R^{6a1} is
(1) hydrogen,
(2) an alkyl group having 1 to 6 carbon atoms, or
(3) -Y^{10a}-W^{10a}-Y^{10b}-W^{10b}
   wherein Y^{10a} is
      1) - (CR^{Y11}R^{Y11'})ₙ₁₀-,
      2) - (CR^{Y11}R^{Y11'})ₙ₁₀-V¹⁰-NR^{N10'}- (CR^{Y12}R^{Y12'})ₘ₁₀-,
   wherein R^{Y11}, R^{Y11'}, R^{Y12}, and R^{Y12'} are each independently
      (a) a hydrogen atom,
   n10 is an integer of 1 to 6,
   V¹⁰ is
      (a) -CO-,
   m10 is an integer of 0 to 6,
   R^{N10'} is
      (a) a hydrogen atom,
   W^{10a} is
      1) an aryl divalent group having 6 to 10 carbon atoms,
      2) a heteroaryl divalent group containing 1 to 4 same or different atoms selected from a nitrogen atom and an oxygen atom, and having 5 or 6 ring-constituting atoms,
   Y^{10b} is
      1) - (CR^{Y13}R^{Y13'})_{ny10c}-A^{Y10b}-(CR^{Y14}R^{Y14'})_{ny10d}-
   wherein R^{Y13}, R^{Y13'}, R^{Y14}, and R^{Y14'} are each independently
      (a) a hydrogen atom,
      (b) a halogen atom,
   ny10c and ny10d are each independently an integer of 0 to 6,
   A^{Y10b} is
      (a) a bond,
      (b) -O-,
   W^{10b} is
      1) a hydrogen atom,
      2) an aryl group having 6 to 10 carbon atoms and optionally substituted by a group selected from an alkyl group having 1 to 6 carbon atoms and optionally substituted by halogen and halogen,
      3) a heteroaryl group containing 1 to 4 same or different atoms selected from a nitrogen atom and a sulfur atom, and having 5 or 6 ring-constituting atoms, which is optionally substituted by an alkyl group having 1 to 6 carbon atoms,
      4) an optionally substituted cycloalkyl group having 3 to 10 carbon atoms,
      5) an optionally substituted aryl group having 6 to 14 carbon atoms,
      6) an optionally substituted heteroaryl group containing 1 to 6 same or different atoms selected from a nitrogen atom, an oxygen atom, and a sulfur atom and having 3 to 10 ring-constituting atoms, or
      7) an optionally substituted heterocyclic group containing 1 to 6 same or different atoms selected from a nitrogen atom, an oxygen atom, and a sulfur atom and having 3 to 10 ring-constituting atoms.

The aforementioned "Definition of each group used in the present specification" can be referred to for the meaning of each group.

R^{6a2} is
(1) a hydrogen atom,
(2) CN,
(3) a halogen atom,
(4) an optionally substituted alkyl group,
(5) an optionally substituted cycloalkyl group,
(6) an optionally substituted aryl group,
(7) an optionally substituted heteroaryl group,
(8) an optionally substituted heterocyclic group,
(9) -V^{a3a}-(optionally substituted alkyl),
(10) -V^{a3a}-(optionally substituted cycloalkyl),
(11) -V^{a3a}-(optionally substituted aryl),
(12) -V^{a3a}-(optionally substituted heteroaryl), or
(13) -V^{a3a}-(optionally substituted heterocyclic group)
   (in the above-mentioned formulas, V^{a3a} is
   1) -CO-,
   2) -NR^{Va3}-
      wherein R^{Va3} is
      (a) a hydrogen atom,
      (b) an optionally substituted alkyl group having 1 to 6 carbon atoms, or
      (c) an optionally substituted cycloalkyl group having 3 to 6 carbon atoms,
   3) -O-,
   4) -S-,
   5) -SO-, or
   6) -SO₂-, or
(14) -V^{a3b}-NR^{Na3}R^{Na3'}
   wherein V^{a3b} is
      1) -CO-
      2) -SO-, or
      3) -SO₂-,
   R^{Na3} and R^{Na3'} are each independently
      1) a hydrogen atom,
      2) an optionally substituted alkyl group,
      3) an optionally substituted cycloalkyl group,
      4) an optionally substituted aryl group,
      5) an optionally substituted heteroaryl group, or
      6) an optionally substituted heterocyclic group,
   preferably,
      (1) a hydrogen atom,
      (2) CN,
      (3) a halogen atom,
      (4) an optionally substituted alkyl group having 1 to 4 carbon atoms,
      (5) an optionally substituted cyclopropyl group,
      (6) an optionally substituted oxetanyl group,
      (7) an optionally substituted azetidinyl group,
      (8) an optionally substituted aryl group having 6 to 10 carbon atoms, or
      (9) -CO-NR^{a2N6}R^{a2N6'}
   wherein R^{a2N6} and R^{a2N6'} are each independently
      1) a hydrogen atom,
      2) an optionally substituted alkyl group having 1 to 4 carbon atoms,
      3) an optionally substituted cyclopropyl group,
      4) an optionally substituted oxetanyl group, or
      5) an optionally substituted azetidinyl group,
   more preferably,
      (1) a hydrogen atom,
      (2) CN,
      (3) a halogen atom,
      (4) an alkyl group having 1 to 4 carbon atoms,
      (5) an optionally substituted cyclopropyl group,
      (6) an optionally substituted oxetanyl group,
      (7) an optionally substituted azetidinyl group,
      (8) an aryl group having 6 to 10 carbon atoms and optionally substituted by an alkoxy group having 1 to 6 carbon atoms, or
      (9) -CO-NR^{a2N6}R^{a2N6'}
   wherein R^{a2N6} and R^{a2N6'} are each independently
      1) a hydrogen atom,
      2) an optionally substituted alkyl group having 1 to 4 carbon atoms,
      3) an optionally substituted cyclopropyl group,
      4) an optionally substituted oxetanyl group, or
      5) an optionally substituted azetidinyl group.

The aforementioned "Definition of each group used in the present specification" can be referred to for the meaning of each group.

R^{6a3} is a group represented by:
(1) a hydrogen atom,
(2) CN,
(3) a halogen atom,
(4) an optionally substituted alkyl group,
(5) an optionally substituted cycloalkyl group,
(6) an optionally substituted aryl group,
(7) an optionally substituted heteroaryl group,
(8) an optionally substituted heterocyclic group,
(9) -V^{a3a}-(optionally substituted alkyl),
(10) -V^{a3a}-(optionally substituted cycloalkyl),
(11) -V^{a3a}-(optionally substituted aryl),
(12) -V^{a3a}-(optionally substituted heteroaryl), or
(13) -V^{a3a}-(optionally substituted heterocyclic group)
   (in the above-mentioned formulas, V^{a3a} is
   1) -CO-,
   2) -NR^{Va3}-
      wherein R^{Va3} is
      (a) a hydrogen atom,
      (b) an optionally substituted alkyl group having 1 to 6 carbon atoms, or
      (c) an optionally substituted cycloalkyl group having 3 to 6 carbon atoms,
   3) -O-,
   4) -S-,
   5) -SO-, or
   6) -SO₂-, or
(14) -V^{a3b}-NR^{Na3}R^{Na3'}
   wherein V^{a3b} is
      1) -CO-
      2) -SO-, or
      3) -SO₂-,
   R^{Na3} and R^{Na3'} are each independently
      1) a hydrogen atom,
      2) an optionally substituted alkyl group,
      3) an optionally substituted cycloalkyl group,
      4) an optionally substituted aryl group,
      5) an optionally substituted heteroaryl group, or
      6) an optionally substituted heterocyclic group,
   preferably,
      (1) a hydrogen atom,
      (2) CN,
      (3) a halogen atom,
      (4) an optionally substituted alkyl group,
      (5) an optionally substituted cycloalkyl group,
      (6) an optionally substituted aryl group,
      (7) an optionally substituted heteroaryl group,
      (8) an optionally substituted heterocyclic group,
      (9) -CO-(optionally substituted heterocyclic group), or
      (10) -CO-NR^{a3N6}R^{a3N6'}
   wherein R^{a3N6} and R^{a3N6'} are each independently
      1) a hydrogen atom,
      2) an optionally substituted alkyl group,
      3) an optionally substituted cycloalkyl group,
      4) an optionally substituted aryl group,
      5) an optionally substituted heteroaryl group, or
      6) an optionally substituted heterocyclic group,
   more preferably,
      (1) a hydrogen atom,
      (2) CN,
      (3) a halogen atom,
      (4) an optionally substituted alkyl group having 1 to 6 carbon atoms,
      (5) an optionally substituted cycloalkyl group having 3 to 10 carbon atoms,
      (6) an optionally substituted aryl group having 6 to 14 carbon atoms,
      (7) an optionally substituted heteroaryl group containing 1 to 6 same or different atoms selected from a nitrogen atom, an oxygen atom, and a sulfur atom and having 3 to 10 ring-constituting atoms,
      (8) an optionally substituted heterocyclic group containing 1 to 6 same or different atoms selected from a nitrogen atom, an oxygen atom, and a sulfur atom and having 3 to 10 ring-constituting atoms,
      (9) -CO-(optionally substituted heterocyclic group containing 1 to 6 same or different atoms selected from a nitrogen atom, an oxygen atom, and a sulfur atom and having 3 to 10 ring-constituting atoms), or
      (10) -CO-NR^{a3N6}R^{a3N6'}
   wherein R^{a3N6} and R^{a3N6'} are each independently
      1) a hydrogen atom,
      2) an optionally substituted alkyl group having 1 to 6 carbon atoms,
      3) an optionally substituted cycloalkyl group having 3 to 10 carbon atoms,
      4) an optionally substituted aryl group having 6 to 14 carbon atoms,
      5) an optionally substituted heteroaryl group containing 1 to 6 same or different atoms selected from a nitrogen atom, an oxygen atom, and a sulfur atom and having 3 to 10 ring-constituting atoms, or
      6) an optionally substituted heterocyclic group containing 1 to 6 same or different atoms selected from a nitrogen atom, an oxygen atom, and a sulfur atom and having 3 to 10 ring-constituting atoms.

Further preferably, R^{6a3} is
(First group)
   a hydrogen atom or an alkyl group having 1 to 6 carbon atoms
(Second group)
   a group represented by -Y^{6a}-W^{6a}
   wherein
   Y^{6a} is
      1) a bond,
      2) - (CR^{Y1}R^{Y1'})_{ny6a}-A^{Y6a}-(CR^{Y2}R^{Y2'})_{ny6b}-wherein
   R^{Y1}, R^{Y1'}, R^{Y2} and R^{Y2'} are each independently
      (a) a hydrogen atom,
      (b) a halogen atom,
      (c) -O-(optionally substituted alkyl having 1 to 6 carbon atoms),
      (d) -O-(optionally substituted cycloalkyl having 3 to 6 carbon atoms),
      (e) an optionally substituted alkyl group having 1 to 6 carbon atoms, or
      (f) an optionally substituted cycloalkyl group having 3 to 6 carbon atoms, or
      (g) R^{Y1} and R^{Y1'}, and R^{Y2} and R^{Y2'}, are optionally each independently bonded to each other to form, together with the adjacent carbon atom, an optionally substituted cycloalkyl group having 3 to 10 carbon atoms, or an optionally substituted heterocycle containing 1 to 6 same or different atoms selected from a nitrogen atom, an oxygen atom, and a sulfur atom and having 3 to 10 ring-constituting atoms,
   ny6a is an integer of 1 to 6,
   A^{Y6a} is
      (a) a bond,
      (b) -CO-,
      (c) -O-,
      (d) -SO-,
      (e) -SO₂-,
      (f) -NR^{NY6a}-,
         wherein R^{NY6a} is
         (i) a hydrogen atom,
         (ii) an optionally substituted alkyl group having 1 to 6 carbon atoms,
         (iii) an optionally substituted cycloalkyl group having 3 to 6 carbon atoms,
         (iv) an optionally substituted heterocyclic group having 3 to 6 ring-constituting atoms,
         (v) -CO-(optionally substituted alkyl having 1 to 6 carbon atoms), or
         (vi) -CO-(optionally substituted cycloalkyl having 3 to 6 carbon atoms), or
      (g) -V^{Y6b}-NR^{NY6b}- or -NR^{NY6b}-V^{Y6b}-
   wherein V^{Y6b} is
      (i) -CO-,
      (ii) -SO-, or
      (iii) -SO₂-,
   R^{NY6b} is
      (i) a hydrogen atom,
      (ii) an optionally substituted alkyl group having 1 to 6 carbon atoms,
      (iii) an optionally substituted cycloalkyl group having 3 to 6 carbon atoms, or
      (iv) an optionally substituted heterocyclic group having 3 to 6 ring-constituting atoms,
   ny6b is an integer of 0 to 6, and
   W^{6a} is
      1) a hydrogen atom,
      2) a halogen atom,
      3) an optionally substituted cycloalkyl group having 3 to 10 carbon atoms,
      4) an optionally substituted aryl group having 6 to 10 carbon atoms,
      5) optionally substituted heteroaryl group containing 1 to 6 same or different atoms selected from a nitrogen atom, an oxygen atom, and a sulfur atom and having 5 to 10 ring-constituting atoms, or
      6) an optionally substituted heterocyclic group containing 1 to 6 same or different atoms selected from a nitrogen atom, an oxygen atom, and a sulfur atom and having 3 to 10 ring-constituting atoms,
(Third group)
   a group represented by the formula:

   -CD-R^{9a}-W^{9a}-Y^{9b}-W^{9b}

   wherein
   R^{9a} is
      (1) a group represented by the formula: 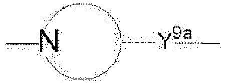 wherein 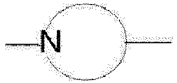 is
         a heterocyclic group containing a nitrogen atom, optionally further containing 1 to 5 same or different atoms selected from a nitrogen atom, an oxygen atom, and a sulfur atom, and having 3 to 10 ring-constituting atoms, which is optionally further substituted in addition to Y^{9a}
         (e.g., azetidinyl group, pyrrolidinyl group, piperidyl group, piperazinyl group, morpholinyl group, tetrahydronaphthyridinyl group, tetrahydrothiazolopyridyl group or tetrahydroisoquinolyl group, each of which is optionally further substituted by an oxo group or a halogen atom in addition to Y^{9a}) , and
         Y^{9a} is
            1) - (CR^{Y5}R^{Y5'})_{ny9a}-A^{Y9a}-(CR^{Y6}R^{Y6'})_{ny9b}-
         wherein R^{Y5}, R^{Y5'}, R^{Y6}, and R^{Y6'} are each independently
            (a) a hydrogen atom,
            (b) a halogen atom,
            (c) -O-(optionally substituted alkyl having 1 to 6 carbon atoms),
            (d) -O-(optionally substituted cycloalkyl having 3 to 6 carbon atoms),
            (e) an optionally substituted alkyl group having 1 to 6 carbon atoms, or
            (f) an optionally substituted cycloalkyl group having 3 to 6 carbon atoms, or
            (g) R^{Y5} and R^{Y5'}, and R^{Y6} and R^{Y6'}, are each independently optionally bonded to each other to form, together with the adjacent carbon atom, an optionally substituted cycloalkyl group having 3 to 10 carbon atoms, or an optionally substituted heterocycle containing 1 to 6 same or different atoms selected from a nitrogen atom, an oxygen atom, and a sulfur atom and having 3 to 10 ring-constituting atoms;
         ny9a and ny9b are each independently an integer of 0 to 6;
         AY9a is
            (a) a bond,
            (b) -O-,
            (c) -SO-,
            (d) -SO₂-,
            (e) -NR^{NY9c}-
               wherein R^{NY9c} is
               (i) a hydrogen atom,
               (ii) an optionally substituted alkyl group having 1 to 6 carbon atoms,
               (iii) an optionally substituted cycloalkyl group having 3 to 6 carbon atoms,
               (iv) an optionally substituted heterocyclic group having 3 to 6 ring-constituting atoms,
               (v) -CO-(optionally substituted alkyl having 1 to 6 carbon atoms), or
               (vi) -CO-(optionally substituted cycloalkyl having 3 to 6 carbon atoms),
            (f) -V^{Y9d}-NR^{NY9d}- or -NR^{NY9d}-V^{Y9d}-
         wherein V^{Y9d} is
            (i) -CO-,
            (ii) -SO-, or
            (iii) -SO₂-, and
         R^{NY9d} is
            (i) a hydrogen atom,
            (ii) an optionally substituted alkyl group having 1 to 6 carbon atoms,
            (iii) an optionally substituted cycloalkyl group having 3 to 6 carbon atoms, or
            (iv) an optionally substituted heterocyclic group having 3 to 6 ring-constituting atoms, or
      (2) a group represented by the formula: -NR^{N9}-, -NR^{N9}-(CR^{Y7}R^{Y7'})ₙ₉-,

         -NR^{N9}-(CR^{Y7}R^{Y7'})ₙ₉-V⁹-(CR^{Y8}R^{Y8'})ₘ₉-,

         -NR^{N9}-(CR^{Y7}R^{Y7'})ₙ₉-V⁹-NR^{N9'}-(CR^{Y8}R^{Y8'})ₘ₉-,

         -NR^{N9}-(CR^{Y7}R^{Y7'})ₒ₉-NR^{N9'}-V⁹-(CR^{Y8}R^{Y8'})ₘ₉-,

         -NR^{N9}-(CR^{Y7}R^{Y7'})ₒ₉-NR^{N9"}-(CR^{Y8}R^{Y8'})ₘ₉-, -NR^{N9}-(CR^{Y7}R^{Y7'})ₒ₉-O-(CR^{Y8}R^{Y8'})ₘ₉-,

         or

         -NR^{N9}-(CR^{Y7}R^{Y7'})₀₉-S-(CR^{Y8}R^{Y8'})ₘ₉-

         in the above-mentioned formulas,
         R^{N9} is
            1) a hydrogen atom,
            2) -(CR^{N9a}R^{N9a'})_{nN9a}-Y^{N9}-W^{N9}
         wherein R^{N9a} and R^{N9a'} are each independently
            (a) a hydrogen atom,
            (b) a halogen atom,
            (c) -O-(optionally substituted alkyl having 1 to 6 carbon atoms),
            (d) -O-(optionally substituted cycloalkyl having 3 to 6 carbon atoms),
            (e) an optionally substituted alkyl group having 1 to 6 carbon atoms, or
            (f) an optionally substituted cycloalkyl group having 3 to 6 carbon atoms, or
            (g) R^{N9a} and R^{N9a'} are optionally bonded to each other to form, together with the adjacent carbon atom, an optionally substituted cycloalkyl group having 3 to 10 carbon atoms, or
            (h) an optionally substituted heterocyclic group containing 1 to 6 same or different atoms selected from a nitrogen atom, an oxygen atom, and a sulfur atom and having 3 to 10 ring-constituting atoms;
         nN9a is an integer of 1 to 6;
         Y^{N9} is
            (a) a bond-,
            (b) -O-,
            (c) -SO-,
            (d) -SO₂-,
            (e) -NR^{N9b}-
               wherein R^{N9b} is
               (i) a hydrogen atom,
               (ii) an optionally substituted alkyl group having 1 to 6 carbon atoms,
               (iii) an optionally substituted cycloalkyl group having 3 to 6 carbon atoms,
               (iv) an optionally substituted heterocyclic group having 3 to 6 ring-constituting atoms,
               (v) -CO-(optionally substituted alkyl having 1 to 6 carbon atoms), or
               (vi) -CO-(optionally substituted cycloalkyl having 3 to 6 carbon atoms, or
            (f) -V^{N9c}-NR^{N9c}- or -NR^{N9c}-V^{N9c}-
         wherein V^{N9c} is
            (i) -CO-,
            (ii) -SO-, or
            (iii) -SO₂-,
         RN9c is
            (i) a hydrogen atom,
            (ii) an optionally substituted alkyl group having 1 to 6 carbon atoms, or
            (iii) an optionally substituted cycloalkyl group having 3 to 6 carbon atoms,
         W^{N9} is
            (a) a hydrogen atom,
            (b) an optionally substituted alkyl group having 1 to 6 carbon atoms,
            (c) an optionally substituted cycloalkyl group having 3 to 10 carbon atoms,
            (d) an optionally substituted aryl group having 6 to 10 carbon atoms,
            (e) an optionally substituted heterocyclic group containing 1 to 6 same or different atoms selected from a nitrogen atom, an oxygen atom, and a sulfur atom and having 3 to 10 ring-constituting atoms, or
            (f) a halogen atom;
         R^{Y7}, R^{Y7'} , R^{Y8}, and R^{Y8'} are each independently
            1) a hydrogen atom,
            2) a halogen atom,
            3) -O-(optionally substituted alkyl having 1 to 6 carbon atoms),
            4) -O-(optionally substituted cycloalkyl having 3 to 6 carbon atoms),
            5) an optionally substituted alkyl group having 1 to 6 carbon atoms, or
            6) an optionally substituted cycloalkyl group having 3 to 6 carbon atoms, or
            7) R^{Y7} and R^{Y7'}, and R^{Y8} and R^{Y8'}, are each independently optionally bonded to each other to form, together with the adjacent carbon atom, an optionally substituted cycloalkyl group having 3 to 10 carbon atoms, or an optionally substituted heterocycle containing 1 to 6 same or different atoms selected from a nitrogen atom, an oxygen atom, and a sulfur atom and having 3 to 10 ring-constituting atoms;
         n9 is an integer of 1 to 6;
         V⁹ is
            1) -CO-,
            2) -SO-,
            3) -SO₂-, or
            4) an optionally substituted heterocyclic divalent group having 3 to 6 ring-constituting atoms;
         m9 is an integer of 0 to 6;
         R^{N9'} is
            1) a hydrogen atom,
            2) an optionally substituted alkyl group having 1 to 6 carbon atoms,
            3) an optionally substituted cycloalkyl group having 3 to 6 carbon atoms, or
            4) an optionally substituted heterocyclic group having 3 to 6 ring-constituting atoms;
         o9 is an integer of 2 to 6;
         R^{N9"} is
            1) a hydrogen atom,
            2) an optionally substituted alkyl group having 1 to 6 carbon atoms,
            3) an optionally substituted cycloalkyl group having 3 to 6 carbon atoms,
            4) an optionally substituted heterocyclic group having 3 to 6 carbon atoms,
            5) -CO-(optionally substituted alkyl having 1 to 6 carbon atoms), or
            6) -CO-(optionally substituted cycloalkyl having 3 to 6 carbon atoms);
   W^{9a} is
      (1) an optionally substituted alkylene group having 1 to 6 carbon atoms,
      (2) an optionally substituted aryl divalent group having 6 to 10 carbon atoms,
      (3) an optionally substituted heteroaryl divalent group containing 1 to 6 same or different atoms selected from a nitrogen atom, an oxygen atom, and a sulfur atom and having 5 to 10 ring-constituting atoms,
      (4) an optionally substituted cycloalkyl divalent group having 3 to 10 carbon atoms, or
      (5) an optionally substituted heterocyclic divalent group containing 1 to 6 same or different atoms selected from a nitrogen atom, an oxygen atom, and a sulfur atom and having 3 to 10 ring-constituting atoms,
   Y^{9b} is
      (1) -(CR^{Y9}R^{Y9'})ny9c-A^{Y9b}-(CR^{Y10}R^{Y10'})ny9d-
   wherein R^{Y9}, R^{Y9'}, R^{Y10}, and R^{Y10'} are each independently
      1) a hydrogen atom,
      2) a halogen atom,
      3) -O-(optionally substituted alkyl having 1 to 6 carbon atoms),
      4) -O-(optionally substituted cycloalkyl having 3 to 6 carbon atoms),
      5) an optionally substituted alkyl having 1 to 6 carbon atoms or
      6) an optionally substituted cycloalkyl group having 3 to 6 carbon atoms, or
      7) R^{Y9} and R^{Y9}', and R^{Y10} and R^{Y10'}, are each independently optionally bonded to each other to form, together with the adjacent carbon atom, an optionally substituted cycloalkyl group having 3 to 10 carbon atoms, or an optionally substituted heterocyclic group containing 1 to 6 same or different atoms selected from a nitrogen atom, an oxygen atom, and a sulfur atom and having 3 to 10 ring-constituting atoms,
   ny9c and ny9d are each independently an integer of 0 to 6,
   A^{Y9b} is
      1) a bond,
      2) -O-,
      3) -SO-,
      4) -SO₂-,
      5) -NR^{NY9e}-,
         wherein R^{NY9e} is
         (a) a hydrogen atom,
         (b) an optionally substituted alkyl group having 1 to 6 carbon atoms,
         (c) an optionally substituted cycloalkyl group having 3 to 6 carbon atoms,
         (d) an optionally substituted heterocyclic group having 3 to 6 ring-constituting atoms,
         (e) -CO-(optionally substituted alkyl having 1 to 6 carbon atoms),
         (f) -CO-(optionally substituted cycloalkyl having 3 to 6 carbon atoms), or
      6) -V^{Y9f}-NR^{NY9f}- or -NR^{NY9f}-V^{Y9f}-
         wherein V^{Y9f} is
            (i) -CO-,
            (ii) -SO-, or
            (iii) -SO₂-,
         R^{NY9f} is
            (i) a hydrogen atom,
            (ii) an optionally substituted alkyl group having 1 to 6 carbon atoms,
            (iii) an optionally substituted cycloalkyl group having 3 to 6 carbon atoms, or
            (iv) an optionally substituted heterocyclic group having 3 to 6 ring-constituting atoms,
   W^{9b} is
      (1) a hydrogen atom,
      (2) a halogen atom,
      (3) an optionally substituted aryl group having 6 to 10 carbon atoms,
      (4) optionally substituted heteroaryl group containing 1 to 6 same or different atoms selected from a nitrogen atom, an oxygen atom, and a sulfur atom and having 5 to 10 ring-constituting atoms,
      (5) an optionally substituted cycloalkyl group having 3 to 10 carbon atoms,
      (6) an optionally substituted heterocyclic group containing 1 to 6 same or different atoms selected from a nitrogen atom, an oxygen atom, and a sulfur atom and having 3 to 10 ring-constituting atoms,
      (7) -CONR^{NW9}R^{NW9'}
   wherein R^{NW9} and R^{NW9'} are each independently
      1) a hydrogen atom,
      2) an optionally substituted alkyl group having 1 to 6 carbon atoms, or
      3) an optionally substituted cycloalkyl group having 3 to 6 carbon atoms, or
      4) -NR^{NW9"}R^{NW9‴}
   wherein R^{NM9"} and R^{NW9‴} are each independently
      (a) a hydrogen atom,
      (b) an optionally substituted alkyl group having 1 to 6 carbon atoms,
      (c) an optionally substituted cycloalkyl group having 3 to 6 carbon atoms,
      (d) an optionally substituted heterocyclic group having 3 to 6 ring-constituting atoms,
      (e) -CO-(optionally substituted alkyl having 1 to 6 carbon atoms), or
      (f) -CO-(optionally substituted cycloalkyl having 3 to 6 carbon atoms).

Further more preferably, R^{6a3} is
(First group)
   a hydrogen atom or an alkyl group having 1 to 6 carbon atoms
(Second group)

   -Y^{6a}-W^{6a}

   wherein
   Y^{6a} is
      1) a bond,
      2) -(CR^{Y1}R^{Y1'})_{ny6a}-A^{Y6a}-(CR^{Y2}R^{Y2'})_{ny6b}- wherein
   R^{Y1}, R^{Y1'}, R^{Y2}, and R^{Y2'} are each independently
   a hydrogen atom,
   ny6a is an integer of 1 to 6,
   A^{Y6a} is
      (a) a bond
      (b) -O-,
      (c) -NR^{NY6a}-,
         wherein R^{NY6a} is
         an alkyl group having 1 to 6 carbon atoms and optionally substituted by a substituent selected from a cycloalkyl group having 3 to 6 carbon atoms and an aryl group having 6 to 10 carbon atoms, or
      (d) -V^{Y6b}-NR^{NY6b}- or -NR^{NY6b}-V^{Y6b}-
   wherein V^{Y6b} is

      -CO-,
   R^{NY6b} is
   an alkyl group having 1 to 6 carbon atoms and optionally substituted by a group selected from a cycloalkyl group having 3 to 6 carbon atoms and an aryl group having 6 to 10 carbon atoms,
   ny6b is an integer of 0 to 6, and
   W^{6a} is
      1) a hydrogen atom,
      2) a cycloalkyl group having 3 to 10 carbon atoms,
      3) an aryl group having 6 to 10 carbon atoms,
      4) -W^{6a'}-(CR^{Y3}R^{Y3'})_{ny6c}-A^{Y6b}-(CR^{Y4}R^{Y4'})_{ny6d}-W^{6b}
         wherein
   W^{6a'} is
      (1) a cycloalkyl divalent group having 3 to 10 carbon atoms,
      (2) an aryl divalent group having 6 to 10 carbon atoms,
   R^{Y3}, R^{Y3'}, R^{Y4}, and R^{Y4'} are each independently
      (1) a hydrogen atom,
      (2) a halogen atom,
   A^{Y6b} is
      (1) a bond,
      (2) -O-, or
      (3) -V^{Y6d}-NR^{NY6d}-
   wherein V^{Y6d} is
      -CO-, and
   R^{NY6d} is
   an alkyl group having 1 to 6 carbon atoms,
   ny6c and ny6d are each independently an integer of 0 to 6,
   W^{6b} is
      (1) a hydrogen atom,
      (2) a halogen atom,
      (4) a heteroaryl group containing 1 to 4 same or different atoms selected from a nitrogen atom and a sulfur atom, and having 5 or 6 ring-constituting atoms, which is optionally substituted by an alkyl group having 1 to 6 carbon atoms,
(Third group)
   a group represented by the formula:

   -CD-R^{9a}-W^{9a}-Y^{9b}-W^{9b}

   wherein
   R^{9a} is
      (1) a group represented by the formula: 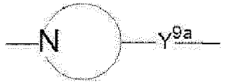 wherein 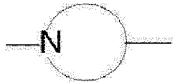 is
         a heterocyclic group containing a nitrogen atom, optionally further containing 1 to 5 same or different atoms selected from a nitrogen atom, an oxygen atom, and a sulfur atom, and having 3 to 10 ring-constituting atoms, which is optionally further substituted in addition to Y^{9a}
         (e.g., azetidinyl group, pyrrolidinyl group, piperidyl group, piperazinyl group, morpholinyl group, tetrahydronaphthyridinyl group, tetrahydrothiazolopyridyl group or tetrahydroisoquinolyl group, each of which is optionally further substituted by an oxo group or a halogen atom in addition to Y^{9a}),
         Y^{9a} is a group represented by
            1) -(CR^{Y5}R^{Y5'})_{ny9a}-A^{Y9a}-(CR^{Y6}R^{Y6'})_{ny9b}-
         wherein R^{Y5}, R^{Y5'}, R^{Y6}, and R^{Y6'} are each independently
            (a) a hydrogen atom,
         ny9a and ny9b are each independently an integer of 0 to 6;
         AY9a is
            (a) a bond,
            (b) -O-,
            (e) -NR^{NY9c}-
               wherein R^{NY9c} is
               (i) a hydrogen atom,
            (f) -V^{Y9d}-NR^{NY9d}- or -NR^{NY9d}-V^{Y9d}-
               wherein V^{Y9d} is
                  (i) -CO-, and
               R^{NY9d} is
                  (i) a hydrogen atom,
                  (ii) an alkyl group having 1 to 6 carbon atoms, or
      (2) a group represented by the formula: -NR^{N9}-, -NR^{N9}-(CR^{Y7}R^{Y7'})ₙ₉-,

         -NR^{N9}-(CR^{Y7}R^{Y7'})ₙ₉-V⁹-(CR^{Y8}R^{Y8'})ₘ₉-,

         -NR^{N9}-(CR^{Y7}R^{Y7'})ₙ₉-V⁹-NR^{N9'}-(CR^{Y8}R^{Y8'})ₘ₉-,

         -NR^{N9}-(CR^{Y7}R^{Y7'})ₒ₉-NR^{N9'}-V⁹-(CR^{Y8}R^{Y8'})ₘ₉-,

         -NR^{N9}-(CR^{Y7}R^{Y7'})ₒ₉-NR^{N9"}-(CR^{Y8}R^{Y8'})ₘ₉-, -NR^{N9}-(CR^{Y7}R^{Y7'})ₒ₉-O-(CR^{Y8}R^{Y8'})ₘ₉-,

         or
         in the above-mentioned formulas,
         R^{N9} is
            1) a hydrogen atom,
            2) -(CR^{N9a}R^{N9a'})_{nN9a-}Y^{N9}-W^{N9}
         wherein R^{N9a} and R^{N9a'} are each independently
            (a) a hydrogen atom,
            (b) a halogen atom,
            (c) an optionally substituted alkyl group having 1 to 6 carbon atoms, or,
            (d) R^{N9a} and R^{N9a'} are optionally bonded to each other to form, together with the adjacent carbon atom, cycloalkyl group having 3 to 6 carbon atoms, or heterocyclic group containing 1 to 2 oxygen atoms, and having 5 or 6 ring-constituting atoms;
         nN9a is an integer of 1 to 6;
         Y^{N9} is
            (a) a bond,
            (b) -O-,
            (c) -NR^{N9c}-V^{N9c}-
         wherein V^{N9c} is
            (i) -CO-,
         RN9c is
            (i) a hydrogen atom, or
            (ii) an optionally substituted alkyl group having 1 to 6 carbon atoms,
         W^{N9} is
            (a) a hydrogen atom,
            (b) an optionally substituted alkyl group having 1 to 6 carbon atoms,
            (c) a cycloalkyl group having 3 to 6 carbon atoms,
            (d) an aryl group having 6 to 10 carbon atoms, or
            (f) a halogen atom;
         R^{Y7}, R^{Y7'}, R^{Y8} and R^{Y8'} are each independently
            1) a hydrogen atom
            2) an optionally substituted alkyl group having 1 to 6 carbon atoms,
         n9 is an integer of 1 to 6;
         V⁹ is
            1) -CO-,
            2) an optionally substituted heterocyclic divalent group having 3 to 6 ring-constituting atoms;
         m9 is an integer of 0 to 6;
         R^{N9'} is
            1) a hydrogen atom,
            2) an alkyl group having 1 to 6 carbon atoms;
         o9 is an integer of 2 to 6;
         R^{N9"} is
            1) a hydrogen atom;
   W^{9a} is
      (1) an optionally substituted alkylene group having 1 to 6 carbon atoms,
      (2) an optionally substituted aryl divalent group having 6 to 10 carbon atoms,
      (3) an optionally substituted heteroaryl divalent group containing 1 to 4 same or different atoms selected from a nitrogen atom and a sulfur atom, and having 5 or 6 ring-constituting atoms, or
      (5) an optionally substituted heterocyclic divalent group containing 1 to 4 nitrogen atoms, and having 5 or 6 ring-constituting atoms,
   Y^{9b} is a group represented by
      (1) -(CR^{Y9}R^{Y9'})ny9c-A^{Y9b}-(CR^{Y10}R^{Y10'}) ny9d-
   wherein R^{Y9}, R^{Y9}', R^{Y10}, and R^{Y10'} are each independently
      1) a hydrogen atom, or
      2) a halogen atom,
   ny9c and ny9d are each independently an integer of 0 to 6,
   A^{Y9b} is
      1) a bond,
      2) -O-,
   W^{9b} is
      (1) a hydrogen atom,
      (2) a halogen atom,
      (3) an aryl group having 6 to 10 carbon atoms and optionally substituted by halogen,
      (4) a heteroaryl group containing 1 to 4 same or different atoms selected from a nitrogen atom and a sulfur atom, and having 5 or 6 ring-constituting atoms, which is optionally substituted by an alkyl group having 1 to 6 carbon atoms,
      (5) -CONR^{NW9}R^{NW9'}
   wherein R^{NW9} and R^{NW9'} are each independently
      1) a hydrogen atom,
      2) an alkyl group having 1 to 6 carbon atoms.

### group (W4) : an alkyl group optionally substituted by a group selected from a halogen atom, a hydroxy group, an alkoxy group, and a heterocyclic group

The group (W4) is preferably an alkyl group having 1 to 6 carbon atoms and optionally substituted by 1 to 3 groups selected from 1) a halogen atom, 2) a hydroxy group, 3) an alkoxy group having 1 to 6 carbon atoms, and 4) an optionally substituted heterocyclic group containing 1 to 6 atoms selected from a nitrogen atom, an oxygen atom, and a sulfur atom and having 3 to 10 ring-constituting atoms.

L is a bond or a chemical linker, preferably, a group represented by the formula (L-I): -(Lₐ)_{q}- wherein q is an integer of 1 to 100 (more preferably, an integer of 1 to 50), and
Lₐ in the number of q are each independently a group selected from
   (1) a bond, (2) CR^{L1}R^{L1'}, (3) O, (4) S, (5) CO, (6) SO, (7) SO₂, (8) NR^{L1}, (9) CONR^{L1}, (10) NR^{L1}CO, (11) NR^{L1}CONR^{L1'}, (12) SONR^{L1}, (13) NR^{L1}SO, (14) SO₂NR^{L1}, (15) NR^{L1}SO₂, (16) NR^{L1}SO₂NR^{L1}', (17) CR^{L1}=CR^{L1'}, (18) C≡C, (19) SiR^{L1}R^{L1'}, (20) P(O)R^{L1}, (21) P(O)OR^{L1}, (22) NR^{L1}C(=NCN)NR^{L1'}, (23) NR^{L1}C (=NCN), (24) an optionally substituted cycloalkyl divalent group, (25) an optionally substituted heterocyclic divalent group, (26) an optionally substituted aryl divalent group, or (27) an optionally substituted heteroaryl divalent group
(in the above-mentioned formulas, R^{L1} and R^{L1'} are each independently
   1) a hydrogen atom, 2) a halogen atom, 3) -CN, 4) -NO₂, 5) -SF₅, 6) -CO₂H, 7) -N(R^{L2}R^{L2}'), 8) -A^{L2}R^{L2}, 9) an optionally substituted alkyl group, 10) an optionally substituted cycloalkyl group, 11) an optionally substituted heterocyclic divalent group, 12) an optionally substituted aryl group, 13) an optionally substituted heteroaryl group, 14) -SO₂R^{L2}, 15) -P(O)(OR^{L2})OR^{L2'}, 16) -C≡ CR^{L2}, 17) -C(R^{L2})=C(R^{L2'}R^{L2"}), 18) -COR^{L2}, 19) -CON(R^{L2}R^{L2'}), 20) -SO₂N(R^{L2}R^{L2'}), 21) -N(R^{L2})CON(R^{L2'}R^{L2"}), or 22)-N(R^{L2})SO₂N(R^{L2'}R^{L2"})
(in the above-mentioned formulas, A^{L2} is oxygen atom or sulfur atom, R^{L2}, R^{L2'}, and R^{L2"} are each independently a) a hydrogen atom, b) an optionally substituted C₁-C₈ alkyl group, or c) an optionally substituted C₃-C₈ cycloalkyl group).

L is more preferably, a group represented by the formula (L-II) : -L_{b1}-L_{b2}-L_{b3}-
wherein L_{b1} and L_{b3} are each independently

   (1) a bond, (2) CR^{L1}R^{L1'}, (3) O, (4) S, (5) SO, (6) SO₂, (7) NR^{L1}, (8) SO₂NR^{L1}, (9) NR^{L1}SO₂, (10) SONR^{L1}, (11) NR^{L1}SO, (12) CONR^{L1}, (13) NR^{L1}CO, (14) NR^{L1}CONR^{L1'}, (15) NR^{L1}SO₂NR^{L1}', or (16) CO, and
L_{b2} is
   (1) a bond, (2) (CH₂)₁₋₁₀, (3) (CH₂)₀₋₆-O-(CH₂)₀₋₆, (4) (CH₂)₀₋₆-CONH-(CH₂)₀₋₆, (5) (CH₂)₀₋₆-NHCO-(CH₂)₀₋₆, (6) (CH₂)₀₋₆-NH-(CH₂)₀₋₆, (7) (CH₂)₀₋₆-NHSO₂-(CH₂)₀₋₆, or (8) (CH₂)₀₋₆-SO₂NH-(CH₂)₀₋₆
wherein R^{L1} and R^{L1'} are as defined above.

L is further preferably, a group represented by the formula (L-III): -L₁-L₂-L₃-
wherein L₁ and L₃ are each independently
   (1) a bond, (2) CR^{L1}R^{L1'}, (3) O, (4) S, (5) SO, (6) SO₂, (7) NR^{L1}, (8) SO₂NR^{L1}, (9) NR^{L1}SO₂, (10) SONR^{L1}, (11) NR^{L1}SO, (12) CONR^{L1}, (13) NR^{L1}CO, (14) NR^{L1}CONR^{L1'}, (15) NR^{L1}SO₂NR^{L1}', or (16) CO, and
L₂ is
   (CH₂)ₚ₁ₐ-O-(CH₂-CH₂-O)ₚ₂ₐ-(CH₂)ₚ₃ₐ
wherein
   p1a and p3a are each an integer of 0 to 10 when an atom that directly binds of the adjacent L₁ or L₃ is a carbon atom or bond, and an integer of 2 to 10 in other cases, and p2a is an integer of 0 to 10.

L is particularly preferably a group represented by -L_{b1}-L_{b2}-L_{b3}-
wherein L_{b1} and L_{b3} are each independently (1) a bond, (2) CR^{L1}R^{L1'}, (3) O, (4) S, (5) SO, (6) SO₂, (7) NR^{L1}, (8) SO₂NR^{L1}, (9) NR^{L1}SO₂, (10) SONR^{L1}, (11) NR^{L1}SO, (12) CONR^{L1}, (13) NR^{L1}CO, (14) NR^{L1}CONR^{L1'}, (15) NR^{L1}SO₂NR^{L1}', or (16) CO, and
L_{b2} is
   (1) a bond, (2) (CH₂)₁₋₁₀, (3) (CH₂)₀₋₆-O-(CH₂) ₀₋₆, (4) (CH₂) ₀₋₆-CONH-(CH₂)₀₋₆, (5) (CH₂)₀₋₆-NHCO-(CH₂)₀₋₆, (6) (CH₂)₀₋₆-NH-(CH₂)₀₋₆, (7) (CH₂)₀₋₆-NHSO₂-(CH₂)₀₋₆, or (8) (CH₂)₀₋₆-SO₂NH-(CH₂)₀₋₆
wherein R^{L1} and R^{L1'} are as defined above.

The aforementioned "Definition of each group used in the present specification" can be referred to for the meaning of the "optionally substituted cycloalkyl divalent group", "optionally substituted heterocyclic divalent group", "optionally substituted aryl divalent group", "optionally substituted heteroaryl divalent group", "halogen atom", "optionally substituted alkyl group", "optionally substituted cycloalkyl group", "optionally substituted aryl group", "optionally substituted heteroaryl group", "optionally substituted C₁-C₈ alkyl group", and "optionally substituted C₃-C₈ cycloalkyl group" in the above-mentioned definitions.

### [A]

A is a target-directed ligand, more specifically, a group having a moiety capable of binding to a target protein or a moiety that binds to the target protein. For example, a preferred specific example of the target-directed ligand is a compound that binds to the following target protein. The "target-directed ligand" of the present invention is not limited thereto.

Those of ordinary skill in the art can appropriately select or design and use a target-directed ligand according to the target protein of interest. Those described in Examples 163 to 165 described later are the examples thereof.

In the present invention, the target protein bound to A is preferably a protein having a biological function selected from the group consisting of structure, regulation, hormone, enzyme, gene regulation, immunity, contraction, storage, transport, and signal transduction. The protein is more preferably selected from the group consisting of structural protein, receptor, enzyme, cell surface protein, and proteins related to integrated cellular function, including proteins involved in catalytic activity, aromatase activity, motor activity, helicase activity, metabolic process (anabolism and catabolism), antioxidant activity, proteolysis, biosynthesis, kinase activity, oxidoreductase activity, transferase activity, hydrolase activity, lyase activity, isomerase activity, ligase activity, enzyme regulatory factor activity, signal transducer activity, structural molecular activity, binding activity (protein, lipid carbohydrates), receptor activity, cellular motility, membrane fusion, intercellular signal transduction, control of biological processes, development, cell differentiation, stimulus response, cell adhesion, cell death, transport(protein transporter activity, nuclear transport, transporter activity, channel transporter activity, carrier activity, permease activity, secretion activity, electron transporter activity), pathogenesis, virus outer shell, chaperon regulatory factor activity, nucleic acid binding activity, transcriptional regulator activity, epigenetics control, aggregation, extracellular organization, biogenetic activity, or translation regulatory factor activity.

In the present invention, the target protein bound to A is preferably selected from the group consisting of proteins related to cancer related proteins, autoimmune disease related proteins, inflammatory disease related proteins, neurodegenerative disease related proteins, muscular disease related proteins, sensory system disease related proteins, circulatory disease related proteins, metabolic disease related proteins, and genetic disease related proteins.

When compound (I) contains optical isomers (enantiomer, diastereomer), stereoisomer, regioisomer, and rotamer, these are also included as compound (I), and can be respectively obtained as single products by synthesis methods and separation methods (e.g., concentration, solvent extraction, column chromatography, recrystallization, etc.) known per se.

Compound (I) may be converted to a prodrug as appropriate, and such embodiment is also included in the scope of the present invention (hereinafter compound (I) and a prodrug thereof are at times collectively referred to as "the compound of the present invention"). The prodrug may be one that changes to compound (I) under physiological conditions, such as the one described in "Drug Development" Vol. 7 "Molecular Design", p. 163-198 published by HIROKAWA SHOTEN (1990).

Compound (I) may be any of hydrate, non-hydrate, solvate, and non-solvate. In addition, compound (I) may be a compound labeled or substituted with an isotope (e.g., ²H, ³H, ¹¹C, ¹⁴C, ¹⁸F, ³⁵S, ¹²⁵I, etc.) . A compound labeled or substituted with an isotope can be used as a tracer (PET tracer) used in positron emission tomography (PET), and may be useful in the fields of medical diagnosis and the like. A deuterium conversion form wherein ¹H is converted to ²H(D) is also encompassed in compound (I).

Tautomers are also encompassed in compound (I).

### [Production method of the target protein degradation-inducing compound of the present invention]

The production method of compound (I) is explained below.

### (General synthesis approach)

The compounds of the present invention can be produced by the following methods A to X. While these methods and steps may be combined but the production method thereof is not limited thereto.

The formula (I) of the present invention: 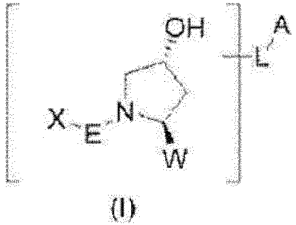 wherein each symbol is as defined above.

The formula (1) of the intermediate of the present invention: 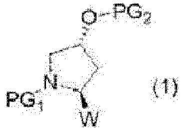 wherein PG₁ is an amine-protecting group, PG₂ is a hydroxy-protecting group, and other symbols are as defined above.

As the protecting group represented by PG₁, a carbamate-protecting group can be mentioned. As the protecting group represented by PG₂, a silyl-protecting group can be mentioned.

When PG₁ is, for example, a Boc group, deprotection can be performed in a suitable solvent in the presence of an acid. When PG₂ is, for example, a TBS group, deprotection can be performed in a suitable solvent in the presence of an acid, base, or fluoride ion.

### Synthesis method A

The production method described here is suitable for producing an intermediate of a compound represented by the formula (I) wherein W is an imidazolyl group, namely, the following compound [A-2]. 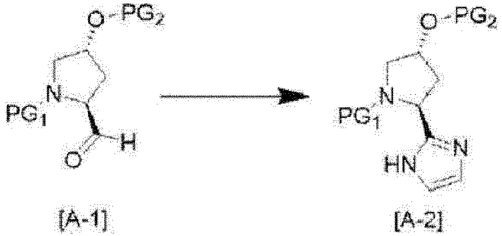 wherein each symbol is as defined above.

The formula [A-2] can be derived from the formula [A-1] according to a known method (e.g., BIOORGANIC & Medicinal Chemistry Letters, 26(21), 5354-5360; 2016).

### Synthesis method B

The production method described here is suitable for producing an intermediate that is a compound represented by the formula (I) wherein W is an imidazolyl group, and R^{a2} and R^{a3} are substituted, i.e., the following compound [B-2]. 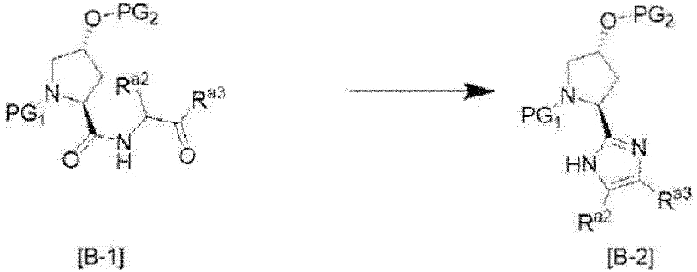 wherein each symbol is as defined above.

The formula [B-2] can be derived from the formula [B-1] according to a known method (e.g., WO2006099060).

### Synthesis method C

The production method described here is suitable for producing an intermediate that is a compound represented by the formula (I) wherein W is an imidazolyl group, R^{a1} and R^{a2} are each a hydrogen atom, and R^{a3} is substituted by carboxylic acid, i.e., the following compound [C-4]. 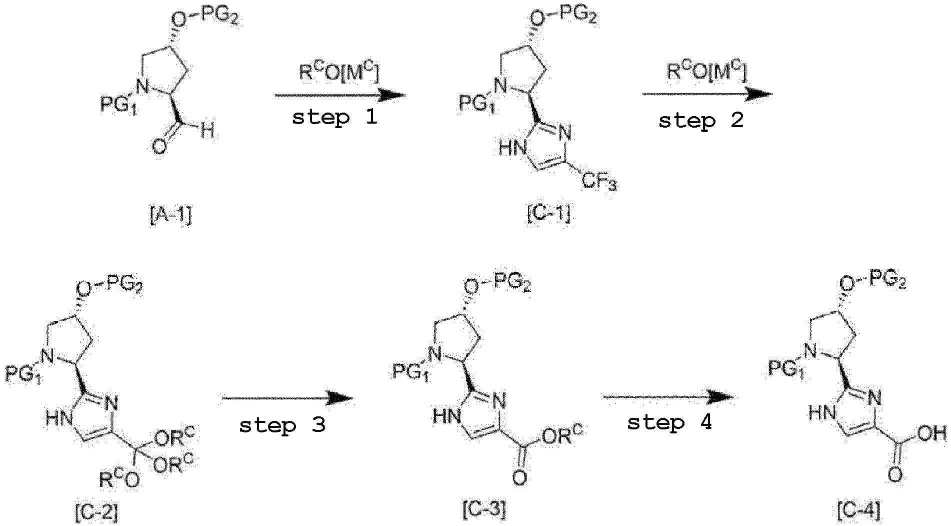 wherein R^{c}O[M^{c}] is an alkali metal alkoxide, and each symbol is as defined above.

The formula [C-1] can be derived from compound [A-1] according to a known method (e.g., Journal of Medicinal Chemistry, 33(1), 317-27; 1990).

The formula [C-2] is obtained by decomposing the trifluoro group in the formula [C-1] with a base. The reaction proceeds using alkali metal alkoxide in an appropriate alcohol solvent generally from room temperature to the refluxing temperature of the solvent. While the reaction time varies depending on the starting materials and solvents to be used and the reaction temperature, it is generally 5 min to 12 hr. Examples of the base having an alkoxy group include alkali metal alkoxide such as sodium methoxide, specifically potassium methoxide, sodium methoxide, lithium methoxide, sodium ethoxide, potassium tert-butoxide, and the like. Examples of the solvent include methanol, ethanol, tert-butanol and the like.

The formula [C-3] is obtained by decomposition of the formula [C-2] under acidic conditions. The reaction proceeds using an acid in an appropriate solvent generally from room temperature to the refluxing temperature of the solvent. While the reaction time varies depending on the starting materials and solvents to be used and the reaction temperature, the reaction generally proceeds in 1 hr to 24 hr. Examples of the acid include trifluoroacetic acid, hydrochloric acid, and the like. Examples of the solvent include dichloromethane, 1,2-dichloroethane, chloroform, methanol, ethyl acetate, toluene, 1,4-dioxane, and the like.

The formula [C-4] is obtained by subjecting the formula [C-3] to a basic condition or an acidic condition. The reaction proceeds using a base or acid in an appropriate solvent generally from room temperature to the refluxing temperature of the solvent. While the reaction time varies depending on the starting materials and solvents to be used and the reaction temperature, the reaction generally proceeds in 1 hr to 48 hr. When a base is used, sodium hydroxide, potassium hydroxide, lithium hydroxide, and the like can be mentioned, and when an acid is used, trifluoroacetic acid, hydrochloric acid, and the like can be mentioned. When a base is used as a solvent, for example, methanol, ethanol, tetrahydrofuran, and water can be mentioned, and when an acid is used, dichloromethane, 1,2-dichloroethane, chloroform, methanol, ethyl acetate, toluene, 1,4-dioxane, water and the like can be mentioned.

### Synthesis method D

The production method described here is suitable for producing an intermediate that is a compound represented by the formula (I) wherein W is a thiazolyl group substituted by R^{a2} and R^{a3}, i.e., the following compound [D-4]. 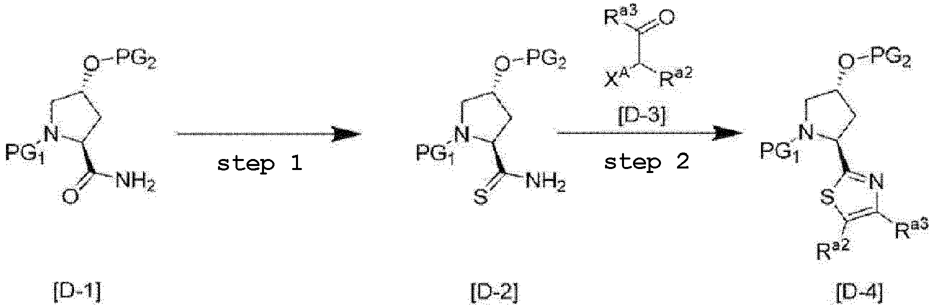 wherein X^{A} is a halogen atom, each symbol is as defined above, and the halogen atom is a chlorine atom, a bromine atom, or an iodine atom.

The formula [D-2] is obtained by thioamidating the formula [D-1]. The thioamidation reaction proceeds using a sulfurizing agent in an appropriate solvent generally from room temperature to the refluxing temperature of the solvent. While the reaction time varies depending on the starting materials and solvents to be used and the reaction temperature, it is generally 0.5 hr to 24 hr. Examples of the sulfurizing agent include Lawesson's reagent, diphosphorus pentasulfide, and the like. Examples of the solvent include 1,2-dichloroethane, tetrahydrofuran, 1,4-dioxane and the like.

The formula [D-4] can be derived from the formula [D-2] and the formula [D-3] according to a known method (e.g., ChemBioChem, 12(15), 2284-2288; 2011, WO 2009098448, WO 2010060952) .

### Synthesis method E

The production method described here is suitable for producing an intermediate that is a compound represented by the formula (I) wherein W is an oxadiazolyl group substituted by R^{a3}, i.e., the following compound [E-3]. 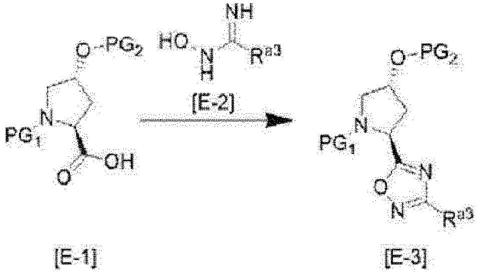 wherein each symbol is as defined above.

the formula [E-3] can be derived from the formula [E-1] and the formula [E-2] according to a known method (e.g., WO 2016044386) .

### Synthesis method F

The production method described here is suitable for producing an intermediate that is a compound represented by the formula (I) wherein W is a triazolyl group substituted by R^{a1} and R^{a3}, i.e., the following compound [F-3]. 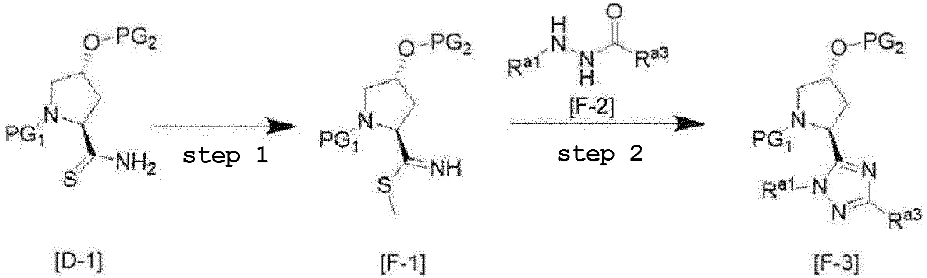 wherein each symbol is as defined above.

The formula [F-1] can be derived from the formula [D-1] according to a known method (e.g., Tetrahedron Letters, 45(52), 9557-9559; 2004).

The formula [F-3] can be derived from the formula [F-1] and the formula [F-2] according to a known method (e.g., US20120264735).

### Synthesis method G

The production method described here is suitable for producing an intermediate that is a compound represented by the formula (I) wherein W is a tetrazolyl group, i.e., the following compound [G-2]. 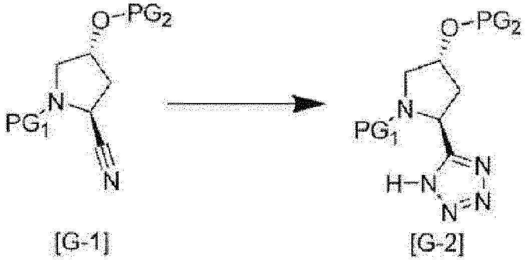 wherein each symbol is as defined above.

The formula [G-2] can be derived from the formula [G-1] according to a known method (e.g., Synthesis, 46(15), 2065-2070; 2014, WO 2011035900).

### Synthesis method H

The production method described here is suitable for producing an intermediate that is a compound represented by the formula (I) W is triazolyl group, substituted by R^{a2}, i.e., the following compound [H-3]. 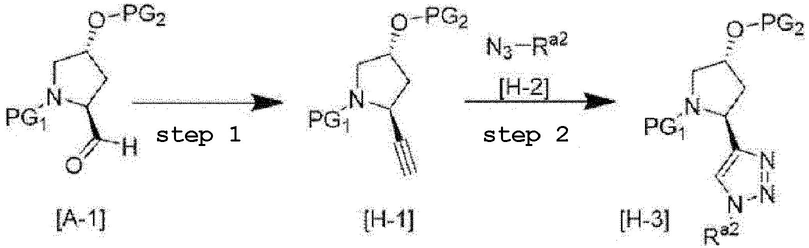 wherein each symbol is as defined above.

The formula [H-1] can be derived from the formula [A-1] according to a known method (e.g., Bioorganic & Medicinal Chemistry Letters, 26(5), 1419-1427; 2016).

The formula [H-3] can be derived from the formula [H-1] and the formula [H-2] according to a known method (e.g., Organometallics, 30(5), 1021-1029; 2011).

### Synthesis method I

The production method described here is suitable for producing an intermediate that is a compound represented by the formula (I) wherein W is a pyrazolyl group substituted by R^{a2} and R^{a3}, i.e., the following compound (1-3). 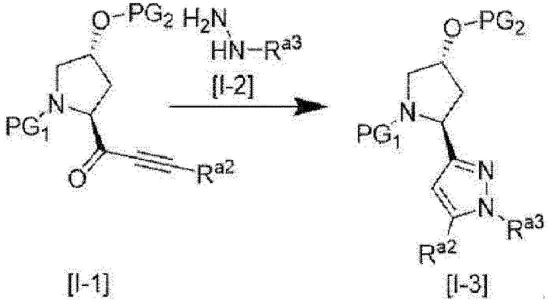 wherein each symbol is as defined above.

The formula [1-3] can be derived from the formula [I-1] and the formula [1-2] according to a known method (e.g., WO 2002034716) .

### Synthesis method J

The production method described here is suitable for producing an intermediate that is a compound represented by the formula (I) wherein W is an oxazolyl group substituted by R^{a2} and R^{a3}, i.e., the following compound [J-2]. 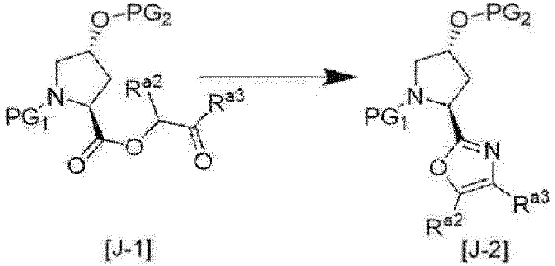 wherein each symbol is as defined above.

The formula [J-2] can be derived from the formula [J-1] according to a known method (e.g., WO 2009080226).

### Synthesis method K

The production method described here is suitable for producing an intermediate that is a compound represented by the formula (I) wherein W is a pyridyl group, i.e., the following compound [K-2]. 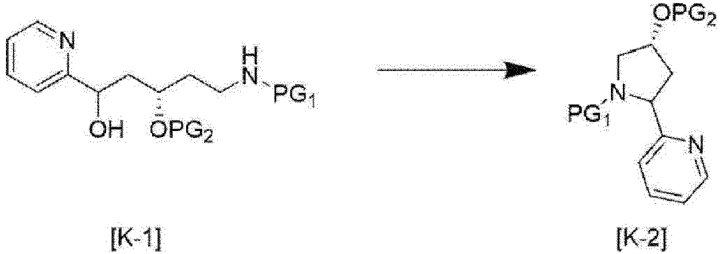 wherein each symbol is as defined above.

The formula [K-2] can be derived from the formula [K-1] according to a known method (e.g., WO 2013061977).

### Synthesis method L

The production method described here is suitable for producing an intermediate that is a compound represented by the formula (I) wherein W is an alkyl group optionally substituted by a group selected from a heterocyclic group and substituted by X^{2aL}, i.e., the following compound [L-5]. 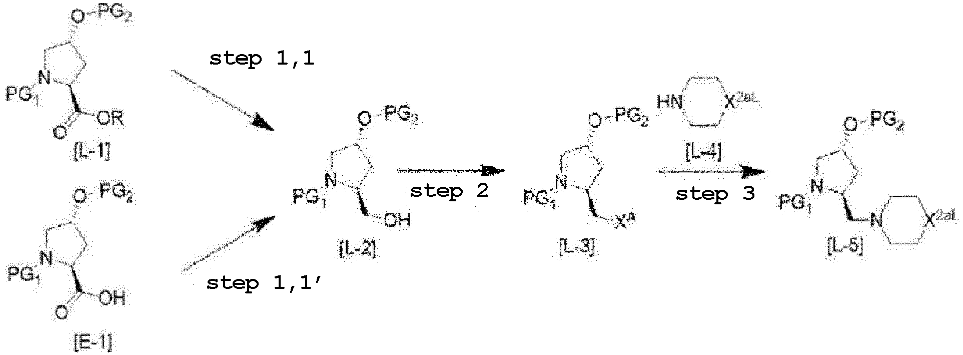 wherein X^{2aL} is a carbon or oxygen atom, and each symbol is as defined above.

The formula [L-2] is obtained by the following two steps.

### Method 1

The formula [L-2] is obtained by a reduction reaction of the formula [L-1]. The reaction proceeds using a reducing agent in an appropriate solvent generally from -78°C to the refluxing temperature of the solvent. While the reaction time varies depending on the starting materials and solvents to be used and the reaction temperature, the reaction proceeds generally for 10 min to 24 hr. When a reducing agent is used, hydrogenated lithium aluminum hydride, DIBAL-H, and the like can be mentioned. Examples of the solvent include methylene chloride, diethyl ether, tetrahydrofuran, and toluene.

### Method 2

The formula [L-2] is obtained by a reduction reaction of the formula [E-1]. The reaction proceeds using a reducing agent in an appropriate solvent generally from -78°C to the refluxing temperature of the solvent. While the reaction time varies depending on the starting materials and solvents to be used and the reaction temperature, the reaction generally proceeds for 10 min to 24 hr. When a reducing agent is used, hydrogenated lithium aluminum hydride, BH3·THF, and the like can be mentioned. Examples of the solvent include diethyl ether and tetrahydrofuran.

The formula [L-3] is obtained by halogenation of the formula [L-2]. The reaction proceeds using a halogenating agent in an appropriate solvent generally from 0°C to the refluxing temperature of the solvent. While the reaction time varies depending on the starting materials and solvents to be used and the reaction temperature, the reaction generally proceeds in 10 min to 24 hr. Examples of the halogenating agent include iodine and triphenyl phosphine, or carbon tetrabromide and triphenyl phosphine, and the like. In the case of iodine and triphenyl phosphine, imidazole or the like is used as a base. Examples of the solvent include diethyl ether, tetrahydrofuran, and dichloromethane.

The formula [L-5] is obtained by an alkylation reaction of the formula [L-3] and the formula [L-4]. The alkylation reaction proceeds using a base and a alkylating agent such as halogenated alkyl and the like, in an appropriate solvent generally from 0°C to the refluxing temperature of the solvent. While the reaction time varies depending on the starting materials and solvents to be used and the reaction temperature, it is generally from 30 min to 24 hr. Examples of the base include inorganic bases such as sodium hydride, potassium hydroxide, cesium carbonate, potassium carbonate, and the like, alkoxides such as potassium tert-butoxide, sodium methoxide, and the like, and the like. Examples of the solvent include N,N-dimethylformamide, tetrahydrofuran, dimethyl sulfoxide, and the like.

### Synthesis method M

The production method described here is suitable for producing an intermediate that is a compound represented by the formula (I) wherein W is a heteroaryl group having 5 ring-constituting atoms, A^{3c} is a carbon atom, substituted by R^{a1} and R^{a2}, further R^{a3} is an optionally substituted aryl group, or substituted by an optionally substituted heteroaryl group, i.e., the following compound [M-4]. 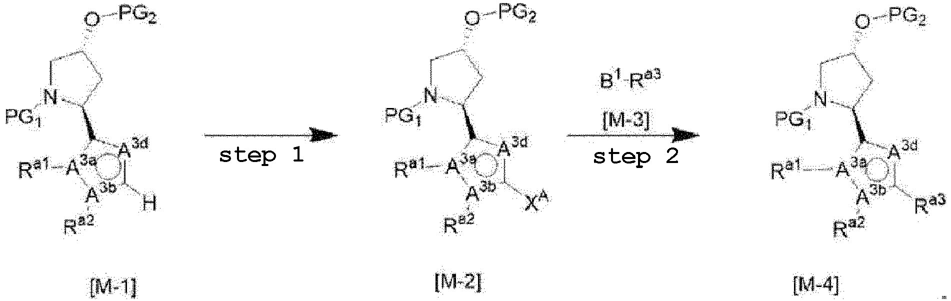 wherein B¹ is boronic acid or boronic acid ester optionally having substituent(s), and other each symbol is as defined above. As used herein, boronic acid ester optionally having substituent(s) for B¹ is pinacolatoboron, neopentyl glycolatoboron or the like.

The formula [M-2] is obtained by halogenation of the formula [M-1]. The reaction proceeds in the presence of a halogenating agent in a suitable solvent from 0°C to solvent refluxing. While the reaction time varies depending on the starting materials and solvents to be used and the reaction temperature, it is generally 1 hr to 24 hr. The halogenating agent is, for example, bromine, iodine, NCS, NBS, NIS, or the like. As the solvent, acetonitrile, methylene chloride, carbon tetrachloride, 1,2-dichloroethane, tetrahydrofuran, and the like can be mentioned.

The formula [M-4] is obtained by a coupling reaction of the formula [M-2] and boronic acid derivative [M-3]. The reaction preferably proceeds in the presence of a palladium catalyst, a phosphine ligand, and a base in a suitable solvent from 0°C to under heating, particularly from room temperature to the boiling point of the solvent. While the reaction time varies depending on the starting materials and solvents to be used and the reaction temperature, it is generally 1 hr to 24 hr. Examples of the palladium catalyst include palladium (II) acetate, palladium (II) chloride, tris(dibenzylideneacetone)dipalladium(0) or chloroform adduct thereof, and the like. Examples the phosphine ligand include triphenylphosphine, 2-(dicyclohexylphosphino)-2',4',6'-triisopropyl-1,1'-biphenyl, 2-(dicyclohexylphosphino)-3,6-dimethoxy-2',4',6'-triisopropyl-1,1'-biphenyl, 2-(dicyclohexylphosphino)-2,6-diisopropoxy-1,1'-biphenyl, 2-di-tert-butylphosphino-2'-4'-6'-triisopropylbiphenyl, 2-dicyclohexylphosphino-2'-6'-dimethoxybiphenyl, 2-(dicyclohexylphosphino)-2-(N,N-dimethylamino)biphenyl, tri-ortho-tolylphosphine, 2-(dicyclohexylphosphino)biphenyl, 2-(di-tert-butylphosphino)biphenyl, 2-(di-tert-butylphosphino)-1,1-binaphthyl, tri-tert-butylphosphine, tri-tert-butylphosphonium tetrafluoroborate, and the like. A reagent in which a palladium catalyst and a phosphine ligand form a complex may also be used. Examples thereof include tetrakis(triphenyl phosphine)palladium(0), 1,1-bis(diphenylphosphino)ferrocene-palladium(II) dichloride, dichlorobis(triphenylphosphine)palladium(II), dichlorobis(tricyclohexylphosphine)palladium(II), bis(tri-tert-butylphosphine)palladium(0), chloro(2-dicyclohexylphosphino-2',4',6'-triisopropyl-1,1'-biphenyl) (2'-amino-1,1'-biphenyl-2-yl)palladium(II), [(2-dicyclohexylphosphino-3,6-dimethoxy-2',4' ,6'-triisopropyl-1,1'-biphenyl)-2-(2'-amino-1,1'-biphenyl)palladium(II) methanesulfonate, (2-dicyclohexyl phosphino-2,6-diisopropoxy-1,1'-biphenyl) [2-(2'-amino-1,1'-biphenyl)]palladium(II) methanesulfonate, [(2-di-tert-butylphosphino-3,6-dimethoxy-2',4',6'-triisopropyl-1,1'-biphenyl)-2-(2'-amino-1,1'-biphenyl)palladium(II) methanesulfonate, and the like. Examples of the base include tert-butoxy sodium, potassium acetate, tripotassium phosphate, cesium carbonate, potassium carbonate, sodium hydrogen carbonate, triethylamine, diisopropyl ethylamine, dicyclohexylethylamine, potassium fluoride, cesium fluoride, and the like. Examples of the solvent include ether solvents such as tetrahydrofuran, 1,2-dimethoxyethane, 1,4-dioxane and the like, alcoholic solvents such as methanol, ethanol, propanol, butanol, and the like, N,N-dimethylformamide, NMP or a mixed solvent of an organic solvent and water, and the like.

### Synthesis method N

The production method described here is suitable for producing an intermediate that is a compound represented by the formula (I) wherein W is an imidazolyl group, a thiazolyl group, or an oxazolyl group, substituted by R^{6a2,} R^{6a3} is -CO-NR^{a3N6}R^{a3N6'} or -CO-(a heterocyclic group containing a nitrogen atom, optionally further containing 1 to 5 same or different atoms selected from a nitrogen atom, an oxygen atom, and a sulfur atom, and having 3 to 10 ring-constituting atoms, which is optionally further substituted in addition to Y^{9a}) , i.e., the following compound [N-4] or [N-5]. 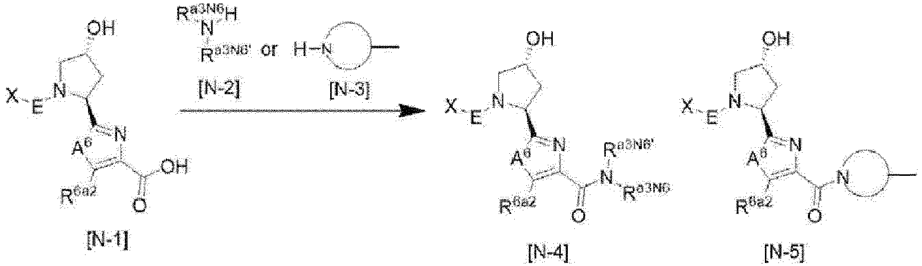 wherein each symbol is as defined above.

As the method for obtaining the formula [N-4] or [N-5], for example, any of the following three methods is used.

### Method 1

The formula [N-4] or [N-5] is obtained by a condensation reaction of the formula [N-1] and the formula [N-2] or the formula [N-3]. The reaction proceeds using a condensing agent in the presence of a suitable base in a suitable solvent at 0°C to the refluxing temperature of the solvent. While the reaction time varies depending on the starting materials and solvents to be used and the reaction temperature, it is generally from 30 min to 24 hr. Examples of the condensing agent include 1-ethyl-3-(3-dimethylaminopropyl)carbodiimide hydrochloride (WSC·HCl), 1-[bis (dimethylamino)methylene]-1H-1,2,3-triazolo [4,5-b]pyridinium 3-oxide hexafluorophosphate (HATU), 4-(4,6-dimethoxy[1.3.5]triazin-2-yl)-4-methylmorpholinium chloride hydrate (DMT-MM), 2-chloro-1-methylpyridinium iodide, and the like. Examples of the solvent include methanol, N,N-dimethylformamide, chloroform, dichloromethane, tetrahydrofuran, and the like. The reaction may be promoted by adding 1-hydroxybenzotriazole (HOBt). Examples of the base include triethylamine, N,N-diisopropyl ethylamine, pyridine, and the like.

### Method 2

The formula [N-4] or [N-5] is obtained by converting the formula [N-1] to an acid halide with a halogenating agent, and then reacting same with the formula [N-2] or the formula [N-3]. The reaction proceeds using a base in an appropriate solvent at generally from -20°C to the refluxing temperature of the solvent. While the reaction time varies depending on the starting materials and solvents to be used and the reaction temperature, it is generally from 0.5 hr to 24 hr. Examples of the halogenating agent include thionyl chloride, oxalyl chloride, phenylphosphonyl dichloride, and the like. Examples of the base include triethylamine, pyridine and the like. Examples of the solvent include dichloromethane, 1,2-dichloroethane, chloroform, pyridine, toluene, and the like.

### Method 3

The formula [N-4] or [N-5] is obtained by converting the formula [N-1] to a mixed acid anhydride, and then reacting same with the formula [N-2] or the formula [N-3]. The reaction proceeds using a base in an appropriate solvent at generally from -20°C to the refluxing temperature of the solvent. While the reaction time varies depending on the starting materials and solvents to be used and the reaction temperature, it is generally from 0.5 hr to 24 hr. Examples of the reagent to form the mixed acid anhydride include acid anhydrides such as methyl chlorocarbonate, ethyl chlorocarbonateic, isobutyloxycarbonyl chloride, pivaloyl chloride, and the like. Examples of the base include triethylamine, DIEPA, pyridine, N-methylmorpholine, and the like. Examples of the solvent include methanol, ethanol, isopropyl alcohol, butanol, ethylene glycol, tetrahydrofuran, chloroform, N,N-dimethylformamide, dimethyl sulfoxide, toluene, and the like. Examples of the base include triethylamine, DIEPA, pyridine, N-methylmorpholine, and the like.

### Synthesis method O

The production method described here is suitable for producing an intermediate that is a compound represented by the formula (I) wherein W is an imidazolyl group, R^{6a1} is substituted by -(CR^{Y11}R^{Y11'})ₙ₁₀-W^{10a}-Y^{10b}-W^{10b}' and further substituted by R^{6a2,} R^{6a3}, i.e., the following compound [O-3]. 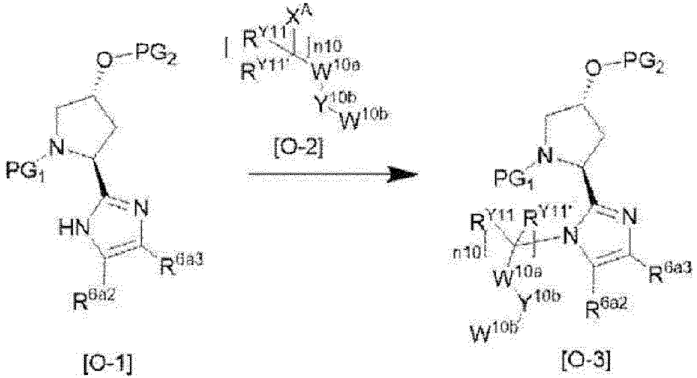 wherein each symbol is as defined above.

The formula [O-3] is obtained by an alkylation reaction of the formula [O-1] and the formula [O-2]. The alkylation reaction proceeds using a base and an alkylating agent such as halogenated alkyl and the like in an appropriate solvent at generally at 0°C to the refluxing temperature of the solvent. While the reaction time varies depending on the starting materials and solvents to be used and the reaction temperature, it is generally from 30 min to 24 hr. Examples of the base include inorganic bases such as sodium hydride, potassium hydroxide, cesium carbonate, potassium carbonate, and the like, alkoxides such as potassium tert-butoxide, sodium methoxide, and the like, and the like. As the reaction promoter, for example, NaI, TBAI, or the like is used at times. Examples of the solvent include N,N-dimethylformamide, tetrahydrofuran, acetonitrile, toluene, NMP, dimethyl sulfoxide, and the like.

### Synthesis method P

The production method described here is suitable for producing an intermediate that is a compound represented by the formula (I) wherein W is an imidazolyl group, R^{6a1} is substituted by -(CR^{Y11}R^{Y11'})ₙ₁₀-W^{10a}-W^{10b,} (W^{10a} is an optionally substituted aryl divalent group having 6 to 10 carbon atoms or an optionally substituted heteroaryl divalent group containing 1 to 6 atoms selected from a nitrogen atom, an oxygen atom, and a sulfur atom and having 5 to 10 ring-constituting atoms, W^{10b} is an optionally substituted aryl group having 6 to 10 carbon atoms or an optionally substituted heteroaryl group containing 1 to 6 same or different atoms selected from a nitrogen atom, an oxygen atom, and a sulfur atom and having 5 to 10 ring-constituting atoms) further substituted by R^{6a2,} R^{6a3}, i.e., the following compound [P-3]. 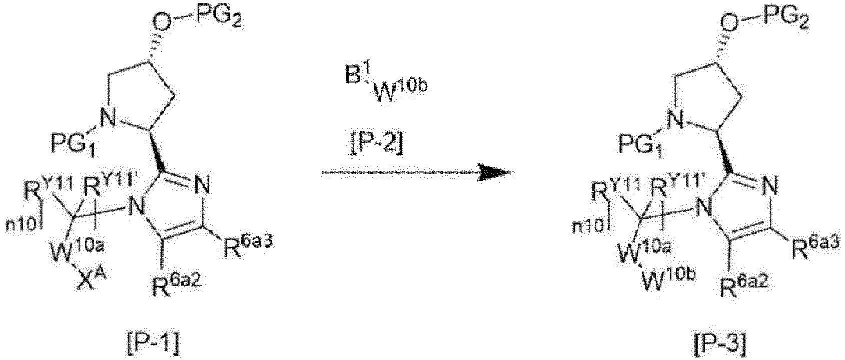 wherein each symbol is as defined above.

The formula [P-3] is obtained by a coupling reaction of the formula [P-1] and the formula [P-2]. The reaction preferably proceeds in the presence of a palladium catalyst, a phosphine ligand, and a base in a suitable solvent at 0°C to under heating, particularly from room temperature to the boiling point of the solvent. The reaction time and the palladium catalyst, phosphine ligand and base, and the solvent to be used are the same as those in step 2 of Synthesis method M.

### Synthesis method Q

The production method described here is suitable for producing an intermediate that is a compound represented by the formula (I) wherein E is a bond, X is an optionally substituted aryl group or an optionally substituted heteroaryl group, i.e., the following compound [Q-3]. 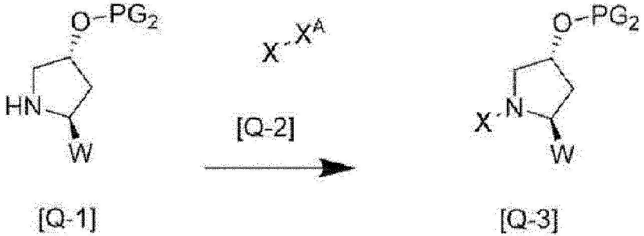 wherein each symbol is as defined above.

As the method for obtaining the formula [Q-3], for example, any of the following two methods is used.

### Method 1

The formula [Q-3] is obtained by an SnAr reaction of the formula [Q-1] and the formula [Q-2]. The reaction proceeds in the presence of a base in an appropriate solvent at generally from room temperature to the refluxing temperature of the solvent. While the reaction time varies depending on the starting materials and solvents to be used and the reaction temperature, it is generally from 30 min to 48 hr. Examples of the base include inorganic bases such as sodium hydride, potassium hydroxide, cesium carbonate, potassium carbonate, and the like, alkoxides such as potassium t-butoxide, sodium methoxide, and the like, and organic bases such as triethylamine, DBU, DIPEA, and the like. Examples of the solvent include tetrahydrofuran, 1,4-dioxane, dimethylformamide, dimethyl sulfoxide, NMP, toluene, xylene, acetonitrile, and the like.

### Method 2

The formula [Q-3] is obtained by a coupling reaction of the formula [Q-1] and the formula [Q-2]. The reaction preferably proceeds in the presence of a palladium catalyst, a phosphine ligand, and a base in a suitable solvent at 0°C to under heating, particularly from room temperature to the boiling point of the solvent. While the reaction time varies depending on the starting materials and solvents to be used and the reaction temperature, it is generally from 1 hr to 72 hr. Examples of the palladium catalyst include palladium(II) acetate, palladium (II) chloride, tris(dibenzyl ideneacetone)dipalladium(0) or chloroform adduct thereof, and the like. Examples of the phosphine ligand include triphenyl phosphine, 2-(dicyclohexylphosphino)-2',4',6'-triisopropyl-1,1'-biphenyl, 2-(dicyclohexylphosphino) -3,6-dimethoxy-2',4',6'-triisopropyl-1,1'-biphenyl, 2-(dicyclohexylphosphino) -2,6-diisopropoxy-1,1'-biphenyl, 2-di-tert-butylphosphino-2'-4'-6'-triisopropylbiphenyl, 2-dicyclohexylphosphino-2'-6'-dimethoxybiphenyl, 2-(dicyclohexylphosphino)-2-(N,N-dimethylamino)biphenyl, tri-ortho-tolylphosphine, 2-(dicyclohexylphosphino)biphenyl, 2-(di-tert-butylphosphino)biphenyl, 2-(di-tert-butylphosphino)-1,1-binaphthyl, tri-tert-butylphosphine, tri-tert-butylphosphonium tetrafluoroborate, and the like. A reagent in which a palladium catalyst and a phosphine ligand form a complex may also be used. Examples thereof include tetrakis(triphenyl phosphine)palladium(0), 1,1-bis(diphenylphosphino)ferrocene-palladium(II) dichloride, dichlorobis(triphenylphosphine)palladium(II), dichlorobis(tricyclohexylphosphine)palladium(II), bis(tri-tert-butylphosphine)palladium(0), chloro(2-dicyclohexylphosphino-2',4',6'-triisopropyl-1,1'-biphenyl) (2'-amino-1,1'-biphenyl-2-yl)palladium(II), [(2-dicyclohexylphosphino-3,6-dimethoxy-2' ,4' ,6'-triisopropyl-1,1'-biphenyl)-2-(2'-amino-1,1'-biphenyl)palladium(II) methanesulfonate, (2-dicyclohexylphosphino-2,6-diisopropoxy-1,1'-biphenyl) [2-(2'-amino-1,1'-biphenyl)]palladium(II) methanesulfonate, [(2-di-tert-butylphosphino-3,6-dimethoxy-2',4',6'-triisopropyl-1,1'-biphenyl)-2-(2'-amino-1,1'-biphenyl)palladium(II) methanesulfonate, and the like. Examples of the base include tert-butoxy sodium, potassium acetate, tripotassium phosphate, cesium carbonate, potassium carbonate, sodium hydrogen carbonate, triethylamine, diisopropyl ethylamine, dicyclohexylethylamine, potassium fluoride, cesium fluoride, and the like. Examples of the solvent include ether solvents such as tetrahydrofuran, 1,2-dimethoxyethane, 1,4-dioxane, and the like, alcoholic solvents such as methanol, ethanol, propanol, butanol, and the like, N,N-dimethylformamide, NMP or a mixed solvent of an organic solvent and water, and the like.

### Synthesis method R1

The production method described here is suitable for producing an intermediate that is a compound represented by the formula (I) wherein E is -CO-, X is -CHR^{m}-X¹-X² wherein X¹ is substituted by a triazolyl group, i.e., the following compound [R1-4] . 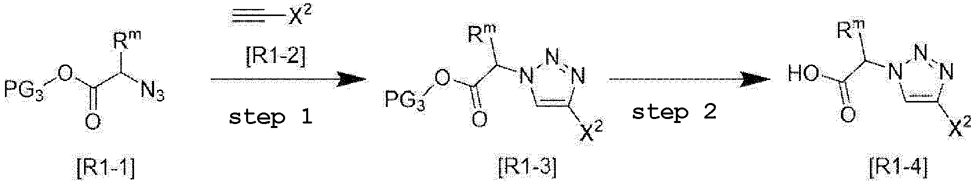 wherein PG₃ is a carboxylic acid-protecting group, as the protecting group represented by PG₃, for example, alkyl can be mentioned. Each symbol is as defined above.

The formula [R1-3] is obtained by a click reaction of the formula [R1-1] and the formula [R1-2]. The reaction proceeds in the presence of a suitable metal catalyst in a suitable solvent at 0°C to the refluxing temperature of the solvent. Examples of the metal catalyst include Cu, CuSO4, Zn(OAc)2, and the like. While the reaction time varies depending on the starting materials and solvents to be used and the reaction temperature, it is generally 5 min to 24 hr. As a reaction promoter, SODIUM ASCORBATE and the like are used at times. As the solvent, water, methanol, ethanol, t-BuOH, dimethyl sulfoxide, and a mixed solvent thereof, and the like are used.

The formula [R1-4] is obtained by subjecting the formula [R1-3] to a basic condition or an acidic condition. The reaction proceeds using a base or acid in an appropriate solvent generally from room temperature to the refluxing temperature of the solvent. The reaction time and the base and the solvent to be used are the same as those in step 4 of Synthesis method C.

### Synthesis method R2

The production method described here is suitable for producing an intermediate that is a compound represented by the formula (I) wherein E is -CO-, X is -CHR^{m}-X¹-X² wherein X¹ is substituted by a pyrazolyl group, i.e., the following compound [R-4]. 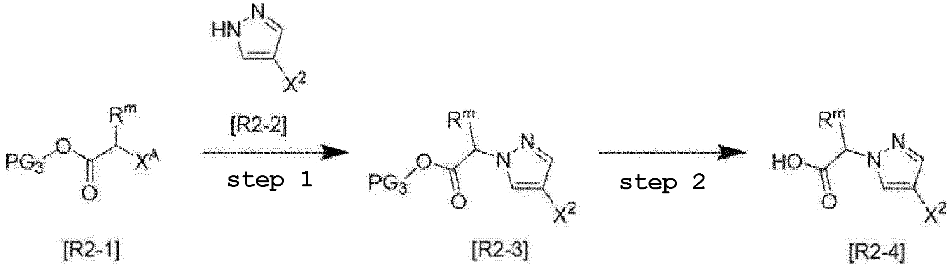 wherein each symbol is as defined above.

The formula [R2-3] is obtained by an alkylation reaction of the formula [R2-1] and the formula [R2-2]. The reaction proceeds using a suitable base in a suitable solvent at 0°C to the refluxing temperature of the solvent. The reaction time and the base, solvent, and reaction promoter to be used are the same as those in Synthesis method O.

The formula [R2-4] is obtained by subjecting the formula [R2-3] to a basic condition or an acidic condition. The reaction proceeds using a base or acid in an appropriate solvent generally from room temperature to the refluxing temperature of the solvent. The reaction time and the base and the solvent to be used are the same as those in step 4 of Synthesis method C.

### Synthesis method R3

The production method described here is suitable for producing an intermediate that is a compound represented by the formula (I) wherein E is -CO-, X is -CHR^{m}-X¹-X², wherein R^{m} is an optionally substituted alkyl group having 1 to 10 carbon atoms, and X¹ is substituted by an oxadiazolyl group, i.e., the following compound [R3-6]. 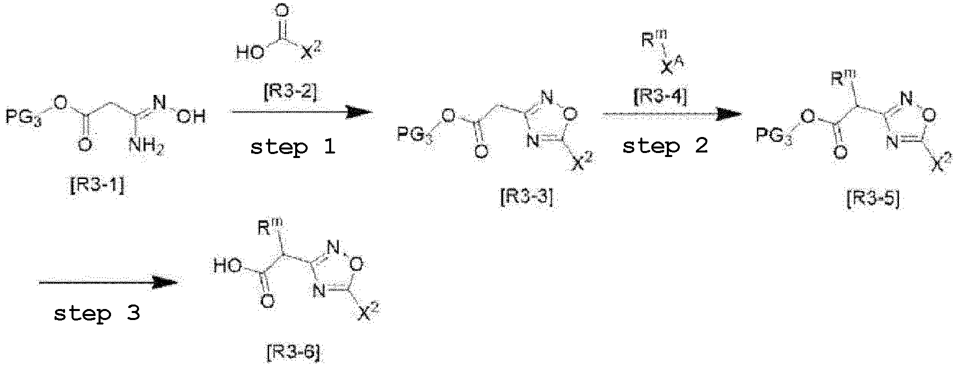 wherein each symbol is as defined above.

The formula [R3-3] can be derived from the formula [R3-1] and the formula [R3-2] according to a known method (e.g., WO 2016044386) .

The formula [R3-5] is obtained by an alkylation reaction of the formula [R3-3] and the formula [R3-4]. The reaction proceeds using a suitable base in a suitable solvent at 0°C to the refluxing temperature of the solvent. The reaction time and the base, solvent, and reaction promoter to be used are the same as those in Synthesis method O.

The formula [R3-6] is obtained by subjecting the formula [R3-5] to a basic condition or an acidic condition. The reaction proceeds using a base or acid in an appropriate solvent generally from room temperature to the refluxing temperature of the solvent. The reaction time and the base and the solvent to be used are the same as those in step 4 of Synthesis method C.

### Synthesis method R4

The production method described here is suitable for producing an intermediate that is a compound represented by the formula (I) wherein E is -CO-, X is -CHR^{m}-X¹-X², wherein R^{m} is an optionally substituted alkyl group having 1 to 10 carbon atoms, and X¹ is substituted by an isoxazolyl group, i.e., the following compound [R4-4]. 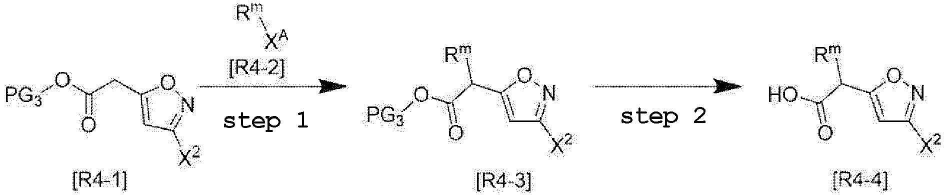 wherein each symbol is as defined above.

The formula [R4-3] is obtained by an alkylation reaction of the formula [R4-1] and the formula [R4-2]. The reaction proceeds using a suitable base in a suitable solvent at 0°C to the refluxing temperature of the solvent. The reaction time and the base, solvent, and reaction promoter to be used are the same as those in Synthesis method O.

The formula [R4-4] is obtained by subjecting the formula [R4-3] to a basic condition or an acidic condition. The reaction proceeds using a base or acid in an appropriate solvent generally from room temperature to the refluxing temperature of the solvent. The reaction time and the base and the solvent to be used are the same as those in step 4 of Synthesis method C.

### Synthesis method S

The production method described here is suitable for producing a compound represented by the formula (I) wherein E is -CO-, X is -CHR^{m}-X¹-X², wherein X¹ is -NR^{1X}-, X² is -CO-, and further substituted by L-A, i.e., the following compound [S-7]. 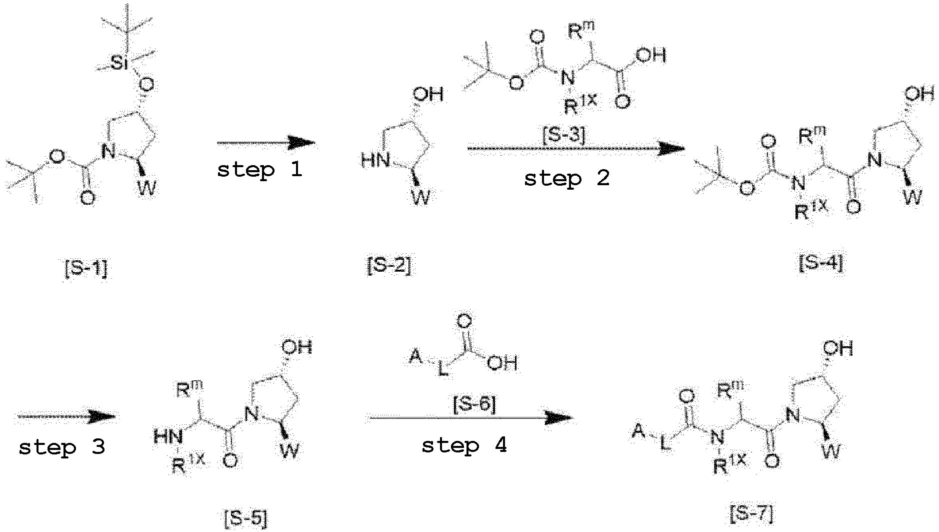 wherein each symbol is as defined above.

The formula [S-2] is obtained by deprotection of the formula [S-1] with an acid. The reaction proceeds using an appropriate acid in an appropriate solvent generally at room temperature to the refluxing temperature of the solvent. While the reaction time varies depending on the starting materials and solvents to be used and the reaction temperature, it is generally from 30 min to 24 hr. Examples of the acid include hydrochloric acid, trifluoroacetic acid, PTSA, PPTs, and the like. Examples of the solvent include 1,4-dioxane, dichloromethane, ethyl acetate, methanol, toluene, and the like.

The formula [S-4] is obtained by a condensation reaction of the formula [S-2] and the formula [S-3]. The reaction proceeds using a condensing agent in the presence of a suitable base in a suitable solvent at 0°C to the refluxing temperature of the solvent. The reaction time and the condensing agent, solvent, reaction promoter, and base to be used are the same as those in Synthesis method N.

The formula [S-5] is obtained by deprotection of the formula [S-4] with an acid. The reaction proceeds using an appropriate acid in an appropriate solvent generally from room temperature to the refluxing temperature of the solvent. The reaction time and the condensing agent, solvent, reaction promoter, and base to be used are the same as those in step 1 of Synthesis method N.

The formula [S-7] is obtained by a condensation reaction of the formula [S-5] and the formula [S-6]. The reaction proceeds using a condensing agent in the presence of a suitable base in a suitable solvent at 0°C to the refluxing temperature of the solvent. The reaction time and the condensing agent, solvent, reaction promoter, and base to be used are the same as those in Synthesis method N.

### Synthesis method T

The production method described here is suitable for producing a compound represented by the formula (I) wherein E is substituted by -CO-, i.e., the following compound [T-3]. 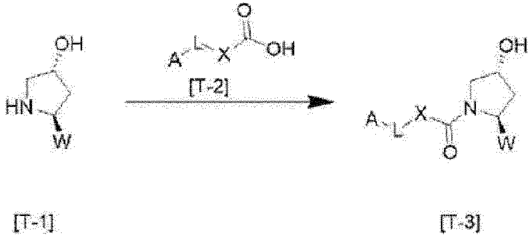 wherein each symbol is as defined above.

The formula [T-3] is obtained by a condensation reaction of the formula [T-1] and the formula [T-2]. The reaction proceeds using a condensing agent in the presence of a suitable base in a suitable solvent at 0°C to the refluxing temperature of the solvent. The reaction time and the condensing agent, solvent, reaction promoter, and base to be used are the same as those in Synthesis method N.

### Synthesis method U

The production method described here is suitable for producing a compound represented by the formula (I) wherein a connection part with a chemical linker is -CONR^{L1}-, i.e., the following compound [U-3]. 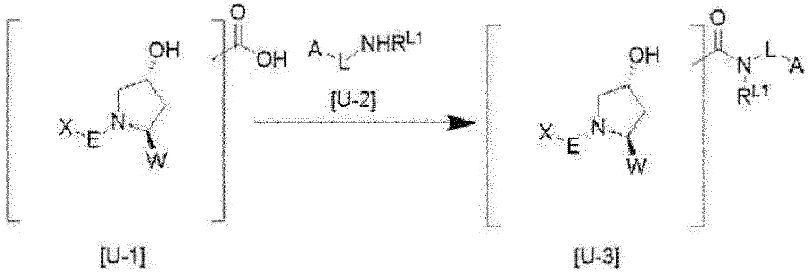 wherein each symbol is as defined above.

The formula [U-3] is obtained by a condensation reaction of the formula [U-1] and the formula [U-2]. The reaction proceeds using a condensing agent in the presence of a suitable base in a suitable solvent at 0°C to the refluxing temperature of the solvent. The reaction time and the condensing agent, solvent, reaction promoter, and base to be used are the same as those in Synthesis method N.

### Synthesis method V

The production method described here is suitable for producing a compound represented by the formula (I) wherein a connection part with a chemical linker is -O-,i.e., the following compound [V-3]. 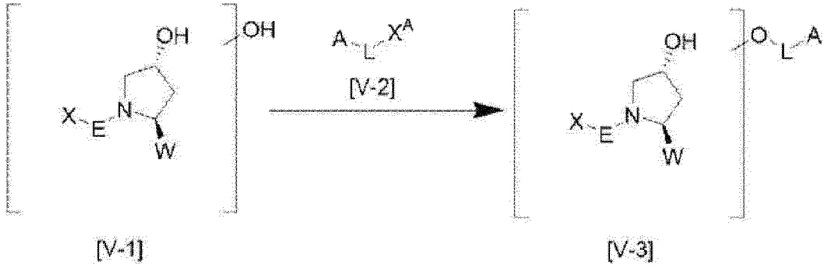 wherein each symbol is as defined above.

The formula [V-3] is obtained by an alkylation reaction of the formula [V-1] and the formula [V-2]. The reaction proceeds using a suitable base in a suitable solvent at 0°C to the refluxing temperature of the solvent. The reaction time and the base, solvent, and reaction promoter to be used are the same as those in Synthesis method O.

### Synthesis method W

The production method described here is suitable for producing a compound represented by the formula (I) wherein a connection part with a chemical linker is -S-, i.e., the following compound [W-3]. 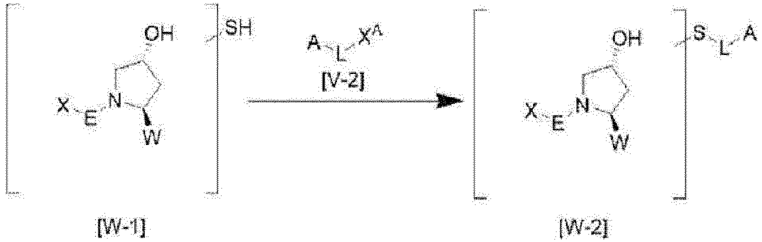 wherein each symbol is as defined above.

The formula [W-2] is obtained by an alkylation reaction of the formula [W-1] and the formula [V-2]. The reaction proceeds using a suitable base in a suitable solvent at 0°C to the refluxing temperature of the solvent. The reaction time and the base, solvent, and reaction promoter to be used are the same as those in Synthesis method O.

### Synthesis method X

The production method described here is suitable for producing a compound represented by the formula (I) wherein a connection part with a chemical linker is -NR^{L1}- or -NR^{L1}CO-, i.e., the following compound [X-3] or compound [X-4]. 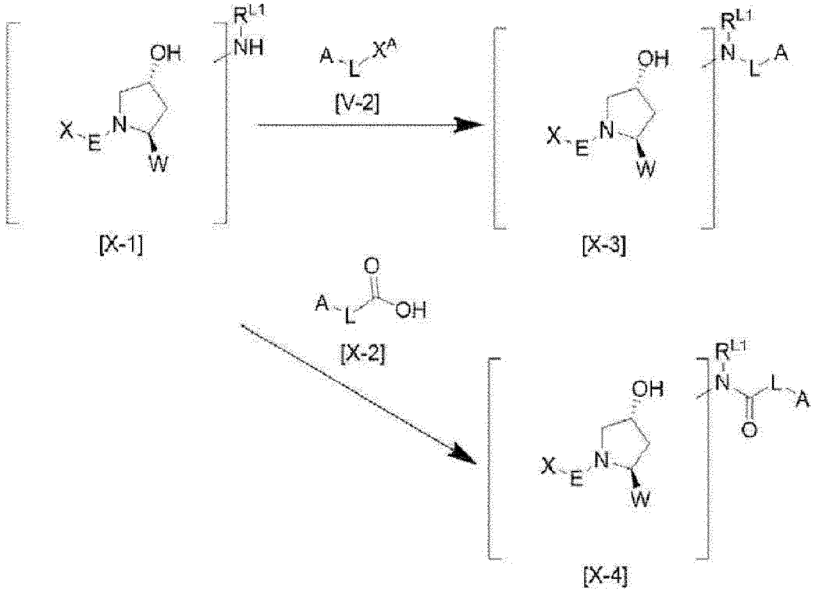 wherein each symbol is as defined above.

The formula [X-3] is obtained by an alkylation reaction of the formula [X-1] and the formula [V-2]. The reaction proceeds using a suitable base in a suitable solvent at 0°C to the refluxing temperature of the solvent. The reaction time and the base, solvent, and reaction promoter to be used are the same as those in Synthesis method O.

The formula [X-4] is obtained by a condensation reaction of the formula [X-1] and the formula [X-2]. The reaction proceeds using a condensing agent in the presence of a suitable base in a suitable solvent at 0°C to the refluxing temperature of the solvent. The reaction time and the condensing agent, solvent, reaction promoter, and base to be used are the same as those in Synthesis method N.

The starting material compounds in the above-mentioned methods can be produced by known methods and/or in the same manner as in the methods described in Examples described later.

The introduction of protecting groups into functional groups and the removal of functional group protecting groups can be performed by reference to a known method (PROTECTIVE GROUPS in ORGANIC SYNTHESIS (Theodora W. Greene, Peter G.M. Wuts) etc.).

In addition, the compound of the present invention and intermediate compounds produced by the above-mentioned methods can be structurally converted to other compound of interest, or intermediate by the method described in Example described later and/or known methods or combination thereof.

A compound represented by the formula (I), which is produced by the aforementioned method, can be purified to any purity by a conventionally used purification means, for example, concentration, extraction, chromatography, reprecipitation, recrystallization, and the like. It can be converted to a pharmacologically acceptable salt as necessary by treating with an acid or a base etc. in a suitable solvent (water, alcohol, ether, etc.). Furthermore, the obtained compound of the present invention or a pharmacologically acceptable salt thereof can be converted to hydrate or solvate by treating with water, water-containing solvent or other solvent.

The compound and a pharmacologically acceptable salt thereof of the present invention include racemic compounds, stereoisomers, and mixture of these compounds, and includes isotope-labeled and radioactive-labeled compounds. Such isomers can be isolated by a standard separation technique including fractional crystallization and chiral column chromatography. In addition, the compound of the present invention has an asymmetric carbon atom. Therefore, it includes enantiomer and diastereomer. A diastereomer mixture can be separated into each diastereomer based on their physical/chemical differences by a method well known in the art, for example, chromatography and/or fractional crystallization. Enantiomer can be separated by chiral column chromatography or by reacting an enantiomer compound with an appropriate optically active compound to give a diastereomer mixture, separating each diastereomer and converting each diastereomer to a corresponding enantiomer. The compound of the present invention may be any of such isomers including diastereomer, enantiomer, and a mixture thereof.

### [Use of the target protein degradation-inducing compound of the present invention]

The compound of the present invention has low toxicity (e.g., acute toxicity, chronic toxicity, genetic toxicity, reproductive toxicity, cardiotoxicity, carcinogenicity), and can be used as a medicament for the prophylaxis or treatment of diseases caused by dysregulation of protein activity in a mammal (e.g., mouse, rat, hamster, rabbit, cat, dog, bovine, sheep, monkey, human, etc.).

While the diseases caused by dysregulation of protein activity are not limited, for example, asthma, multiple sclerosis, cancer, cilium associated disease, cleft palate, diabetes, cardiac disease, hypertension, inflammatory bowel disease, mental retardation, mood disorder, obesity, refractive error, infertility, Angelman syndrome, Canavan disease, coeliac disease, Charcot-Marie-Tooth disease, cystic fibrosis, Duchenne muscular dystrophy, haemochromatosis, haemophilia, Klinefelter's syndrome, neurofibromatosis, phenylketonuria, polycystic kidney disease, (PKD1) or 4(PKD2) Prader-Willi syndrome, sickle-cell disease, Tay-Sachs disease, Turner's syndrome;
Alzheimer's disease, amyotrophic lateral sclerosis (Lou Gehrig's disease), anorexia nervosa, anxiety disorder, atherosclerosis, attention deficit hyperactivity disorder, autism, bipolar disorder, chronic fatigue syndrome, chronic obstructive pulmonary diseases, Crohn's disease, coronary heart disease, dementia, depression, diabetes mellitus type 1, diabetes mellitus type 2, epilepsy, Guillain-Barre syndrome, irritable bowel syndrome, lupus, metabolic syndrome, multiple sclerosis, myocardial infarction, obesity, obsessive-compulsive disorder, panic disorder, Parkinson's disease, psoriasis, rheumatoid arthritis, sarcoidosis, schizophrenia, stroke, thromboangiitis obliterans, Tourette's syndrome, Vasculitis;
aceruloplasminemia, achondrogenesis type II, achondroplasia, acrocephaly, Gaucher's disease type 2, acute intermittent porphyria, Canavan disease, adenomatous polyposis coli, ALA dehydratase deficiency, adenylosuccinate lyase deficiency, adrenogenital syndrome, adrenoleukodystrophy, ALA-D porphyria, ALA dehydratase deficiency, alkaptonuria, Alexander's disease, alkaptonuric ochronosis, Alpha 1-antitrypsin deficiency, Alpha-1 proteinase inhibitor, emphysema, amyotrophic lateral sclerosis, Alström's syndrome, Alexander's disease, amelogenesis imperfecta, ALA dehydratase deficiency, Anderson-Fabry disease, androgen insensitivity syndrome, anemia, angiokeratoma corporis diffusum, angiomatosis retinae (von Hippel-Lindau disease), Apert syndrome, arachnodactyly (Marfan's syndrome), Stickler's syndrome, arthrochalasis multiplex congenital (Ehlers-Danlos syndrome # arthrochalasia type), telangiectatic ataxia, Rett syndrome, primary pulmonary hypertension, Sandhoff's disease, neurofibromatosis type II, Beare-Stevenson cutis gyrata syndrome, Mediterranean fever, familial, Benjamin syndrome, beta-thalassemia, bilateral acoustic neurofibromatosis (neurofibromatosis type II), Factor V Leiden thrombophilia, Bloch-Sulzberger syndrome (incontinentia pigmenti), Bloom's syndrome, X-linked sideroblastic anemia, Bonnevie-Ullrich syndrome (Turner syndrome), Bourneville's disease (tuberous sclerosis), Birt-Hogg-Dube syndrome, osteoporosis (osteogenesis imperfecta), Broad Thumb-Hallux syndrome (Rubinstein-Taybi syndrome), bronze diabetes/bronzed cirrhosis (hemochromatosis), Bulbospinal muscular atrophy (Kennedy's disease), Buerger-Gruetz syndrome (lipoprotein lipase deficiency), CGD chronic granulomatosis, Campomelic dysplasia, biotinidase deficiency, cardiomyopathy (Noonan syndrome), Cri-du-Chat syndrome, CAVD (congenital absence of the vas deferens), Caylor cardiofacial syndrome (CBAVD), CEP (Congenital erythropoietic porphyria), cystic fibrosis, congenital hypothyroidism, Chondrodystrophy syndromes (achondroplasia), otospondylomegaepiphyseal dysplasia, Lesch-Nyhan syndrome, galactosemia, Ehlers-Danlos syndrome, thanatophoric dysplasia, Coffin-Lowry syndrome, Cockayne syndrome, familial adenomatous polyposis, Congenital erythropoietic porphyria, congenital heart disease, methemoglobinemia/congenital methemoglobinemia, achondroplasia, X-linked sideroblastic anemia, connective tissue disease, Conotruncal anomaly face syndrome, Cooley's anemia (beta-thalassemia), copper storage disease(Wilson's disease), copper transport disease (Menkes disease), hereditary coproporphyria, Cowden syndrome, Craniofacial dysarthrosis (Crouzon syndrome), Creutzfeldt-Jakob disease (prion disease), Cowden syndrome, Curschmann-Batten-Steinert syndrome (myotonic dystrophy), Beare-Stevenson cutis gyrata syndrome, primary hyperoxaluria, Spondyloepimetaphyseal dysplasia (Strudwick type), muscular dystrophy, Duchenne and Becker types (DBMD), Usher syndrome, degenerative neurological diseases including de Grouchy syndrome and Dejerine-Sottas syndrome, developmental disability, distal spinal muscular atrophy, type V, androgen insensitivity syndrome, diffuse globoid body sclerosis (Krabbe disease), DiGeorge syndrome, dihydrotestosterone receptor deficiency, androgen insensitivity syndrome, Down syndrome, dwarfism, erythropoietic protoporphyria, erythroid 5-aminolevulinate synthase deficiency, erythropoietic porphyria, erythropoietic protoporphyria, erythropoietic uroporphyria, Friedreich's ataxia, familial paroxysmal polyserositis, porphyria cutanea tarda, familial pressure-sensitive neuropathy, primary pulmonary hypertension (PPH), pancreas fibrocystic disease, fragile X syndrome, galactosemia, genetic brain disorder, giant cell hepatitis (neonatal hemochromatosis), Gronblad-Strandberg syndrome (pseudoxanthoma elasticum), Gunther's disease (congenital erythropoietic porphyria), hemochromatosis, Hallgren's syndrome, sickle cell anemia, hemophilia, hepatoerythropoietic porphyria (HEP), Hippel-Lindau disease (von Hippel-Lindau disease), Huntington's disease, Hutchinson-Gilford progeria syndrome (progeria), hyperandrogenism, hypochondroplasia, hypochromic anemia, immune system disorders including X-linked severe combined immunodeficiency, Insley-Astley syndrome, Jackson-Weiss syndrome, Joubert syndrome, Lesch-Nyhan syndrome, renal diseases including hyperoxaluria, Klinefelter's syndrome, Kniest dysplasia, lacunar dementia, Langer-Saldino achondrogenesis, ataxia telangiectasia, Lynch syndrome, lysyl-hydroxylase deficiency, Machado-Joseph disease, metabolic disorders including Kniest dysplasia, Marfan syndrome, movement disorder, Mowat-Wilson syndrome, cystic fibrosis, Muenke syndrome, multiple neurofibromatosis, Nance-Insley syndrome, Nance-Sweeney chondrodysplasia, Niemann-Pick disease, Noack syndrome (Pfeiffer syndrome), Osler-Weber-Rendu disease, Peutz-Jeghers syndrome, polycystic kidney disease, polyostotic fibrous dysplasia (McCune-Albright syndrome), Prader-Labhart-Willi syndrome, hemochromatosis, primary hyperuricemia syndrome (Lesch-Nyhan syndrome), primary pulmonary hypertension, primary senile degenerative dementia, progressive chorea, chronic hereditary (Huntington) (Huntington's disease), progressive muscular atrophy, spinal muscular atrophy, propionic acidemia, protoporphyria, proximal myotonic dystrophy, pulmonary arterial hypertension, PXE (pseudoxanthoma elasticum), Rb (retinoblastoma), Recklinghausen disease (neurofibromatosis type 1), Recurrent polyserositis, retinal disorder, retinoblastoma, Rett syndrome, RFALS type 3, Ricker syndrome, Riley-Day syndrome, Roussy-Levy syndrome, severe achondroplasia with developmental delay and acanthosis nigricans (SADDAN), Li-Fraumeni syndrome, sarcoma, breast, leukemia, and adrenal gland (SBLA) syndrome, tuberous sclerosis, SDAT, congenital SED (spondyloepiphyseal dysplasia congenita), SED Strudwick (spondyloepimetaphyseal dysplasia, Strudwick type), SEDc (spondyloepiphyseal dysplasia congenita) SEMD, Strudwick type (spondyloepimetaphyseal dysplasia, Strudwick type), Shprintzen syndrome, skin pigmentation disorder, Smith-Lemli-Opitz syndrome, South-African genetic porphyria (variegate porphyria), infantile-onset ascending hereditary spastic paralysis, speech and communication disorder, sphingolipidosis, Tay-Sachs disease, spinocerebellar ataxia, Stickler syndrome, stroke, androgen insensitivity syndrome, tetrahydrobiopterin deficiency, beta-thalassemia, thyroid gland disease, Tomaculous neuropathy (hereditary neuropathy with liability to pressure palsies), Treacher Collins syndrome, Triple X syndrome (triple X syndrome), trisomy 21 (Down syndrome), trisomy X, VHL syndrome (von Hippel-Lindau disease), vision impairment and blindness (Alstrom syndrome), Vrolik disease, Waardenburg syndrome, micro syndrome (Warburg Sjo Fledelius syndrome), Weissenbacher-Zweymuller syndrome, Wolf-Hirschhorn syndrome, Wolff Periodic disease, Weissenbacher-Zweymuller syndrome and xeroderma pigmentosum;
virus infectious disease such as HIV, HCV and the like, and the like can be mentioned.

In the present specification, the "prophylaxis" includes preventing the onset of a disease (all or one or more pathologies) and delaying the onset of the disease. The "prophylactically effective amount" refers to a dose of compound (I) sufficient to achieve such purpose.

In the present specification, the "treatment" includes curing a disease (all or one or more pathologies), improving the disease, and suppressing the progression of the severity of the disease. The "therapeutically effective amount" refers to a dose of compound (I) sufficient to achieve such purpose.

In practicing the present invention, the compound of the present invention (compound (I) or a pharmacologically acceptable salt thereof) can be used either in a single form, or in the form of a pharmaceutical composition containing the compound of the present invention as an active ingredient, together with a pharmaceutically acceptable carrier.

Examples of such pharmaceutical composition include tablet (including sugar-coated tablet, film-coated tablet, sublingual tablet, orally disintegrating tablet, buccal tablet, and the like), pill, powder, granule, capsule (including soft capsule and microcapsule), syrup, liquid, emulsion, suspension, controlled-release preparation (e.g., immediate-release preparation, sustained-release preparation, sustained-release microcapsule), aerosol, films (e.g., orally disintegrable films, mouth cavity mucosa pasting film), injection (e.g., subcutaneous injection, intravenous injection (e.g., bolus), intramuscular injection, intraperitoneal injection), drip transfusion, transdermal absorption type preparation, ointment, lotion, adhesive preparation, suppository (e.g., rectal suppository, vaginal suppository), pellet, nasal preparation, pulmonary preparation (inhalant), eye drop, and the like.

In the present specification, as the "pharmaceutically acceptable carrier", various carriers conventionally used in the field of formulation technology can be used.

Specific examples of the "pharmaceutically acceptable carrier" for solid preparations include excipient (e.g., lactose, sucrose, D-mannitol, starch, cornstarch, crystalline cellulose, light anhydrous silicic acid, etc.), lubricant (e.g., magnesium stearate, talc, colloid silica, etc.), binder (e.g., crystalline cellulose, sucrose, D-mannitol, dextrin, hydroxypropylcellulose, hydroxypropylmethylcellulose, polyvinylpyrrolidone, starch, sucrose, gelatin, methylcellulose, carboxymethylcellulose sodium, etc.), disintegrant (e.g., starch, carboxymethylcellulose, carboxymethylcellulose calcium, sodium carboxymethyl starch, L-hydroxypropylcellulose etc.), and the like.

For liquid preparations, solvent (e.g., water for injection, isotonic brine, alcohol, propylene glycol, macrogol, sesame oil, etc.), solubilizing agent (e.g., polyethylene glycol, propylene glycol, D-mannitol, benzoic acid benzyl, ethanol, triethanolamine, sodium carbonate, sodium citrate, etc.), suspending agent (e.g., surfactants such as stearyltriethanolamine, sodium lauryl sulfate, lauryl aminopropionic acid, lecithin, benzalkonium chloride, mono stearic acid glycerol, and the like; hydrophilic polymers such as poly(vinyl alcohol), polyvinylpyrrolidone, carboxymethylcellulose sodium, methylcellulose, hydroxymethylcellulose, hydroxypropylcellulose, and the like), isotonicity agent (e.g., glucose, D-Sorbitol, sodium chloride, glycerol, D-mannitol, etc.), buffering agent (e.g., buffers such as phosphate, citrate, and the like), soothing agent (e.g., benzyl alcohol, etc.), and the like can be used.

Where necessary, preparation additives such as antiseptic (e.g., p-hydroxybenzoic acid esters, chlorobutanol, benzyl alcohol, sorbic acid, etc.), antioxidant (e.g., sulfite, ascorbic acid, α-tocopherol, etc.), colorant, sweetening agent, and the like may be further added.

The pharmaceutical composition of the present invention can be produced by adding the compound of the present invention in a proportion of 0.01 to 99% (w/w), preferably 0.1 to 85% (w/w), based on the total amount of the preparation, though subject to change depending on the dosage form, administration method, carrier, and the like. The pharmaceutical composition can be produced by a method conventionally used in the field of formulation technology, according to its form. The pharmaceutical composition of the present invention may be formulated into a sustained-release preparation containing the active ingredient.

### (Subject of administration)

The compound of the present invention can be expected to have low toxicity and few side effects, and also has superior properties as a pharmaceutical product. Therefore, the compound of the present invention can be safely administered to mammals (particularly human).

### (Administration route)

In practicing the present invention, the compound of the present invention may be administered either alone or as a pharmaceutical composition orally or parenterally (e.g., intravenous, intramuscular, subcutaneous, intraorgan, intranasal, intradermal, ophthalmic, intracerebral, intrarectal, intravaginal, intraperitoneal administrations, and administration to lesion).

### (Dose)

The dose of the compound of the present invention varies depending on the administration subject, administration route, and the age and symptoms of the administration subject, and is not particularly limited. For example, the dose of the compound of the present invention is 1 to 100 mg per dose for oral administration, and 0.1 to 1000 mg per dose for parenteral administration.

### (Use as prodrug)

The compound (I) may also be used in the form of a prodrug thereof. A prodrug of the compound (I) means a compound which is converted to the compound (I) of the present invention with a reaction due to an enzyme, an gastric acid, etc. under the physiological condition in the living body, that is, a compound which is converted to the compound (I) of the present invention with oxidation, reduction, hydrolysis, etc. according to an enzyme; a compound which is converted to the compound (I) of the present invention by hydrolysis etc. due to gastric acid, etc. A prodrug for compound (I) may be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound (I) to an acylation, alkylation, phosphorylation or boration (e.g., a compound obtained by subjecting an hydroxy group in compound (I) to an acetylation, palmitoylation, propanoylation, pivaloylation, succinylation, fumarylation, alanylation, dimethylaminomethylcarbonylation, etc.); a compound obtained by subjecting a carboxyl group in compound (I) to an esterification or amidation (e.g., a compound obtained by subjecting a carboxyl group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation, etc.) and the like. These compounds can be produced from compound (I) by a method known per se.

A prodrug for compound (I) may also be one which is converted to compound (I) under physiological conditions, such as those described in IYAKUHIN no KAIHATSU (Development of Pharmaceuticals), Vol. 7, Design of Molecules, p.163-198, Published by HIROKAWA SHOTEN (1990).

### (Combined use with other drugs)

As described above, the compound of the present invention has extremely low toxicity, can be used in combination with other medicaments for the prophylaxis or treatment of target diseases, and is expected to exhibit superior prophylactic and/or therapeutic effects in combination with other medicaments. Such combination therapy is also expected to lower the dose of other medicaments and reduce the side effects they have.

Such medicament that can be used in combination with the compound of the present invention (hereinafter to be abbreviated as concomitant drug) can be appropriately selected in consideration of the type of disease of the patients, the severity of its symptoms, and the like.

The administration mode of the concomitant drug is not particularly limited, and the compound of the present invention and the concomitant drug may be combined at the time of administration. For example, they can be used in the administration modes of (1) administration of a preparation containing the compound of the present invention and the concomitant drug in combination, (2) simultaneous or separate administration of two kinds of preparations of the compound of the present invention and the concomitant drug, which have been separately produced, by the same administration route, (3) simultaneous or separate administration of two kinds of preparations of the compound of the present invention and the concomitant drug, which have been separately produced, by different administration routes, and the like. A preferable form, according to the medical practice, can be appropriately selected.

A preparation containing the above-mentioned compound of the present invention and a concomitant drug in combination can be appropriately produced by those of ordinary skill in the art according to the pharmaceutical composition described above containing the compound of the present invention.

The dose of the concomitant drug can be appropriately determined based on the dose employed in clinical situations. The mixing ratio of the compound of the present invention and a concomitant drug can be appropriately determined depending on the disease and symptoms of the administration subject, administration route, the kind of the concomitant drug to be used, and the like. Generally, it can be appropriately determined based on the general clinical dose of the concomitant drug to be used and according to the actual situation in medical practice.

### [Example]

The present invention is explained in more detail in the following by referring to Reference Examples and Examples relating to the synthesis of compound (I), as well as Experimental Examples relating to the pharmacological activity of compound (I). However, these are only examples, and the present invention is not limited to these.

The meanings of the following notations attached to the structural formulas in the table describing the "structural formulas of Reference Example compounds" (Table 1) and the table describing the "structural formulas of Example compounds" (Table 2) described later are explained.

The notation "or 1" indicates that the carbon atom to which it is attached has been identified to have a single steric configuration, but the absolute steric configuration thereof has not been identified.

The notation "wavy line" indicates that in a compound having two or more asymmetric carbon atoms, the stereochemistry of the carbon atom to which it is attached is a mixture of R form and S form.

The notation "solid line" (bonded to asymmetric carbon atom)" indicates a racemate.

Representative abbreviations used in the description of the following Reference Examples and Examples are explained.

### (Abbreviation table)

BH3·THF: tetrahydrofuran-borane
Boc: t-butoxycarbonyl
Boc2O: di-tert-butyl dicarbonate
Cu: copper
CuSO4: Copper(II) Sulfate
DBU: 1,8-diazabicyclo[5.4.0]-7-undecene
DCM: dichloromethane
DIBAL-H: diisobutylaluminum hydride
DIPEA: N,N-diisopropyl ethylamine
DMAP: 4-dimethylaminopyridine
DME: 1,2-dimethoxyethane
DMF: N,N-dimethylformamide
DMSO: dimethyl sulfoxide
DMT -MM: 1-[bis(dimethylamino)methylene]-1H-1,2,3-triazolo[4,5-b]pyridinium 3-oxide hexafluorophosphate (HATU), 4-(4,6-dimethoxy[1.3.5]triazin-2-yl)-4-methylmorpholinium chloride hydrate
Et3N: triethylamine
ESI: Electrospray ionization
g: gram
HATU: O-(7-azabenzotriazol-1-yl)-N,N,N',N',-tetramethyluronium hexafluorophosphate
HOAt: 1-hydroxy-7-azabenzotriazole
HOBt: 1-hydroxybenzotriazole
HPLC: high performance liquid chromatography
IPE: diisopropyl ether
M: mol concentration
MeOH: methyl alcohol
mmol: millimol
MS: mass spectrometry
n-: normal (non-branched)
Na2SO4: anhydrous sodium sulfate
NaI: sodium iodide
NBS: N-bromosuccinimide
NCS: N-chlorosuccinimide
NIS: N-iodosuccinimide
NMP: N-methyl-2-pyrrolidone
NMR: nuclear magnetic resonance
p-: para
Pd(dppf)Cl2·CH2Cl2: [1,1'-bis(diphenylphosphino)ferrocene]palladium(II) dichloride dichloromethane adduct
PdCl2(AmPhos)2: dichlorobis[di-tert-butyl(4-dimethylaminophenyl)phosphine]palladium(II) PdCl2(PPh3)2: dichlorobis(triphenylphosphine)palladium(II) pH: -log[H+]
PPTs: pyridinium p-toluenesulfonate
PTSA: p-toluenesulfonic acid
RuPhos: 2-dicyclohexylphosphino-2',6'-diisopropoxy -1,1'-biphenyl
SNAr: aromatic nucleophilic substitution reaction TBAI: tetrabutylammonium iodine
TBS: tert-butyldimethylsilyl
t-BuOH: tert-butyl alcohol
tert-: tertiary
THF: tetrahydrofuran
WSC·HCl: 1-ethyl-3-(3-dimethylaminopropyl)carbodiimide hydrochloride
XantPhos: 4,5-bis(diphenylphosphino)-9,9-dimethylxanthene XPhos Pd G2: chloro(2-dicyclohexylphosphino-2',4',6'-triisopropyl-1,1'-biphenyl)[2-(2'-amino-1,1'-
biphenyl)]palladium(II)
Zn(OAc)2: zinc acetate

### [Reference Example]

### Reference Example 1: Synthesis of tert-butyl (2S,4R)-4-[tert-butyl(dimethyl)silyl]oxy-2-[4-(trifluoromethyl)-1H-imidazol-2-yl]pyrrolidine-1-carboxylate

3,3-Dibromo-1,1,1-trifluoro-propan-2-one (5 g) was dissolved in water (61.8 mL), sodium acetate (4.4 g) was added at room temperature, and the mixture was stirred at 100°C for 30 min. After completion of the reaction, the reaction solution was added to a mixed solution of tert-butyl (2S,4R)-4-[tert-butyl(dimethyl)silyl]oxy-2-formylpyrrolidine-1-carboxylate (3 g), methanol (152 mL), and 7 M ammonia-methanol (26 mL) at 0°C, and the mixture was stirred at room temperature overnight. After completion of the reaction, the reaction solution was concentrated under reduced pressure, water was added, and the mixture was extracted with ethyl acetate. The organic layer was washed with saturated brine, and dried over anhydrous sodium sulfate. The organic layer was filtered, and the filtrate was concentrated under reduced pressure. The obtained residue was purified by silica gel column chromatography (hexane:ethyl acetate=85:15) to give the title compound (3.4 g) as a colorless powder. MS(ESI)m/z:436.4[M+H]+

### Reference Example 2: Synthesis of tert-butyl (2S,4R)-4-[tert-butyl(dimethyl)silyl]oxy-2-[4-(trimethoxymethyl)-1H-imidazol-2-yl]pyrrolidine-1-carboxylate

tert-Butyl (2S,4R)-4-[tert-butyl(dimethyl)silyl]oxy-2-[4-(trifluoromethyl)-1H-imidazol-2-yl]pyrrolidine-1-carboxylate (2.2 g) described in Reference Example 1 was dissolved in methanol (12 mL), 5 M sodium methoxide-methanol (3.6 mL) was added, and the mixture was stirred under microwave radiation at 100°C for 10 min. After completion of the reaction, the reaction solution was concentrated under reduced pressure, water was added, and the mixture was extracted with ethyl acetate. The organic layer was washed with saturated brine, and dried over anhydrous sodium sulfate. The organic layer was filtered, and the filtrate was concentrated under reduced pressure to give the title compound (2.3 g) as a brown powder. MS(ESI)m/z:470.4[M-H]-

### Reference Example 3: Synthesis of methyl 2-[(2S,4R)-4-[tert-butyl(dimethyl)silyl]oxy-1-[(2-methylpropan-2-yl)oxycarbonyl]pyrrolidin-2-yl]-1H-imidazole-5-carboxylate

To a solution of tert-butyl (2S,4R)-4-[tert-butyl(dimethyl)silyl]oxy-2-[4-(trifluoromethyl)-1H-imidazol-2-yl]pyrrolidine-1-carboxylate (1312 mg) described in Reference Example 1 in methanol (6.0 mL) was added 5 M sodium methoxide-methanol solution (2100 µL), and the mixture was stirred under microwave radiation at 100°C for 10 min. After cooling to room temperature, water and saturated brine were added and the mixture was extracted with ethyl acetate, dried over anhydrous sodium sulfate, filtered, and concentrated under reduced pressure. To the obtained residue were added DCM (30 mL) and trifluoroacetic acid (7.5 mL), and the mixture was stirred at room temperature for 3 hr. After cooling to -15°C, 1 M sodium hydroxide aqueous solution (88 mL) was added dropwise over 8 min. After the completion of the dropwise addition, the mixture was allowed to warm to room temperature and adjusted to around pH=8 by adding sodium hydrogen carbonate (1680 mg). Ethyl acetate (30 mL) and Boc2O (1047 mg) were added, and the mixture was stirred at room temperature for 2.5 hr. After completion of the reaction, the reaction mixture was extracted with ethyl acetate, dried over anhydrous sodium sulfate, filtered, and concentrated under reduced pressure. The obtained residue was purified by silica gel column chromatography (hexane:ethyl acetate=76:24 - 55:45) to give the title compound (1033 mg) as a pale-red powder. MS(ESI)m/z:426.3[M+H]+

### Reference Example 4: Synthesis of 2-[(2S,4R)-4-hydroxy-1-[(2-methylpropan-2-yl)oxycarbonyl]pyrrolidin-2-yl]-1H-imidazole-4-carboxylic acid

Methyl 2-[(2S,4R)-4-[tert-butyl(dimethyl)silyl]oxy-1-[(2-methylpropan-2-yl)oxycarbonyl]pyrrolidin-2-yl]-1H-imidazole-5-carboxylate (528 mg) described in Reference Example 3 was dissolved in methanol (5 mL), 1 M sodium hydroxide aqueous solution (6.2 mL) was added and the mixture was stirred at 50°C for 3 days. After completion of the reaction, 1 M hydrochloric acid (5 mL) was added and the reaction solution was concentrated under reduced pressure. The reaction solution was adsorbed onto PoraPak Rxn RP 60cc, and washed with water (60 mL). Then it was eluted with methanol (60 mL), and the eluate was concentrated under reduced pressure, and azeotropically distilled with ethanol and toluene in this order to give the title compound (299 mg) as a white powder.
MS(ESI)m/z:298.1[M+H]+

### Reference Example 5: Synthesis of tert-butyl (2S,4R)-4-hydroxy-2-[4-[methyl-[[4-(4-methyl-1,3-thiazol-5-yl)phenyl]methyl]carbamoyl]-1H-imidazol-2-yl]pyrrolidine-1-carboxylate

2-[(2S,4R)-4-Hydroxy-1-[(2-methylpropan-2-yl)oxycarbonyl]pyrrolidin-2-yl]-1H-imidazole-4-carboxylic acid (140 mg) described in Reference Example 4 was dissolved in N-methylpyrrolidone (940 µL), HATU (179 mg) was added at room temperature and the mixture was stirred for 20 min. A mixed solution of N-methyl-1-[4-(4-methyl-1,3-thiazol-5-yl)phenyl]methanamine (154 mg), diisopropyl amine (240 µL), and N-methylpyrrolidone (470 µL) was added at room temperature and the mixture was stirred for about 17 hr. At room temperature, HATU (56 mg) was added and the mixture was stirred for about 3 hr. After completion of the reaction, water and dimethyl sulfoxide were added, and the mixture was purified by preparative HPLC (10 mM ammonium carbonate aqueous solution-acetonitrile) to give the title compound (133 mg) as a pale-yellow viscous substance. MS(ESI)m/z:496.5[M-H]-

### Reference Example 6: Synthesis of 2-[(2S,4R)-4-hydroxypyrrolidin-2-yl]-N-methyl-N-[[4-(4-methyl-1,3-thiazol-5-yl)phenyl]methyl]-1H-imidazole-4-carboxamide hydrochloride

tert-Butyl (2S,4R)-4-hydroxy-2-[4-[methyl-[[4-(4-methyl-1,3-thiazol-5-yl)phenyl]methyl]carbamoyl]-1H-imidazol-2-yl]pyrrolidine-1-carboxylate (133 mg) described in Reference Example 5 was dissolved in methanol (1.1 mL), 4 M hydrochloric acid-dioxane (284 µL) was added at room temperature, and the mixture was stirred overnight. After completion of the reaction, the reaction solution was concentrated under reduced pressure to give the title compound (122 mg) as a pale-brown viscous substance. MS(ESI)m/z:398.2[M+H]+

### Reference Example 7: Synthesis of methyl 2-[(2S,4R)-4-hydroxy-1-[2-(3-methoxy-1,2-oxazol-5-yl)-3-methylbutanoyl]pyrrolidin-2-yl]-1H-imidazole-4-carboxylate

Methyl 2-[(2S,4R)-4-[tert-butyl(dimethyl)silyl]oxy-1-[(2-methylpropan-2-yl)oxycarbonyl]pyrrolidin-2-yl]-1H-imidazole-5-carboxylate (150 mg) described in Reference Example 3 was dissolved in 1,4-dioxane (4 mL), 4 M hydrochloric acid-dioxane (6.2 mL) was added at room temperature and the mixture was stirred for 5 hr. After completion of the reaction, the reaction solution was concentrated under reduced pressure, and azeotropically distilled 3 times with toluene. To the obtained residue were added DMF (4 mL), 2-(3-methoxy-1,2-oxazol-5-yl)-3-methylbutanoic acid (77 mg), diisopropyl ethylamine (183 µL), and HATU (174 mg), and the mixture was stirred at room temperature for 1 hr. After completion of the reaction, saturated brine was added, and the mixture was extracted with ethyl acetate. The organic layer was washed with saturated brine, and dried over anhydrous sodium sulfate. The organic layer was filtered, and the filtrate was concentrated under reduced pressure. The obtained residue was purified by silica gel chromatography (ethyl acetate:methanol=100:0 - 95:5). The obtained residue was dissolved in ethyl acetate, washed with saturated brine, and dried over anhydrous sodium sulfate. The organic layer was filtered, and the filtrate was concentrated under reduced pressure to give the title compound (98 mg) as a pale-yellow powder. MS(ESI)m/z:393.4[M+H]+

### Reference Example 8: Synthesis of 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid

Methyl 2-[(2S,4R)-4-hydroxy-1-[2-(3-methoxy-1,2-oxazol-5-yl)-3-methylbutanoyl]pyrrolidin-2-yl]-1H-imidazole-4-carboxylate (266 mg) described in Reference Example 7 was dissolved in methanol (6.8 mL), 1 M sodium hydroxide aqueous solution (6 mL) was added and the mixture was stirred at 60°C for 19 hr. After completion of the reaction, 1 M hydrochloric acid (6 mL) was added, and the reaction solution was concentrated under reduced pressure. The reaction solution was adsorbed onto PoraPak Rxn RP 60cc, and washed with water. It was then eluted with methanol, and the eluate was concentrated under reduced pressure to give the title compound (253 mg) as a colorless powder. MS(ESI)m/z:379.4[M+H]+

### Reference Example 9: Synthesis of 4-(4-methylthiazol-5-yl)benzaldehyde

To 4-bromobenzaldehyde (10 g), palladium acetate (243 mg), and potassium carbonate (10.6 g) were added dimethylacetamide (180 mL) and 4-methylthiazole (10720 mg), and the mixture was substituted with nitrogen and stirred at 150°C for 1.5 hr. After completion of the reaction, ethyl acetate was added, and the mixture was filtered through Celite. The filtrate was washed with water and saturated brine, and dried over anhydrous sodium sulfate. The organic layer was filtered, and the filtrate was concentrated under reduced pressure. The obtained residue was purified by silica gel column chromatography (hexane:ethyl acetate=85:15 - 70:30) to give the title compound (9.2 g) as a yellow powder. MS(ESI)m/z:204.0[M+H]+

### Reference Example 10: Synthesis of N-(cyclobutylmethyl)-1-[4-(4-methyl-1,3-thiazol-5-yl)phenyl]methanamine

4-(4-Methylthiazol-5-yl)benzaldehyde (100 mg) described in Reference Example 9 was dissolved in dichloromethane (5 mL), cyclobutylmethanamine (63 mg) was added at room temperature and stirred for 30 min. Sodium triacetoxyborohydride (150 mg) was added, and the mixture was stirred for about 1.5 hr. After completion of the reaction, 1 M sodium hydroxide aqueous solution and chloroform were added, and the organic layer was separated by a Phase-separator (registered trade mark), and concentrated under reduced pressure. The obtained residue was purified by NH silica gel chromatography (hexane:ethyl acetate=95:5 - 70:30) to give the title compound (103 mg) as a pale-yellow oil. MS(ESI)m/z:273.1[M+H]+

### Reference Example 11: Synthesis of 2-methyl-N-[[4-(4-methyl-1,3-thiazol-5-yl)phenyl]methyl]propan-1-amine

4-(4-Methylthiazol-5-yl)benzaldehyde (100 mg) described in Reference Example 9 was dissolved in DCM (5 mL), 2-methylpropan-1-amine (74 µL) was added and the mixture was stirred at room temperature for 30 min. Sodium triacetoxyborohydride (150 mg) was added and the mixture was stirred for 3 hr. After completion of the reaction, to the reaction mixture were added chloroform and 1 M sodium hydroxide aqueous solution, and the organic layer was separated by a Phase-separator (registered trade mark) and concentrated under reduced pressure. It was purified by NH silica gel column chromatography (hexane:ethyl acetate=95:5 - 70:30) to give the title compound (108 mg) as a pale-yellow oil.
MS(ESI)m/z:261.1[M+H]+

### Reference Example 12: Synthesis of methyl 2-[(2S,4R)-4-[tert-butyl(dimethyl)silyl]oxy-1-[(2S)-2-(9H-fluorene-9-ylmethoxycarbonylamino)-3,3-dimethylbutanoyl]pyrrolidin-2-yl]-1H-imidazole-4-carboxylate

tert-Butyl (2S,4R)-4-[tert-butyl(dimethyl)silyl]oxy-2-[4-(trimethoxymethyl)-1H-imidazol-2-yl]pyrrolidine-1-carboxylate (2.3 g) described in Reference Example 2 was dissolved in dichloromethane (12 mL), TFA (48 mL) was added at 0°C, and the mixture was stirred at room temperature for 1 hr. After completion of the reaction, the reaction solution was concentrated under reduced pressure. The obtained residue was extracted by adding water and 1 M sodium hydroxide aqueous solution, and a mixed solvent of ethyl acetate and methanol (methanol 10%). The organic layer was washed with saturated brine, and dried over anhydrous magnesium sulfate. The organic layer was filtered, and the filtrate was concentrated under reduced pressure. The obtained residue was purified by NH silica gel chromatography (ethyl acetate:methanol=95:5 - 50:50). The obtained residue was dissolved in DMF (10 mL), HATU (1.12 g), (2S)-2-(9H-fluorene-9-ylmethoxycarbonylamino)-3,3-dimethylbutanoic acid (1085 mg), diisopropyl ethylamine (1.5 mL) were added at room temperature and the mixture was stirred for 30 min. After completion of the reaction, it was extracted with water and ethyl acetate. The organic layer was dried over anhydrous magnesium sulfate and filtered. The filtrate was concentrated under reduced pressure. The obtained residue was purified by silica gel chromatography (hexane:ethyl acetate=95:5 - 50:50) to give the title compound (1.5 g) as a pale-red viscous substance. MS(ESI)m/z:661.4[M+H]+

### Reference Example 13: Synthesis of methyl 2-[(2S,4R)-1-[(2S)-2-acetamido-3,3-dimethylbutanoyl]-4-[tert-butyl(dimethyl)silyl]oxypyrrolidin-2-yl]-1H-imidazole-4-carboxylate

Methyl 2-[(2S,4R)-4-[tert-butyl(dimethyl)silyl]oxy-1-[(2S)-2-(9H-fluorene-9-ylmethoxycarbonylamino)-3,3-dimethylbutanoyl]pyrrolidin-2-yl]-1H-imidazole-4-carboxylate (1.5 g) described in Reference Example 12 was dissolved in methanol (7.6 mL), diethylamine (2.4 mL) was added under ice-cooling and the mixture was stirred at room temperature overnight. After completion of the reaction, the reaction solution was concentrated under reduced pressure and purified by silica gel chromatography (ethyl acetate:methanol=98:2 - 20:80). The obtained residue was dissolved in dichloromethane (23 mL), triethylamine (1.3 mL) and acetic anhydride (240 µL) were added at 0°C and the mixture was stirred at room temperature overnight. After completion of the reaction, the reaction solution was concentrated under reduced pressure, and extracted with water and chloroform. The organic layer was washed with saturated brine, and dried over anhydrous magnesium sulfate. The organic layer was filtered, and the filtrate was concentrated under reduced pressure. The obtained residue was purified by silica gel chromatography (ethyl acetate:methanol=100:0 - 95:5) to give the title compound (774 mg) as a pale-yellow viscous substance. MS(ESI)m/z:481.3[M+H]+

### Reference Example 14: Synthesis of 2-[(2S,4R)-1-[(2S)-2-acetamido-3,3-dimethylbutanoyl]-4-hydroxypyrrolidin-2-yl]-1H-imidazole-4-carboxylic acid

Methyl 2-[(2S,4R)-1-[(2S)-2-acetamido-3,3-dimethylbutanoyl]-4-[tert-butyl(dimethyl)silyl]oxypyrrolidin-2-yl]-1H-imidazole-4-carboxylate (709 mg) described in Reference Example 13 was dissolved in methanol (10 mL), 1 M sodium hydroxide aqueous solution (5.9 mL) was added while stirring at 0°C, and the mixture was heated to 50°C and stirred overnight. After completion of the reaction, the reaction mixture was cooled to 0°C, 1 M hydrochloric acid (5.9 mL) was added, and the mixture was concentrated under reduced pressure. The reaction mixture was dissolved in water, adsorbed onto PoraPak^{™} Rxn RP, and washed with water (45 mL). Then the mixture was eluted with MeOH (90 mL), and concentrated under reduced pressure to give the title compound (489 mg) as a colorless powder. MS(ESI)m/z:353.2[M+H]+

### Reference Example 15: Synthesis of 1-[3-(4-methylthiazol-5-yl)phenyl]ethanone

The title compound was obtained by the same reaction and treatment as in Reference Example 9 using 1-(3-bromophenyl)ethanone. MS(ESI)m/z:218.1[M+H]+

### Reference Example 16: Synthesis of 1-O-tert-butyl 2-0-[2-[3-(4-methyl-1,3-thiazol-5-yl)phenyl]-2-oxoethyl] (2S,4R)-4-hydroxypyrrolidine-1,2-dicarboxylate

1-[3-(4-Methylthiazol-5-yl)phenyl]ethanone (615 mg) described in Reference Example 15 was dissolved in acetic acid (5.6 mL), trimethylphenylammonium tribromide (1596 mg) was added and the mixture was stirred with heating at 90°C for 2 hr. After completion of the reaction, the reaction mixture was cooled to 0°C, added dropwise to saturated aqueous sodium hydrogen carbonate (70 mL) to pH10, and extracted twice with ethyl acetate (20 mL). The organic layer was washed with saturated brine, dried over sodium sulfate and filtered. The filtrate was concentrated under reduced pressure. To the obtained residue were added (2S,4R)-1-tert-butoxycarbonyl-4-hydroxypyrrolidine-2-carboxylic acid (785 mg) and Et3N (1.9 mL), and the mixture was stirred at room temperature for 20 hr. To the reaction mixture was added water (10 mL) and the mixture was extracted with ethyl acetate (20 mL). The organic layer was washed with saturated brine, dried over anhydrous sodium sulfate and filtered. The filtrate was concentrated under reduced pressure and purified by silica gel column chromatography (hexane:ethyl acetate=50:50 - 0:100) to give the title compound (489 mg) as an orange viscous substance. MS(ESI)m/z:447.4[M+H]+

### Reference Example 17: Synthesis of tert-butyl (2S,4R)-4-hydroxy-2-[5-[3-(4-methyl-1,3-thiazol-5-yl)phenyl]-1H-imidazol-2-yl]pyrrolidine-1-carboxylate

1-O-tert-Butyl 2-O-[2-[3-(4-methyl-1,3-thiazol-5-yl)phenyl]-2-oxoethyl] (2S,4R)-4-hydroxypyrrolidine-1,2-dicarboxylate (485 mg) described in Reference Example 16 was dissolved in xylene (5.4 mL), ammonium acetate (418 mg) was added and the mixture was stirred with heating at 140°C for 1.5 hr. The reaction mixture was allowed to cool to room temperature, saturated aqueous sodium hydrogen carbonate (10 mL) was added, and the mixture was extracted with ethyl acetate (20 mL). The organic layer was washed with saturated brine, dried over anhydrous sodium sulfate and filtered. The filtrate was concentrated under reduced pressure and purified by silica gel column chromatography (chloroform: methanol=100:0 - 90:10) to give the title compound (384 mg) as a pale-brown powder. MS(ESI)m/z:427.4[M+H]+

### Reference Example 18: Synthesis of (3R,5S)-5-[5-[3-(4-methyl-1,3-thiazol-5-yl)phenyl]-1H-imidazol-2-yl]pyrrolidin-3-ol hydrochloride

tert-Butyl (2S,4R)-4-hydroxy-2-[5-[3-(4-methyl-1,3-thiazol-5-yl)phenyl]-1H-imidazol-2-yl]pyrrolidine-1-carboxylate (380 mg) described in Reference Example 17 was dissolved in methanol (4.4 mL), 4 M hydrochloric acid-methanol (2.2 mL) was added, and the mixture was stirred with heating at 60°C for 3 hr. The reaction mixture was concentrated under reduced pressure to give the title compound (415 mg) as a yellow powder. MS(ESI)m/z:327.3[M+H]+

### Reference Example 19: Synthesis of methyl 2-[(2S,4R)-4-[tert-butyl(dimethyl)silyl]oxy-1-[(2S)-3,3-dimethyl-2-[(2-methylpropan-2-yl)oxycarbonylamino]butanoyl]pyrrolidin-2-yl]-1H-imidazole-4-carboxylate

tert-Butyl (2S,4R)-4-[tert-butyl(dimethyl)silyl]oxy-2-[4-(trimethoxymethyl)-1H-imidazol-2-yl]pyrrolidine-1-carboxylate (1.2 g) described in Reference Example 2 was dissolved in DCM (25 mL), trifluoroacetic acid (6.3 mL) was added, and the mixture was stirred with heating at room temperature for 3 hr. After completion of the reaction, the mixture was azeotropically distilled with toluene, quenched with saturated aqueous sodium hydrogen carbonate, and the aqueous layer was extracted three times with ethyl acetate/10% methanol. The combined organic layer was washed with saturated brine, dried over anhydrous sodium sulfate, filtered, and the filtrate was concentrated under reduced pressure. The obtained residue was dissolved in DMF (21 mL), DIPEA (1.4 mL), (2S)-2-(tert-butoxycarbonylamino)-3,3-dimethylbutanoic acid (686 mg), and HATU (1209 mg) were added, and the mixture was stirred at room temperature for 30 min. After completion of the reaction, saturated brine was added, and the aqueous layer was extracted with ethyl acetate. The organic layer was washed with saturated brine, dried over anhydrous sodium sulfate, and filtered. The filtrate was concentrated and purified by silica gel column chromatography (hexane:ethyl acetate=80:20 - 60:40) to give the title compound (1058 mg) as a pale-red powder. MS(ESI)m/z:539.5[M+H]+

### Reference Example 20: Synthesis of N-methyl-1-[3-(4-methylthiazol-5-yl)phenyl]methanamine

The title compound was obtained by the same reaction and treatment as in Example 56 using 1-(3-bromophenyl)-N-methyl-methanamine, 4-methyl-5-(4,4,5,5-tetramethyl-1,3,2-dioxaborolan-2-yl)thiazole, and potassium carbonate instead of sodium carbonate. MS(ESI)m/z:219.2[M+H]+

### Reference Example 21: Synthesis of tert-butyl (2S,4R)-4-[tert-butyl(dimethyl)silyl]oxy-2-(1H-imidazol-2-yl)pyrrolidine-1-carboxylate

tert-Butyl (2S,4R)-4-[tert-butyl(dimethyl)silyl]oxy-2-formylpyrrolidine-1-carboxylate (4551 mg) was dissolved in 7 M ammonia-methanol (19 mL), 39% glyoxal aqueous solution (6.3 mL) was added, and the mixture was stirred with heating at room temperature for 8 hr. After quenching with saturated brine, the aqueous layer was extracted with ethyl acetate. The organic layer was washed with saturated brine, dried over anhydrous sodium sulfate, and filtered. The filtrate was concentrated under reduced pressure and purified by silica gel column chromatography (hexane:ethyl acetate=50:50) to give the title compound (2.6 g) as a colorless viscous substance.
MS(ESI)m/z:368.3[M+H]+

### Reference Example 22: Synthesis of tert-butyl (2S,4R)-4-[tert-butyl(dimethyl)silyl]oxy-2-[1-[(3-phenylphenyl)methyl]imidazol-2-yl]pyrrolidine-1-carboxylate

tert-Butyl (2S,4R)-4-[tert-butyl(dimethyl)silyl]oxy-2-(1H-imidazol-2-yl)pyrrolidine-1-carboxylate (170 mg) described in Reference Example 21 was dissolved in DMF (1 mL), cesium carbonate (450 mg) and 3-phenylbenzylbromide (174 mg) were added and the mixture was stirred with heating at 70°C for 3 hr. To the reaction mixture were added chloroform and water, and the organic layer was separated by a Phase-separator (registered trade mark), concentrated under reduced pressure, and purified by silica gel column chromatography (hexane:ethyl acetate=90:10 - 50:50) to give the title compound (191 mg) as a pale-yellow oil. MS(ESI)m/z:534.7[M+H]+

### Reference Example 23: Synthesis of tert-butyl (2S,4R)-4-[tert-butyl(dimethyl)silyl]oxy-2-[1-[(3-chlorophenyl)methyl]imidazol-2-yl]pyrrolidine-1-carboxylate

tert-Butyl (2S,4R)-4-[tert-butyl(dimethyl)silyl]oxy-2-(1H-imidazol-2-yl)pyrrolidine-1-carboxylate (307 mg) described in Reference Example 21 was dissolved in DMF (1 mL), cesium carbonate (826 mg) and 3-chlorobenzylbromide (160 µL) were added, and the mixture was stirred with heating at 70°C for 3.5 hr. To the reaction mixture were added chloroform and water, and the organic layer was separated by a Phase-separator (registered trade mark), concentrated under reduced pressure, and purified by silica gel column chromatography (hexane:ethyl acetate=90:10 - 50:50) to give the title compound (319 mg) as a pale-yellow oil. MS(ESI)m/z:492.6,494.6[M+H]+

### Reference Example 24: Synthesis of tert-butyl (2S,4R)-4-[tert-butyl(dimethyl)silyl]oxy-2-[1-[[3-(4-methyl-1,3-thiazol-5-yl)phenyl]methyl]imidazol-2-yl]pyrrolidine-1-carboxylate

tert-Butyl (2S,4R)-4-[tert-butyl(dimethyl)silyl]oxy-2-[1-[(3-chlorophenyl)methyl]imidazol-2-yl]pyrrolidine-1-carboxylate (104 mg) described in Reference Example 23, 4-methyl-5-(4,4,5,5-tetramethyl-1,3,2-dioxaborolan-2-yl)thiazole (72 mg), XPhos Pd G2 (18 mg), XPhos (30 mg), sodium carbonate (90 mg), 1,4-dioxane (1 mL), and water (200 µL) were added, and the mixture was substituted with nitrogen and stirred with heating at 100°C for 3.5 hr. To the reaction mixture were added chloroform and water, and the organic layer was separated by a Phase-separator (registered trade mark), concentrated under reduced pressure, and purified by silica gel column chromatography (hexane:ethyl acetate=80:20 - 20:80) to give the title compound (38 mg) as a pale-yellow oil.
MS(ESI)m/z:555.4[M+H]+

### Reference Example 25: Synthesis of tert-butyl (2S,4R)-4-[tert-butyl(dimethyl)silyl]oxy-2-[1-[2-(3-chlorophenyl)ethyl]imidazol-2-yl]pyrrolidine-1-carboxylate

tert-Butyl (2S,4R)-4-[tert-butyl(dimethyl)silyl]oxy-2-(1H-imidazol-2-yl)pyrrolidine-1-carboxylate (216 mg) described in Reference Example 21 was dissolved in DMF (1 mL), cesium carbonate (611 mg) and 1-(2-bromoethyl)-3-chlorobenzene (260 µL) were added, and the mixture was stirred with heating at 70°C overnight. 1-(2-Bromoethyl)-3-chlorobenzene (260 µL) was added and the mixture was stirred with heating at 70°C for 6 hr. To the reaction mixture were added chloroform and water, and the organic layer was separated by a Phase-separator (registered trade mark), concentrated under reduced pressure, and purified by silica gel column chromatography (hexane:ethyl acetate=90:10 - 50:50) to give the title compound (70 mg) as a pale-yellow oil. MS(ESI)m/z:506.4/508.4[M+H]+

### Reference Example 26: Synthesis of tert-butyl (2S,4R)-4-[tert-butyl(dimethyl)silyl]oxy-2-[1-[2-[3-(4-methyl-1,3-thiazol-5-yl)phenyl]ethyl]imidazol-2-yl]pyrrolidine-1-carboxylate

tert-Butyl (2S,4R)-4-[tert-butyl(dimethyl)silyl]oxy-2-[1-[2-(3-chlorophenyl)ethyl]imidazol-2-yl]pyrrolidine-1-carboxylate (65 mg) described in Reference Example 25, 4-methyl-5-(4,4,5,5-tetramethyl-1,3,2-dioxaborolan-2-yl)thiazole (56 mg), XPhos Pd G2 (10 mg), XPhos (18 mg), sodium carbonate (56 mg), 1,4-dioxane (1 mL), and water (200 µL) were added, and the mixture was substituted with nitrogen and stirred with heating at 100°C for 1.5 hr. It was purified by silica gel column chromatography (hexane:ethyl acetate=80:20 - 20:80, chloroform:methanol=95:5) to give the title compound (74 mg) as a brown oil. MS(ESI)m/z:569.7[M+H]+

### Reference Example 27: Synthesis of N-(cyclopropylmethyl)-4-phenyl-butan-1-amine

4-Phenylbutan-1-amine (257 mg) was dissolved in THF (5 mL), cyclopropanecarboxyaldehyde (160 µL), sodium triacetoxyborohydride (1050 mg), and acetic acid (100 µL) were added at 0°C, and the mixture was stirred at room temperature for 22 hr. After completion of the reaction, to the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (90 mg) as a colorless oil. MS(ESI)m/z:204.1[M+H]+

### Reference Example 28: Synthesis of N-(cyclopropylmethyl)-4-(4-pyridyl)butan-1-amine

THF (5.0 mL) was added to 4-(4-pyridyl)butan-1-amine (204 mg), cyclopropanecarboxyaldehyde (120 µL), acetic acid (80 µL), and sodium triacetoxyborohydride (845 mg) were added at 0°C, and the mixture was stirred at room temperature for 22 hr. After completion of the reaction, to the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (13 mg) as a yellow oil. MS(ESI)m/z:205.1[M+H]+

### Reference Example 29: Synthesis of N-[[4-(4-methylthiazol-5-yl)phenyl]methyl]propane-2-amine

DCM (3.0 mL) was added to 4-(4-methylthiazol-5-yl)benzaldehyde (60 mg) described in Reference Example 9, isopropylamine (38 µL) was added, and the mixture was stirred at room temperature for about 30 min. Sodium triacetoxyborohydride (90 mg) was further added, and the mixture was stirred at room temperature for about 2 hr. After completion of the reaction, to the reaction mixture were added chloroform and 1 M sodium hydroxide aqueous solution, and the organic layer was separated by a Phase-separator (registered trade mark), and concentrated under reduced pressure. The residue was purified by NH silica gel column chromatography (hexane:ethyl acetate=95:5 - 70:30) to give the title compound (47 mg) as a pale-yellow oil. MS(ESI)m/z:247.1[M+H]+

### Reference Example 30: Synthesis of 2,2,2-trifluoro-N-[[4-(4-methylthiazol-5-yl)phenyl]methyl]ethaneamine

The title compound was obtained by the same reaction and treatment as in Reference Example 10 using 4-(4-methylthiazol-5-yl)benzaldehyde described in Reference Example 9 and 2,2,2-trifluoroethaneamine. MS(ESI)m/z:287.1[M+H]+

### Reference Example 31: Synthesis of ethyl 2-(4-bromopyrazol-1-yl)-3-methylbutanoate

The title compound was obtained by the same reaction and treatment as in Reference Example 22 using 4-bromo-1H-pyrazole, ethyl 2-bromo-3-methylbutanoate, and DMSO instead of DMF. MS(ESI)m/z:275.1,277.1[M+H]+

### Reference Example 32: Synthesis of ethyl 2-[4-(2-methoxypyridin-4-yl)pyrazol-1-yl]-3-methylbutanoate

The title compound was obtained by the same reaction and treatment as in Example 56 using ethyl 2-(4-bromopyrazol-1-yl)-3-methylbutanoate described in Reference Example 31 and (2-methoxy-4-pyridyl)boronic acid. MS(ESI)m/z:304.1[M+H]+

### Reference Example 33: Synthesis of 2-[4-(2-methoxypyridin-4-yl)pyrazol-1-yl]-3-methylbutanoic acid

The title compound was obtained by the same reaction and treatment as in Reference Example 8 using ethyl 2-[4-(2-methoxypyridin-4-yl)pyrazol-1-ylJ-3 methylbutanoate described in Reference Example 32, and ethanol instead of methanol. MS(ESI)m/z:276.2[M+H]+

### Reference Example 34: Synthesis of 2-[(2S,4R)-4-hydroxy-1-[2-(3-methoxy-1,2-oxazol-5-yl)-3-methylbutanoyl]pyrrolidin-2-yl]-N-methyl-N-[[4-(4-methyl-1,3-thiazol-5-yl)phenyl]methyl]-1H-imidazole-4-carboxamide

2-[(2S,4R)-4-hydroxypyrrolidin-2-yl]-N-methyl-N-[[4-(4-methyl-1,3-thiazol-5-yl)phenyl]methyl]-1H-imidazole-4-carboxamide hydrochloride (40 mg) described in Reference Example 6, 2-(3-methoxy-1,2-oxazol-5-yl)-3-methylbutanoic acid (25 mg), and WSC·HCl (24 mg) were dissolved in DCM (420 µL), Et3N (35 µL) and HOBt (17 mg) were added, and the mixture was stirred at room temperature for 2.5 hr. MeOH was added and the mixture was concentrated under reduced pressure. The residue was purified by preparative HPLC (10 mM ammonium carbonate aqueous solution-acetonitrile) to give the title compound (19 mg) as a white powder. MS(ESI)m/z:577.3[M-H]-

### Reference Example 35: Synthesis of tert-butyl (2S,4R)-2-[4-[cyclopropylmethyl-[[4-(4-methyl-1,3-thiazol-5-yl)phenyl]methyl]carbamoyl]-1H-imidazol-2-yl]-4-hydroxypyrrolidine-1-carboxylate

NMP (0.35 mL) was added to 2-[(2S,4R)-4-hydroxy-1-[(2-methylpropan-2-yl)oxycarbonyl]pyrrolidin-2-yl]-1H-imidazole-4-carboxylic acid (20 mg) described in Reference Example 4, HATU (26 mg) was added, and the mixture was stirred at room temperature for 30 min. Separately, a solution of N-(cyclopropylmethyl)-1-[4-(4-methylthiazol-5-yl)phenyl]methanamine (18 mg) described in Reference Example 106 in NMP (0.35 mL) and DIPEA (0.035 mL) was prepared, added to the reaction mixture, and the mixture was stirred at room temperature for 1 hr. After completion of the reaction, water was added, and the aqueous layer was extracted with ethyl acetate. The organic layer was washed with saturated brine/water, dried over anhydrous sodium sulfate, filtered, and the filtrate was concentrated under reduced pressure. The residue was purified by NH silica gel column chromatography (ethyl acetate:methanol=100:0 - 90:10) to give the title compound (26 mg) as a colorless oil. MS(ESI)m/z:538.5[M+H]+

### Reference Example 36: Synthesis of N-(cyclopropylmethyl)-2-[(2S,4R)-4-hydroxy-1-[2-(3-methoxy-1,2-oxazol-5-yl)-3-methylbutanoyl]pyrrolidin-2-yl]-N-[[4-(4-methyl-1,3-thiazol-5-yl)phenyl]methyl]-1H-imidazole-4-carboxamide

tert-Butyl (2S,4R)-2-[4-[cyclopropylmethyl-[[4-(4-methyl-1,3-thiazol-5-yl)phenyl]methyl]carbamoyl]-1H-imidazol-2-yl]-4-hydroxypyrrolidine-1-carboxylate (26 mg) described in Reference Example 35 was dissolved in 1,4-dioxane (0.4 mL), 4 M hydrochloric acid-dioxane (0.4 mL) was added at room temperature, and the mixture was stirred overnight. After completion of the reaction, the reaction solution was concentrated under reduced pressure, and further azeotropically distilled 3 times with toluene. The obtained residue was dissolved in N,N-dimethylformamide (0.5 mL), 2-(3-methoxy-1,2-oxazol-5-yl)-3-methylbutanoic acid (13 mg), HATU (22 mg), and diisopropyl ethylamine (34 µL) were added, and the mixture was stirred at room temperature for 3 hr. After completion of the reaction, water was added, and the mixture was extracted with ethyl acetate, and concentrated under reduced pressure. The obtained residue was purified by NH silica gel column chromatography (chloroform: methanol=100:0 - 90:10) to give the title compound (19 mg) as a white powder. MS(ESI)m/z:617.4[M-H]-

### Reference Example 37: Synthesis of tert-butyl N-[[4-(4-methyl-1,3-thiazol-5-yl)phenyl]methyl]-N-[2-(oxan-2-yloxy)ethyl]carbamate

To sodium hydride (20 mg) were added DMF (1.6 mL) and tert-butyl N-[[4-(4-methylthiazol-5-yl)phenyl]methyl]carbamate (50 mg), and the mixture was stirred at room temperature for about 20 min. To the reaction mixture was added 2-(2-bromoethoxy)tetrahydropyran (18 mg), and the mixture was stirred at room temperature overnight. After completion of the reaction, to the reaction mixture were added chloroform and saturated ammonium chloride aqueous solution, and the organic layer was separated by a Phase-separator (registered trade mark), concentrated under reduced pressure, and purified by preparative HPLC (10 mM ammonium carbonate aqueous solution-acetonitrile) to give the title compound (18 mg) as a colorless oil. MS(ESI)m/z:443.4[M+H]+

### Reference Example 38: Synthesis of tert-butyl (2S,4R)-4-[tert-butyl(dimethyl)silylJoxy-2-(4,5-dibromo-1H-imidazol-2-yl)pyrrolidine-1-carboxylate

DCM (20 mL) was added to tert-butyl (2S,4R)-4-[tert-butyl(dimethyl)silyl]oxy-2-(1H-imidazol-2-yl)pyrrolidine-1-carboxylate (2 g) described in Reference Example 21, NBS (2130 mg) was added, and the mixture was stirred at room temperature for about 2 hr. After completion of the reaction, saturated aqueous sodium hydrogen carbonate was added, and the aqueous layer was extracted with ethyl acetate. The organic layer was washed with saturated brine, dried over anhydrous sodium sulfate, filtered, and the filtrate was concentrated under reduced pressure. The residue was purified by silica gel column chromatography (hexane:ethyl acetate=90:10 - 60:40) to give the title compound (2.4 g) as a colorless powder. MS(ESI)m/z:524.2/526.2/528.2[M+H]+

### Reference Example 39: Synthesis of tert-butyl (2S,4R)-2-(5-bromo-1H-imidazol-2-yl)-4-[tert-butyl(dimethyl)silyl]oxypyrrolidine-1-carboxylate

To tert-butyl (2S,4R)-4-[tert-butyl(dimethyl)silyl]oxy-2-(4,5-dibromo-1H-imidazol-2-yl)pyrrolidine-1-carboxylate (791 mg) described in Reference Example 38 were added ethanol (14 mL) and water (7 mL), sodium sulfite (9488 mg) was added, and the mixture was stirred at 100°C for about 2 days. The mixture was allowed to cool to room temperature, saturated brine was added, and the aqueous layer was extracted with ethyl acetate. The organic layer was washed with saturated brine, dried over anhydrous sodium sulfate, filtered, and the filtrate was concentrated under reduced pressure. The residue was purified by silica gel column chromatography (hexane:ethyl acetate=90:10 - 80:20) to give the title compound (463 mg) as a white powder. MS(ESI)m/z:446.1/448.1[M+H]+

### Reference Example 40: Synthesis of tert-butyl (2S,4R)-4-[tert-butyl(dimethyl)silyl]oxy-2-[5-(3-methoxyphenyl)-1H-imidazol-2-yl]pyrrolidine-1-carboxylate

To tert-butyl (2S,4R)-2-(5-bromo-1H-imidazol-2-yl)-4-[tert-butyl(dimethyl)silyl]oxypyrrolidine-1-carboxylate (33 mg) described in Reference Example 39 were added DME (1 mL) and water (0.30 mL), potassium carbonate (31 mg), (3-methoxyphenyl) boronic acid (20 mg), and PdCl2(AmPhos)2 (5 mg) were added, and the mixture was stirred at 115°C overnight. DME (1 mL) and water (0.30 mL) were further added, PdCl2(AmPhos)2 (5 mg) and (3-methoxyphenyl)boronic acid (17 mg) were added, and the mixture was stirred at 115°C for 3 hr. The mixture was allowed to cool to room temperature, and filtered through Celite. The filtrate was concentrated under reduced pressure. The residue was purified by silica gel column chromatography (hexane:ethyl acetate=80:20 - 67:33) to give the title compound (30 mg) as a colorless oil.
MS(ESI)m/z:474.2[M+H]+

### Reference Example 41: Synthesis of tert-butyl (2S,4R)-4-hydroxy-2-[5-[(4-methoxyphenyl)methyl]-1H-imidazol-2-yl]pyrrolidine-1-carboxylate

(2S,4R)-1-tert-Butoxycarbonyl-4-hydroxypyrrolidine-2-carboxylic acid (200 mg) was dissolved in DMF (8.6 mL), 1-amino-3-(4-methoxyphenyl)propan-2-one hydrochloride (205 mg), DIPEA (0.45 mL), and HATU (395 mg) were added at room temperature, and the mixture was stirred for about 9 hr. After completion of the reaction, the reaction mixture was extracted with water and ethyl acetate. The organic layer was dried over anhydrous sodium sulfate, filtered and the filtrate was concentrated. The obtained residue was purified by silica gel chromatography (ethyl acetate:methanol=100:0 - 95:5). The obtained residue was dissolved in xylene (2.9 mL), ammonium acetate (113 mg) was added, and the mixture was stirred at 150°C for about 4 hr. After completion of the reaction, sodium hydroxide aqueous solution and ethyl acetate were added, and the mixture was extracted. The organic layer was washed with saturated brine, and dried over anhydrous sodium sulfate. The organic layer was filtered, and the filtrate was concentrated, and purified by preparative HPLC (10 mM ammonium carbonate aqueous solution-acetonitrile) to give the title compound (8.2 mg) as a yellow powder. MS(ESI)m/z:374.4[M+H]+

### Reference Example 42: Synthesis of tert-butyl (2S,4R)-4-[tert-butyl(dimethyl)silyl]oxy-2-[5-[(3-methoxyphenyl)methyl]-1H-imidazol-2-yl]pyrrolidine-1-carboxylate

The title compound was obtained by the same reaction and treatment as in Reference Example 40 using tert-butyl (2S,4R)-2-(5-bromo-1H-imidazol-2-yl)-4-[tert-butyl(dimethyl)silyl]oxypyrrolidine-1-carboxylate described in Reference Example 39, and 2-[(3-methoxyphenyl)methyl]-4,4,5,5-tetramethyl-1,3,2-dioxaborolane. MS(ESI)m/z:488.3[M+H]+

### Reference Example 43: Synthesis of tert-butyl (2S,4R)-4-[tert-butyl(dimethyl)silyl]oxy-2-[5-[2-(methylcarbamoyl)phenyl]-1H-imidazol-2-yl]pyrrolidine-1-carboxylate

The title compound was obtained by the same reaction and treatment as in Reference Example 40 using tert-butyl (2S,4R)-2-(5-bromo-1H-imidazol-2-yl)-4-[tert-butyl(dimethyl)silyl]oxypyrrolidine-1-carboxylate described in Reference Example 39, and [2-(methylcarbamoyl)phenyl]boronic acid. MS(ESI)m/z:501.4[M+H]+

### Reference Example 44: Synthesis of tert-butyl (2S,4R)-4-[tert-butyl(dimethyl)silyl]oxy-2-[1-[(3-phenoxyphenyl)methyl]imidazol-2-yl]pyrrolidine-1-carboxylate

The title compound was obtained by the same reaction and treatment as in Reference Example 23 using tert-butyl (2S,4R)-4-[tert-butyl(dimethyl)silyl]oxy-2-(1H-imidazol-2-yl)pyrrolidine-1-carboxylate described in Reference Example 21, and 1-(chloromethyl)-3-phenoxy-benzene. MS(ESI)m/z:550.6[M+H]+

### Reference Example 45: Synthesis of (3R,5S)-5-[1-[(3-phenoxyphenyl)methyl]imidazol-2-yl]pyrrolidin-3-ol hydrochloride

The title compound was obtained by the same reaction and treatment as in Reference Example 6 using tert-butyl (2S,4R)-4-[tert-butyl(dimethyl)silyl]oxy-2-[1-[(3-phenoxyphenyl)methyl]imidazol-2-yl]pyrrolidine-1-carboxylate described in Reference Example 44. MS(ESI)m/z:336.3[M+H]+

### Reference Example 46: Synthesis of tert-butyl (2S,4R)-4-[tert-butyl(dimethyl)silyl]oxy-2-[1-[(2-phenylmethoxyphenyl)methyl]imidazol-2-yl]pyrrolidine-1-carboxylate

The title compound was obtained by the same reaction and treatment as in Reference Example 23 using 1-(bromomethyl)-2-phenylmethoxybenzene, and tert-butyl (2S,4R)-4-[tert-butyl(dimethyl)silyl]oxy-2-(1H-imidazol-2-yl)pyrrolidine-1-carboxylate described in Reference Example 21. MS(ESI)m/z:562.4[M-H]-

### Reference Example 47: Synthesis of tert-butyl (2S,4R)-4-[tert-butyl(dimethyl)silyl]oxy-2-[1-[(3-phenylmethoxyphenyl)methyl]imidazol-2-yl]pyrrolidine-1-carboxylate

The title compound was obtained by the same reaction and treatment as in Reference Example 23 using tert-butyl (2S,4R)-4-[tert-butyl(dimethyl)silyl]oxy-2-(1H-imidazol-2-yl)pyrrolidine-1-carboxylate described in Reference Example 21, 1-benzyloxy-3-(bromomethyl)benzene. MS(ESI)m/z:564.4[M+H]+

### Reference Example 48: Synthesis of tert-butyl (2S,4R)-4-[tert-butyl(dimethyl)silyl]oxy-2-[1-[(2-phenoxyphenyl)methyl]imidazol-2-yl]pyrrolidine-1-carboxylate

The title compound was obtained by the same reaction and treatment as in Reference Example 23 using tert-butyl (2S,4R)-4-[tert-butyl(dimethyl)silyl]oxy-2-(1H-imidazol-2-yl)pyrrolidine-1-carboxylate described in Reference Example 21, and 1-(bromomethyl)-2-phenoxy-benzene. MS(ESI)m/z:550.4[M+H]+

### Reference Example 49: Synthesis of tert-butyl (2S,4R)-2-[4-[ethyl-[[4-(4-methyl-1,3-thiazol-5-yl)phenyl]methyl]carbamoyl]-1H-imidazol-2-yl]-4-hydroxypyrrolidine-1-carboxylate

The title compound was obtained by the same reaction and treatment as in Reference Example 35 using 2-[(2S,4R)-4-hydroxy-1-[(2-methylpropan-2-yl)oxycarbonyl]pyrrolidin-2-yl]-1H-imidazole-4-carboxylic acid described in Reference Example 4, and N-[[4-(4-methyl-1,3-thiazol-5-yl)phenyl]methyl]ethaneamine. MS(ESI)m/z:512.3[M+H]+

### Reference Example 50: Synthesis of tert-butyl N-methyl-N-[(1S)-1-[4-(4-methyl-1,3-thiazol-5-yl)phenyl]ethyl]carbamate

The title compound was obtained by the same reaction and treatment as in Reference Example 9 using tert-butyl N-[(1S)-1-(4-bromophenyl)ethyl]-N-methylcarbamate, 4-methylthiazole, and potassium acetate instead of potassium carbonate. MS(ESI)m/z:333.2[M+H]+

### Reference Example 51: Synthesis of (1S)-N-methyl-1-[4-(4-methyl-1,3-thiazol-5-yl)phenyl]ethaneaminehydrochloride

The title compound was obtained by the same reaction and treatment as in Reference Example 6 using tert-butyl N-methyl-N-[(1S)-1-[4-(4-methyl-1,3-thiazol-5-yl)phenyl]ethyl]carbamate described in Reference Example 50 and 1,4-dioxane instead of methanol. MS(ESI)m/z:233.2[M+H]+

### Reference Example 52: Synthesis of tert-butyl (2S,4R)-4-hydroxy-2-[4-[methyl-[(1S)-1-[4-(4-methyl-1,3-thiazol-5-yl)phenyl]ethyl]carbamoyl]-1H-imidazol-2-yl]pyrrolidine-1-carboxylate

The title compound was obtained by the same reaction and treatment as in Reference Example 35 using 2-[(2S,4R)-4-hydroxy-1-[(2-methylpropan-2-yl)oxycarbonyl]pyrrolidin-2-yl]-1H-imidazole-4-carboxylic acid described in Reference Example 4, and (1S)-N-methyl-1-[4-(4-methyl-1,3-thiazol-5-yl)phenyl]ethaneamine hydrochloride described in Reference Example 51. MS(ESI)m/z:512.5[M+H]+

### Reference Example 53: Synthesis of tert-butyl (2S,4R)-4-[tert-butyl(dimethyl)silyl]oxy-2-[1-[(2-hydroxyphenyl)methyl]imidazol-2-yl]pyrrolidine-1-carboxylate

To a solution of tert-butyl (2S,4R)-4-[tert-butyl(dimethyl)silyl]oxy-2-[1-[(2-phenylmethoxyphenyl)methyl]imidazol-2-yl]pyrrolidine-1-carboxylate (20 mg) described in Reference Example 46 in ethyl acetate (0.33 mL) was added 10% Pd/C (4 mg), and the mixture was stirred under hydrogen atmosphere at room temperature overnight. After completion of the reaction, nitrogen substitution was performed, insoluble material was filtered off through celite, washed with chloroform and the filtrate was concentrated under reduced pressure to give the title compound (15 mg) as a yellow oil. MS(ESI)m/z:474.4[M+H]+

### Reference Example 54: Synthesis of tert-butyl (2S,4R)-4-[tert-butyl(dimethyl)silyl]oxy-2-[1-[[2-[(4-methyl-1,3-thiazol-5-yl)methoxy]phenyl]methyl]imidazol-2-yl]pyrrolidine-1-carboxylate

To a solution of tert-butyl (2S,4R)-4-[tert-butyl(dimethyl)silyl]oxy-2-[1-[(2-hydroxyphenyl)methyl]imidazol-2-yl]pyrrolidine-1-carboxylate (15 mg)described in Reference Example 53 in DMF (0.32 mL) were added cesium carbonate (32 mg) and 5-(bromomethyl)-4-methylthiazole hydrogen bromide (13 mg), and the mixture was stirred at 60°C overnight. The reaction mixture was cooled to room temperature, chloroform and water were added, and the organic layer was separated by a Phase-separator (registered trade mark), and concentrated under reduced pressure. The residue was purified by preparative HPLC (10 mM ammonium carbonate aqueous solution-acetonitrile) to give the title compound (6.4 mg) as a brown oil. MS(ESI)m/z:585.5[M+H]+

### Reference Example 55: Synthesis of tert-butyl (2S,4R)-4-[tert-butyl(dimethyl)silyl]oxy-2-[1-[(3-hydroxyphenyl)methyl]imidazol-2-yl]pyrrolidine-1-carboxylate

The title compound was obtained by the same reaction and treatment as in Reference Example 53 using tert-butyl (2S,4R)-4-[tert-butyl(dimethyl)silyl]oxy-2-[1-[(3-phenylmethoxyphenyl)methyl]imidazol-2-yl]pyrrolidine-1-carboxylate described in Reference Example 47. MS(ESI)m/z:474.3[M+H]+

### Reference Example 56: Synthesis of tert-butyl (2S,4R)-4-[tert-butyl(dimethyl)silyl]oxy-2-[1-[[3-[(4-methyl-1,3-thiazol-5-yl)methoxy]phenyl]methyl]imidazol-2-yl]pyrrolidine-1-carboxylate

The title compound was obtained by the same reaction and treatment as in Reference Example 54 using tert-butyl (2S,4R)-4-[tert-butyl(dimethyl)silyl]oxy-2-[1-[(3-hydroxyphenyl)methyl]imidazol-2-yl]pyrrolidine-1-carboxylate described in Reference Example 55, and 5-(bromomethyl)-4-methyl-thiazole hydrogen bromide. MS(ESI)m/z:585.5[M+H]+

### Reference Example 57: Synthesis of tert-butyl (2S,4R)-4-[tert-butyl(dimethyl)silyl]oxy-2-[5-iodo-4-(trifluoromethyl)-1H-imidazol-2-yl]pyrrolidine-1-carboxylate

tert-Butyl (2S,4R)-4-[tert-butyl(dimethyl)silyl]oxy-2-[4-(trifluoromethyl)-1H-imidazol-2-yl]pyrrolidine-1-carboxylate (241 mg) described in Reference Example 1 was dissolved in DCM (5.5 mL), NIS (250 mg) was added, and the mixture was stirred under shading at room temperature overnight. Then, p-toluenesulfonic acid pyridinium (140 mg) was added, and the mixture was stirred under shading at room temperature for 6 hr. Sodium sulfite saturated aqueous solution was added, and the aqueous layer was extracted with dichloromethane, and the organic layer was separated by a Phase-separator (registered trade mark), and concentrated under reduced pressure. The residue was purified by silica gel column chromatography (chloroform: ethyl acetate=98:2 - 96:4) to give the title compound (201 mg) as a white powder. MS(ESI)m/z:560.1[M-H]-

### Reference Example 58: Synthesis of tert-butyl (2S,4R)-4-[tert-butyl(dimethyl)silyl]oxy-2-[5-(4-methoxyphenyl)-4-(trifluoromethyl)-1H-imidazol-2-yl]pyrrolidine-1-carboxylate

The title compound was obtained by the same reaction and treatment as in Example 56 using tert-butyl (2S,4R)-4-[tert-butyl(dimethyl)silyl]oxy-2-[5-iodo-4-(trifluoromethyl)-1H-imidazol-2-yl]pyrrolidine-1-carboxylate described in Reference Example 57, and (4-methoxyphenyl)boronic acid.
MS(ESI)m/z:542.3[M+H]+

### Reference Example 59: Synthesis of 2-[(2S,4R)-4-hydroxy-1-[(2-methylpropan-2-yl)oxycarbonyl]pyrrolidin-2-yl]-5-(4-methoxyphenyl)-1H-imidazole-4-carboxylic acid

tert-Butyl (2S,4R)-4-[tert-butyl(dimethyl)silyl]oxy-2-[5-(4-methoxyphenyl)-4-(trifluoromethyl)-1H-imidazol-2-yl]pyrrolidine-1-carboxylate (52 mg) described in Reference Example 58 was dissolved in 1,4-dioxane (1.3 mL), 1 M sodium hydroxide aqueous solution (0.67 mL) was added, and the mixture was stirred at room temperature for 7 hr. The mixture was stirred at 40°C overnight, and further stirred at 50°C for 5 hr. 1 M Sodium hydroxide aqueous solution (0.10 mL) was added, and the mixture was further stirred at 50°C for 2 hr. 1 M Hydrochloric acid (770 µL) was added, and the reaction mixture was concentrated under reduced pressure. After azeotropic distillation with toluene, the residue was purified by preparative HPLC (10 mM ammonium carbonate aqueous solution-acetonitrile) to give the title compound (6.5 mg) as a colorless oil. MS(ESI)m/z:404.1[M+H]+

### Reference Example 60: Synthesis of tert-butyl (2S,4R)-4-hydroxy-2-[5-(4-methoxyphenyl)-4-(methylcarbamoyl)-1H-imidazol-2-yl]pyrrolidine-1-carboxylate

The title compound was obtained by the same reaction and treatment as in Example 54 using 2-[(2S,4R)-4-hydroxy-1-[(2-methylpropan-2-yl)oxycarbonyl]pyrrolidin-2-yl]-5-(4-methoxyphenyl)-1H-imidazole-4-carboxylic acid described in Reference Example 59, methylamine hydrochloride, and DMF instead of DCM. MS(ESI)m/z:417.1[M+H]+

### Reference Example 61: Synthesis of tert-butyl (2S,4R)-2-[4,5-bis(3-methoxyphenyl)-1H-imidazol-2-yl]-4-[tert-butyl(dimethyl)silyl]oxypyrrolidine-1-carboxylate

The title compound was obtained by the same reaction and treatment as in Reference Example 40 using tert-butyl (2S,4R)-4-[tert-butyl(dimethyl)silyl]oxy-2-(4,5-dibromo-1H-imidazol-2-yl)pyrrolidine-1-carboxylate described in Reference Example 38, and (3-methoxyphenyl)boronic acid. MS(ESI)m/z:580.3[M+H]+

### Reference Example 62: Synthesis of tert-butyl (2S,4R)-4-[tert-butyl(dimethyl)silyl]oxy-2-[1-(naphthalen-1-ylmethyl) imidazol-2-yl]pyrrolidine-1-carboxylate

To a solution of tert-butyl (2S,4R)-4-[tert-butyl(dimethyl)silyl]oxy-2-formylpyrrolidine-1-carboxylate (150 mg) in DCM (1518 µL) were added 1-naphthylmethanamine (100 µL) and magnesium sulfate (83 mg), and the mixture was stirred at room temperature overnight. Insoluble material was filtered off, and the filtrate was concentrated under reduced pressure. To the concentrated residue were added a solution of ammonium acetate (47 mg) in methanol (1517 µL) and 39% glyoxal aqueous solution (78 µL), and the mixture was stirred at room temperature for 7 hr. A solution of ammonium acetate (29 mg) in methanol (758 µL) and 39% glyoxal aqueous solution (39 µL) were added, and the mixture was stirred at room temperature overnight. The reaction mixture was concentrated under reduced pressure, and residue was purified by silica gel column chromatography (hexane:ethyl acetate=67:33 - 47:53 - 0:100) to give the title compound (77 mg) as a pale-yellow oil. MS(ESI)m/z:508.4[M+H]+

### Reference Example 63: Synthesis of tert-butyl (2S,4R)-4-[tert-butyl(dimethyl)silyl]oxy-2-[5-(4-methoxyphenyl)-1H-imidazol-2-yl]pyrrolidine-1-carboxylate

The title compound was obtained by the same reaction and treatment as in Reference Example 40 using tert-butyl (2S,4R)-2-(5-bromo-1H-imidazol-2-yl)-4-[tert-butyl(dimethyl)silyl]oxypyrrolidine-1-carboxylate described in Reference Example 39, and (4-methoxyphenyl)boronic acid. MS(ESI)m/z:474.2[M+H]+

### Reference Example 64: Synthesis of tert-butyl-[(3R,5S)-5-[5-(4-methoxyphenyl)-1H-imidazol-2-yl]pyrrolidin-3-yl]oxy-dimethylsilane

tert-Butyl (2S,4R)-4-[tert-butyl(dimethyl)silyl]oxy-2-[5-(4-methoxyphenyl)-1H-imidazol-2-yl]pyrrolidine-1-carboxylate (27 mg) described in Reference Example 63 was dissolved in DCM (1.5 mL), trifluoroacetic acid (0.50 mL) was added, and the mixture was stirred at room temperature for 3 hr. After completion of the reaction, the reaction solution was concentrated under reduced pressure, and then azeotropically distilled with toluene to give the title compound (34 mg) as a yellow powder. MS(ESI)m/z:374.1[M+H]+

### Reference Example 65: Synthesis of 1-amino-3-[4-(trifluoromethyl)phenyl]propan-2-one hydrochloride

To a solution of 1-bromo-3-[4-(trifluoromethyl)phenyl]propan-2-one (3765 mg) in DMF (13 mL) was added sodium diformylamide (1337 mg) under ice-cooling, and the mixture was stirred for 1 hr, after which stirred at room temperature overnight. After completion of the reaction, water was added, and the mixture was extracted with ethyl acetate. The organic layer was washed with water and saturated brine, dried over anhydrous sodium sulfate, and filtered. The filtrate was concentrated under reduced pressure. To the obtained residue were added isopropanol (26 mL) and concentrated hydrochloric acid (26 mL), and the mixture was stirred at room temperature for 3 days. The reaction mixture was concentrated under reduced pressure, the obtained residue was washed with IPE, collected by filtration, and dried under reduced pressure to give the title compound (3.0 g) as a white powder. MS(ESI)m/z:218.2[M+H]+

### Reference Example 66: Synthesis of tert-butyl (2S,4R)-4-hydroxy-2-[[2-oxo-3-[4-(trifluoromethyl)phenyl]propyl]carbamoyl]pyrrolidine-1-carboxylate

The title compound was obtained by the same reaction and treatment as in Example 54 using (2S,4R)-1-tert-butoxycarbonyl-4-hydroxypyrrolidine-2-carboxylic acid, 1-amino-3-[4-(trifluoromethyl)phenyl]propan-2-one hydrochloride described in Reference Example 65, and DMF instead of DCM.
MS(ESI)m/z:429.3[M-H]-

### Reference Example 67: Synthesis of tert-butyl (2S,4R)-4-hydroxy-2-[5-[[4-(trifluoromethyl)phenyl]methyl]-1H-imidazol-2-yl]pyrrolidine-1-carboxylate

The title compound was obtained by the same reaction and treatment as in Reference Example 82 using tert-butyl (2S,4R)-4-hydroxy-2-[[2-oxo-3-[4-(trifluoromethyl)phenyl]propyl]carbamoyl]pyrrolidine-1-carboxylate described in Reference Example 66. MS(ESI)m/z:412.2[M+H]+

### Reference Example 68: Synthesis of tert-butyl (2S,4R)-4-[tert-butyl(dimethyl)silyl]oxy-2-[5-[3-(methylcarbamoyl)phenyl]-1H-imidazol-2-yl]pyrrolidine-1-carboxylate

The title compound was obtained by the same reaction and treatment as in Reference Example 40 using tert-butyl (2S,4R)-2-(5-bromo-1H-imidazol-2-yl)-4-[tert-butyl(dimethyl)silyl]oxypyrrolidine-1-carboxylate described in Reference Example 39, and 3-(methylaminocarbonyl)phenylboronic acid instead of (3-methoxyphenyl)boronic acid.
MS(ESI)m/z:501.3[M+H]+

### Reference Example 69: Synthesis of tert-butyl (2S,4R)-2-[5-[(4-bromophenyl)methyl]-1H-imidazol-2-yl]-4-hydroxypyrrolidine-1-carboxylate

(2S,4R)-1-tert-Butoxycarbonyl-4-hydroxypyrrolidine-2-carboxylic acid (200 mg) was dissolved in DMF (5 mL), 1-amino-3-(4-bromophenyl)propan-2-one hydrochloride (252 mg), DIPEA (0.45 mL), and HATU (395 mg) were added, and the mixture was stirred at room temperature for about 1 hr. After completion of the reaction, water was added to the reaction solution, and the aqueous layer was extracted with ethyl acetate. The organic layer was dried over anhydrous sodium sulfate, filtered, and the filtrate was concentrated under reduced pressure. The residue was purified by NH silica gel column chromatography (ethyl acetate:methanol=100:0 - 95:5). To the obtained crude purification product were added xylene (10 mL) and ammonium acetate (338 mg), and the mixture was stirred at 150°C for about 3 hr. After completion of the reaction, the reaction mixture was allowed to cool to room temperature, 1 M sodium hydroxide aqueous solution was added, and the aqueous layer was extracted with ethyl acetate. The organic layer was washed with saturated brine, dried over anhydrous sodium sulfate, filtered, and the filtrate was concentrated under reduced pressure. The residue was purified by silica gel column chromatography (ethyl acetate:methanol=100:0 - 90:10) to give the title compound (66 mg) as a yellow oil.
MS(ESI)m/z:422.3/424.3[M+H]+

### Reference Example 70: Synthesis of tert-butyl N-[(2S)-1-[(2S,4R)-2-[5-[(4-bromophenyl)methyl]-1H-imidazol-2-yl]-4-hydroxypyrrolidin-1-yl]-3,3-dimethyl-1-oxobutan-2-yl]carbamate

tert-Butyl (2S,4R)-2-[5-[(4-bromophenyl)methyl]-1H-imidazol-2-yl]-4-hydroxypyrrolidine-1-carboxylate (66 mg) described in Reference Example 69 was dissolved in 1,4-dioxane (3 mL), 4 M hydrochloric acid-dioxane (1.2 mL) was added, and the mixture was stirred at room temperature for about 16 hr. After completion of the reaction, the reaction solution was concentrated under reduced pressure, and further azeotropically distilled with toluene. To the residue were added DMF (5.0 mL), N-Boc-L-tert-leucine (47 mg), DIPEA (0.081 mL), and HATU (83 mg), and the mixture was stirred at room temperature for about 1 hr. After completion of the reaction, 1 M sodium hydroxide aqueous solution was added, and the aqueous layer was extracted with ethyl acetate. The organic layer was washed with saturated brine, dried over anhydrous sodium sulfate, filtered, and the filtrate was concentrated under reduced pressure. The residue was purified by silica gel column chromatography (ethyl acetate:methanol=100:0 - 90:10) to give the title compound (34 mg) as a pale-yellow oil. MS(ESI)m/z:535.4/537.5[M+H]+

### Reference Example 71: Synthesis of tert-butyl (2S,4R)-4-[tert-butyl(dimethyl)silylJoxy-2-carbamothioylpyrrolidine-1-carboxylate

tert-Butyl (2S,4R)-4-[tert-butyl(dimethyl)silyl]oxy-2-carbamoylpyrrolidine-1-carboxylate (976 mg) was dissolved in THF (10 mL), Lawesson reagent (916 mg) was added, and the mixture was stirred at room temperature for 3 hr. After completion of the reaction, the reaction mixture was concentrated under reduced pressure. The residue was purified by NH silica gel column chromatography (hexane:ethyl acetate=80:20 - 0:100) to give the title compound (978 mg) as a colorless powder. MS(ESI)m/z:359.5[M-H]-

### Reference Example 72: Synthesis of benzyl (2S,4R)-4-[tert-butyl(dimethyl)silyl]oxy-2-(1H-imidazol-2-yl)pyrrolidine-1-carboxylate

The title compound was obtained by the same reaction and treatment as in Reference Example 21 using benzyl (2S,4R)-4-[tert-butyl(dimethyl)silylJoxy-2-formylpyrrolidine-1-carboxylate. MS(ESI)m/z:402.4[M+H]+

### Reference Example 73: Synthesis of tert-butyl (2S,4R)-4-[tert-butyl(dimethyl)silyl]oxy-2-[1-[2-(4-chlorophenyl)ethyl]imidazol-2-yl]pyrrolidine-1-carboxylate

The title compound was obtained by the same reaction and treatment as in Reference Example 22 using tert-butyl (2S,4R)-4-[tert-butyl(dimethyl)silyl]oxy-2-(1H-imidazol-2-yl)pyrrolidine-1-carboxylate described in Reference Example 21, and 1-(2-bromoethyl)-4-chloro-benzene.
MS(ESI)m/z:506.2/508.2[M+H]+

### Reference Example 74: Synthesis of tert-butyl (2S,4R)-2-[[3-(2-chlorophenyl)-2-oxopropyl]carbamoyl]-4-hydroxypyrrolidine-1-carboxylate

The title compound was obtained by the same reaction and treatment as in Example 44 using (2S,4R)-1-tert-butoxycarbonyl-4-hydroxypyrrolidine-2-carboxylic acid, and 1-amino-3-(2-chlorophenyl)propan-2-one hydrochloride.
MS(ESI)m/z:395.2/397.0[M-H]-

### Reference Example 75: Synthesis of tert-butyl (2S,4R)-2-[5-[(2-chlorophenyl)methyl]-1H-imidazol-2-yl]-4-hydroxypyrrolidine-1-carboxylate

The title compound was obtained by the same reaction and treatment as in Reference Example 91 using tert-butyl (2S,4R)-2-[[3-(2-chlorophenyl)-2-oxopropyl]carbamoyl]-4-hydroxypyrrolidine-1-carboxylate described in Reference Example 74. MS(ESI)m/z:378.1/380.1[M+H]+

### Reference Example 76: Synthesis of tert-butyl N-[(2S)-1-[(2S,4R)-2-[5-[(2-chlorophenyl)methyl]-1H-imidazol-2-yl]-4-hydroxypyrrolidin-1-yl]-3,3-dimethyl-1-oxobutan-2-yl]carbamate

The title compound was obtained by the same reaction and treatment as in Example 59 using tert-butyl (2S,4R)-2-[5-[(2-chlorophenyl)methyl]-1H-imidazol-2-yl]-4-hydroxypyrrolidine-1-carboxylate described in Reference Example 75, and (2S)-2-(tert-butoxycarbonylamino)-3,3-dimethylbutanoic acid. MS(ESI)m/z:491.2/493.2[M+H]+

Reference Example 77: Synthesis of tert-butyl (2S,4R)-4-[tert-butyl(dimethyl)silyl]oxy-2-[5-[2-(trifluoromethyl)phenyl]-1H-imidazol-2-yl]pyrrolidine-1-carboxylate

The title compound was obtained by the same reaction and treatment as in Reference Example 40 using tert-butyl (2S,4R)-2-(5-bromo-1H-imidazol-2-yl)-4-[tert-butyl(dimethyl)silyl]oxypyrrolidine-1-carboxylate described in Reference Example 39, and [2-(trifluoromethyl)phenyl]boronic acid. MS(ESI)m/z:512.5[M+H]+

### Reference Example 78: Synthesis of tert-butyl N-[(2S)-1-[(2S,4R)-4-hydroxy-2-[5-[2-(trifluoromethyl)phenyl]-1H-imidazol-2-yl]pyrrolidin-1-yl]-3,3-dimethyl-1-oxobutan-2-yl]carbamate

The title compound was obtained by the same reaction and treatment as in Example 59 using tert-butyl (2S,4R)-4-[tert-butyl(dimethyl)silyl]oxy-2-[5-[2-(trifluoromethyl)phenyl]-1H-imidazol-2-yl]pyrrolidine-1-carboxylate described in Reference Example 77, and (2S)-2-(tert-butoxycarbonylamino)-3,3-dimethylbutanoic acid. MS(ESI)m/z:511.4[M+H]+

### Reference Example 79: Synthesis of N-formyl-N-[2-oxo-3-[2-(trifluoromethyl)phenyl]propyl]formamide

Under nitrogen atmosphere, 1-bromo-3-[2-(trifluoromethyl)phenyl]propan-2-one (1.0 g) was dissolved in DMF (4 mL), sodium diformylamide (379 mg) was gradually added under ice-cooling, and the mixture was stirred for 1 hr. The mixture was warmed to room temperature and stirred overnight. After completion of the reaction, the reaction was quenched by adding water, and the aqueous layer was extracted with ethyl acetate. The organic layer was washed with saturated brine, dried over Na2SO4, filtered, concentrated under reduced pressure, and purified by silica gel column chromatography (hexane:ethyl acetate=90:10 - 67:33) to give the title compound (664 mg) as a gray powder. MS(ESI)m/z:272.0[M-H]-

### Reference Example 80: Synthesis of 1-amino-3-[2-(trifluoromethyl)phenyl]propan-2-one hydrochloride

N-Formyl-N-[2-oxo-3-[2-(trifluoromethyl)phenyl]propyl]formamide (663 mg) described in Reference Example 79 was dissolved in a mixed solution of concentrated hydrochloric acid (3.7 mL)/isopropanol (3.7 mL), and the mixture was stirred at room temperature for 2 days. After completion of the reaction, the reaction mixture was concentrated under reduced pressure, and azeotropically distilled with toluene. The obtained residue was washed with IPE in a suspension, collected by filtration and dried under reduced pressure to give the title compound (510 mg) as a gray powder. MS(ESI)m/z:218.2[M+H]+

### Reference Example 81: Synthesis of tert-butyl (2S,4R)-4-hydroxy-2-[[2-oxo-3-[2-(trifluoromethyl)phenyl]propyl]carbamoyl]pyrrolidine-1-carboxylate

(2S,4R)-1-tert-Butoxycarbonyl-4-hydroxypyrrolidine-2-carboxylic acid (170 mg) was dissolved in DMF (7.3 mL), WSC·HCl (183 mg) and HOBt (149 mg) were added, and the mixture was stirred for about 10 min. Then, 1-amino-3-[2-(trifluoromethyl)phenyl]propan-2-one hydrochloride (205 mg) described in Reference Example 80, and Et3N (409 µL) were added, and the mixture was stirred for 4 hr. After completion of the reaction, the reaction was quenched by adding water, and the aqueous layer was extracted with ethyl acetate. The organic layer was washed with saturated brine, dried over Na2SO4, filtered, concentrated under reduced pressure, and purified by silica gel column chromatography (ethyl acetate:methanol=100:0 - 85:15) to give the title compound (249 mg) as a brown oil. MS(ESI)m/z:429.3[M-H]-

### Reference Example 82: Synthesis of tert-butyl (2S,4R)-4-hydroxy-2-[5-[[2-(trifluoromethyl)phenyl]methyl]-1H-imidazol-2-yl]pyrrolidine-1-carboxylate

Xylene (3.4 mL) was added to tert-butyl (2S,4R)-4-hydroxy-2-[[2-oxo-3-[2-(trifluoromethyl)phenyl]propyl]carbamoyl]pyrrolidine-1-carboxylate (148 mg) described in Reference Example 81, ammonium acetate (265 mg) was added, and the mixture was stirred at 160°C for about 1 hr in a microwave reactor. After completion of the reaction, 1 M sodium hydroxide aqueous solution was added to the reaction solution, and the aqueous layer was extracted with ethyl acetate. The organic layer was washed with saturated brine/water, dried over anhydrous sodium sulfate, filtered, and the filtrate was concentrated under reduced pressure. The residue was purified by silica gel column chromatography (ethyl acetate:methanol=100:0 - 80:20) to give the title compound (83 mg) as a brown oil. MS(ESI)m/z:412.4[M+H]+

### Reference Example 83: Synthesis of tert-butyl N-[(2S)-1-[(2S,4R)-4-hydroxy-2-[5-[[2-(trifluoromethyl)phenyl]methyl]-1H-imidazol-2-yl]pyrrolidin-1-yl]-3,3-dimethyl-1-oxobutan-2-yl]carbamate

The title compound was obtained by the same reaction and treatment as in Example 60 using tert-butyl (2S,4R)-4-hydroxy-2-[5-[[2-(trifluoromethyl)phenyl]methyl]-1H-imidazol-2-yl]pyrrolidine-1-carboxylate described in Reference Example 82, and (2S)-2-(tert-butoxycarbonylamino)-3,3-dimethylbutanoic acid instead of 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid. MS(ESI)m/z:525.2[M+H]+

### Reference Example 84: Synthesis of tert-butyl N-[(2S)-1-[(2S,4R)-4-hydroxy-2-[5-[[3-(trifluoromethyl)phenyl]methyl]-1H-imidazol-2-yl]pyrrolidin-1-yl]-3,3-dimethyl-1-oxobutan-2-yl]carbamate

The title compound was obtained by the same reaction and treatment as in Example 60 using tert-butyl (2S,4R)-4-[tert-butyl(dimethyl)silyl]oxy-2-[5-[2-(trifluoromethyl)phenyl]-1H-imidazol-2-yl]pyrrolidine-1-carboxylate described in Reference Example 77, and (2S)-2-(tert-butoxycarbonylamino)-3,3-dimethylbutanoic acid instead of 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid. MS(ESI)m/z:525.5[M+H]+

### Reference Example 85: Synthesis of benzyl (2S,4R)-2-[1-[(4-bromophenyl)methyl]imidazol-2-yl]-4-[tert-butyl(dimethyl)silylloxypyrrolidine-1-carboxylate

The title compound was obtained by the same reaction and treatment as in Reference Example 25 using benzyl (2S,4R)-4-[tert-butyl(dimethyl)silyl]oxy-2-(1H-imidazol-2-yl)pyrrolidine-1-carboxylate described in Reference Example 72, and 1-bromo-4-(bromomethyl)benzene instead of 1-(2-bromoethyl)-3-chlorobenzene. MS(ESI)m/z:570.4/572.4[M+H]+

### Reference Example 86: Synthesis of benzyl (2S,4R)-4-[tert-butyl(dimethyl)silyl]oxy-2-[1-[[4-(4-methyl-1,3-thiazol-5-yl)phenyl]methyl]imidazol-2-yl]pyrrolidine-1-carboxylate

The title compound was obtained by the same reaction and treatment as in Example 56 using benzyl (2S,4R)-2-[1-[(4-bromophenyl)methyl]imidazol-2-yl]-4-[tert-butyl(dimethyl)silyl]oxypyrrolidine-1-carboxylate described in Reference Example 85. MS(ESI)m/z:589.6[M+H]+

### Reference Example 87: Synthesis of tert-butyl N-[(2S)-1-[(2S,4R)-4-hydroxy-2-[1-[[4-(4-methyl-1,3-thiazol-5-yl)phenyl]methyl]imidazol-2-yl]pyrrolidin-1-yl]-3,3-dimethyl-1-oxobutan-2-yl]carbamate

Acetic acid (3 mL) was added to benzyl (2S,4R)-4-[tert-butyl(dimethyl)silyl]oxy-2-[1-[[4-(4-methyl-1,3-thiazol-5-yl)phenyl]methyl]imidazol-2-yl]pyrrolidine-1-carboxylate (128 mg) described in Reference Example 86, HBr in acetic acid (0.38 mL) was added, and the mixture was stirred at room temperature for about 2 hr. After completion of the reaction, chloroform and water were added, and reversed-phase extraction was performed. To the aqueous layer was added 4 M sodium hydroxide aqueous solution, and the aqueous layer was extracted with ethyl acetate. The organic layer was washed with saturated brine, dried over anhydrous sodium sulfate, filtered, and the filtrate was concentrated under reduced pressure. To the residue were added DMF (5 mL), N-Boc-L-tert-leucine (50 mg), HATU (88 mg), and DIPEA (0.075 mL), and the mixture was stirred at room temperature for about 30 min. After completion of the reaction, water was added, and the aqueous layer was extracted with ethyl acetate. The organic layer was washed with saturated brine/water, dried over anhydrous sodium sulfate, filtered, and the filtrate was concentrated under reduced pressure. The residue was purified by silica gel column chromatography (ethyl acetate:methanol=100:0 - 95:5) to give the title compound (67 mg) as a colorless oil.
MS(ESI)m/z:554.5[M+H]+

### Reference Example 88: Synthesis of tert-butyl N-[(2S)-1-[(2S,4R)-2-[5-(4-bromophenyl)-1H-imidazol-2-yl]-4-hydroxypyrrolidin-1-yl]-3,3-dimethyl-1-oxobutan-2-yl]carbamate

The title compound was obtained by the same reaction and treatment as in Example 60 using tert-butyl (2S,4R)-2-[5-(4-bromophenyl)-1H-imidazol-2-yl]-4-hydroxypyrrolidine-1-carboxylate, and (2S)-2-(tert-butoxycarbonylamino)-3,3-dimethylbutanoic acid instead of 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid. MS(ESI)m/z:521.3/523.3[M+H]+

### Reference Example 89: Synthesis of tert-butyl N-[(2S)-1-[(2S,4R)-4-hydroxy-2-[5-[4-(4-methyl-1,3-thiazol-5-yl)phenyl]-1H-imidazol-2-yl]pyrrolidin-1-yl]-3,3-dimethyl-1-oxobutan-2-yl]carbamate

The title compound was obtained by the same reaction and treatment as in Example 56 using tert-butyl N-[(2S)-1-[(2S,4R)-2-[5-(4-bromophenyl)-1H-imidazol-2-yl]-4-hydroxypyrrolidin-1-yl]-3,3-dimethyl-1-oxobutan-2-yl]carbamate described in Reference Example 88. MS(ESI)m/z:540.5[M+H]+

### Reference Example 90: Synthesis of tert-butyl (2S,4R)-2-[[3-(3-bromophenyl)-2-oxopropyl]carbamoyl]-4-hydroxypyrrolidine-1-carboxylate

The title compound was obtained by the same reaction and treatment as in Example 44 using 1-amino-3-(3-bromophenyl)propan-2-one hydrochloride and (2S,4R)-1-tert-butoxycarbonyl-4-hydroxypyrrolidine-2-carboxylic acid instead of 2-(3-methoxy-1,2-oxazol-5-yl)-3-methylbutanoic acid. MS(ESI)m/z:439.1/441.9[M-H]-

### Reference Example 91: Synthesis of tert-butyl (2S,4R)-2-[5-[(3-bromophenyl)methyl]-1H-imidazol-2-yl]-4-hydroxypyrrolidine-1-carboxylate

Xylene (6.5 mL) and 1,4-dioxane (1 mL) were added to tert-butyl (2S,4R)-2-[[3-(3-bromophenyl)-2-oxopropyl]carbamoyl]-4-hydroxypyrrolidine-1-carboxylate (581 mg) described in Reference Example 90, ammonium acetate (2001 mg) was added, and the mixture was stirred at 150°C for about 24 hr. After completion of the reaction, the reaction mixture was allowed to cool to room temperature and methanol and 1 M sodium hydroxide aqueous solution were added, and the aqueous layer was extracted with ethyl acetate. The organic layer was washed with saturated brine, dried over anhydrous sodium sulfate, filtered, and the filtrate was concentrated under reduced pressure. The residue was purified by silica gel column chromatography (ethyl acetate:methanol=100:0 - 90:10) to give the title compound (222 mg) as a yellow powder. MS(ESI)m/z:422.3/424.3[M+H]+

### Reference Example 92: Synthesis of tert-butyl N-[(2S)-1-[(2S,4R)-2-[5-[(3-bromophenyl)methyl]-1H-imidazol-2-yl]-4-hydroxypyrrolidin-1-yl]-3,3-dimethyl-1-oxobutan-2-yl]carbamate

The title compound was obtained by the same reaction and treatment as in Example 60 using tert-butyl (2S,4R)-2-[5-[(3-bromophenyl)methyl]-1H-imidazol-2-yl]-4-hydroxypyrrolidine-1-carboxylate described in Reference Example 91, and (2S)-2-(tert-butoxycarbonylamino)-3,3-dimethylbutanoic acid instead of 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid. MS(ESI)m/z:535.4/537.3[M+H]+

### Reference Example 93: Synthesis of tert-butyl (2S,4R)-4-hydroxy-2-[5-[[3-(4-methylthiazol-5-yl)phenyl]methyl]-1H-imidazol-2-yl]pyrrolidine-1-carboxylate

The title compound was obtained by the same reaction and treatment as in Example 56 using tert-butyl (2S,4R)-2-[5-[(3-bromophenyl)methyl]-1H-imidazol-2-yl]-4-hydroxypyrrolidine-1-carboxylate described in Reference Example 91.
MS(ESI)m/z:441.4[M+H]+

### Reference Example 94: Synthesis of tert-butyl N-[(1S)-1-[(2S,4R)-4-hydroxy-2-[5-[[3-(4-methylthiazol-5-yl)phenyl]methyl]-1H-imidazol-2-yl]pyrrolidine-1-carbonyl]-2,2-dimethyl-propyl]carbamate

The title compound was obtained by the same reaction and treatment as in Example 60 using tert-butyl (2S,4R)-4-hydroxy-2-[5-[[3-(4-methylthiazol-5-yl)phenyl]methyl]-1H-imidazol-2-yl]pyrrolidine-1-carboxylate described in Reference Example 93, and (2S)-2-(tert-butoxycarbonylamino)-3,3-dimethylbutanoic acid instead of 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid. MS(ESI)m/z:554.3[M+H]+

### Reference Example 95: Synthesis of tert-butyl N-[(2S)-1-[(2S,4R)-4-hydroxy-2-[5-[[4-(trifluoromethyl)phenyl]methyl]-1H-imidazol-2-yl]pyrrolidin-1-yl]-3,3-dimethyl-1-oxobutan-2-yl]carbamate

The title compound was obtained by the same reaction and treatment as in Example 60 using tert-butyl (2S,4R)-4-hydroxy-2-[5-[[4-(trifluoromethyl)phenyl]methyl]-1H-imidazol-2-yl]pyrrolidine-1-carboxylate described in Reference Example 67 and (2S)-2-(tert-butoxycarbonylamino)-3,3-dimethylbutanoic acid. MS(ESI)m/z:525.5[M+H]+

### Reference Example 96: Synthesis of N-[(2S)-1-[(2S,4R)-2-[5-[(4-bromophenyl)methyl]-1H-imidazol-2-yl]-4-hydroxypyrrolidin-1-yl]-3,3-dimethyl-1-oxobutan-2-yl]acetamide

tert-Butyl N-[(2S)-1-[(2S,4R)-2-[5-[(4-bromophenyl)methyl]-1H-imidazol-2-yl]-4-hydroxypyrrolidin-1-yl]-3,3-dimethyl-1-oxobutan-2-yl]carbamate (34 mg) described in Reference Example 70 was dissolved in 1,4-dioxane (3 mL), 4 M hydrochloric acid-dioxane (0.5 mL) was added at room temperature and the mixture was stirred for 6 hr. After completion of the reaction, the reaction solution was concentrated under reduced pressure, and then azeotropically distilled with toluene. The obtained residue was dissolved in dichloromethane (5 mL), triethylamine (53 µL) and acetic anhydride (12 µL) were added, and the mixture was stirred at room temperature for 30 min. After completion of the reaction, 1 M sodium hydroxide aqueous solution was added, and the mixture was extracted with ethyl acetate, washed with saturated brine, and dried over anhydrous sodium sulfate. The organic layer was filtered, and the filtrate was concentrated under reduced pressure. The obtained residue was purified by silica gel column chromatography (ethyl acetate:methanol=90:10) to give the title compound (16 mg) as a colorless powder. MS(ESI)m/z:477.2/479.2[M+H]+

### Reference Example 97: Synthesis of N-[(2S)-1-[(2S,4R)-2-[1-[2-(4-chlorophenyl)ethyl]imidazol-2-yl]-4-hydroxypyrrolidin-1-yl]-3,3-dimethyl-1-oxobutan-2-yl]acetamide

tert-Butyl (2S,4R)-4-[tert-butyl(dimethyl)silyl]oxy-2-[1-[2-(4-chlorophenyl)ethyl]imidazol-2-yl]pyrrolidine-1-carboxylate (52 mg) described in Reference Example 73 was dissolved in dichloromethane (2 mL), TFA (2 mL) was added, and the mixture was stirred at room temperature for 30 min. After completion of the reaction, the reaction solution was concentrated under reduced pressure, and then azeotropically distilled twice with toluene. The obtained residue was dissolved in DMF (3 mL), DIPEA (89 µL), (2S)-2-(tert-butoxycarbonylamino)-3,3-dimethylbutanoic acid (29 mg), and HATU (51 mg) were added, and the mixture was stirred at room temperature for 30 min. The reaction mixture was diluted with ethyl acetate, and the organic layer was washed with water and saturated brine, dried over anhydrous sodium sulfate, filtered, and the filtrate was concentrated under reduced pressure. The obtained residue was dissolved in 1,4-dioxane (2 mL), 4 M hydrochloric acid-dioxane (2 mL) was added, and the mixture was stirred at room temperature for 2 hr. The reaction mixture was concentrated under reduced pressure, and the obtained residue was suspended in THF (3 mL). Triethylamine (71 µL) and acetic anhydride (29 µL) were added, and the mixture was stirred at room temperature for 30 min. After completion of the reaction, water and chloroform were added, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (26 mg) as a pale-yellow oil. MS(ESI)m/z:447.4/449.4[M+H]+

### Reference Example 98: Synthesis of methyl 2-[(2S,4R)-4-[tert-butyl(dimethyl)silyl]oxy-1-[(2-methylpropan-2-yl)oxycarbonyl]pyrrolidin-2-yl]-1-[(3-chlorophenyl)methyl]imidazole-4-carboxylate

DMF (2 mL), cesium carbonate (694 mg), and 3-chlorobenzylbromide (140 µL) were added to methyl 2-[(2S,4R)-4-[tert-butyl(dimethyl)silyl]oxy-1-[(2-methylpropan-2-yl)oxycarbonyl]pyrrolidin-2-yl]-1H-imidazole-5-carboxylate (299 mg) described in Reference Example 3, and the mixture was stirred at 70°C for about 3 hr. After completion of the reaction, to the reaction mixture were added chloroform and water, and the organic layer was separated by a Phase-separator (registered trade mark), and concentrated under reduced pressure. The residue was purified by silica gel column chromatography (hexane:ethyl acetate=90:10 - 40:60) to give the title compound (191 mg) as a white powder.
MS (ESI) m/z:550.3/552.3[M+H] +

### Reference Example 99: Synthesis of methyl 2-[(2S,4R)-4-[tert-butyl(dimethyl)silyl]oxy-1-[(2-methylpropan-2-yl)oxycarbonyl]pyrrolidin-2-yl]-1-[[3-(4-methyl-1,3-thiazol-5-yl)phenyl]methyl]imidazole-4-carboxylate

4-Methyl-5-(4,4,5,5-tetramethyl-1,3,2-dioxoborolan-2-yl)thiazole (102 mg), XPhos Pd G2 (25 mg), RuPhos (42 mg), sodium carbonate (130 mg), 1,4-dioxane (2 mL), and water (400 µL) were added to methyl 2-[(2S,4R)-4-[tert-butyl(dimethyl)silyl]oxy-1-[(2-methylpropan-2-yl)oxycarbonyl]pyrrolidin-2-yl]-1-[(3-chlorophenyl)methyl]imidazole-4-carboxylate (160 mg) described in Reference Example 98, and the mixture was stirred under nitrogen atmosphere at 100°C for about 2.5 hr. After completion of the reaction, the reaction mixture was purified by silica gel column chromatography (hexane:ethyl acetate=80:20 - 20:80) to give the title compound (185 mg) as a pale-yellow oil. MS(ESI)m/z:613.7[M+H]+

### Reference Example 100: Synthesis of tert-butyl (2S,4R)-4-[tert-butyl(dimethyl)silyl]oxy-2-[1-(2-methoxy-2-oxoethyl)imidazol-2-yl]pyrrolidine-1-carboxylate

DMF (2 mL), cesium carbonate (283 mg), and methyl 2-bromoacetate (60 µL) were added to tert-butyl (2S,4R)-4-[tert-butyl(dimethyl)silyl]oxy-2-(1H-imidazol-2-yl)pyrrolidine-1-carboxylate (231 mg) described in Reference Example 21, and the mixture was stirred at room temperature for about 20 hr. After completion of the reaction, the reaction mixture was purified by silica gel column chromatography (hexane:ethyl acetate=90:10 - 40:60) to give the title compound (220 mg) as a pale-yellow oil. MS(ESI)m/z:440.5[M+H]+

### Reference Example 101: Synthesis of 4-[2-[(2S,4R)-4-[tert-butyl(dimethyl)silyl]oxy-1-[(2-methylpropan-2-yl)oxycarbonyl]pyrrolidin-2-yl]-1H-imidazol-5-yl]benzoic acid

4-Carboxyphenylboronic acid (22 mg), PdCl2(AmPhos)2 (4.8 mg), potassium carbonate (28 mg), DME (0.67 mL), and water (0.22 mL) were added to tert-butyl (2S,4R)-2-(5-bromo-1H-imidazol-2-yl)-4-[tert-butyl(dimethyl)silyl]oxypyrrolidine-1-carboxylate (30 mg) described in Reference Example 39, and the mixture was stirred under nitrogen atmosphere and heating under reflux conditions for about 3 hr. After cooling to room temperature, 4-carboxyphenylboronic acid (22 mg), PdCl2(AmPhos)2 (4.8 mg), potassium carbonate (28 mg), DME (1.3 mL), and water (0.4 mL) were added, and the mixture was stirred under nitrogen atmosphere and heating under reflux conditions for about 18 hr. After completion of the reaction, to the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (28 mg) as a yellow oil. MS(ESI)m/z:488.2[M+H]+

### Reference Example 102: Synthesis of methyl 2-[(2S,4R)-4-[tert-butyl(dimethyl)silyl]oxy-1-[(2-methylpropan-2-yl)oxycarbonyl]pyrrolidin-2-yl]-1-[(4-methoxyphenyl)methyl]imidazole-4-carboxylate

The title compound was obtained by the same reaction and treatment as in Reference Example 22 using methyl 2-[(2S,4R)-4-[tert-butyl(dimethyl)silyl]oxy-1-[(2-methylpropan-2-yl)oxycarbonyl]pyrrolidin-2-yl]-1H-imidazole-5-carboxylate described in Reference Example 3 and 4-methoxybenzylchloride instead of 3-phenylbenzylbromide. MS(ESI)m/z:546.5[M+H]+

### Reference Example 103: Synthesis of tert-butyl (2S,4R)-4-[tert-butyl(dimethyl)silyl]oxy-2-[4-(hydroxymethyl)-1-[(4-methoxyphenyl)methyl]imidazol-2-yl]pyrrolidine-1-carboxylate

DCM (3 mL) was added to methyl 2-[(2S,4R)-4-[tert-butyl(dimethyl)silyl]oxy-1-[(2-methylpropan-2-yl)oxycarbonyl]pyrrolidin-2-yl]-1-[(4-methoxyphenyl)methyl]imidazole-4-carboxylate (195 mg) described in Reference Example 102, DIBAL-H (1.0 mol/L toluene solution, 1.5 mL) was added at 0°C, and the mixture was stirred for about 2 hr. Ethyl acetate and Rochelle salt were added and stirred at 0°C. To the reaction mixture were added chloroform and water, and the organic layer was separated by a Phase-separator (registered trade mark), and concentrated under reduced pressure. The residue was purified by silica gel column chromatography (chloroform: methanol=99:1 - 80:20) to give the title compound (132 mg) as a white powder.
MS (ESI) m/z:518.6[M+H] +

### Reference Example 104: Synthesis of tert-butyl (2S,4R)-4-[tert-butyl(dimethyl)silyl]oxy-2-[1-[(4-methoxyphenyl)methyl]-4-(phenylmethoxymethyl)imidazol-2-yl]pyrrolidine-1-carboxylate

THF (1 mL) was added to tert-butyl (2S,4R)-4-[tert-butyl(dimethyl)silyl]oxy-2-[4-(hydroxymethyl)-1-[(4-methoxyphenyl)methyl]imidazol-2-yl]pyrrolidine-1-carboxylate (56 mg) described in Reference Example 103, benzyl bromide (25 µL), sodium hydride (8 mg), and TBAI (6 mg) were added at 0°C, and the mixture was stirred while gradually allowing to warm to room temperature, and the mixture was stirred at room temperature for about 8 hr. To the reaction mixture were added chloroform and water, and the organic layer was separated by a Phase-separator (registered trade mark), and concentrated under reduced pressure. The residue was purified by silica gel column chromatography (hexane:ethyl acetate=90:10 - 50:50) to give the title compound (64 mg) as a pale-yellow oil. MS(ESI)m/z:608.7[M+H]+

### Reference Example 105: Synthesis of tert-butyl N-[(2S)-1-[(2S,4R)-4-hydroxy-2-[1-[(4-methoxyphenyl)methyl]-4-(phenylmethoxymethyl)imidazol-2-yl]pyrrolidin-1-yl]-3,3-dimethyl-1-oxobutan-2-yl]carbamate

The title compound was obtained by the same reaction and treatment as in Example 60 using tert-butyl (2S,4R)-4-[tert-butyl(dimethyl)silyl]oxy-2-[1-[(4-methoxyphenyl)methyl]-4-(phenylmethoxymethyl)imidazol-2-yl]pyrrolidine-1-carboxylate described in Reference Example 104, and (2S)-2-(tert-butoxycarbonylamino)-3,3-dimethylbutanoic acid instead of 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid. MS (ESI)m/z:607.4 [M+H]+

### Reference Example 106: Synthesis of N-(cyclopropylmethyl)-1-[4-(4-methylthiazol-5-yl)phenyl]methanamine

To 4-methyl-5-(4,4,5,5-tetramethyl-1,3,2-dioxaborolan-2-yl)thiazole (65 mg), 1-(4-bromophenyl)-N-(cyclopropylmethyl)methanamine (57 mg), PdCl2(PPh3)2 (20 mg), potassium carbonate (67 mg), and Pd(dppf)Cl2·CH2Cl2 (20 mg) were added water (0.48 mL) and 1,4-dioxane (1.2 mL), and the mixture was stirred under nitrogen atmosphere at 100°C overnight. To the reaction mixture were added anhydrous sodium sulfate and chloroform, and the organic layer was separated by a Phase-separator (registered trade mark), and concentrated under reduced pressure. The residue was purified by silica gel column chromatography (hexane:ethyl acetate=90:10 - 50:50) to give the title compound (18 mg) as a colorless oil. MS(ESI)m/z:259.1[M+H]+

### Reference Example 107: Synthesis of benzyl (2S,4R)-4-[tert-butyl(dimethyl)silyl]oxy-2-(5-iodo-1H-imidazol-2-yl)pyrrolidine-1-carboxylate

To a solution of benzyl (2S,4R)-4-[tert-butyl(dimethyl)silyl]oxy-2-(4,5-diiodo-1H-imidazol-2-yl)pyrrolidine-1-carboxylate (324 mg) described in Reference Example 147 in water (4 mL) and ethanol (4 mL) was added sodium sulfite (688 mg), and the mixture was stirred with heating under reflux for 2 days. After cooling to room temperature, saturated brine was added, and the mixture was extracted with ethyl acetate. The organic layer was dried over anhydrous sodium sulfate, filtered, and concentrated under reduced pressure. The obtained residue was purified by preparative HPLC (10 mM ammonium carbonate aqueous solution-acetonitrile) to give the title compound (120 mg) as a colorless viscous substance. MS(ESI)m/z:528.3[M+H]+

### Reference Example 108: Synthesis of benzyl (2S,4R)-4-[tert-butyl(dimethyl)silyl]oxy-2-[5-[2-(4-chlorophenyl)ethynyl]-1H-imidazol-2-yl]pyrrolidine-1-carboxylate

To a solution of benzyl (2S,4R)-4-[tert-butyl(dimethyl)silylJoxy-2-(5-iodo-1H-imidazol-2-yl)pyrrolidine-1-carboxylate described in Reference Example 107 (120 mg) in THF (5 mL) was added 1-chloro-4-ethynylbenzene (40 mg), DIPEA (0.24 mL) deaerate. PdCl2(PPh3)2 (8.0 mg), copper (I) iodide (6.5 mg), under nitrogen atmosphere 80°C for 2 hr stirred. After cooling to room temperature, anhydrous sodium carbonate and silica gel, filtered, and concentrated under reduced pressure. The obtained residue was purified by silica gel column chromatography (hexane:ethyl acetate=100:0 - 90:10) to give the title compound (120 mg) as a yellow oil. MS(ESI)m/z:536.4/538.4[M+H]+

### Reference Example 109: Synthesis of tert-butyl N-[(1S)-1-[(2S,4R)-2-[5-[2-(4-chlorophenyl)ethyl]-1H-imidazol-2-yl]-4-hydroxypyrrolidine-1-carbonyl]-2,2-dimethyl-propyl]carbamate

To a solution of benzyl (2S,4R)-4-[tert-butyl(dimethyl)silyl]oxy-2-[5-[2-(4-chlorophenyl)ethynyl]-1H-imidazol-2-yl]pyrrolidine-1-carboxylate (120 mg) described in Reference Example 108 in methanol (5 mL) were added 4 M hydrochloric acid-dioxane (1.1 mL) and 10%Pd/C (24 mg), and the mixture was stirred under hydrogen atmosphere at room temperature overnight. After nitrogen substitution, the mixture was filtered, and the filtrate was concentrated under reduced pressure. To the obtained residue were added acetic acid (3 mL) and 33% hydrogen bromide-acetic acid solution (1 mL), and the mixture was stirred at room temperature for 6 hr. The reaction mixture was concentrated under reduced pressure, and azeotropically distilled 3 times with toluene. To the obtained residue were added DMF (5 mL), (2S)-2-(tert-butoxycarbonylamino)-3,3-dimethylbutanoic acid (155 mg), DIPEA (387 µL), and HATU (340 mg), and the mixture was stirred at room temperature for 3 hr. After completion of the reaction, saturated brine was added, and the mixture was extracted with ethyl acetate, dried over anhydrous sodium sulfate, filtered, and concentrated under reduced pressure. The obtained residue was purified by silica gel column chromatography (ethyl acetate:methanol=100:0 - 90:10) and concentrated under reduced pressure. To the obtained residue were added methanol (10 mL) and 1 M sodium hydroxide aqueous solution (10 mL), and the mixture was stirred at room temperature for 1 hr. After completion of the reaction, the reaction mixture was concentrated under reduced pressure to remove methanol, extracted with ethyl acetate, dried over anhydrous sodium sulfate, filtered, and concentrated under reduced pressure. The obtained residue was purified by silica gel column chromatography (ethyl acetate:methanol=100:0 - 90:10) to give the title compound (40 mg) as a pale-yellow oil. MS(ESI)m/z:505.4/507.4[M+H]+

### Reference Example 110: Synthesis of tert-butyl (2S,4R)-4-[tert-butyl(dimethyl)silyl]oxy-2-(4,5-diiodo -1H-imidazol-2-yl)pyrrolidine-1-carboxylate

To a solution of tert-butyl (2S,4R)-4-[tert-butyl(dimethyl)silyl]oxy-2-(1H-imidazol-2-yl)pyrrolidine-1-carboxylate (500 mg) described in Reference Example 21 in DCM (9 mL) was added NIS (612 mg), and the mixture was stirred at room temperature for 2 hr. After completion of the reaction, the reaction mixture was concentrated under reduced pressure, and the obtained residue was purified by silica gel column chromatography (hexane:ethyl acetate=80:20 - 40:60) to give the title compound (739 mg) as a colorless powder. MS (ESI)m/z:620.2[M+H] +

### Reference Example 111: Synthesis of tert-butyl (2S,4R)-4-[tert-butyl(dimethyl)silyl]oxy-2-[5-[2-(3-chlorophenyl)ethynyl]-1H-imidazol-2-yl]pyrrolidine-1-carboxylate

To a solution of tert-butyl (2S,4R)-4-[tert-butyl(dimethyl)silyl]oxy-2-(5-iodo -1H-imidazol-2-yl)pyrrolidine-1-carboxylate described in Reference Example 112 (369 mg) in THF (5 mL) were added 1-chloro-3-ethynyl-benzene (133 mg) and DIPEA (0.78 mL), and the mixture was deaerated. PdCl2(PPh3)2 (26 mg) and copper (I) iodide (21 mg) were added, and the mixture was stirred under nitrogen atmosphere at 80°C for 2 hr. After cooling to room temperature, anhydrous sodium carbonate and silica gel were added, and the mixture was filtered and concentrated under reduced pressure. The obtained residue was purified by silica gel column chromatography (hexane:ethyl acetate=80:20 - 50:50) to give the title compound (298 mg) as a pale-yellow oil. MS(ESI)m/z:502.4/504.4[M+H]+

### Reference Example 112: Synthesis of tert-butyl (2S,4R)-4-[tert-butyl(dimethyl)silylJoxy-2-(5-iodo-1H-imidazol-2-yl)pyrrolidine-1-carboxylate

To a solution of tert-butyl (2S,4R)-4-[tert-butyl(dimethyl)silyl]oxy-2-(4,5-diiodo -1H-imidazol-2-yl)pyrrolidine-1-carboxylate (739 mg) described in Reference Example 110 in water (10 mL) and ethanol (30 mL) was added sodium sulfite (7519 mg), and the mixture was stirred with heating under reflux overnight. After cooling to room temperature, saturated brine was added, and the mixture was extracted with ethyl acetate. The organic layer was dried over anhydrous sodium sulfate, filtered, and concentrated under reduced pressure. The obtained residue was purified by silica gel column chromatography (hexane:ethyl acetate=90:10 - 85:15) to give the title compound (369 mg) as a colorless viscous substance. MS(ESI)m/z:494.3[M+H]+

### Reference Example 113: Synthesis of tert-butyl N-[(1S)-1-[(2S,4R)-2-[5-[2-(3-chlorophenyl)ethyl]-1H-imidazol-2-yl]-4-hydroxypyrrolidine-1-carbonyl]-2,2-dimethyl-propyl]carbamate

To a solution of tert-butyl (2S,4R)-4-[tert-butyl(dimethyl)silyl]oxy-2-[5-[2-(3-chlorophenyl)ethynyl]-1H-imidazol-2-yl]pyrrolidine-1-carboxylate (145 mg) described in Reference Example 111 in methanol (5 mL) were added 4 M hydrochloric acid-dioxane (2.2 mL) and 10%Pd/C (174 mg), and the mixture was stirred under hydrogen atmosphere at room temperature overnight. After nitrogen substitution, the mixture was filtered, and the filtrate was concentrated under reduced pressure. To the obtained residue were added (2S)-2-(tert-butoxycarbonylamino)-3,3-dimethylbutanoic acid (100 mg), DMF (5 mL), DIPEA (0.30 mL), and HATU (176 mg), and the mixture was stirred at room temperature for 2 hr. After completion of the reaction, saturated brine was added, and the mixture was extracted with ethyl acetate, dried over anhydrous sodium sulfate, filtered, and concentrated under reduced pressure. The obtained residue was purified by silica gel column chromatography (ethyl acetate:methanol=100:0 - 90:10) to give the title compound (74 mg) as a colorless oil. MS(ESI)m/z:505.4/507.4[M+H]+

### Reference Example 114: Synthesis of tert-butyl (2S,4R)-2-[1-[(3-bromophenyl)methyl]imidazol-2-yl]-4-[tert-butyl(dimethyl)silyl]oxy-pyrrolidine-1-carboxylate

tert-Butyl (2S,4R)-4-[tert-butyl(dimethyl)silyl]oxy-2-(1H-imidazol-2-yl)pyrrolidine-1-carboxylate (1256 mg) described in Reference Example 21, 1-bromo-3-(bromomethyl)benzene (1281 mg), cesium carbonate (3349 mg), and DMF (4.3 mL) were mixed and stirred at 70°C for 1.5 hr. After cooling to room temperature, acetic acid (195 µL) was added and the mixture was stirred for 15 min. Saturated ammonium chloride aqueous solution was added, and the mixture was extracted with ethyl acetate, dried over anhydrous sodium sulfate, filtered, and concentrated under reduced pressure. The obtained residue was purified by silica gel column chromatography (hexane:ethyl acetate=32:68 - 11:89) to give the title compound (1358 mg) as a yellow oil. MS(ESI)m/z:536.4/538.4[M+H]+

### Reference Example 115: Synthesis of methyl 2-[(2S,4R)-1-tert-butoxycarbonyl-4-[tert-butyl(dimethyl)silyl]oxy-pyrrolidin-2-yl]-4-iodo-1H-imidazole-5-carboxylate

To a solution of methyl 2-[(2S,4R)-4-[tert-butyl(dimethyl)silyl]oxy-1-[(2-methylpropan-2-yl)oxycarbonyl]pyrrolidin-2-yl]-1H-imidazole-5-carboxylate (50 mg) described in Reference Example 3 in DCM (1176 µL) was added NIS (53 mg), and the mixture was stirred under shading at room temperature overnight. After completion of the reaction, sodium sulfite and saturated aqueous sodium hydrogen carbonate solution were added, and the mixture was extracted with chloroform. The organic layer was separated by a Phase-separator (registered trade mark), and concentrated under reduced pressure. The obtained residue was purified by silica gel column chromatography (hexane:ethyl acetate=90:10 - 70:30) to give the title compound (56 mg) as a colorless viscous substance. MS(ESI)m/z:552.4[M+H]+

### Reference Example 116: Synthesis of methyl 2-[(2S,4R)-1-tert-butoxycarbonyl-4-[tert-butyl(dimethyl)silyl]oxy-pyrrolidin-2-yl]-4-methyl-1H-imidazole-5-carboxylate

DME (1 mL) was added to methyl 2-[(2S,4R)-1-tert-butoxycarbonyl-4-[tert-butyl(dimethyl)silyl]oxy-pyrrolidin-2-yl]-4-iodo-1H-imidazole-5-carboxylate (56 mg) described in Reference Example 115, 2,4,6-trimethyl-1,3,5,2,4,6-trioxatriborinane (14 µL), Pd(dppf)Cl2·CH2Cl2 (8.9 mg), and tripotassium phosphate (67 mg), and the mixture was stirred under nitrogen atmosphere at 80°C overnight. The reaction mixture was concentrated under reduced pressure, to the concentrated residue were added XPhos Pd G2 (9.5 mg), 1,4-dioxane (1 mL), water (100 µL), and 2,4,6-trimethyl-1,3,5,2,4,6-trioxatriborinane (14 µL), and the mixture was stirred under nitrogen atmosphere at 80°C for 6 hr. XPhos Pd G2 (13 mg) and 2,4,6-trimethyl-1,3,5,2,4,6-trioxatriborinane (14 µL) were added, and the mixture was stirred under nitrogen atmosphere at 80°C for 3 days. The reaction mixture was concentrated under reduced pressure, and the obtained residue was purified by silica gel column chromatography (hexane:ethyl acetate=87:13 - 66:34) to give the title compound (26 mg) as a white powder. MS(ESI)m/z:440.5[M+H]+

### Reference Example 117: Synthesis of tert-butyl (2S,4R)-4-hydroxy-2-[4-methyl-5-[methyl-[[4-(4-methylthiazol-5-yl)phenyl]methyl]carbamoyl]-1H-imidazol-2-yl]pyrrolidine-1-carboxylate

To a solution of methyl 2-[(2S,4R)-1-tert-butoxycarbonyl-4-[tert-butyl(dimethyl)silyl]oxy-pyrrolidin-2-yl]-4-methyl-1H-imidazole-5-carboxylate (27 mg) described in Reference Example 116 in methanol (610 µL) was added 1 M sodium hydroxide aqueous solution (244 µL), and the mixture was stirred at 50°C overnight. 1 M Sodium hydroxide aqueous solution (244 µL) was added and the mixture was further stirred for 4 hr and cooled to room temperature. 1 M Hydrochloric acid (488 µL) and ethanol were added, and the mixture was concentrated under reduced pressure, and azeotropically distilled with ethanol and toluene. To the obtained residue were added N-methyl-1-[4-(4-methyl-1,3-thiazol-5-yl)phenyl]methanamine (36 mg), HATU (32 mg), DMF (610 µL), and DIPEA (31 µL), and the mixture was stirred at room temperature overnight. HATU (20 mg) was added, and the mixture was stirred at room temperature overnight, and the reaction was discontinued by adding water (100 µL). The reaction mixture was diluted with DMSO and purified by preparative HPLC (10 mM ammonium carbonate aqueous solution-acetonitrile) to give the title compound (14 mg) as a pale-yellow powder. MS(ESI)m/z:512.3[M+H]+

### Reference Example 118: Synthesis of tert-butyl (2S,4R)-4-[tert-butyl(dimethyl)silyl]oxy-2-[1-[(1R)-1-(1-naphthyl)ethyl]imidazol-2-yl]pyrrolidine-1-carboxylate

To a solution of tert-butyl (2S,4R)-4-[tert-butyl(dimethyl)silyl]oxy-2-formylpyrrolidine-1-carboxylate (301 mg) in DCM (3.0 mL) were added magnesium sulfate (170 mg) and (1R)-1-(1-naphthyl)ethaneamine (219 µL), and the mixture was stirred at room temperature overnight. The mixture was filtered and concentrated under reduced pressure. To the concentrated residue was added a solution of ammonium acetate (118 mg) in methanol (3.0 mL), 39% glyoxal aqueous solution (160 µL) was added, and the mixture was stirred at room temperature overnight. The reaction mixture was concentrated under reduced pressure, and the concentrated residue was purified by silica gel column chromatography (hexane:ethyl acetate=85:15 - 64:36) to give the title compound (138 mg) as an orange oil. MS(ESI)m/z:522.6[M+H]+

### Reference Example 119: Synthesis of tert-butyl (2S,4R)-4-[tert-butyl(dimethyl)silyl]oxy-2-[1-[[2-[4-(trifluoromethyl)phenyl]-4-pyridyl]methyl]imidazol-2-yl]pyrrolidine-1-carboxylate

The title compound was obtained by the same reaction and treatment as in Reference Example 118 using tert-butyl (2S,4R)-4-[tert-butyl(dimethyl)silyl]oxy-2-formylpyrrolidine-1-carboxylate, [2-[4-(trifluoromethyl)phenyl]-4-pyridyl]methanamine hydrochloride described in Reference Example 148, and triethylamine. MS(ESI)m/z:603.6[M+H]+

### Reference Example 120: Synthesis of tert-butyl (2S,4R)-2-[1-[2-(4-bromophenyl)ethyl]imidazol-2-yl]-4-[tert-butyl(dimethyl)silyl]oxy-pyrrolidine-1-carboxylate

DMF (2 mL), cesium carbonate (326 mg), and 1-bromo-4-(2-bromoethyl)benzene (300 µL) were added to tert-butyl (2S,4R)-4-[tert-butyl(dimethyl)silyl]oxy-2-(1H-imidazol-2-yl)pyrrolidine-1-carboxylate (236 mg) described in Reference Example 21, and the mixture was stirred at 70°C for about 18 hr. Furthermore, 1-bromo-4-(2-bromoethyl)benzene (300 µL) was added and the mixture was stirred for 1.5 hr. Again, 1-bromo-4-(2-bromoethyl)benzene (300 µL) was added and the mixture was stirred for 1 hr. After completion of the reaction, to the reaction mixture were added chloroform and water, and the organic layer was separated by a Phase-separator (registered trade mark), and concentrated under reduced pressure. The residue was purified by preparative HPLC (10 mM ammonium carbonate aqueous solution-acetonitrile) to give the title compound (174 mg) as an orange oil.
MS(ESI)m/z:550.7/552.7[M+H]+

### Reference Example 121: Synthesis of tert-butyl (2S,4R)-4-[tert-butyl(dimethyl)silyl]oxy-2-[1-[[5-(4-fluorophenyl)-3-pyridyl]methyl]imidazol-2-yl]pyrrolidine-1-carboxylate

The title compound was obtained by the same reaction and treatment as in Reference Example 22 using tert-butyl (2S,4R)-4-[tert-butyl (dimethyl)silyl]oxy-2-(1H-imidazol-2-yl)pyrrolidine-1-carboxylate described in Reference Example 21, and 3-(chloromethyl)-5-(4-fluorophenyl)pyridine salt acid. MS(ESI)m/z:553.3[M+H]+

### Reference Example 122: Synthesis of (3R,5S)-5-(1H-imidazol-2-yl)pyrrolidin-3-ol hydrochloride

1,4-Dioxane (30 mL) and 4 M hydrochloric acid-dioxane (22 mL) were added to tert-butyl (2S,4R)-4-[tert-butyl(dimethyl)silyl]oxy-2-(1H-imidazol-2-yl)pyrrolidine-1-carboxylate (1.1 g) described in Reference Example 21 at room temperature, and the mixture was stirred for about 4 hr. After completion of the reaction, the reaction solution was concentrated under reduced pressure and azeotropically distilled with toluene to give the title compound (716 mg) as a pale-brown powder. MS(ESI)m/z:153.9[M+H]+

### Reference Example 123: Synthesis of tert-butyl (2S,4R)-2-(1H-benzimidazol-2-yl)-4-hydroxypyrrolidine-1-carboxylate

(2S,4R)-1-tert-Butoxycarbonyl-4-hydroxypyrrolidine-2-carboxylic acid (151 mg) was dissolved in DMF (2 mL), benzene-1,2-diamine (78 mg), HATU (287 mg), and DIPEA (400 µL) were added at room temperature, and the mixture was stirred for 2 hr. After completion of the reaction, chloroform and water were added, and the organic layer was separated by a Phase-separator (registered trade mark), and concentrated under reduced pressure. To the obtained residue was added acetic acid (2 mL), and the mixture was stirred at 60°C for 4 hr. After completion of the reaction, the reaction solution was concentrated under reduced pressure. The residue was purified by preparative HPLC (10 mM ammonium carbonate aqueous solution-acetonitrile) to give the title compound (143 mg) as a white powder.
MS(ESI)m/z:304.4[M+H]+

### Reference Example 124: Synthesis of tert-butyl (2S,4R)-4-[tert-butyl(dimethyl)silyl]oxy-2-[1-[2-[4-(4-methylthiazol-5-yl)phenyl]ethyl]imidazol-2-yl]pyrrolidine-1-carboxylate

The title compound was obtained by the same reaction and treatment as in Reference Example 24 using tert-butyl (2S,4R)-2-[1-[2-(4-bromophenyl)ethyl]imidazol-2-yl]-4-[tert-butyl(dimethyl)silylJoxy-pyrrolidine-1-carboxylate described in Reference Example 120, and 4-methyl-5-(4,4,5,5-tetramethyl-1,3,2-dioxaborolan-2-yl)thiazole. MS(ESI)m/z:569.7[M+H]+

### Reference Example 125: Synthesis of tert-butyl (2S,4R)-4-[tert-butyl(dimethyl)silyl]oxy-2-[1-[(3-phenyl-1,2,4-oxadiazol-5-yl)methyl]imidazol-2-yl]pyrrolidine-1-carboxylate

The title compound was obtained by the same reaction and treatment as in Reference Example 22 using tert-butyl (2S,4R)-4-[tert-butyl (dimethyl)silyl]oxy-2-(1H-imidazol-2-yl)pyrrolidine-1-carboxylate described in Reference Example 21, and 5-(chloromethyl)-3-phenyl-1,2,4-oxadiazole. MS(ESI)m/z:526.5[M+H]+

### Reference Example 126: Synthesis of tert-butyl (2S,4R)-4-[tert-butyl(dimethyl)silyl]oxy-2-[1-[(3,5-dimethyl-1-phenyl-pyrazol-4-yl)methyl]imidazol-2-yl]pyrrolidine-1-carboxylate

The title compound was obtained by the same reaction and treatment as in Reference Example 22 using tert-butyl (2S,4R)-4-[tert-butyl(dimethyl)silyl]oxy-2-(1H-imidazol-2-yl)pyrrolidine-1-carboxylate described in Reference Example 21, and 4-(chloromethyl)-3,5-dimethyl-1-phenyl-pyrazole.
MS(ESI)m/z:552.6[M+H]+

### Reference Example 127: Synthesis of tert-butyl N-[(1S)-1-[(2S,4R)-2-[1-[(3-bromophenyl)methyl]imidazol-2-yl]-4-[tert-butyl(dimethyl)silyl]oxy-pyrrolidine-1-carbonyl]-2,2-dimethylpropyl]carbamate

tert-Butyl (2S,4R)-2-[1-[(3-bromophenyl)methyl]imidazol-2-yl]-4-[tert-butyl(dimethyl)silyl]oxy-pyrrolidine-1-carboxylate (1358 mg) described in Reference Example 114 was dissolved in DCM (17 mL), trifluoroacetic acid (7.7 mL) was added at room temperature, and the mixture was stirred for 2 hr. After completion of the reaction, the reaction solution was concentrated under reduced pressure and azeotropically distilled with toluene. To the obtained residue were added ethyl acetate and saturated aqueous sodium hydrogen carbonate, and sodium chloride was added. The organic layer was extracted and concentrated under reduced pressure. The obtained residue was dissolved in DMF (13 mL), (2S)-2-(tert-butoxycarbonylamino)-3,3-dimethylbutanoic acid (761 mg), DIPEA (1.8 mL), and HATU (1443 mg) were added at room temperature, and the mixture was stirred overnight. After completion of the reaction, ethyl acetate and water were added, and the organic layer was extracted and concentrated under reduced pressure. The residue was purified by silica gel column chromatography (hexane:ethyl acetate=90:10 - 50:50) to give the title compound (1.4 g) as a pale-yellow powder. MS(ESI)m/z:649.3/651.3[M+H]+

### Reference Example 128: Synthesis of (2S)-2-amino-1-[(2S,4R)-4-hydroxy-2-[1-[[3-(4-methylthiazol-5-yl)phenyl]methyl]imidazol-2-yl]pyrrolidin-1-yl]-3,3-dimethylbutan-1-one hydrochloride

tert-Butyl N-[(1S)-1-[(2S,4R)-2-[1-[(3-bromophenyl)methyl]imidazol-2-yl]-4-[tert-butyl(dimethyl)silyl]oxy-pyrrolidine-1-carbonyl]-2,2-dimethylpropyl]carbamate (315 mg) described in Reference Example 127, 4-methyl-5-(4,4,5,5-tetramethyl-1,3,2-dioxaborolan-2-yl)thiazole (131 mg), Pd(dppf)Cl2·CH2Cl2 (40 mg), and sodium carbonate (154 mg) were dissolved in 1,4-dioxane (4 mL) and water (1 mL). After nitrogen substitution, the mixture was stirred at 90°C for 2 hr. 4-Methyl-5-(4,4,5,5-tetramethyl-1,3,2-dioxaborolan-2-yl)thiazole (131 mg), and Pd(dppf)Cl2·CH2Cl2 (40 mg) were added. After nitrogen substitution again, the mixture was stirred at 90°C for 1 hr. After completion of the reaction, anhydrous sodium sulfate was added, and the reaction solution was filtered through celite. The filtrate was concentrated under reduced pressure, and the obtained residue was purified by silica gel column chromatography (hexane:ethyl acetate=80:20 - 30:70). The obtained residue was dissolved in 1,4-dioxane (4.8 mL), 4 M hydrochloric acid-dioxane (5 mL) was added at room temperature, and the mixture was stirred for 2 hr. After completion of the reaction, the reaction solution was concentrated under reduced pressure, and azeotropically distilled with toluene to give the title compound (205 mg) as a pale-yellow powder. MS(ESI)m/z:454.4[M+H]+

### Reference Example 129: Synthesis of tert-butyl N-[(1S)-1-[(2S,4R)-4-[tert-butyl(dimethyl)silyl]oxy-2-[1-[2-[3-(4-methylthiazol-5-yl)phenyl]ethyl]imidazol-2-yl]pyrrolidine-1-carbonyl]-2,2-dimethyl-propyl]carbamate

tert-Butyl (2S,4R)-4-[tert-butyl(dimethyl)silyl]oxy-2-[1-[2-[3-(4-methyl-1,3-thiazol-5-yl)phenyl]ethyl]imidazol-2-yl]pyrrolidine-1-carboxylate (244 mg) described in Reference Example 26 was dissolved in DCM (2.9 mL), trifluoroacetic acid (1.3 mL) was added under ice-cooling, and the mixture was stirred at room temperature for 2 hr. After completion of the reaction, the reaction solution was concentrated under reduced pressure and azeotropically distilled with toluene. To the obtained residue were added ethyl acetate and saturated aqueous sodium hydrogen carbonate, and sodium chloride was added. The organic layer was extracted and concentrated under reduced pressure. The obtained residue was dissolved in DMF (2.1 mL), (2S)-2-(tert-butoxycarbonylamino)-3,3-dimethylbutanoic acid (129 mg), DIPEA (0.30 mL), and HATU (245 mg) were added at room temperature, and the mixture was stirred overnight. After completion of the reaction, ethyl acetate and water were added, and the organic layer was extracted and concentrated under reduced pressure. The residue was purified by silica gel column chromatography (hexane:ethyl acetate=80:20 - 40:60) to give the title compound (257 mg) as a colorless oil. MS (ES I) m/z: 682.7 [M+H]+

### Reference Example 130: Synthesis of (2S)-2-amino-1-[(2S,4R)-4-hydroxy-2-[1-[2-[3-(4-methylthiazol-5-yl)phenyl]ethyl]imidazol-2-yl]pyrrolidin-1-yl]-3,3-dimethylbutan-1-one hydrochloride

The title compound was obtained by the same reaction and treatment as in Reference Example 18 using tert-butyl N-[(1S)-1-[(2S,4R)-4-[tert-butyl(dimethyl)silyl]oxy-2-[1-[2-[3-(4-methylthiazol-5-yl)phenyl]ethyl]imidazol-2-yl]pyrrolidine-1-carbonyl]-2,2-dimethyl-propyl]carbamate described in Reference Example 129 and 1,4-dioxane instead of methanol. MS(ESI)m/z:468.5[M+H]+

### Reference Example 131: Synthesis of methyl 2-[(2S,4R)-1-[(2S)-2-[(1-fluorocyclopropanecarbonyl)amino]-3,3-dimethylbutanoyl]-4-hydroxypyrrolidin-2-yl]-1H-imidazole-4-carboxylate

Methyl 2-[(2S,4R)-4-[tert-butyl(dimethyl)silyl]oxy-1-[(2S)-3,3-dimethyl-2-[(2-methylpropan-2-yl)oxycarbonylamino]butanoyl]pyrrolidin-2-yl]-1H-imidazole-4-carboxylate (1000 mg) described in Reference Example 19 was dissolved in 1,4-dioxane (9 mL), 4 M hydrochloric acid-dioxane (14 mL) was added at room temperature, and the mixture was stirred for 3 hr. After completion of the reaction, the reaction mixture was washed with diisopropyl ether in a suspension, and the solid was collected by filtration and dried under reduced pressure. The obtained residue was dissolved in DMF (9 mL), 1-fluorocyclopropanecarboxylic acid (213 mg), DIPEA (0.96 mL), and HATU (776 mg) were added at room temperature and the mixture was stirred for 15 hr. After completion of the reaction, 2 M ammonia-methanol was added, and the mixture was concentrated under reduced pressure. The residue was purified by silica gel column chromatography (chloroform: methanol=100:0 - 90:10) to give the title compound (734 mg) as a colorless powder. MS(ESI)m/z:411.4[M+H]+

### Reference Example 132: Synthesis of tert-butyl N-[(1S)-1-[(2S,4R)-4-[tert-butyl(dimethyl)silyl]oxy-2-[4-(hydroxymethyl)-1H-imidazol-2-yl]pyrrolidine-1-carbonyl]-2,2-dimethylpropyl]carbamate

DCM (2.8 mL) was added to methyl 2-[(2S,4R)-4-[tert-butyl(dimethyl)silyl]oxy-1-[(2S)-3,3-dimethyl-2-[(2-methylpropan-2-yl)oxycarbonylamino]butanoyl]pyrrolidin-2-yl]-1H-imidazole-4-carboxylate (152 mg) described in Reference Example 19 at an outer temperature of 0°C, DIBAL-H (1.0 mol/L toluene solution) (0.83 mL) was added, and the mixture was stirred under nitrogen atmosphere at the same temperature for 30 min, and stirred at room temperature overnight. After cooling to 0°C, the reaction mixture was diluted with diethyl ether, water (34 uL), 15% sodium hydroxide aqueous solution (34 uL), and water (85 uL) were added, and the mixture was stirred at room temperature for 30 min. Magnesium sulfate was added and the mixture was further stirred for 15 min. Insoluble material was removed from the reaction mixture by filtration through diatomaceous earth, and the filtrate was concentrated under reduced pressure. The obtained residue was purified by silica gel column chromatography to give the title compound (60 mg) as a colorless oil. MS(ESI)m/z:511.3[M+H]+

### Reference Example 133: Synthesis of 2-[(2S,4R)-1-[(2S)-2-[(1-fluorocyclopropanecarbonyl)amino]-3,3-dimethylbutanoyl]-4-hydroxypyrrolidin-2-yl]-1H-imidazole-4-carboxylic acid

Methyl 2-[(2S,4R)-1-[(2S)-2-[(1-fluorocyclopropanecarbonyl)amino]-3,3-dimethylbutanoyl]-4-hydroxypyrrolidin-2-yl]-1H-imidazole-4-carboxylate (734 mg) described in Reference Example 131, 1 M sodium hydroxide aqueous solution (5.7 mL), and methanol (9.5 mL) were added, and the mixture was stirred at an outer temperature of 50°C for 15 hr. 1 M sodium hydroxide aqueous solution (5.7 mL) was added, and the mixture was further stirred at the same temperature for 21 hr. 1 M hydrochloric acid (11 mL) was added to adjust the pH to about 3, and the mixture was concentrated under reduced pressure. The concentrated residue was washed with water in a suspension and collected by filtration. The filtrate was concentrated, the residue was dissolved in water, applied to PoraPakTM Rxn Rp (conditioned with MeOH:45 mL, then conditioned with water: 45 mL), washed with water: 45 mL, eluted with methanol: 90 mL, and mixed with the residue obtained earlier. Three times of azeotropic distillation with toluene gave the title compound (624 mg) as a pale-brown powder. MS(ESI)m/z:395.3[M-H]-

### Reference Example 134: Synthesis of N-[[4-(4-methylthiazol-5-yl)phenyl]methyl]cyclopropaneamine

4-(4-Methylthiazol-5-yl)benzaldehyde (100 mg) described in Reference Example 9, DCM (5 mL), and cyclopropylamine (0.051 mL) were added, and the mixture was stirred at room temperature for 30 min. Sodium triacetoxyborohydride (150 mg) was added, and the mixture was further stirred at the same temperature overnight. To the reaction mixture were added chloroform and 1 M sodium hydroxide aqueous solution, and the mixture was stirred. The organic layer was separated by a Phase-separator (registered trade mark), and concentrated under reduced pressure. The obtained residue was purified by NH silica gel column chromatography (hexane:ethyl acetate=95:5 - 72:28) to give the title compound (61 mg) as a pale-yellow oil. MS(ESI)m/z:245.1[M+H]+

### Reference Example 135: Synthesis of N-[[4-(4-methylthiazol-5-yl)phenyl]methyl]tetrahydrofuran-3-amine

4-(4-Methylthiazol-5-yl)benzaldehyde (60 mg) described in Reference Example 9, DCM (3 mL), and tetrahydrofuran-3-amine (0.038 mL) were added, and the mixture was stirred at room temperature for 30 min. Sodium triacetoxyborohydride (90 mg) was added, and the mixture was stirred at the same temperature for 1 hr. To the reaction mixture were added chloroform and saturated aqueous sodium hydrogen carbonate and the mixture was stirred. The organic layer was separated by a Phase-separator (registered trade mark), and concentrated under reduced pressure. The obtained residue was purified by preparative HPLC (10 mM ammonium carbonate aqueous solution-acetonitrile) to give the title compound (58 mg) as a pale-yellow oil. MS(ESI)m/z:275.2[M+H]+

### Reference Example 136: Synthesis of N-[[4-(4-methylthiazol-5-yl)phenyl]methyl]tetrahydropyran-4-amine

4-(4-Methylthiazol-5-yl)benzaldehyde (60 mg) described in Reference Example 9, DCM (3 mL), and tetrahydropyran-4-amine (0.046 mL) were added, and the mixture was stirred at room temperature for 30 min. Sodium triacetoxyborohydride (90 mg) was added, and the mixture was stirred at the same temperature for 2 hr was stirred. acetic acid (0.017 mL), and the mixture was further stirred at the same temperature for 3.5 hr. To the reaction mixture were added chloroform and saturated aqueous sodium hydrogen carbonate and the mixture was stirred. The organic layer was separated by a Phase-separator (registered trade mark), and concentrated under reduced pressure. The obtained residue was purified by preparative HPLC (10 mM ammonium carbonate aqueous solution-acetonitrile) to give the title compound (11 mg) as a pale-yellow oil.
MS (ESI)m/z:289.3[M+H] +

### Reference Example 137: Synthesis of N-[[4-(4-methylthiazol-5-yl)phenyl]methyl]cyclobutanamine

4-(4-Methylthiazol-5-yl)benzaldehyde (60 mg) described in Reference Example 9, DCM (3 mL), and cyclobutanamine (0.038 mL) were added, and the mixture was stirred at room temperature for 30 min. Sodium triacetoxyborohydride (90 mg) was added, and the mixture was further stirred at the same temperature for 2 hr. To the reaction mixture were added chloroform and saturated aqueous sodium hydrogen carbonate and the mixture was stirred. The organic layer was separated by a Phase-separator (registered trade mark), and concentrated under reduced pressure. The obtained residue was purified by preparative HPLC (10 mM ammonium carbonate aqueous solution-acetonitrile) to give the title compound (53 mg) as a pale-yellow oil. MS(ESI)m/z:259.0[M+H]+

### Reference Example 138: Synthesis of N-[[4-(4-methylthiazol-5-yl)phenyl]methyl]cyclopentanamine

4-(4-Methylthiazol-5-yl)benzaldehyde (80 mg) described in Reference Example 9, DCM (4 mL), and cyclopentanamine (0.058 mL) were added, and the mixture was stirred at room temperature for 30 min. Sodium triacetoxyborohydride (125 mg) was added, and the mixture was further stirred at the same temperature for 2 hr. To the reaction mixture were added chloroform and saturated aqueous sodium hydrogen carbonate and the mixture was stirred. The organic layer was separated by a Phase-separator (registered trade mark), and concentrated under reduced pressure. The obtained residue was purified by preparative HPLC (10 mM ammonium carbonate aqueous solution-acetonitrile) to give the title compound (75 mg) as a colorless oil. MS(ESI)m/z:273.1[M+H]+

### Reference Example 139: Synthesis of N-[[4-(4-methylthiazol-5-yl)phenyl]methyl]cyclohexanamine

4-(4-Methylthiazol-5-yl)benzaldehyde (80 mg) described in Reference Example 9, DCM (4 mL), and cyclohexanamine (0.067 mL) were added, and the mixture was stirred at room temperature for 1 hr. Sodium triacetoxyborohydride (125 mg) was added, and the mixture was further stirred at the same temperature for 2 hr. To the reaction mixture were added chloroform and saturated aqueous sodium hydrogen carbonate and the mixture was stirred. The organic layer was separated by a Phase-separator (registered trade mark), and concentrated under reduced pressure. The obtained residue was purified by preparative HPLC (10 mM ammonium carbonate aqueous solution-acetonitrile) to give the title compound (81 mg) as a colorless oil. MS (ESI)m/z:287.1[M+H] +

### Reference Example 140: Synthesis of tert-butyl N-[2-(4-phenylbutanoylamino)ethyl]carbamate

To a solution of 4-phenylbutanoic acid (1.6 g) in THF (20 mL) were added WSC·HCl (1.9 g) and N-ethylmorpholine (2.2 g) at room temperature, and the mixture was stirred at room temperature for 1 hr. tert-Butyl N-(2-aminoethyl)carbamate (1.6 mL) was added, and the mixture was further stirred at the same temperature for 20 hr. To the reaction solution were added saturated aqueous sodium hydrogen carbonate and water and the mixture was stirred and extracted with chloroform. The organic layer was washed with saturated brine, dried over anhydrous sodium sulfate, and the solvent was evaporated under reduced pressure. The obtained residue was purified by silica gel column chromatography (chloroform: methanol=100:0 - 90:10) to give the title compound (2.1 g) as a white powder. MS(ESI)m/z:207.1[M+H-Boc]+

### Reference Example 141: Synthesis of tert-butyl N-[2-(4-phenylbutylamino)ethyl]carbamate

To a solution of tert-butyl N-[2-(4-phenylbutanoylamino)ethyl]carbamate (1.0 g) described in Reference Example 140 and THF (10 mL) was added dropwise a borane-THF complex (11 mL) over 15 min, and the mixture was stirred at room temperature for 1 hr. To the reaction mixture was added methanol 15 mL, and the mixture was stirred and concentrated under reduced pressure. The obtained residue was purified by silica gel column chromatography (chloroform: methanol=100:0 - 90:10), and further purified by preparative HPLC (10 mM ammonium carbonate aqueous solution-acetonitrile) to give the title compound (590 mg) as a colorless oil. MS (ESI)m/z:293.4[M+H] +

### Reference Example 142: Synthesis of tert-butyl N-[2-[[2-[(2S,4R)-1-[(2S)-2-[(1-fluorocyclopropanecarbonyl)amino]-3,3-dimethylbutanoyl]-4-hydroxypyrrolidin-2-yl]-1H-imidazole-4-carbonyl]-(4-phenylbutyl)amino]ethyl]carbamate

To a solution of 2-[(2S,4R)-1-[(2S)-2-[(1-fluorocyclopropanecarbonyl)amino]-3,3-dimethylbutanoyl]-4-hydroxypyrrolidin-2-yl]-1H-imidazole-4-carboxylic acid (300 mg) described in Reference Example 133 in DMF (6.0 mL) were added DIPEA (420 µL), HATU (300 mg), and tert-butyl N-[2-(4-phenylbutylamino)ethyl]carbamate (225 mg) described in Reference Example 141 at room temperature, and the mixture was stirred at room temperature for 1 hr. The reaction mixture was diluted with DMSO and purified by preparative HPLC (10 mM ammonium carbonate aqueous solution-acetonitrile) to give the title compound (400 mg) as a white powder.
MS(ESI)m/z:671.4[M+H]+

### Reference Example 143: Synthesis of N-(2-aminoethyl)-2-[(2S,4R)-1-[(2S)-2-[(1-fluorocyclopropanecarbonyl)amino]-3,3-dimethylbutanoyl]-4-hydroxypyrrolidin-2-yl]-N-(4-phenylbutyl)-1H-imidazole-4-carboxamide hydrochloride

To a solution of tert-butyl N-[2-[[2-[(2S,4R)-1-[(2S)-2-[(1-fluorocyclopropanecarbonyl)amino]-3,3-dimethylbutanoyl]-4-hydroxypyrrolidin-2-yl]-1H-imidazole-4-carbonyl]-(4-phenylbutyl)amino]ethyl]carbamate (400 mg) described in Reference Example 142 in 1,4-dioxane (3.0 mL) was added 4 M hydrochloric acid-methanol (1.5 mL) at room temperature, and the mixture was stirred at room temperature for 3 hr. The reaction mixture was concentrated, azeotropically distilled with diethyl ether, and dried under reduced pressure overnight to give the title compound (372 mg) as a white powder. MS (ESI)m/z:571.3 [M+H]+

### Reference Example 144: Synthesis of tert-butyl N-methyl-N-[2-(4-phenylbutylamino)ethyl]carbamate

To a solution of 4-phenylbutanal (580 mg) and tert-butyl N-(2-aminoethyl)-N-methyl-carbamate (0.77 mL) in methanol (20 mL) were added DIPEA (0.75 mL), acetic acid (0.45 mL), and borane; 2-methylpyridine (628 mg) at room temperature, and the mixture was stirred at room temperature for 20 hr. The reaction mixture was diluted with methanol, purified by preparative HPLC (10 mM ammonium carbonate aqueous solution-acetonitrile), and further purified by preparative HPLC (10 mM ammonium carbonate aqueous solution-acetonitrile) to give the title compound (125 mg) as a colorless oil.
MS(ESI)m/z:307.2[M+H]+

### Reference Example 145: Synthesis of tert-butyl N-[(1S)-1-[(2S,4R)-4-[tert-butyl(dimethyl)silyl]oxy-2-[4-[[cyclopropylmethyl(4-phenylbutyl)amino]methyl]-1H-imidazol-2-yl]pyrrolidine-1-carbonyl]-2,2-dimethyl-propyl]carbamate

tert-Butyl N-[(1S)-1-[(2S,4R)-4-[tert-butyl(dimethyl)silyl]oxy-2-[4-(hydroxymethyl)-1H-imidazol-2-yl]pyrrolidine-1-carbonyl]-2,2-dimethyl-propyl]carbamate (60 mg) described in Reference Example 132, DCM (1.2 mL), and manganese dioxide (51 mg) were added, and the mixture was stirred at room temperature for 2 hr. Manganese dioxide (51 mg) was further added, and the mixture was stirred at room temperature for 15 hr. Insoluble material was removed from the reaction mixture by filtration through diatomaceous earth, and the filtrate was concentrated under reduced pressure. To the concentrated residue were added N-(cyclopropylmethyl)-4-phenyl-butan-1-amine (36 mg)described in Reference Example 27, methanol (1.2 mL), and decaborane (7.2 mg), and the mixture was stirred at room temperature for 3 hr. N-(cyclopropylmethyl)-4-phenyl-butan-1-amine (36 mg) described in Reference Example 27 and decaborane (7.2 mg) were added, and the mixture was further stirred for 1 hr. The reaction mixture was concentrated under reduced pressure, and the residue was purified by silica gel column chromatography (chloroform: methanol=100:0 - 90:10) to give the title compound (70 mg) as a colorless oil. MS(ESI)m/z:696.4[M+H]+

### Reference Example 146: Synthesis of tert-butyl N-[(1S)-1-[(2S,4R)-2-[4-[cyclopropylmethyl(4-phenylbutyl)carbamoyl]-1H-imidazol-2-yl]-4-hydroxypyrrolidine-1-carbonyl]-2,2-dimethylpropyl]carbamate

2-[(2S,4R)-1-[(2S)-2-(tert-Butoxycarbonylamino)-3,3-dimethylbutanoyl]-4-hydroxypyrrolidin-2-yl]-1H-imidazole-4-carboxylic acid (208 mg) described in Reference Example 149 was dissolved in NMP (2.5 mL), HATU (212 mg), and DIPEA (0.26 mL) were added at room temperature, and the mixture was stirred for 5 min. Then N-(cyclopropylmethyl)-4-phenyl-butan-1-amine (124 mg) described in Reference Example 27 was added and the mixture was stirred for 2 hr. To the reaction mixture were added water and the mixture was extracted with ethyl acetate. The organic layer was washed with water and saturated brine, dried over sodium sulfate and then filtered. The filtrate was concentrated under reduced pressure. The residue was purified by silica gel column chromatography (hexane:ethyl acetate=50:50 - 0:100, ethyl acetate:methanol=9:1). The residue was dissolved in 1,4-dioxane (2.1 mL), 4 M hydrochloric acid-dioxane (1.57 mL) was added at room temperature and the mixture was stirred for 2 hr. After completion of the reaction, the reaction solution was concentrated under reduced pressure and diluted with IPE. The precipitate was collected by filtration from the reaction mixture, washed with IPE and dried. The obtained residue was dissolved in dichloromethane (0.35 mL), acetic anhydride (4 µL) and triethylamine (20 µL)were added, and the mixture was stirred at room temperature for 2 hr. To the reaction mixture was added saturated aqueous sodium hydrogen carbonate, chloroform and water were added, and the mixture was stirred. The organic layer was separated by a Phase-separator (registered trade mark), and concentrated under reduced pressure. The obtained residue was purified by silica gel column chromatography (chloroform: methanol=100:0 - 90:10), and further purified by preparative HPLC (10 mM ammonium carbonate aqueous solution-acetonitrile) to give the title compound (12 mg) as a colorless powder. MS(ESI)m/z:538.6[M+H]+

### Reference Example 147: Synthesis of benzyl (2S,4R)-4-[tert-butyl(dimethyl)silyl]oxy-2-(4,5-diiodo-1H-imidazol-2-yl)pyrrolidine-1-carboxylate

To a solution of benzyl (2S,4R)-4-[tert-butyl(dimethyl)silyl]oxy-2-(1H-imidazol-2-yl)pyrrolidine-1-carboxylate (200 mg) described in Reference Example 72 in DCM (5 mL) was added NIS (247 mg), and the mixture was stirred at room temperature for 3 hr. After completion of the reaction, the reaction mixture was concentrated under reduced pressure, and the residue was purified by silica gel column chromatography (hexane:ethyl acetate=80:20 - 50:50) to give the title compound (324 mg) as a colorless oil.
MS (ES I) m/z: 654.3 [M+H]+

### Reference Example 148: Synthesis of [2-[4-(trifluoromethyl)phenyl]-4-pyridyl]methanamine hydrochloride

To 2-[4-(trifluoromethyl)phenyl]pyridine-4-carbonitrile (11 g) were added methanol (230 mL), 10%Pd/C (1.1 g), and 4 M hydrochloric acid-dioxane (23 mL), and the mixture was stirred under hydrogen atmosphere at room temperature overnight. After celite filtration, the filtrate was diluted with ethyl acetate, and the precipitated powder was collected by filtration and dried to give the title compound (14 g) as a brown powder. MS (ESI) m/z:253.1[M+H] +

### Reference Example 149: Synthesis of 2-[(2S,4R)-1-[(2S)-2-(tert-butoxycarbonylamino)-3,3-dimethylbutanoyl]-4-hydroxypyrrolidin-2-yl]-1H-imidazole-4-carboxylic acid

Under ice-cooling, methanol (10 mL) and 1 M sodium hydroxide aqueous solution (6.8 mL) were added to methyl 2-[(2S,4R)-4-[tert-butyl(dimethyl)silyl]oxy-1-[(2S)-3,3-dimethyl-2-[(2-methylpropan-2-yl)oxycarbonylamino]butanoyl]pyrrolidin-2-yl]-1H-imidazole-4-carboxylate (920 mg) described in Reference Example 19, and the mixture was stirred at 50°C overnight. After completion of the reaction, the mixture was ice-cooled, adjusted to pH=4 by adding 1 M hydrochloric acid, and concentrated under reduced pressure. The obtained residue was washed with water and hexane:ethyl acetate=1:1 and dried under reduced pressure to give (625 mg) as a pale-yellow powder. MS(ESI)m/z:411.3[M+H]+

### Reference Example 150: Synthesis of tert-butyl (2S,4R)-4-[tert-butyl(dimethyl)silyl]oxy-2-[1-(2-naphthylmethyl)imidazol-2-yl]pyrrolidine-1-carboxylate

2-Naphthylmethanamine (358 mg) was dissolved in a mixed solvent of ethyl acetate (8.4 mL) and THF (8.4 mL), tert-butyl (2S,4R)-4-[tert-butyl(dimethyl)silyl]oxy-2-formylpyrrolidine-1-carboxylate (501 mg) and magnesium sulfate (279 mg)were added, and the mixture was stirred at room temperature for 2 days. Insoluble material was filtered off through celite, and the filtrate was concentrated under reduced pressure. To the obtained residue were added methanol (10 mL), ammonium acetate (212 mg), and 39%glyoxal aqueous solution (260 µL), and the mixture was stirred at room temperature for 4 days. The reaction mixture was concentrated under reduced pressure, and the residue was purified by silica gel column chromatography (hexane:ethyl acetate=55:45 - 34:66 - 0:100). The obtained crude product was purified again by preparative HPLC (10 mM ammonium carbonate aqueous solution-acetonitrile) to give the title compound (18 mg) as a pale-yellow oil.
MS(ESI)m/z:508.5[M+H]+

### Reference Example 151: Synthesis of (3R,5S)-5-[1-(2-naphthylmethyl)imidazol-2-yl]pyrrolidin-3-ol

To a solution of tert-butyl (2S,4R)-4-[tert-butyl(dimethyl)silyl]oxy-2-[1-(2-naphthylmethyl)imidazol-2-yl]pyrrolidine-1-carboxylate (18 mg) described in Reference Example 150 in methanol (350 µL) was added 4 M hydrochloric acid-dioxane (90 µL), and the mixture was stirred at room temperature for 3 weeks. To the reaction mixture was added DIPEA (200 µL), and the mixture was purified by preparative HPLC (10 mM ammonium carbonate aqueous solution-acetonitrile) to give the title compound (8.4 mg) as a white powder. MS(ESI)m/z:294.1[M+H]+

### Reference Example 152: Synthesis of tert-butyl (2S)-3-methyl-2-[4-(3-methylisoxazol-5-yl)triazol-1-yl]butanoate

tert-Butyl (2S)-2-amino-3-methylbutanoate hydrochloride (62 mg) and DMAP (102 mg) were dissolved in acetonitrile (1 mL), 2-azido-1,3-dimethylimidazolinium hexafluorophosphate (102 mg) was added and the mixture was stirred at 30°C for 2.5 hr. Then, 5-ethynyl-3-methyl-isoxazole (65 mg), Copper(II) Sulfate (II) (20 mg), and sodium ascorbate (75 mg) were added at room temperature and the mixture was stirred for 19 hr. After completion of the reaction, the reaction solution was purified by silica gel column chromatography (hexane:ethyl acetate=95:5 - 60:40) to give the title compound (51 mg) as a white powder. MS (ESI) m/z:307.3[M+H] +

### Reference Example 153: Synthesis of (2S)-3-methyl-2-[4-(3-methylisoxazol-5-yl)triazol-1-yl]butanoic acid

tert-Butyl (2S)-3-methyl-2-[4-(3-methylisoxazol-5-yl)triazol-1-yl]butanoate (48 mg) described in Reference Example 152 was dissolved in DCM (1 mL), trifluoroacetic acid (1 mL) was added at room temperature, and the mixture was stirred for 22 hr. After completion of the reaction, the reaction solution was concentrated under reduced pressure to give the title compound (60 mg) as an orange oil. MS (ESI) m/z:251.2[M+H] +

### Reference Example 154: Synthesis of tert-butyl (2S,3S)-2-(4-ethoxytriazol-1-yl)-3-methyl-pentanoate

tert-Butyl (2S,3S)-2-amino-3-methyl-pentanoate hydrochloride (106 mg) and DMAP (174 mg) were dissolved in acetonitrile (3 mL), 2-azido-1,3-dimethylimidazolinium hexafluorophosphate (153 mg) was added and the mixture was stirred at 30°C for 2.5 hr. Then, ethoxyacetylene (150 µL) , Copper(II) Sulfate (II) (31 mg), sodium ascorbate (102 mg), and water (1 mL) were added at room temperature and the mixture was stirred for 24 hr. After completion of the reaction, the reaction solution was purified by silica gel column chromatography (hexane:ethyl acetate=95:5 - 70:30) to give the title compound (67 mg) as a colorless oil.
MS (ESI)m/z:284.3[M+H] +

### Reference Example 155: Synthesis of (2S,3S)-2-(4-ethoxytriazol-1-yl)-3-methyl-pentanoic acid

tert-Butyl (2S,3S)-2-(4-ethoxytriazol-1-yl)-3-methyl-pentanoate (65 mg) described in Reference Example 154 was dissolved in DCM (1 mL), trifluoroacetic acid (1 mL) was added at room temperature and the mixture was stirred for 22 hr. After completion of the reaction, the reaction solution was concentrated under reduced pressure to give the title compound (78 mg) as a pale-yellow oil. MS(ESI)m/z:228.1[M+H]+

### Reference Example 156: Synthesis of tert-butyl N-[(1S)-1-[(2S,4R)-4-[tert-butyl(dimethyl)silyl]oxy-2-[4-[[cyclopropanecarbonyl(4-phenylbutyl)amino]methyl]-1H-imidazol-2-yl]pyrrolidine-1-carbonyl]-2,2-dimethyl-propyl]carbamate

tert-Butyl N-[(1S)-1-[(2S,4R)-4-[tert-butyl(dimethyl)silyl]oxy-2-[4-(hydroxymethyl)-1H-imidazol-2-yl]pyrrolidine-1-carbonyl]-2,2-dimethyl-propyl]carbamate (75 mg) described in Reference Example 132 was dissolved in DCM (1.5 mL), manganese dioxide (127 mg) was added at room temperature, and the mixture was stirred for 15 hr. After completion of the reaction, the reaction solution was filtered through celite, and the filtrate was concentrated under reduced pressure. The obtained residue was dissolved in methanol (1.5 mL), decaborane (8.9 mg) and 4-phenylbutylamine (65 mg) were added at room temperature and the mixture was stirred for 2 days. After completion of the reaction, the reaction solution was concentrated under reduced pressure. The obtained residue was purified by basic silica gel column chromatography (chloroform: methanol=90:10) to give a crude product. The obtained crude product was dissolved in DMF (1.5 mL), HATU (222 mg), DIPEA (127 µL), and cyclopropanecarboxylic acid (46 µL) were added at room temperature and the mixture was stirred for 3 hr. After completion of the reaction, the mixture was extracted with water and ethyl acetate. The organic layer was washed with water and saturated brine, dried over anhydrous sodium sulfate and then filtered. The filtrate was concentrated under reduced pressure. The obtained residue was purified by silica gel column chromatography (chloroform: methanol=100:0 - 90:10) to give the title compound (65 mg) as a yellow oil. MS(ESI)m/z:710.3[M+H]+

### Reference Example 157: Synthesis of tert-butyl N-[(1S)-1-[(2S,4R)-4-[tert-butyl(dimethyl)silyl]oxy-2-[4-[[cyclopropylmethyl(4-phenylbutanoyl)amino]methyl]-1H-imidazol-2-yl]pyrrolidine-1-carbonyl]-2,2-dimethyl-propyl]carbamate

tert-Butyl N-[(1S)-1-[(2S,4R)-4-[tert-butyl(dimethyl)silyl]oxy-2-[4-(hydroxymethyl)-1H-imidazol-2-yl]pyrrolidine-1-carbonyl]-2,2-dimethyl-propyl]carbamate (100 mg) described in Reference Example 132 was dissolved in DCM (2.0 mL), manganese dioxide (170 mg) was added at room temperature and the mixture was stirred for 15 hr. After completion of the reaction, the reaction solution was filtered through celite, and the filtrate was concentrated under reduced pressure. The obtained residue was dissolved in methanol (2.0 mL), decaborane (12 mg) and cyclopropylmethylamine (42 mg) were added at room temperature and the mixture was stirred for 18 hr. After completion of the reaction, the reaction solution was concentrated under reduced pressure. The obtained residue was purified by basic silica gel column chromatography (chloroform: methanol=90:10) to give a crude product. The obtained crude product was dissolved in DMF (2.0 mL), HATU (297 mg), DIPEA (169 µL), and 4-phenylbutyric acid (128 mg) were added at room temperature and the mixture was stirred for 3 hr. After completion of the reaction, the reaction solution was extracted with water and ethyl acetate. The organic layer was washed with water and saturated brine, dried over anhydrous sodium sulfate, and then filtered. The filtrate was concentrated under reduced pressure. The obtained residue was purified by silica gel column chromatography (hexane:ethyl acetate=50:50 - 0:100) to give the title compound (50 mg) as an orange oil. MS(ESI)m/z:710.3[M+H]+

### Reference Example 158: Synthesis of tert-butyl (2S,4R)-4-hydroxy-2-[4-(4-phenylpiperidine-1-carbonyl)-1H-imidazol-2-yl]pyrrolidine-1-carboxylate

tert-Butyl (2S,4R)-4-[tert-butyl(dimethyl)silyl]oxy-2-[4-(trifluoromethyl)-1H-imidazol-2-yl]pyrrolidine-1-carboxylate (226 mg) described in Reference Example 1, 4-phenylpiperidine (251 mg), and potassium carbonate (215 mg) were dissolved in NMP (1 mL), and the mixture was stirred under microwave radiation at 100°C for 10 min, and then stirred at 160°C for 40 min. After completion of the reaction, the reaction mixture was extracted with water and ethyl acetate. The organic layer was washed with water and saturated brine, dried over anhydrous sodium sulfate, filtered, and the filtrate was concentrated under reduced pressure. The obtained residue was purified by silica gel column chromatography (chloroform: methanol=100:0 - 90:10) to give the title compound (186 mg) as a brown viscous substance. MS(ESI)m/z:441.1[M+H]+

### Reference Example 159: Synthesis of tert-butyl N-[(1S)-1-[(2S,4R)-4-hydroxy-2-[1-methyl-5-(4-phenylpiperidine-1-carbonyl)imidazol-2-yl]pyrrolidine-1-carbonyl]-2,2-dimethylpropyl]carbamate

tert-Butyl (2S,4R)-4-hydroxy-2-[4-(4-phenylpiperidine-1-carbonyl)-1H-imidazol-2-yl]pyrrolidine-1-carboxylate (186 mg) described in Reference Example 158 was dissolved in DMF (1.6 mL), cesium carbonate (419 mg) and methyl iodide (60 µL) were added at room temperature, and the mixture was stirred for 3 hr. After completion of the reaction, the reaction mixture was extracted with water and ethyl acetate. The organic layer was washed with water and saturated brine, dried over anhydrous sodium sulfate, filtered, and the filtrate was concentrated under reduced pressure. The obtained residue was purified by NH silica gel column chromatography (ethyl acetate:methanol=95:5). Then, the obtained residue was dissolved in DCM (742 µL), trifluoroacetic acid (742 µL) was added at room temperature and the mixture was stirred for 3 hr. After completion of the reaction, the reaction mixture was eluted with a cation exchange resin (PoraPakTM Rxn CX 6cc), washed with methanol, extracted with 2 M ammonia-methanol, and concentrated under reduced pressure. The obtained residue, N-Boc-L-tert-leucine (28 mg), HATU (46 mg), and DIPEA (53 µL) were dissolved in DMF (1 mL), and the mixture was stirred at room temperature for 4 hr. After completion of the reaction, the reaction mixture was extracted with water and ethyl acetate. The organic layer was washed with water and saturated brine, dried over anhydrous sodium sulfate, filtered, and the filtrate was concentrated under reduced pressure. The obtained residue was purified by NH silica gel column chromatography (ethyl acetate:methanol=95:5). The obtained crude product was purified by NH thin layer silica gel chromatography to give the title compound (8.5 mg) as a colorless oil.
MS(ESI)m/z:568.4[M+H]+

### Reference Example 160: Synthesis of N-(4-phenylbutyl)cyclopropaneamine

DMF (8 mL) was added to molecular sieve 4A (powder) (500 mg) and cesium hydroxide monohydrate (400 mg), and the mixture was stirred at room temperature for 10 min. Cyclopropylamine (0.15 mL) was added, and the mixture was stirred for 30 min. 4-Bromobutylbenzene (500 mg) was added, and the mixture was stirred at room temperature overnight and purified by silica gel column chromatography (hexane:ethyl acetate=100:0 - 0:100) to give the title compound (83 mg) as a pale-yellow oil. MS (ESI) m/z:190.1[M+H] +

### Reference Example 161: Synthesis of N-(5-phenylpentyl)cyclopropaneamine

To a solution of 5-bromopentylbenzene (700 mg) in acetonitrile (6 mL) were added cyclopropylamine (0.42 mL) and potassium carbonate (425 mg) and the mixture was stirred at 70 overnight. Insoluble material was filtered off, and the filtrate was concentrated under reduced pressure and purified by NH silica gel column chromatography (hexane:ethyl acetate=100:0 - 0:100) to give the title compound (45 mg) as a colorless oil. MS(ESI)m/z:204.1[M+H]+

### Reference Example 162: Synthesis of tert-butyl N-[(1S,2S)-1-[(2S,4R)-4-hydroxy-2-(1H-imidazol-2-yl)pyrrolidine-1-carbonyl]-2-methylbutyl]carbamate

DMF (1 mL) was added to (3R,5S)-5-(1H-imidazol-2-yl)pyrrolidin-3-ol hydrochloride (50 mg) described in Reference Example 122, HATU (116 mg), DIPEA (116 µL), and N-(tert-butoxycarbonyl)-L-isoleucine 0.5 hydrate (78 mg) were added, and the mixture was stirred at room temperature for about 1 hr. To the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (41 mg) as a colorless oil. MS(ESI)m/z:367.3[M+H]+

### Reference Example 163: Synthesis of N-(2-benzyloxyethyl)cyclopropaneamine

DMF (8 mL) and cesium hydroxide monohydrate (390 mg) were added to molecular sieve 4A (powder) (500 mg), and the mixture was stirred at room temperature for 10 min. Cyclopropylamine (0.15 mL) was added, and the mixture was stirred for 30 min. 2-Bromoethoxymethylbenzene (500 mg) was added, and the mixture was stirred at room temperature overnight. Insoluble material was collected by filtration, water was added to the filtrate and the mixture was extracted with ethyl acetate. The organic layer was washed with water and saturated brine, dried over sodium sulfate and filtered. The filtrate was concentrated under reduced pressure and purified by silica gel column chromatography (ethyl acetate:methanol=100:0 - 80:20) to give the title compound (41 mg) as a colorless oil. MS(ESI)m/z:192.0[M+H]+

### Reference Example 164: Synthesis of (3R,5S)-5-(4-methylthiazol-2-yl)pyrrolidin-3-ol

tert-Butyl (2S,4R)-4-[tert-butyl(dimethyl)silyl]oxy-2-carbamothioylpyrrolidine-1-carboxylate (200 mg) described in Reference Example 71 was dissolved in DMF (10 mL), 1-chloropropan-2-one (0.049 mL) and DIPEA (0.096 mL) were added, and the mixture was stirred at 80°C for 5 hr. After completion of the reaction, saturated brine was added, and the aqueous layer was extracted with ethyl acetate. The organic layer was washed with saturated brine, dried over anhydrous sodium sulfate, filtered, and the filtrate was concentrated under reduced pressure. The residue was purified by silica gel column chromatography (hexane:ethyl acetate=75:25). To the obtained crudely purified product was added 4 M hydrochloric acid-dioxane (2.8 mL), and the mixture was stirred at room temperature for about 5 hr. This was dissolved in DMSO and purified by preparative HPLC (10 mM ammonium carbonate aqueous solution-acetonitrile) to give the title compound (10 mg) as a white powder. MS(ESI)m/z:185.0[M+H]+

### Reference Example 165: Synthesis of 4-(ethylaminomethyl)-N-methyl-benzamide hydrochloride

4-[[tert-Butoxycarbonyl (ethyl)amino]methyl]benzoic acid (300 mg) was dissolved in DMF (4.0 mL), 12 M-methylamine aqueous solution (0.25 mL) and HATU (490 mg) were added at room temperature, and the mixture was stirred for 15 hr. HATU (325 mg) was added and the mixture was further stirred with heating for 25 hr. After completion of the reaction, the reaction mixture was extracted with water and ethyl acetate. The organic layer was washed successively with water and saturated brine and dried over anhydrous sodium sulfate. The organic layer was filtered, and the filtrate was concentrated under reduced pressure. The obtained residue was purified by silica gel chromatography (hexane:ethyl acetate=50:50 - 0:100). The obtained residue was dissolved in THF (2.5 mL), 4 M hydrochloric acid-dioxane (2.5 mL) was added and the mixture was stirred for 8 hr. After completion of the reaction, the reaction mixture was diluted with ethyl acetate. The precipitate was collected by filtration, washed with ethyl acetate, and dried to give the title compound (246 mg) as a white powder. MS(ESI)m/z:193.0[M+H]+

### Reference Example 166: Synthesis of tert-butyl N-methyl-N-[(1-phenyl-4-piperidyl)methyl]carbamate

1,4-Dioxane (10 mL) was added to tert-butyl N-methyl-N-(4-piperidylmethyl)carbamate (500 mg), bromobenzene (0.46 mL), palladium(II) acetate (49 mg), XantPhos (127 mg), and cesium carbonate (1.4 g), and the mixture was stirred under nitrogen atmosphere at 110°C for 5 hr. Palladium (II) acetate (49 mg) and XantPhos (127 mg) were added, and the mixture was further stirred at 110°C for 6 hr. After completion of the reaction, the reaction mixture was extracted with water and ethyl acetate. The organic layer was washed with saturated brine, and dried over anhydrous sodium sulfate. The organic layer was filtered, and the filtrate was concentrated under reduced pressure. The obtained residue was purified by silica gel chromatography (hexane:ethyl acetate=85:15 - 75:25) to give the title compound (100 mg) as a pale-yellow oil. MS(ESI)m/z:305.3[M+H]+

### Reference Example 167: Synthesis of N-methyl-1-(1-phenyl-4-piperidyl)methanamine dihydrochloride

tert-Butyl N-methyl-N-[(1-phenyl-4-piperidyl)methyl]carbamate (100 mg) described in Reference Example 166 was dissolved in DCM (3 mL), trifluoroacetic acid (1.5 mL) was added and the mixture was stirred for 2.5 hr. The reaction mixture was concentrated under reduced pressure, and the obtained residue was dissolved in THF (4.0 mL). 4 M Hydrochloric acid-ethyl acetate (0.50 mL) was added, and the mixture was concentrated under reduced pressure to give the title compound (110 mg) as a pale-green powder. MS (ESI) m/z:205.2[M+H] +

### Reference Example 168: Synthesis of tert-butyl N-[[1-[(2-fluorophenyl)methyl]-4-piperidyl]methyl]-N-methyl-carbamate

tert-Butyl N-methyl-N-(4-piperidylmethyl)carbamate (400 mg), 2-fluorobenzaldehyde (0.46 mL) was dissolved in dichloromethane (5.0 mL), sodium triacetoxyborohydride (695 mg) was added, and the mixture was stirred for 23 hr. After completion of the reaction, the reaction mixture was extracted with saturated aqueous sodium hydrogen carbonate and chloroform. The organic layer was washed with saturated brine, and dried over anhydrous sodium sulfate. The organic layer was filtered, and the filtrate was concentrated under reduced pressure. The obtained residue was purified by silica gel chromatography (hexane:ethyl acetate=70:30 - 50:50) to give the title compound (461 mg) as a colorless oil. MS(ESI)m/z:337.4[M+H]+

### Reference Example 169: Synthesis of tert-butyl N-methyl-N-[[4-(2-methyl-4-pyridyl)phenyl]methyl]carbamate

To tert-butyl N-[(4-bromophenyl)methyl]-N-methylcarbamate (400 mg), (2-methyl-4-pyridyl)boronic acid (270 mg), PdCl2(PPh3)2 (105 mg), and potassium carbonate (365 mg) were added water (1 mL) and 1,4-dioxane (6 mL), and the mixture was stirred under microwave radiation at 120°C for 1 hr. After completion of the reaction, the reaction mixture was extracted with water and ethyl acetate. The organic layer was washed with saturated brine, and dried over anhydrous sodium sulfate. The organic layer was filtered, and the filtrate was concentrated under reduced pressure. The obtained residue was purified by silica gel chromatography (hexane:ethyl acetate=70:30 - 35:65) to give the title compound (372 mg) as a pale-brown oil. MS(ESI)m/z:313.3[M+H]+

### Reference Example 170: Synthesis of N-methyl-1-[4-(2-methyl-4-pyridyl)phenyl]methanamine dihydrochloride

tert-Butyl N-methyl-N-[[4-(2-methyl-4-pyridyl)phenyl]methyl]carbamate (372 mg) described in Reference Example 169 was dissolved in DCM (6 mL), trifluoroacetic acid (2 mL) was added and the mixture was stirred for 2 hr. The reaction mixture was concentrated under reduced pressure, the obtained residue was dissolved in THF (4.0 mL), and 4 M hydrochloric acid -ethyl acetate (1.0 mL) was added. The reaction mixture was diluted with ethyl acetate and the precipitate was collected by filtration, washed with ethyl acetate, and dried to give the title compound (395 mg) as a pale-red powder. MS(ESI)m/z:213.2[M+H]+

### Reference Example 171: Synthesis of tert-butyl N-methyl-N-[[4-(6-methyl-3-pyridyl)phenyl]methyl]carbamate

To tert-butyl N-[(4-bromophenyl)methyl]-N-methylcarbamate (300 mg), (6-methyl-3-pyridyl)boronic acid (175 mg), PdCl2(PPh3)2 (80 mg), and potassium carbonate (275 mg) were added water (1 mL) and 1,4-dioxane (5 mL), and the mixture was stirred under microwave radiation at 120°C for 1 hr. After completion of the reaction, the reaction mixture was extracted with water and ethyl acetate. The organic layer was washed with saturated brine, and dried over anhydrous sodium sulfate. The organic layer was filtered, and the filtrate was concentrated under reduced pressure. The obtained residue was purified by silica gel chromatography (hexane:ethyl acetate=80:20 - 45:55) to give the title compound (243 mg) as a pale-yellow oil. MS(ESI)m/z:313.3[M+H]+

### Reference Example 172: Synthesis of N-methyl-1-[4-(6-methyl-3-pyridyl)phenyl]methanamine dihydrochloride

tert-Butyl N-methyl-N-[[4-(6-methyl-3-pyridyl)phenyl]methyl]carbamate (243 mg) described in Reference Example 171 was dissolved in DCM (4.5 mL), trifluoroacetic acid (1.5 mL) was added and the mixture was stirred for 4 hr. The reaction mixture was concentrated under reduced pressure, the obtained residue was dissolved in THF (4.0 mL), and 4 M hydrochloric acid-ethyl acetate (0.80 mL) was added. The reaction mixture was diluted with ethyl acetate and the precipitate was collected by filtration, washed with ethyl acetate, and dried to give the title compound (175 mg) as a white powder. MS(ESI)m/z:213.2[M+H]+

### Reference Example 173: Synthesis of 1-[1-[(2-fluorophenyl)methyl]-4-piperidyl]-N-methyl-methanamine dihydrochloride

The title compound was obtained by the same reaction and treatment as in Reference Example 170 using tert-butyl N-[[1-[(2-fluorophenyl)methyl]-4-piperidyl]methyl]-N-methyl-carbamate described in Reference Example 168. MS(ESI)m/z:237.3[M+H]+

### Reference Example 174: Synthesis of ethyl 2-(5-propyl-1,3,4-oxadiazol-2-yl)acetate

To a solution of 1H-tetrazole -5-ethyl acetate (500 mg) in pyridine (3 mL) was added butyryl chloride (0.37 mL) and the mixture was stirred at room temperature for 1 hr and then heated under reflux for 5 hr. After cooling to room temperature, the mixture was concentrated under reduced pressure. Ethyl acetate was added to the residue, and the residue was successively washed with 1 M hydrochloric acid and water. The organic layer was concentrated under reduced pressure and the residue was purified by silica gel column chromatography (hexane:ethyl acetate=95:5 - 50:50) to give the title compound (110 mg) as a yellow oil.
MS (ESI)m/z:199.2[M+H] +

### Reference Example 175: Synthesis of ethyl 3-methyl-2-(5-propyl-1,2,4-oxadiazol-3-yl)butanoate

A solution of potassium tert-butoxide (388 mg) in DMF (3 mL) was cooled to 0°C, a solution of ethyl 2-(5-propyl-1,2,4-oxadiazol-3-yl)acetate (456 mg) in DMF (2 mL) was added and the mixture was stirred for 30 min. 2-Iodopropane (0.30 mL) was added, and the mixture was stirred for 3 hr while gradually allowing to warm to room temperature. Water was added to the reaction mixture and the mixture was extracted with ethyl acetate. The organic layer was washed with water, and concentrated under reduced pressure. The obtained residue was purified by silica gel column chromatography (hexane:ethyl acetate=99:1 - 75:25) to give the title compound (406 mg) as a colorless oil. MS(ESI)m/z:241.0[M+H]+

### Reference Example 176: Synthesis of ethyl 3-methyl-2-(5-propyl-1,3,4-oxadiazol-2-yl)butanoate

The title compound was obtained by the same reaction and treatment as in Reference Example 175 using ethyl 2-(5-propyl-1,3,4-oxadiazol-2-yl)acetate described in Reference Example 174. MS (ESI) m/z:241.0[M+H] +

### Reference Example 177: Synthesis of ethyl 3-methyl-2-[4-(3-methylisoxazol-5-yl)pyrazol-1-yl]butanoate

To a solution of ethyl 2-(4-bromopyrazol-1-yl)-3-methylbutanoate (200 mg) described in Reference Example 31 in THF (4 mL) were added 3-methyl-5-(4,4,5,5-tetramethyl-1,3,2-dioxaborolan-2-yl)isoxazole (167 mg), tetrakis (triphenyl phosphine) palladium (0) (84 mg), tripotassium phosphate (463 mg), and water (0.052 mL). After nitrogen substitution, and the mixture was stirred under microwave radiation at 120°C for 30 min, and concentrated under reduced pressure. The residue was purified by silica gel column chromatography (hexane:ethyl acetate=99:1 - 65:35) to give the title compound (16 mg) as a pale-yellow powder. MS(ESI)m/z:278.0[M+H]+

### Reference Example 178: Synthesis of 3-methyl-2-(5-propyl-1,2,4-oxadiazol-3-yl)butanoic acid

The title compound was obtained by the same reaction and treatment as in Reference Example 8 using ethyl 3-methyl-2-(5-propyl-1,2,4-oxadiazol-3-yl)butanoate described in Reference Example 175. MS(ESI)m/z:213.0[M+H]+

### Reference Example 179: Synthesis of 3-methyl-2-(5-propyl-1,3,4-oxadiazol-2-yl)butanoic acid

The title compound was obtained by the same reaction and treatment as in Reference Example 8 using ethyl 3-methyl-2-(5-propyl-1,3,4-oxadiazol-2-yl)butanoate described in Reference Example 176. MS(ESI)m/z:213.0[M+H]+

### [Table 1-1]

**Table 1: Structural formulas of Reference Example compounds**

| Ref. Ex. No. | structural formula | Ref. Ex. No. | structural formula |
|---|---|---|---|
| 1 | 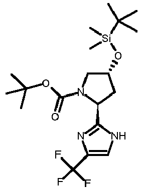 | 2 | 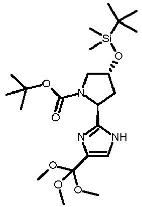 |
| 3 | 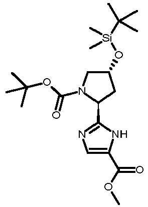 | 4 | 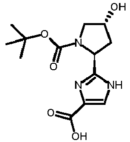 |
| 5 | 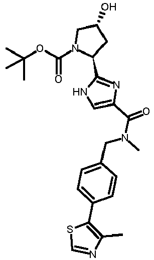 | 6 | 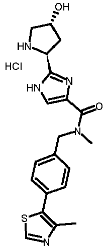 |
| 7 | 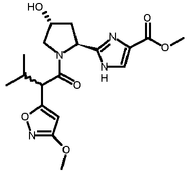 | 8 | 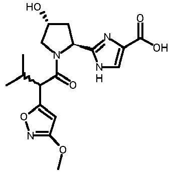 |
| 9 | 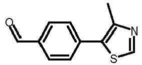 | 10 | 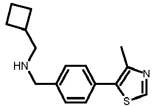 |
| 11 | 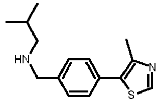 | 12 | 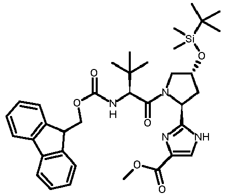 |

**[Table 1-2]**

| Ref. Ex. No. | structural formula | Ref. Ex. No. | structural formula |
|---|---|---|---|
| 13 | 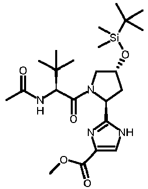 | 14 | 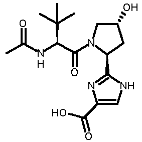 |
| 15 | 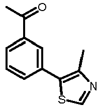 | 16 | 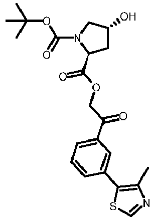 |
| 17 | 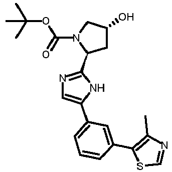 | 18 | 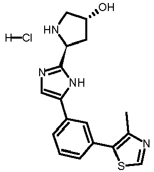 |
| 19 | 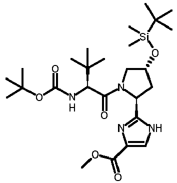 | 20 | 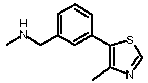 |
| 21 | 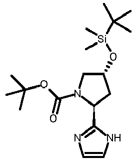 | 22 | 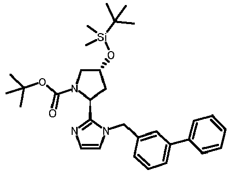 |
| 23 | 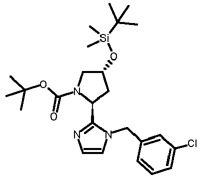 | 24 | 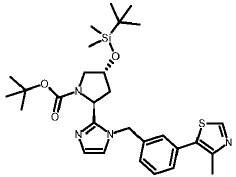 |
| 25 | 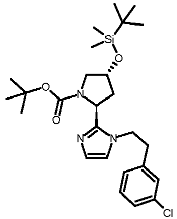 | 26 | 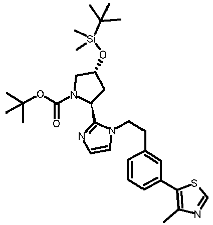 |

**[Table 1-3]**

| Ref. Ex. No. | structural formula | Ref. Ex. No. | structural formula |
|---|---|---|---|
| 27 | 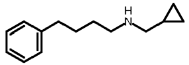 | 28 | 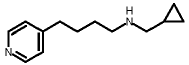 |
| 29 | 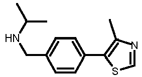 | 30 | 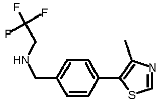 |
| 31 | 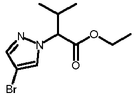 | 32 | 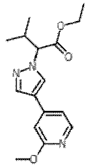 |
| 33 | 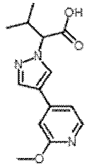 | 34 | 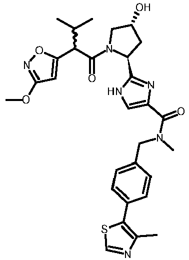 |
| 35 | 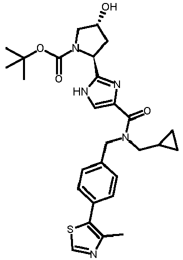 | 36 | 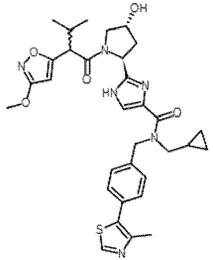 |
| 37 | 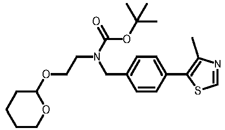 | 38 | 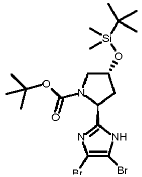 |
| 39 | 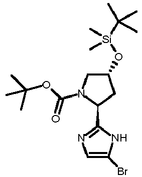 | 40 | 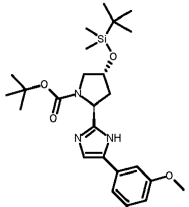 |

**[Table 1-4]**

| Ref. Ex. No. | structural formula | Ref. Ex. No. | structural formula |
|---|---|---|---|
| 41 | 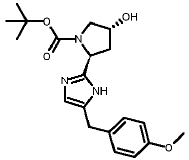 | 42 | 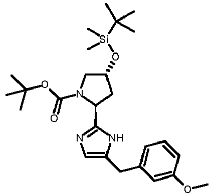 |
| 43 | 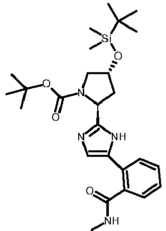 | 44 | 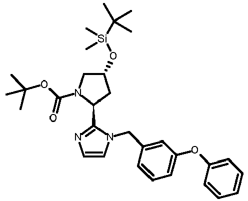 |
| 45 | 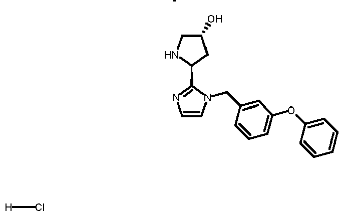 | 46 | 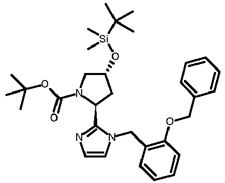 |
| 47 | 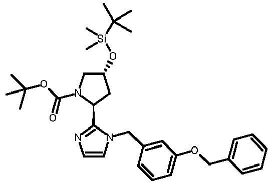 | 48 | 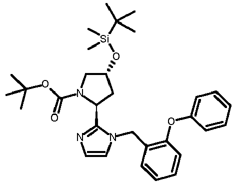 |
| 49 | 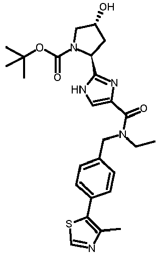 | 50 | 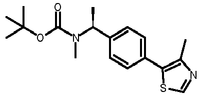 |
| 51 | 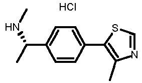 | 52 | 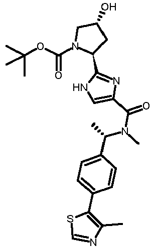 |

**[Table 1-5]**

| Ref. Ex. No. | structural formula | Ref. Ex. No. | structural formula |
|---|---|---|---|
| 53 | 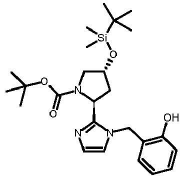 | 54 | 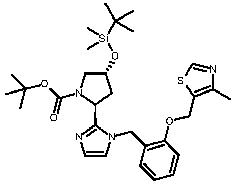 |
| 55 | 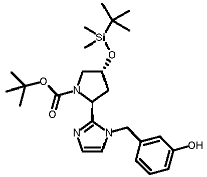 | 56 | 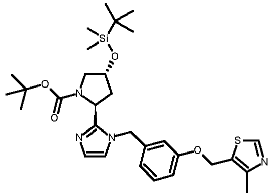 |
| 57 | 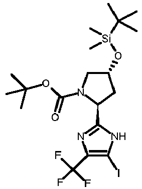 | 58 | 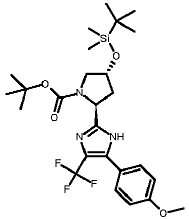 |
| 59 | 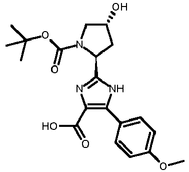 | 60 | 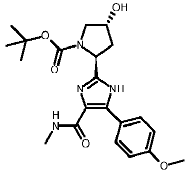 |
| 61 | 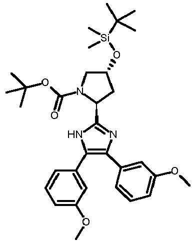 | 62 | 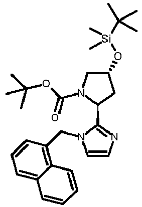 |
| 63 | 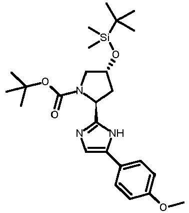 | 64 | 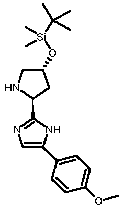 |

**[Table 1-6]**

| Ref. Ex. No. | structural formula | Ref. Ex. No. | structural formula |
|---|---|---|---|
| 65 | 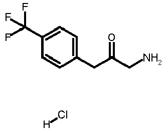 | 66 | 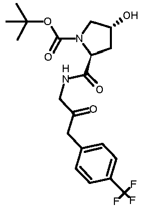 |
| 67 | 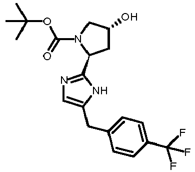 | 68 | 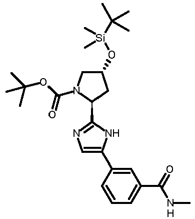 |
| 69 | 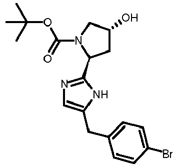 | 70 | 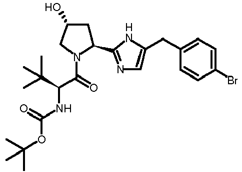 |
| 71 | 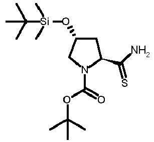 | 72 | 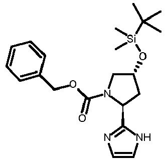 |
| 73 | 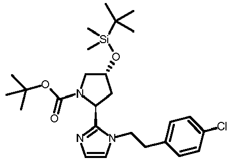 | 74 | 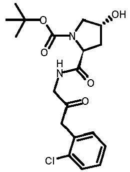 |
| 75 | 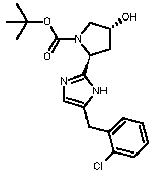 | 76 | 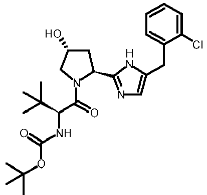 |
| 77 | 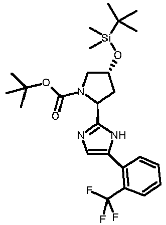 | 78 | 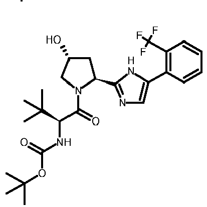 |

**[Table 1-7]**

| Ref. Ex. No. | structural formula | Ref. Ex. No. | structural formula |
|---|---|---|---|
| 79 | 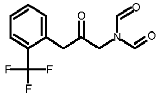 | 80 | 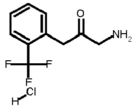 |
| 81 | 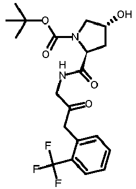 | 82 | 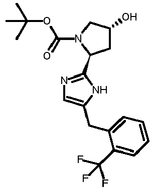 |
| 83 | 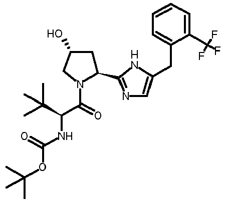 | 84 | 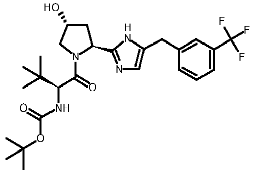 |
| 85 | 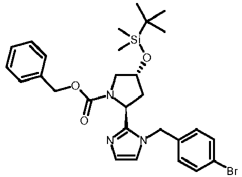 | 86 | 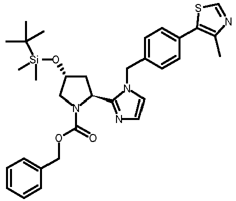 |
| 87 | 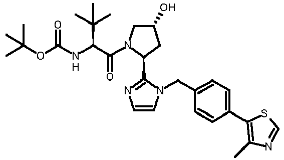 | 88 | 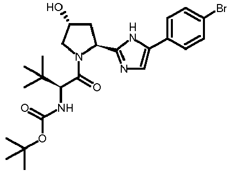 |
| 89 | 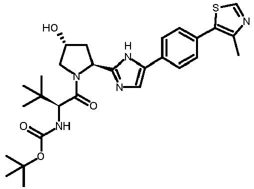 | 90 | 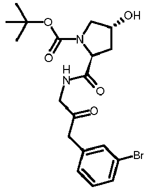 |
| 91 | 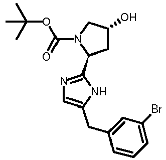 | 92 | 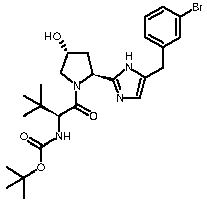 |

**[Table 1-8]**

| Ref. Ex. No. | structural formula | Ref. Ex. No. | structural formula |
|---|---|---|---|
| 93 | 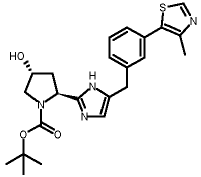 | 94 | 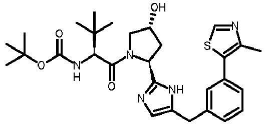 |
| 95 | 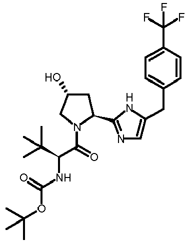 | 96 | 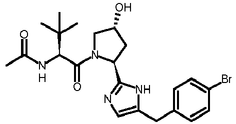 |
| 97 | 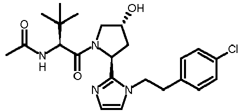 | 98 | 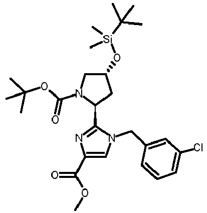 |
| 99 | 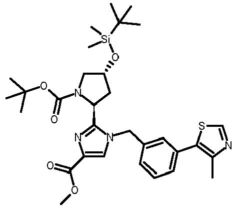 | 100 | 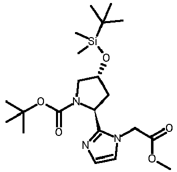 |
| 101 | 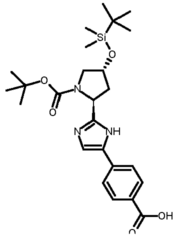 | 102 | 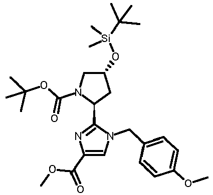 |
| 103 | 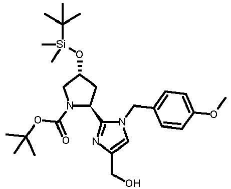 | 104 | 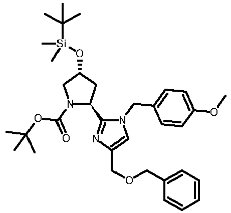 |

**[Table 1-9]**

| Ref. Ex. No. | structural formula | Ref. Ex. No. | structural formula |
|---|---|---|---|
| 105 | 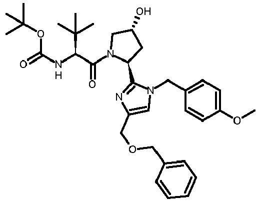 | 106 | 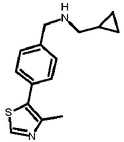 |
| 107 | 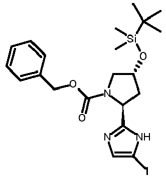 | 108 | 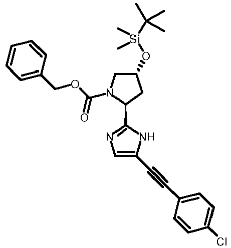 |
| 109 | 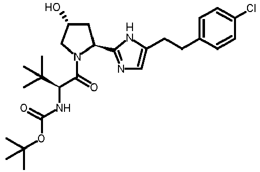 | 110 | 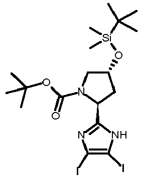 |
| 111 | 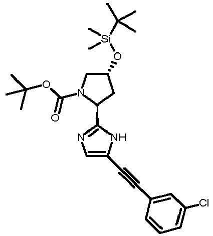 | 112 | 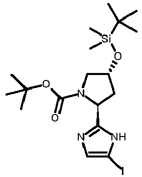 |
| 113 | 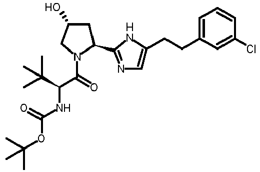 | 114 | 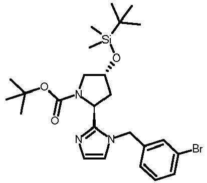 |
| 115 | 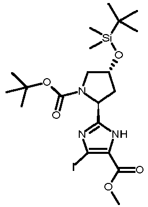 | 116 | 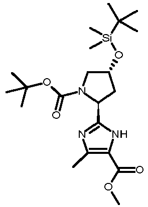 |

**[Table 1-10]**

| Ref. Ex. No. | structural formula | Ref. Ex. No. | structural formula |
|---|---|---|---|
| 117 | 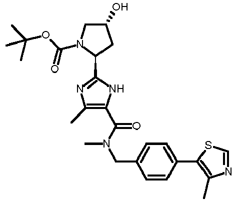 | 118 | 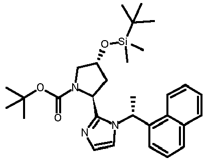 |
| 119 | 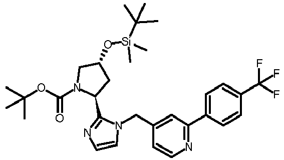 | 120 | 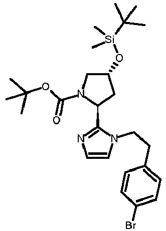 |
| 121 | 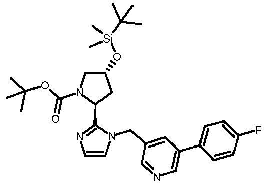 | 122 | 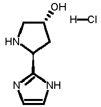 |
| 123 | 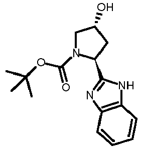 | 124 | 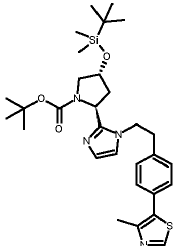 |
| 125 | 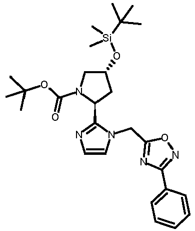 | 126 | 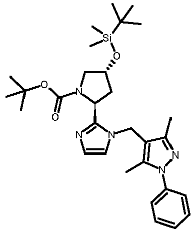 |
| 127 | 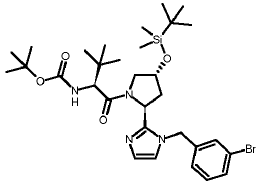 | 128 | 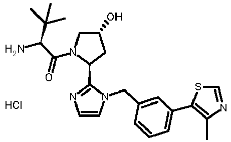 |

**[Table 1-11]**

| Ref. Ex. No. | structural formula | Ref. Ex. No. | structural formula |
|---|---|---|---|
| 129 | 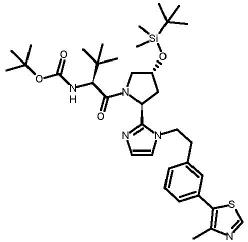 | 130 | 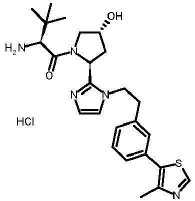 |
| 131 | 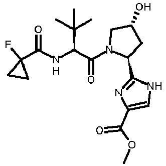 | 132 | 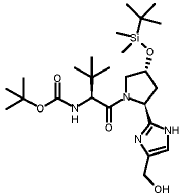 |
| 133 | 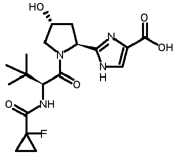 | 134 | 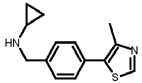 |
| 135 | 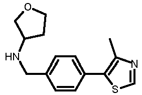 | 136 | 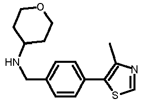 |
| 137 | 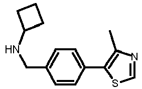 | 138 | 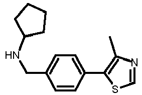 |
| 139 | 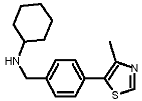 | 140 | 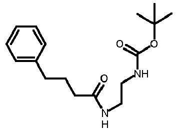 |
| 141 | 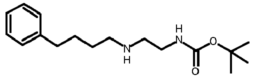 | 142 | 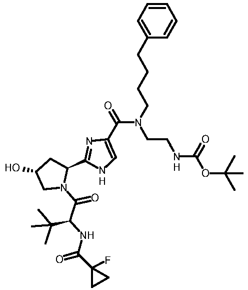 |

**[Table 1-12]**

| Ref. Ex. No. | structural formula | Ref. Ex. No. | structural formula |
|---|---|---|---|
| 143 | 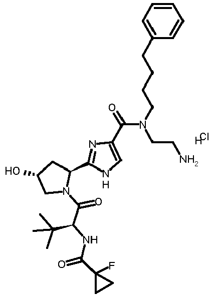 | 144 | 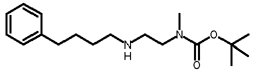 |
| 145 | 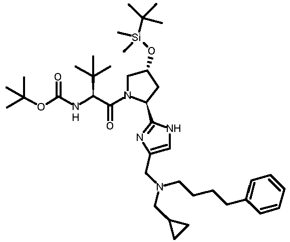 | 146 | 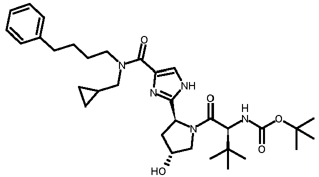 |
| 147 | 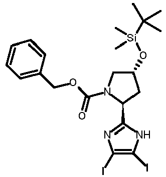 | 148 | 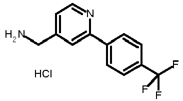 |
| 149 | 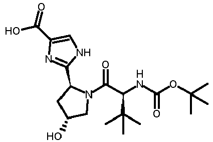 | 150 | 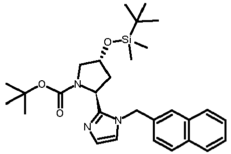 |
| 151 | 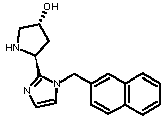 | 152 | 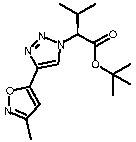 |
| 153 | 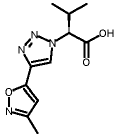 | 154 | 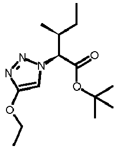 |

**[Table 1-13]**

| Ref. Ex. No. | structural formula | Ref. Ex. No. | structural formula |
|---|---|---|---|
| 155 | 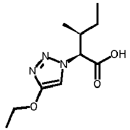 | 156 | 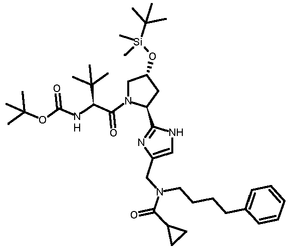 |
| 157 | 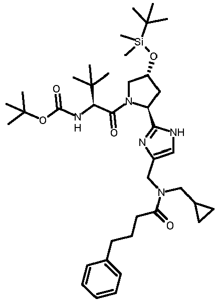 | 158 | 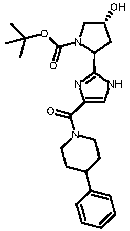 |
| 159 | 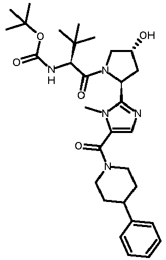 | 160 | 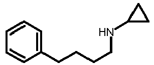 |
| 161 | 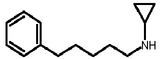 | 162 | 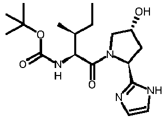 |
| 163 | 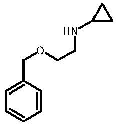 | 164 | 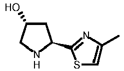 |
| 165 | 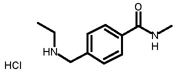 | 166 | 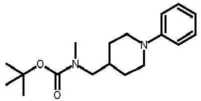 |
| 167 | 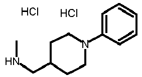 | 168 | 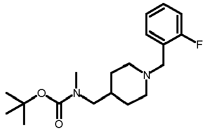 |

**[Table 1-14]**

| Ref. Ex. No. | structural formula | Ref. Ex. No. | structural formula |
|---|---|---|---|
| 169 | 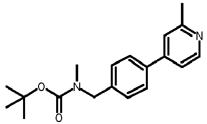 | 170 | 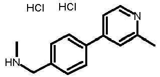 |
| 171 | 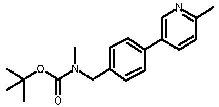 | 172 | 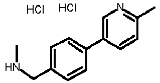 |
| 173 | 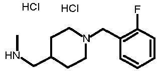 | 174 | 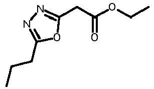 |
| 175 | 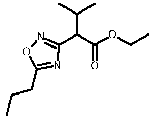 | 176 | 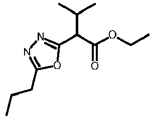 |
| 177 | 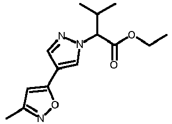 | 178 | 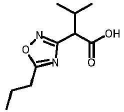 |
| 179 | 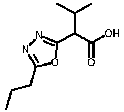 | | 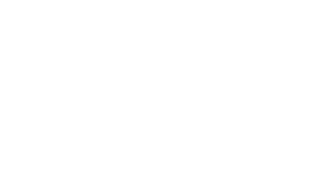 |

### Reference Example 180: Synthesis of N-[5-[4-(2-aminoethoxy)phenyl]-4-fluoro-2-[rel-(3S,5R)-3,4,5-trimethylpiperazin-1-yl]phenyl]-4-(trifluoromethyl)-6-(2-trimethylsilylethoxy)pyridine-3-carboxamide (180-1): benzyl N-[2-[4-[2-fluoro-4-[rel-(3S,5R)-3,4,5-trimethylpiperazin-1-yl]-5-[[4-(trifluoromethyl)-6-(2-trimethylsilylethoxy)pyridine-3-carbonyl]amino]phenyl]phenoxy]ethyl]carbamate (Reference Example compound 180-1)

N-{5-Bromo-4-fluoro-2-[rel-(3R,5S)-3,4,5-trimethylpiperazin-1-yl]phenyl}-4-(trifluoromethyl)-6-[2-(trimethylsilyl)ethoxy]pyridine-3-carboxamide (1.0 g), benzyl {2-[4-(4,4,5,5-tetramethyl-1,3,2-dioxaborolan-2-yl)phenoxy]ethyl}carbamate (1.0 g), bis[tert-butyl(4-dimethylaminophenyl)phosphine]dichloropalladium(II) (120 mg), tripotassium phosphate (700 mg) were added to 1,4-dioxane (30 mL) and water (3 mL), and the mixture was stirred at 100°C for 2 hr. The mixture was allowed to cool to room temperature, diluted with ethyl acetate, and dried over sodium sulfate. Insoluble material was filtered off, and the filtrate was concentrated under reduced pressure, and the obtained residue was purified by silica gel column chromatography (chloroform: methanol=100:0 - 95:5) and NH silica gel column chromatography (hexane:ethyl acetate=80:20 - 50:50)purified to give the title compound (1.22 g) as a colorless solid.
MS(ESI)m/z:796.6[M+H]⁺

### (180-2): N-[5-[4-(2-aminoethoxy)phenyl]-4-fluoro-2-[rel-(3S,5R)-3,4,5-trimethylpiperazin-1-yl]phenyl]-4-(trifluoromethyl)-6-(2-trimethylsilylethoxy)pyridine-3-carboxamide (Reference Example 180)

To a solution of Reference Example compound 180-1 (1.22 g) in methanol (30 mL) was added 10%Pd/C (500 mg), and the mixture was stirred under hydrogen atmosphere at room temperature for 2 hr. After completion of the reaction, insoluble material was filtered off with celite, and the filtrate was concentrated under reduced pressure to give the title compound (1.05 g) as a gray solid.
MS(ESI)m/z:662.5[M+H]⁺

### Reference Example 181: Synthesis of 2-[(2S,4R)-1-[(2S)-2-amino-3,3-dimethylbutanoyl]-4-hydroxypyrrolidin-2-yl]-N-(cyclopropylmethyl)-N-(4-phenylbutyl)-1H-imidazole-4-carboxamide hydrochloride

To Reference Example compound 146 (50 mg) was added 4 M hydrochloric acid-dioxane (20 mL) at room temperature and the mixture was stirred for 0.5 hr. After completion of the reaction, the reaction solution was concentrated under reduced pressure to give the title compound as an unpurified yellow oil (50 mg). MS(ESI)m/z:496.2[M+H]⁺

### Reference Example 182: Synthesis of 2-[2-[2-[2-[4-[(6-isopropoxy-1,3-dimethyl-2-oxo-benzoimidazol-5-yl)carbamoyl]-3-methoxy-phenoxy]ethoxy]ethoxy]ethoxy]acetic acid (182-1): tert-butyl 2-[2-[2-[2-[4-[(6-isopropoxy-1,3-dimethyl-2-oxo-benzoimidazol-5-yl)carbamoyl]-3-methoxy-phenoxy]ethoxy]ethoxy]ethoxy]acetate (Reference Example compound 182-1)

To a solution of tert-butyl [2-(2-{2-[(4-methylbenzene-1-sulfonyl) oxy]ethoxy}ethoxy)ethoxy]acetate (6.0 g), N-{1,3-dimethyl-2-oxo-6-[(propan-2-yl)oxy]-2,3-dihydro-1H-benzoimidazol-5-yl}-4-hydroxy-2-methoxybenzamide (5.53 g), and N,N-dimethylformamide (100 mL) was added potassium carbonate (5.94 g) and the mixture was stirred at 60°C for 12 hr. After completion of the reaction, water was added, and the mixture was extracted with ethyl acetate. The organic layer was dried over anhydrous sodium sulfate, filtered, and concentrated under reduced pressure. The obtained residue was purified by preparative HPLC (formic acid addition) to give the title compound (3.0 g) as a yellow oil.
MS(ESI)m/z:386.2[M+H]⁺

### (182-2): 2-[2-[2-[2-[4-[(6-isopropoxy-1,3-dimethyl-2-oxo-benzoimidazol-5-yl)carbamoyl]-3-methoxy-phenoxy]ethoxy]ethoxy]ethoxy]acetic acid (Reference Example 182)

To a solution of Reference Example compound 182-1 (3.0 g) in DCM (20 mL) was added trifluoroacetic acid (5.0 mL) and the mixture was stirred with heating at room temperature for 19 hr. After completion of the reaction, the reaction solution was concentrated under reduced pressure. The obtained residue was purified by preparative HPLC (water (0.1%TFA)-acetonitrile) to give the title compound (1.0 g) as a colorless oil. MS(ESI)m/z:576.3[M+H]⁺

### Reference Example 183: Synthesis of benzyl 5-[[(1S)-1-[(2S,4R)-4-hydroxy-2-[5-[3-(4-methylthiazol-5-yl)phenyl]-1H-imidazol-2-yl]pyrrolidine-1-carbonyl]-2,2-dimethyl-propyl]amino]-5-oxo-pentanoate

Using Reference Example compound 18, the same reaction and treatment as in Reference Example 70 were performed, and then the same reaction and treatment as in Reference Example 96 were performed using 5-benzyl-5-oxopentanoic acid to give the title compound (25 mg) as a pale-yellow solid. MS(ESI)m/z:644.3[M+H]⁺

**[Table 1-15]**

| Ref. Ex. No. | structural formula | Ref. Ex. No. | structural formula |
|---|---|---|---|
| 180 | 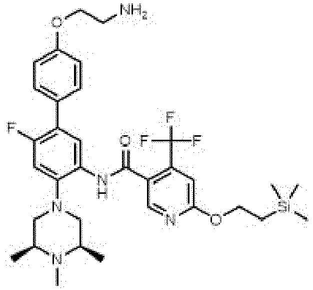 | 181 | 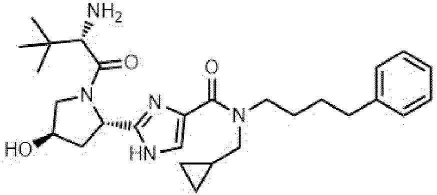 |
| 182 | 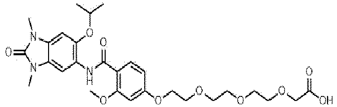 | 183 | 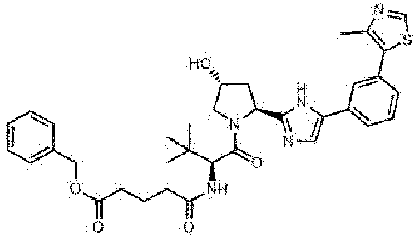 |

### [Example]

### Example 1: Synthesis of 1-[(2S,4R)-4-hydroxy-2-[4-(4-phenoxypiperidine-1-carbonyl)-1H-imidazol-2-yl]pyrrolidin-1-yl]-2-(3-methoxy-1,2-oxazol-5-yl)-3-methylbutan-1-one

2-[(2S,4R)-4-Hydroxy-1-[2-(3-methoxy-1,2-oxazol-5-yl)-3-methylbutanoyl]pyrrolidin-2-yl]-1H-imidazole-4-carboxylic acid (20 mg) described in Reference Example 8, HATU (30 mg), and 4-phenoxypiperidine (14 mg) were dissolved in DMF (600 µL), DIPEA (40 µL) was added and the mixture was stirred at room temperature for about 1 hr. To the reaction mixture were added chloroform and water, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (16 mg) as a white powder. MS(ESI)m/z:536.3[M-H]-

### Example 2: Synthesis of 1-[(2S,4R)-4-hydroxy-2-[4-(4-phenylpiperidine-1-carbonyl)-1H-imidazol-2-yl]pyrrolidin-1-yl]-2-(3-methoxy-1,2-oxazol-5-yl)-3-methylbutan-1-one

2-[(2S,4R)-4-Hydroxy-1-[2-(3-methoxy-1,2-oxazol-5-yl)-3-methylbutanoyl]pyrrolidin-2-yl]-1H-imidazole-4-carboxylic acid (20 mg) described in Reference Example 8, HATU (30 mg), and 4-phenylpiperidine (13 mg) were dissolved in DMF (600 µL), DIPEA (36 µL) was added and the mixture was stirred at room temperature for about 1 hr. To the reaction mixture were added chloroform and water, and the organic layer was separated by a Phase-separator (registered trade mark), and concentrated under reduced pressure. The residue was purified by preparative HPLC (10 mM ammonium carbonate aqueous solution-acetonitrile) to give the title compound (20 mg) as a white powder.
MS(ESI)m/z:520.3[M-H]-

### Example 3: Synthesis of N-(cyclobutylmethyl)-2-[(2S,4R)-4-hydroxy-1-[2-(3-methoxy-1,2-oxazol-5-yl)-3-methylbutanoyl]pyrrolidin-2-yl]-N-[[4-(4-methyl-1,3-thiazol-5-yl)phenyl]methyl]-1H-imidazole-4-carboxamide

HATU (30 mg) and 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (20 mg) described in Reference Example 8 was dissolved in DMF (500 µL), were stirred for about 10 min. N-(cyclobutylmethyl)-1-[4-(4-methyl-1,3-thiazol-5-yl)phenyl]methanamine (18 mg) described in Reference Example 10 and DIPEA (27 µL) were added and the mixture was stirred at room temperature for about 2 hr. To the reaction mixture were added chloroform and saturated aqueous sodium hydrogen carbonate, and the organic layer was separated by a Phase-separator (registered trade mark), and concentrated under reduced pressure. The residue was purified by preparative HPLC (10 mM ammonium carbonate aqueous solution-acetonitrile) to give the title compound (27 mg) as a white powder. MS(ESI)m/z:633.3[M+H]+

### Example 4: Synthesis of 2-[(2S,4R)-4-hydroxy-1-[2-(3-methoxy-1,2-oxazol-5-yl)-3-methylbutanoyl]pyrrolidin-2-yl]-N-(2-methylpropyl)-N-[[4-(4-methyl-1,3-thiazol-5-yl)phenyl]methyl]-1H-imidazole-4-carboxamide

HATU (30 mg) and 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (20 mg) described in Reference Example 8 were dissolved in DMF (500 µL), and the mixture was stirred for about 10 min. 2-Methyl-N-[[4-(4-methyl-1,3-thiazol-5-yl)phenyl]methyl]propan-1-amine (17 mg) described in Reference Example 11 and DIPEA (27 µL) were added and the mixture was stirred at room temperature for about 2 hr. To the reaction mixture were added chloroform and saturated aqueous sodium hydrogen carbonate, and the organic layer was separated by a Phase-separator (registered trade mark), and concentrated under reduced pressure. The residue was purified by preparative HPLC (10 mM ammonium carbonate aqueous solution-acetonitrile). The obtained crude product was further purified by silica gel column chromatography (ethyl acetate:methanol=100:0 - 85:15) to give the title compound (15 mg) as a white powder. MS(ESI)m/z:621.3[M+H]+

### Example 5: Synthesis of 2-[(2S,4R)-1-[(2S)-2-acetamido-3,3-dimethylbutanoyl]-4-hydroxypyrrolidin-2-yl]-N-methyl-N-[(4-phenylphenyl)methyl]-1H-imidazole-4-carboxamide

The title compound was obtained by the same reaction and treatment as in Example 3 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-(4-phenylphenyl)methanamine. MS(ESI)m/z:532.4[M+H]+

### Example 6: Synthesis of N-[(2S)-1-[(2S,4R)-4-hydroxy-2-[5-[3-(4-methyl-1,3-thiazol-5-yl)phenyl]-1H-imidazol-2-yl]pyrrolidin-1-yl]-3,3-dimethyl-1-oxobutan-2-yl]acetamide

The title compound was obtained by the same reaction and treatment as in Example 3 using (3R,5S)-5-[5-[3-(4-methyl-1,3-thiazol-5-yl)phenyl]-1H-imidazol-2-yl]pyrrolidin-3-ol hydrochloride described in Reference Example 18 and (2S)-2-acetamido-3,3-dimethylbutanoic acid. MS(ESI)m/z:482.3[M+H]+

### Example 7: Synthesis of 2-[(2S,4R)-1-[(2S)-2-acetamido-3,3-dimethylbutanoyl]-4-hydroxypyrrolidin-2-yl]-N-(2-pyridin-2-yl ethyl)-1H-imidazole-4-carboxamide

2-[(2S,4R)-1-[(2S)-2-acetamido-3,3-dimethylbutanoyl]-4-hydroxypyrrolidin-2-yl]-1H-imidazole-4-carboxylic acid (9.5 mg) described in Reference Example 14 was dissolved in NMP (1.0 mL), HATU (30 mg) was added and the mixture was stirred. DIPEA (14 µL) and 2-(2-pyridyl)ethaneamine (5.0 mg) were added and the mixture was stirred at room temperature for about 30 min. The reaction mixture was adsorbed onto PoraPak^{™} Rxn Rp and washed with water. After elution with acetonitrile-water, the eluate was concentrated by centrifugation. The residue was purified by silica gel column chromatography (ethyl acetate:methanol=95:5 - 70:30) to give the title compound (4.6 mg) as a colorless powder. MS(ESI)m/z:457.3[M+H]+

### Example 8: Synthesis of 2-[(2S,4R)-1-[(2S)-2-acetamido-3,3-dimethylbutanoyl]-4-hydroxypyrrolidin-2-yl]-N-methyl-N-(4-phenylbutyl)-1H-imidazole-4-carboxamide

2-[(2S,4R)-1-[(2S)-2-acetamido-3,3-dimethylbutanoyl]-4-hydroxypyrrolidin-2-yl]-1H-imidazole-4-carboxylic acid (13 mg) described in Reference Example 14 was dissolved in NMP (4.0 mL), HATU (30 mg) was added and the mixture was stirred for about 10 min. N-methyl-4-phenyl-butan-1-amine (7.1 mg) and DIPEA (18 µL) were added and the mixture was stirred at room temperature for about 10 min. The reaction mixture was purified by preparative HPLC (10 mM ammonium carbonate aqueous solution-acetonitrile) to give the title compound (9.4 mg) as a colorless oil. MS(ESI)m/z:498.4[M+H]+

### Example 9: Synthesis of 2-[(2S,4R)-1-[(2S)-2-acetamido-3,3-dimethylbutanoyl]-4-hydroxypyrrolidin-2-yl]-N-(2-phenylethyl)-1H-imidazole-4-carboxamide

The title compound was obtained by the same reaction and treatment as in Example 7 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 2-phenylethaneamine. MS(ESI)m/z:456.3[M+H]+

### Example 10: Synthesis of 2-[(2S,4R)-1-[(2S)-2-acetamido-3,3-dimethylbutanoyl]-4-hydroxypyrrolidin-2-yl]-N-[2-(2-methoxyphenyl)ethyl]-1H-imidazole-4-carboxamide

The title compound was obtained by the same reaction and treatment as in Example 7 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 2-(2-methoxyphenyl)ethaneamine. MS(ESI)m/z:486.3[M+H]+

### Example 11: Synthesis of 2-[(2S,4R)-1-[(2S)-2-acetamido-3,3-dimethylbutanoyl]-4-hydroxypyrrolidin-2-yl]-N-[2-(3-methoxyphenyl)ethyl]-1H-imidazole-4-carboxamide

The title compound was obtained by the same reaction and treatment as in Example 7 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 2-(3-methoxyphenyl)ethaneamine. MS(ESI)m/z:486.3[M+H]+

### Example 12: Synthesis of 2-[(2S,4R)-1-[(2S)-2-acetamido-3,3-dimethylbutanoyl]-4-hydroxypyrrolidin-2-yl]-N-[2-(4-methoxyphenyl)ethyl]-1H-imidazole-4-carboxamide

The title compound was obtained by the same reaction and treatment as in Example 7 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 2-(4-methoxyphenyl)ethaneamine. MS(ESI)m/z:486.3[M+H]+

### Example 13: Synthesis of N-[(2S)-1-[(2S,4R)-2-[4-(3,4-dihydro-1H-2,7-naphthyridine -2-carbonyl)-1H-imidazol-2-yl]-4-hydroxypyrrolidin-1-yl]-3,3-dimethyl-1-oxobutan-2-yl]acetamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 1,2,3,4-tetrahydro-2,7-naphthyridine dihydrochloride.
MS (ESI)m/z:469.3[M+H] +

### Example 14: Synthesis of 2-[(2S,4R)-1-[(2S)-2-acetamido-3,3-dimethylbutanoyl]-4-hydroxypyrrolidin-2-yl]-N-[(2-chlorophenyl)methyl]-N-methyl-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 1-(2-chlorophenyl)-N-methyl-methanamine.
MS(ESI)m/z:490.2/492.2[M+H]+

### Example 15: Synthesis of 2-[(2S,4R)-1-[(2S)-2-acetamido-3,3-dimethylbutanoyl]-4-hydroxypyrrolidin-2-yl]-N-[(3-chlorophenyl)methyl]-N-methyl-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 1-(3-chlorophenyl)-N-methyl-methanamine.
MS(ESI)m/z:490.2/492.2[M+H]+

### Example 16: Synthesis of 2-[(2S,4R)-1-[(2S)-2-acetamido-3,3-dimethylbutanoyl]-4-hydroxypyrrolidin-2-yl]-N-[(4-chlorophenyl)methyl]-N-methyl-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 1-(4-chlorophenyl)-N-methyl-methanamine.
MS(ESI)m/z:490.2/492.2[M+H]+

### Example 17: Synthesis of 2-[(2S,4R)-1-[(2S)-2-acetamido-3,3-dimethylbutanoyl]-4-hydroxypyrrolidin-2-yl]-N-[(2-fluorophenyl)methyl]-N-methyl-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 1-(2-fluorophenyl)-N-methyl-methanamine. MS(ESI)m/z:474.3[M+H]+

### Example 18: Synthesis of 2-[(2S,4R)-1-[(2S)-2-acetamido-3,3-dimethylbutanoyl]-4-hydroxypyrrolidin-2-yl]-N-[(3-fluorophenyl)methyl]-N-methyl-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 1-(3-fluorophenyl)-N-methyl-methanamine. MS(ESI)m/z:474.3[M+H]+

### Example 19: Synthesis of 2-[(2S,4R)-1-[(2S)-2-acetamido-3,3-dimethylbutanoyl]-4-hydroxypyrrolidin-2-yl]-N-[(4-fluorophenyl)methyl]-N-methyl-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 1-(4-fluorophenyl)-N-methyl-methanamine. MS(ESI)m/z:474.3[M+H]+

### Example 20: Synthesis of 2-[(2S,4R)-1-[(2S)-2-acetamido-3,3-dimethylbutanoyl]-4-hydroxypyrrolidin-2-yl]-N-methyl-N-[[2-(trifluoromethyl)phenyl]methyl]-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-[2-(trifluoromethyl)phenyl]methanamine.
MS(ESI)m/z:524.3[M+H]+

### Example 21: Synthesis of 2-[(2S,4R)-1-[(2S)-2-acetamido-3,3-dimethylbutanoyl]-4-hydroxypyrrolidin-2-yl]-N-methyl-N-[[3-(trifluoromethyl)phenyl]methyl]-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-[3-(trifluoromethyl)phenyl]methanamine.
MS (ESI)m/z:524.3 [M+H]+

### Example 22: Synthesis of 2-[(2S,4R)-1-[(2S)-2-acetamido-3,3-dimethylbutanoyl]-4-hydroxypyrrolidin-2-yl]-N-methyl-N-[[4-(trifluoromethyl)phenyl]methyl]-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-[4-(trifluoromethyl)phenyl]methanamine.
MS(ESI)m/z:524.4[M+H]+

### Example 23: Synthesis of 2-[(2S,4R)-1-[(2S)-2-acetamido-3,3-dimethylbutanoyl]-4-hydroxypyrrolidin-2-yl]-N-[(2-methoxyphenyl)methyl]-N-methyl-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 1-(2-methoxyphenyl)-N-methyl-methanamine. MS(ESI)m/z:486.3[M+H]+

### Example 24: Synthesis of 2-[(2S,4R)-1-[(2S)-2-acetamido-3,3-dimethylbutanoyl]-4-hydroxypyrrolidin-2-yl]-N-[(3-methoxyphenyl)methyl]-N-methyl-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 1-(3-methoxyphenyl)-N-methyl-methanamine. MS(ESI)m/z:486.3[M+H]+

### Example 25: Synthesis of 2-[(2S,4R)-1-[(2S)-2-acetamido-3,3-dimethylbutanoyl]-4-hydroxypyrrolidin-2-yl]-N-[(4-methoxyphenyl)methyl]-N-methyl-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 1-(4-methoxyphenyl)-N-methyl-methanamine. MS(ESI)m/z:486.3[M+H]+

### Example 26: Synthesis of 2-[(2S,4R)-1-[(2S)-2-acetamido-3,3-dimethylbutanoyl]-4-hydroxypyrrolidin-2-yl]-N-methyl-N-(pyrimidin-2-ylmethyl)-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-pyrimidin-2-yl-methanamine. MS(ESI)m/z:458.3[M+H]+

### Example 27: Synthesis of 2-[(2S,4R)-1-[(2S)-2-acetamido-3,3-dimethylbutanoyl]-4-hydroxypyrrolidin-2-yl]-N-methyl-N-[(1-methylpyrazol-3-yl)methyl]-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-(1-methylpyrazol-3-yl)methanamine. MS(ESI)m/z:460.3[M+H]+

### Example 28: Synthesis of 2-[(2S,4R)-1-[(2S)-2-acetamido-3,3-dimethylbutanoyl]-4-hydroxypyrrolidin-2-yl]-N-methyl-N-(3-phenylpropyl)-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-3-phenyl-propan-1-amine. MS(ESI)m/z:484.3[M+H]+

### Example 29: Synthesis of 2-[(2S,4R)-1-[(2S)-2-acetamido-3,3-dimethylbutanoyl]-4-hydroxypyrrolidin-2-yl]-N-methyl-N-[[3-(4-methyl-1,3-thiazol-5-yl)phenyl]methyl]-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-[3-(4-methylthiazol-5-yl)phenyl]methanamine described in Reference Example 20. MS(ESI)m/z:553.4[M+H]+

### Example 30: Synthesis of 2-[(2S,4R)-1-[(2S)-2-acetamido-3,3-dimethylbutanoyl]-4-hydroxypyrrolidin-2-yl]-N-methyl-N-(naphthalen-2-ylmethyl)-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-(2-naphthyl)methanamine. MS(ESI)m/z:506.3[M+H]+

### Example 31: Synthesis of 2-[(2S,4R)-1-[(2S)-2-acetamido-3,3-dimethylbutanoyl]-4-hydroxypyrrolidin-2-yl]-N-methyl-N-(naphthalen-1-ylmethyl)-1H-imidazole-4-carboxamide

The title compound was obtained by the same reaction and treatment as in Example 8 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-(1-naphthyl)methanamine. MS(ESI)m/z:506.3[M+H]+

### Example 32: Synthesis of 1-[(2S,4R)-4-hydroxy-2-[1-[(3-phenylphenyl)methyl]imidazol-2-yl]pyrrolidin-1-yl]-2-(3-methoxy-1,2-oxazol-5-yl)-3 methylbutan-1-one

4 M Hydrochloric acid-dioxane (2.0 mL) was added to tert-butyl (2S,4R)-4-[tert-butyl(dimethyl)silyl]oxy-2-[1-[(3-phenylphenyl)methyl]imidazol-2-yl]pyrrolidine-1-carboxylate (34 mg) described in Reference Example 22, and the mixture was stirred at room temperature for about 14 hr. After completion of the reaction, the precipitated solid was collected by filtration. To the collected powder were added dichloromethane (1.0 mL), 2-(3-methoxy-1,2-oxazol-5-yl)-3-methylbutanoic acid (16 mg), HATU (30 mg), and DIPEA (30 µL), and the mixture was stirred at room temperature for about 1 hr. To the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (22 mg) as a white powder. MS(ESI)m/z:501.6[M+H]+

### Example 33: Synthesis of 1-[(2S,4R)-4-hydroxy-2-[1-[[3-(4-methyl-1,3-thiazol-5-yl)phenyl]methyl]imidazol-2-yl]pyrrolidin-1-yl]-2-(3-methoxy-1,2-oxazol-5-yl)-3 methylbutan-1-one

tert-Butyl (2S,4R)-4-[tert-butyl(dimethyl)silyl]oxy-2-[1-[[3-(4-methyl-1,3-thiazol-5-yl)phenyl]methyl]imidazol-2-yl]pyrrolidine-1-carboxylate (35 mg) described in Reference Example 24 was dissolved in 1,4-dioxane (1.0 mL), 4 M hydrochloric acid-dioxane (200 µL) was added, and the mixture was stirred at room temperature for about 16 hr. After completion of the reaction, the reaction solution was concentrated under reduced pressure. To the residue were added dichloromethane (1.0 mL), 2-(3-methoxy-1,2-oxazol-5-yl)-3-methylbutanoic acid (16 mg), HATU (29 mg), and DIPEA (40 µL), and the mixture was stirred at room temperature for about 1 hr. To the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (26 mg) as a white powder. MS (ESI)m/z:522.3 [M+H]+

### Example 34: Synthesis of 1-[(2S,4R)-4-hydroxy-2-[1-[2-[3-(4-methyl-1,3-thiazol-5-yl)phenyl]ethyl]imidazol-2-yl]pyrrolidin-1-yl]-2-(3-methoxy-1,2-oxazol-5-yl)-3 methylbutan-1-one

4 M Hydrochloric acid-dioxane (1.0 mL) was added to tert-butyl (2S,4R)-4-[tert-butyl(dimethyl)silyl]oxy-2-[1-[2-[3-(4-methyl-1,3-thiazol-5-yl)phenyl]ethyl]imidazol-2-yl]pyrrolidine-1-carboxylate (70 mg) described in Reference Example 26, and the mixture was stirred at room temperature for about 2 hr. After completion of the reaction, the precipitated solid was collected by filtration. To the collected powder were added dichloromethane (1.0 mL), 2-(3-methoxy-1,2-oxazol-5-yl)-3-methylbutanoic acid (32 mg), HATU (55 mg), and DIPEA (60 µL), and the mixture was stirred at room temperature for about 1.5 hr. To the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (5.6 mg) as a white powder. MS (ESI)m/z:536.3 [M+H]+

### Example 35: Synthesis of 2-[(2S,4R)-1-[(2S)-2-acetamido-3,3-dimethylbutanoyl]-4-hydroxypyrrolidin-2-yl]-N-methyl-N-[[4-(4-methyl-1,3-thiazol-5-yl)phenyl]methyl]-1H-imidazole-4-carboxamide

2-[(2S,4R)-1-[(2S)-2-acetamido-3,3-dimethylbutanoyl]-4-hydroxypyrrolidin-2-yl]-1H-imidazole-4-carboxylic acid described in Reference Example 14 (23 mg), N-methyl-1-[4-(4-methyl-1,3-thiazol-5-yl)phenyl]methanamine (22 mg), DCM (1252 µL), DIPEA (16 µL), and HATU (30 mg) were successively added and the mixture was stirred at room temperature overnight. N-Methyl-1-[4-(4-methyl-1,3-thiazol-5-yl)phenyl]methanamine (22 mg) and DIPEA (16 µL) were added and the mixture was stirred at room temperature for about 2 hr. After completion of the reaction, the reaction mixture was purified by silica gel column chromatography (ethyl acetate:methanol=100:0 - 75:25). Furthermore, the obtained crude product was purified by preparative HPLC (10 mM ammonium carbonate aqueous solution-acetonitrile) to give the title compound (11 mg) as a white powder. MS(ESI)m/z:553.3[M+H]+

### Example 36: Synthesis of N-(cyclopropylmethyl)-2-[(2S,4R)-4-hydroxy-1-[2-(3-methoxy-1,2-oxazol-5-yl)-3-methylbutanoyl]pyrrolidin-2-yl]-N-(4-phenylbutyl)-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (17 mg) described in Reference Example 8, N-(cyclopropylmethyl)-4-phenyl-butan-1-amine (27 mg) described in Reference Example 27, and HATU (22 mg) were dissolved in DCM (1 mL), DIPEA (30 µL) was added, and the mixture was stirred at room temperature for 3 hr. After completion of the reaction, to the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (10 mg) as a white powder. MS(ESI)m/z:564.6[M+H]+

### Example 37: Synthesis of N-(cyclopropylmethyl)-2-[(2S,4R)-4-hydroxy-1-[2-(3-methoxy-1,2-oxazol-5-yl)-3-methylbutanoyl]pyrrolidin-2-yl]-N-(4-pyridin-4-ylbutyl)-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (18 mg) described in Reference Example 8, N-(cyclopropylmethyl)-4-(4-pyridyl)butan-1-amine (12 mg) described in Reference Example 28, and HATU (25 mg) was dissolved in DCM (1 mL), DIPEA (30 µL) was added, and the mixture was stirred at room temperature for 2.5 hr. After completion of the reaction, to the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (15 mg) as a white powder.
MS (ESI)m/z:565.7[M+H] +

### Example 38: Synthesis of 2-[(2S,4R)-4-hydroxy-1-[2-(3-methoxy-1,2-oxazol-5-yl)-3-methylbutanoyl]pyrrolidin-2-yl]-N-[[4-(4-methyl-1,3-thiazol-5-yl)phenyl]methyl]-N-propan-2-yl-1H-imidazole-4-carboxamide

N-[[4-(4-methylthiazol-5-yl)phenyl]methyl]propane-2-amine (27 mg) described in Reference Example 29, 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (20 mg) described in Reference Example 8, and HATU (26 mg) was dissolved in DCM (1 mL), DIPEA (30 µL) was added, and the mixture was stirred at room temperature for 2.5 hr. After completion of the reaction, to the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (14 mg) as a white powder.
MS(ESI)m/z:607.6[M+H]+

### Example 39: Synthesis of N-[(2S)-1-[(2S,4R)-2-[4-(6,7-dihydro-4H-[1,3]thiazolo[5,4-c]pyridine-5-carbonyl)-1H-imidazol-2-yl]-4-hydroxypyrrolidin-1-yl]-3,3-dimethyl-1-oxobutan-2-yl]acetamide

The title compound was obtained by the same reaction and treatment as in Example 38 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 4,5,6,7-tetrahydrothiazolo-[5,4-c]pyridine salt acid. MS (ESI)m/z:475.2 [M+H]+

### Example 40: Synthesis of 2-[(2S,4R)-4-hydroxy-1-[2-(3-methoxy-1,2-oxazol-5-yl)-3-methylbutanoyl]pyrrolidin-2-yl]-N-[[4-(4-methyl-1,3-thiazol-5-yl)phenyl]methyl]-N-(2,2,2-trifluoroethyl)-1H-imidazole-4-carboxamide

The title compound was obtained by the same reaction and treatment as in Example 38 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 2,2,2-trifluoro-N-[[4-(4-methylthiazol-5-yl)phenyl]methyl]ethaneamine described in Reference Example 30. MS(ESI)m/z:647.6[M+H]+

### Example 41: Synthesis of 2-[(2S,4R)-4-hydroxy-1-[2-(3-methoxy-1,2-oxazol-5-yl)-3-methylbutanoyl]pyrrolidin-2-yl]-N-methyl-N-(1-pyridin-4-ylpiperidin-4-yl)-1H-imidazole-4-carboxamide

The title compound was obtained by the same reaction and treatment as in Example 38 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and N-methyl-1-(4-pyridyl)piperidine-4-amine.
MS(ESI)m/z:552.6[M+H]+

### Example 42: Synthesis of 2-[(2S,4R)-4-hydroxy-1-[2-(3-methoxy-1,2-oxazol-5-yl)-3-methylbutanoyl]pyrrolidin-2-yl]-N-methyl-N-[(4-phenyl-1,3-thiazol-2-yl)methyl]-1H-imidazole-4-carboxamide

The title compound was obtained by the same reaction and treatment as in Example 38 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and N-methyl-1-(4-phenylthiazol-2-yl)methanamine. MS(ESI)m/z:565.5[M+H]+

### Example 43: Synthesis of 2-[(2S,4R)-4-hydroxy-1-[2-(3-methoxy-1,2-oxazol-5-yl)-3-methylbutanoyl]pyrrolidin-2-yl]-N-methyl-N-[(1-pyridin-4-ylpiperidin-4-yl)methyl]-1H-imidazole-4-carboxamide

The title compound was obtained by the same reaction and treatment as in Example 38 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and N-methyl-1-[1-(4-pyridyl)-4-piperidyl]methanamine. MS(ESI)m/z:566.6[M+H]+

### Example 44: Synthesis of 1-[(2S,4R)-4-hydroxy-2-[5-[3-(4-methyl-1,3-thiazol-5-yl)phenyl]-1H-imidazol-2-yl]pyrrolidin-1-yl]-2-(3-methoxy-1,2-oxazol-5-yl)-3-methylbutan-1-one

(3R,5S)-5-[5-[3-(4-methyl-1,3-thiazol-5-yl)phenyl]-1H-imidazol-2-yl]pyrrolidin-3-ol hydrochloride (30 mg) described in Reference Example 18 was dissolved in DMF (3 mL), 2-(3-methoxy-1,2-oxazol-5-yl)-3-methylbutanoic acid (18 mg), DIPEA (57 µL), and HATU (41 mg) were added, and the mixture was stirred at room temperature for 1 hr. After completion of the reaction, the reaction was quenched by adding saturated brine, and the aqueous layer was extracted with ethyl acetate. The organic layer was washed with saturated brine, dried over anhydrous sodium sulfate, filtered, concentrated under reduced pressure, and purified by silica gel column chromatography (ethyl acetate:methanol=100:0 - 90:10) to give the title compound (29 mg) as a colorless powder. MS(ESI)m/z:508.4[M+H]+

### Example 45: Synthesis of 1-[(2S,4R)-4-hydroxy-2-[5-[3-(4-methyl-1,3-thiazol-5-yl)phenyl]-1H-imidazol-2-yl]pyrrolidin-1-yl]-2-[4-(2-methoxypyridin-4-yl)pyrazol-1-yl]-3-methylbutan-1-one

The title compound was obtained by the same reaction and treatment as in Example 44 using (3R,5S)-5-[5-[3-(4-methyl-1,3-thiazol-5-yl)phenyl]-1H-imidazol-2-yl]pyrrolidin-3-ol hydrochloride described in Reference Example 18 and 2-[4-(2-methoxypyridin-4-yl)pyrazol-1-yl]-3-methylbutanoic acid described in Reference Example 33. MS(ESI)m/z:584.4[M+H]+

### Example 46: Synthesis of 2-[(2S,4R)-1-[(2S)-2-acetamido-3,3-dimethylbutanoyl]-4-hydroxypyrrolidin-2-yl]-N-(pyridin-2-ylmethyl)-1H-imidazole-4-carboxamide

A solution (0.10 mL) of 2-pyridylmethanamine (121 mg) and DIPEA (0.60 mL) in NMP (3.4 mL), and a solution (0.65 mL) of 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 (120 mg) and HATU (142 mg) in NMP (8 mL) were injected into a flow reactor, mixed and reacted at 100°C for 10 min. The reaction mixture was adsorbed onto PoraPak^{™} Rxn Rp and washed with water. The eluate was concentrated by centrifugation and the residue was purified by preparative HPLC (10 mM ammonium carbonate aqueous solution-acetonitrile) to give the title compound (2.7 mg) as a pale-yellow oil. MS(ESI)m/z:443.3[M+H]+

### Example 47: Synthesis of 2-[(2S,4R)-1-[(2S)-2-acetamido-3,3-dimethylbutanoyl]-4-hydroxypyrrolidin-2-yl]-N-(pyridin-3-ylmethyl)-1H-imidazole-4-carboxamide

The title compound was obtained by the same reaction and treatment as in Example 46 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and 3-pyridylmethanamine. MS(ESI)m/z:443.3[M+H]+

### Example 48: Synthesis of 2-[(2S,4R)-1-[(2S)-2-acetamido-3,3-dimethylbutanoyl]-4-hydroxypyrrolidin-2-yl]-N-methyl-N-(pyridin-2-ylmethyl)-1H-imidazole-4-carboxamide

The title compound was obtained by the same reaction and treatment as in Example 46 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-(2-pyridyl)methanamine. MS(ESI)m/z:457.3[M+H]+

### Example 49: Synthesis of 2-[(2S,4R)-1-[(2S)-2-acetamido-3,3-dimethylbutanoyl]-4-hydroxypyrrolidin-2-yl]-N-methyl-N-(pyridin-4-ylmethyl)-1H-imidazole-4-carboxamide

The title compound was obtained by the same reaction and treatment as in Example 46 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-(4-pyridyl)methanamine. MS(ESI)m/z:457.3[M+H]+

### Example 50: Synthesis of 2-[(2S,4R)-1-[(2S)-2-acetamido-3,3-dimethylbutanoyl]-4-hydroxypyrrolidin-2-yl]-N-benzyl-1H-imidazole-4-carboxamide

The title compound was obtained by the same reaction and treatment as in Example 46 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and phenylmethanamine. MS(ESI)m/z:442.3[M+H]+

### Example 51: Synthesis of 2-[(2S,4R)-1-[(2S)-2-acetamido-3,3-dimethylbutanoyl]-4-hydroxypyrrolidin-2-yl]-N-benzyl-N-methyl-1H-imidazole-4-carboxamide

The title compound was obtained by the same reaction and treatment as in Example 46 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-phenyl-methanamine. MS(ESI)m/z:456.3[M+H]+

### Example 52: Synthesis of 2-[(2S,4R)-1-[(2S)-2-acetamido-3,3-dimethylbutanoyl]-4-hydroxypyrrolidin-2-yl]-N-methyl-N-(2-phenylethyl)-1H-imidazole-4-carboxamide

The title compound was obtained by the same reaction and treatment as in Example 46 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-2-phenylethaneamine. MS(ESI)m/z:470.3[M+H]+

### Example 53: Synthesis of 2-[(2S,4R)-1-[(2S)-2-acetamido-3,3-dimethylbutanoyl]-4-hydroxypyrrolidin-2-yl]-N-methyl-N-(pyridin-3-ylmethyl)-1H-imidazole-4-carboxamide

The title compound was obtained by the same reaction and treatment as in Example 46 using 2-[(2S,4R)-1-[(2S)-2-acetamido-3,3-dimethylbutanoyl]-4-hydroxypyrrolidin-2-yl]-1H-imidazole-4-carboxylic acid described in Reference Example 14 and N-methyl-1-(3-pyridyl)methanamine. MS(ESI)m/z:457.3[M+H]+

### Example 54: Synthesis of 2-[(2S,4R)-4-hydroxy-1-[2-(3-methoxy-1,2-oxazol-5-yl)-3-methylbutanoyl]pyrrolidin-2-yl]-N-methyl-N-[[4-(4-methyl-1,3-thiazol-5-yl)phenyl]methyl]-1H-imidazole-4-carboxamide

2-[(2S,4R)-4-hydroxypyrrolidin-2-yl]-N-methyl-N-[[4-(4-methyl-1,3-thiazol-5-yl)phenyl]methyl]-1H-imidazole-4-carboxamide hydrochloride (40 mg) described in Reference Example 6, 2-(3-methoxy-1,2-oxazol-5-yl)-3-methylbutanoic acid (24 mg), and HOBt (17 mg) were dissolved in DCM (420 µL), Et3N (35 µL) and WSC·HCl (24 mg) were added, and the mixture was stirred at room temperature for 2.5 hr. MeOH was added and the mixture was concentrated under reduced pressure. The residue was purified by preparative HPLC (10 mM ammonium carbonate aqueous solution-acetonitrile) to give the title compound (18 mg) as a white powder. MS(ESI)m/z:577.3[M-H]-

### Example 55: Synthesis of diastereomer eluted earlier under chiral HPLC conditions described in the experiment term of 2-[(2S,4R)-4-hydroxy-1-[2-(3-methoxy-1,2-oxazol-5-yl)-3-methylbutanoyl]pyrrolidin-2-yl]-N-methyl-N-[[4-(4-methyl-1,3-thiazol-5-yl)phenyl]methyl]-1H-imidazole-4-carboxamide

2-[(2S,4R)-4-hydroxy-1-[2-(3-methoxy-1,2-oxazol-5-yl)-3-methylbutanoyl]pyrrolidin-2-yl]-N-methyl-N-[[4-(4-methyl-1,3-thiazol-5-yl)phenyl]methyl]-1H-imidazole-4-carboxamide (10 mg) described in Reference Example 34 was separated using a chiral column [CHIRALPAK IC(30*250), methanol]. The fraction eluted earlier was recovered, and concentrated under reduced pressure to give the title compound (3.5 mg) as a white powder. MS(ESI)m/z:579.3[M+H]+

### Example 56: Synthesis of diastereomer eluted later under chiral HPLC conditions described in the experiment term of 2-[(2S,4R)-4-hydroxy-1-[2-(3-methoxy-1,2-oxazol-5-yl)-3-methylbutanoyl]pyrrolidin-2-yl]-N-methyl-N-[[4-(4-methyl-1,3-thiazol-5-yl)phenyl]methyl]-1H-imidazole-4-carboxamide

2-[(2S,4R)-4-hydroxy-1-[2-(3-methoxy-1,2-oxazol-5-yl)-3-methylbutanoyl]pyrrolidin-2-yl]-N-methyl-N-[[4-(4-methyl-1,3-thiazol-5-yl)phenyl]methyl]-1H-imidazole-4-carboxamide (10 mg) described in Reference Example 34 was separated using a chiral column [CHIRALPAK IC(30*250), methanol]. The fraction eluted later was recovered, and concentrated under reduced pressure to give the title compound (2.8 mg) as a white powder. MS(ESI)m/z:579.3[M+H]+

### Example 57: Synthesis of diastereomer eluted earlier under chiral HPLC conditions described in the experiment term of N-(cyclopropylmethyl)-2-[(2S,4R)-4-hydroxy-1-[2-(3-methoxy-1,2-oxazol-5-yl)-3-methylbutanoyl]pyrrolidin-2-yl]-N-[[4-(4-methyl-1,3-thiazol-5-yl)phenyl]methyl]-1H-imidazole-4-carboxamide

N-(cyclopropylmethyl)-2-[(2S,4R)-4-hydroxy-1-[2-(3-methoxy-1,2-oxazol-5-yl)-3-methylbutanoyl]pyrrolidin-2-yl]-N-[[4-(4-methyl-1,3-thiazol-5-yl)phenyl]methyl]-1H-imidazole-4-carboxamide (14 mg) described in Reference Example 36 was separated using a chiral column [CHIRALPAK IE(30*250), methyl tert-butyl ether:methanol=87:13]. The fraction eluted earlier was recovered, and concentrated to give the title compound (4 mg) as a white solid. MS(ESI)m/z(ESI)m/z:619.3[M+H]+

### Example 58: Synthesis of diastereomer eluted later under chiral HPLC conditions described in the experiment term of N-(cyclopropylmethyl)-2-[(2S,4R)-4-hydroxy-1-[2-(3-methoxy-1,2-oxazol-5-yl)-3-methylbutanoyl]pyrrolidin-2-yl]-N-[[4-(4-methyl-1,3-thiazol-5-yl)phenyl]methyl]-1H-imidazole-4-carboxamide

N-(cyclopropylmethyl)-2-[(2S,4R)-4-hydroxy-1-[2-(3-methoxy-1,2-oxazol-5-yl)-3-methylbutanoyl]pyrrolidin-2-yl]-N-[[4-(4-methyl-1,3-thiazol-5-yl)phenyl]methyl]-1H-imidazole-4-carboxamide (14 mg) described in Reference Example 36 was separated using a chiral column [CHIRALPAK IE(30*250), methyl tert-butyl ether:methanol=87:13]. The fraction eluted later was recovered, and concentrated to give the title compound (4 mg) as a white solid. MS(ESI)m/z(ESI)m/z:619.3[M+H]+

### Example 59: Synthesis of N-(cyclopropylmethyl)-2-[(2S,4R)-4-hydroxy-1-[2-(3-methoxy-1,2-oxazol-5-yl)-3-methylbutanoyl]pyrrolidin-2-yl]-N-[[4-(4-methyl-1,3-thiazol-5-yl)phenyl]methyl]-1H-imidazole-4-carboxamide

tert-Butyl (2S,4R)-2-[4-[cyclopropylmethyl-[[4-(4-methyl-1,3-thiazol-5-yl)phenyl]methyl]carbamoyl]-1H-imidazol-2-yl]-4-hydroxypyrrolidine-1-carboxylate (26 mg) described in Reference Example 35 was dissolved in 1,4-dioxane (0.4 mL), 4 M hydrochloric acid-dioxane (0.4 mL) was added at room temperature, and the mixture was stirred overnight. After completion of the reaction, the reaction solution was concentrated under reduced pressure and azeotropically distilled 3 times with toluene. The obtained residue was dissolved in N,N-dimethylformamide (0.5 mL), 2-(3-methoxy-1,2-oxazol-5-yl)-3-methylbutanoic acid (13 mg), HATU (22 mg), and diisopropyl ethylamine (34 µL) were added, and the mixture was stirred at room temperature for 3 hr. After completion of the reaction, water was added, and the mixture was extracted with ethyl acetate, and concentrated under reduced pressure. The obtained residue was purified by NH silica gel column chromatography (chloroform: methanol=100:0 - 90:10) to give the title compound (19 mg) as a white powder. MS(ESI)m/z:617.4[M-H]-

### Example 60: Synthesis of N-(2-hydroxyethyl)-2-[(2S,4R)-4-hydroxy-1-[2-(3-methoxy-1,2-oxazol-5-yl)-3-methylbutanoyl]pyrrolidin-2-yl]-N-[[4-(4-methyl-1,3-thiazol-5-yl)phenyl]methyl]-1H-imidazole-4-carboxamide

tert-Butyl N-[[4-(4-methyl-1,3-thiazol-5-yl)phenyl]methyl]-N-[2-(oxan-2-yloxy)ethyl]carbamate (18 mg) described in Reference Example 37 was dissolved in 1,4-dioxane (0.4 mL), 4 M hydrochloric acid-dioxane (0.2 mL) was added at room temperature, and the mixture was stirred overnight. After completion of the reaction, the reaction solution was concentrated under reduced pressure, and azeotropically distilled 3 times with toluene. The obtained residue was dissolved in N,N-dimethylformamide (0.5 mL), 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 (16 mg), HATU (24 mg), and diisopropyl ethylamine (30 µL) were added, and the mixture was stirred at room temperature for 1 hr. After completion of the reaction, water and chloroform were added, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (14 mg) as a colorless oil. MS(ESI)m/z:607.4[M-H]-

### Example 61: Synthesis of 1-[(2S,4R)-4-hydroxy-2-[5-(3-methoxyphenyl)-1H-imidazol-2-yl]pyrrolidin-1-yl]-2-(3-methoxy-1,2-oxazol-5-yl)-3-methylbutan-1-one

tert-Butyl (2S,4R)-4-[tert-butyl(dimethyl)silyl]oxy-2-[5-(3-methoxyphenyl)-1H-imidazol-2-yl]pyrrolidine-1-carboxylate (27 mg) described in Reference Example 40 was dissolved in 1,4-dioxane (1 mL), 4 M hydrochloric acid-dioxane (0.5 mL) was added at room temperature and the mixture was stirred for 2 hr. After completion of the reaction, the reaction solution was concentrated under reduced pressure, 1,4-dioxane (10 mL) was added and the mixture was concentrated. The obtained residue was dissolved in N,N-dimethylformamide (1.1 mL), diisopropyl ethylamine (39 µL), 2-(3-methoxy-1,2-oxazol-5-yl)-3-methylbutanoic acid (15 mg), and HATU (28 mg) were added, and the mixture was stirred at room temperature for 1 hr. After completion of the reaction, water and saturated brine were added, and the mixture was extracted with ethyl acetate and hexane and concentrated under reduced pressure. The obtained residue was purified by silica gel column chromatography (ethyl acetate:methanol=95:5 - 70:30), and again purified by preparative HPLC (10 mM ammonium carbonate aqueous solution-acetonitrile) to give the title compound (7 mg) as a white powder. MS(ESI)m/z:441.4[M+H]+

### Example 62: Synthesis of 1-[(2S,4R)-4-hydroxy-2-[5-[(4-methoxyphenyl)methyl]-1H-imidazol-2-yl]pyrrolidin-1-yl]-2-(3-methoxy-1,2-oxazol-5-yl)-3-methylbutan-1-one

The title compound was obtained by the same reaction and treatment as in Example 61 using tert-butyl (2S,4R)-4-hydroxy-2-[5-[(4-methoxyphenyl)methyl]-1H-imidazol-2-yl]pyrrolidine-1-carboxylate described in Reference Example 41 and 2-(3-methoxy-1,2-oxazol-5-yl)-3 methylbutanoic acid. MS(ESI)m/z:455.4[M+H]+

### Example 63: Synthesis of 1-[(2S,4R)-4-hydroxy-2-[5-[(3-methoxyphenyl)methyl]-1H-imidazol-2-yl]pyrrolidin-1-yl]-2-(3-methoxy-1,2-oxazol-5-yl)-3-methylbutan-1-one

The title compound was obtained by the same reaction and treatment as in Example 61 using 2-(3-methoxy-1,2-oxazol-5-yl)-3-methylbutanoic acid and tert-butyl (2S,4R)-4-[tert-butyl(dimethyl)silyl]oxy-2-[5-[(3-methoxyphenyl)methyl]-1H-imidazol-2-yl]pyrrolidine-1-carboxylate described in Reference Example 42. MS(ESI)m/z:455.3[M+H]+

### Example 64: Synthesis of 2-[2-[(2S,4R)-4-hydroxy-1-[2-(3-methoxy-1,2-oxazol-5-yl)-3-methylbutanoyl]pyrrolidin-2-yl]-1H-imidazol-5-yl]-N-methylbenzamide

The title compound was obtained by the same reaction and treatment as in Example 61 using tert-butyl (2S,4R)-4-[tert-butyl(dimethyl)silyl]oxy-2-[5-[2-(methylcarbamoyl)phenyl]-1H-imidazol-2-yl]pyrrolidine-1-carboxylate described in Reference Example 43 and 2-(3-methoxy-1,2-oxazol-5-yl)-3-methylbutanoic acid. MS (ESI)m/z:468.3 [M+H]+

### Example 65: Synthesis of 1-[(2S,4R)-4-hydroxy-2-[1-[(3-phenoxyphenyl)methyl]imidazol-2-yl]pyrrolidin-1-yl]-2-(3-methoxy-1,2-oxazol-5-yl)-3-methylbutan-1-one

The title compound was obtained by the same reaction and treatment as in Example 61 using (3R,5S)-5-[1-[(3-phenoxyphenyl)methyl]imidazol-2-yl]pyrrolidin-3-ol hydrochloride described in Reference Example 45 and 2-(3-methoxy-1,2-oxazol-5-yl)-3-methylbutanoic acid. MS(ESI)m/z:517.3[M+H]+

### Example 66: Synthesis of 1-[(2S,4R)-4-hydroxy-2-[1-[(2-phenylmethoxyphenyl)methyl]imidazol-2-yl]pyrrolidin-1-yl]-2-(3-methoxy-1,2-oxazol-5-yl)-3-methylbutan-1-one

The title compound was obtained by the same reaction and treatment as in Example 61 using tert-butyl (2S,4R)-4-[tert-butyl(dimethyl)silyl]oxy-2-[1-[(2-phenylmethoxyphenyl)methyl]imidazol-2-yl]pyrrolidine-1-carboxylate described in Reference Example 46 and 2-(3-methoxy-1,2-oxazol-5-yl)-3-methylbutanoic acid. MS(ESI)m/z:531.6[M+H]+

### Example 67: Synthesis of either one diastereomer of 1-[(2S,4R)-4-hydroxy-2-[1-[(3-phenylmethoxyphenyl)methyl]imidazol-2-yl]pyrrolidin-1-yl]-2-(3-methoxy-1,2-oxazol-5-yl)-3 methylbutan-1-one

The title compound was obtained by the same reaction and treatment as in Example 61 using tert-butyl (2S,4R)-4-[tert-butyl(dimethyl)silyl]oxy-2-[1-[(3-phenylmethoxyphenyl)methyl]imidazol-2-yl]pyrrolidine-1-carboxylate described in Reference Example 47 and 2-(3-methoxy-1,2-oxazol-5-yl)-3-methylbutanoic acid. MS(ESI)m/z:531.3[M+H]+

### Example 68: Synthesis of 1-[(2S,4R)-4-hydroxy-2-[1-[(3-phenylmethoxyphenyl)methyl]imidazol-2-yl]pyrrolidin-1-yl]-2-(3-methoxy-1,2-oxazol-5-yl)-3-methylbutan-1-one diastereomer mixture

The title compound was obtained by the same reaction and treatment as in Example 61 using tert-butyl (2S,4R)-4-[tert-butyl(dimethyl)silyl]oxy-2-[1-[(3-phenylmethoxyphenyl)methyl]imidazol-2-yl]pyrrolidine-1-carboxylate described in Reference Example 47 and 2-(3-methoxy-1,2-oxazol-5-yl)-3-methylbutanoic acid. MS(ESI)m/z:531.3[M+H]+

### Example 69: Synthesis of 1-[(2S,4R)-4-hydroxy-2-[1-[(2-phenoxyphenyl)methyl]imidazol-2-yl]pyrrolidin-1-yl]-2-(3-methoxy-1,2-oxazol-5-yl)-3-methylbutan-1-one

The title compound was obtained by the same reaction and treatment as in Example 61 using 2-(3-methoxy-1,2-oxazol-5-yl)-3-methylbutanoic acid described in Reference Example 48 and tert-butyl (2S,4R)-4-[tert-butyl(dimethyl)silyl]oxy-2-[1-[(2-phenoxyphenyl)methyl]imidazol-2-yl]pyrrolidine-1-carboxylate. MS (ESI)m/z:517.5[M+H] +

### Example 70: Synthesis of N-ethyl-2-[(2S,4R)-4-hydroxy-1-[2-(3-methoxy-1,2-oxazol-5-yl)-3-methylbutanoyl]pyrrolidin-2-yl]-N-[[4-(4-methyl-1,3-thiazol-5-yl)phenyl]methyl]-1H-imidazole-4-carboxamide

The title compound was obtained by the same reaction and treatment as in Example 61 using tert-butyl (2S,4R)-2-[4-[ethyl-[[4-(4-methyl-1,3-thiazol-5-yl)phenyl]methyl]carbamoyl]-1H-imidazol-2-yl]-4-hydroxypyrrolidine-1-carboxylate described in Reference Example 49 and 2-(3-methoxy-1,2-oxazol-5-yl)-3-methylbutanoic acid. MS(ESI)m/z:593.3[M+H]+

### Example 71: Synthesis of 2-[(2S,4R)-4-hydroxy-1-[2-(3-methoxy-1,2-oxazol-5-yl)-3-methylbutanoyl]pyrrolidin-2-yl]-N-methyl-N-[(1S)-1-[4-(4-methyl-1,3-thiazol-5-yl)phenyl]ethyl]-1H-imidazole-4-carboxamide

The title compound was obtained by the same reaction and treatment as in Example 61 using tert-butyl (2S,4R)-4-hydroxy-2-[4-[methyl-[(1S)-1-[4-(4-methyl-1,3-thiazol-5-yl)phenyl]ethyl]carbamoyl]-1H-imidazol-2-yl]pyrrolidine-1-carboxylate described in Reference Example 52 and 2-(3-methoxy-1,2-oxazol-5-yl)-3-methylbutanoic acid. MS(ESI)m/z:593.3[M+H]+

### Example 72: Synthesis of 1-[(2S,4R)-4-hydroxy-2-[1-[[2-[(4-methyl-1,3-thiazol-5-yl)methoxy]phenyl]methyl]imidazol-2-yl]pyrrolidin-1-yl]-2-(3-methoxy-1,2-oxazol-5-yl)-3 methylbutan-1-one

The title compound was obtained by the same reaction and treatment as in Example 61 using tert-butyl (2S,4R)-4-[tert-butyl(dimethyl)silyl]oxy-2-[1-[[2-[(4-methyl-1,3-thiazol-5-yl)methoxy]phenyl]methyl]imidazol-2-yl]pyrrolidine-1-carboxylate described in Reference Example 54 and 2-(3-methoxy-1,2-oxazol-5-yl)-3 methylbutanoic acid. MS(ESI)m/z:552.4[M+H]+

### Example 73: Synthesis of 1-[(2S,4R)-4-hydroxy-2-[1-[[3-[(4-methyl-1,3-thiazol-5-yl)methoxy]phenyl]methyl]imidazol-2-yl]pyrrolidin-1-yl]-2-(3-methoxy-1,2-oxazol-5-yl)-3 methylbutan-1-one

The title compound was obtained by the same reaction and treatment as in Example 61 using tert-butyl (2S,4R)-4-[tert-butyl(dimethyl)silyl]oxy-2-[1-[[3-[(4-methyl-1,3-thiazol-5-yl)methoxy]phenyl]methyl]imidazol-2-yl]pyrrolidine-1-carboxylate described in Reference Example 56 and 2-(3-methoxy-1,2-oxazol-5-yl)-3 methylbutanoic acid. MS(ESI)m/z:552.4[M+H]+

### Example 74: Synthesis of 1-[(2S,4R)-4-hydroxy-2-[5-(4-methoxyphenyl)-4-(trifluoromethyl)-1H-imidazol-2-yl]pyrrolidin-1-yl]-2-(3-methoxy-1,2-oxazol-5-yl)-3-methylbutan-1-one

The title compound was obtained by the same reaction and treatment as in Example 61 using 2-(3-methoxy-1,2-oxazol-5-yl)-3-methylbutanoic acid and tert-butyl (2S,4R)-4-[tert-butyl(dimethyl)silyl]oxy-2-[5-(4-methoxyphenyl)-4-(trifluoromethyl)-1H-imidazol-2-yl]pyrrolidine-1-carboxylate described in Reference Example 58. MS(ESI)m/z:509.1[M+H]+

### Example 75: Synthesis of 2-[(2S,4R)-4-hydroxy-1-[2-(3-methoxy-1,2-oxazol-5-yl)-3-methylbutanoyl]pyrrolidin-2-yl]-5-(4-methoxyphenyl)-N-methyl-1H-imidazole-4-carboxamide

The title compound was obtained by the same reaction and treatment as in Example 61 using tert-butyl (2S,4R)-4-hydroxy-2-[5-(4-methoxyphenyl)-4-(methylcarbamoyl)-1H-imidazol-2-yl]pyrrolidine-1-carboxylate described in Reference Example 60 and 2-(3-methoxy-1,2-oxazol-5-yl)-3-methylbutanoic acid. MS (ESI) m/z:498.2[M+H] +

### Example 76: Synthesis of 1-[(2S,4R)-2-[4,5-bis(3-methoxyphenyl)-1H-imidazol-2-yl]-4-hydroxypyrrolidin-1-yl]-2-(3-methoxy-1,2-oxazol-5-yl)-3-methylbutan-1-one

The title compound was obtained by the same reaction and treatment as in Example 61 using 2-(3-methoxy-1,2-oxazol-5-yl)-3-methylbutanoic acid and tert-butyl (2S,4R)-2-[4,5-bis(3-methoxyphenyl)-1H-imidazol-2-yl]-4-[tert-butyl(dimethyl)silyl]oxypyrrolidine-1-carboxylate described in Reference Example 61. MS(ESI)m/z:547.2[M+H]+

### Example 77: Synthesis of 1-[(2S,4R)-4-hydroxy-2-[1-(naphthalen-1-ylmethyl)-imidazol-2-yl]pyrrolidin-1-yl]-2-(3-methoxy-1,2-oxazol-5-yl)-3-methylbutan-1-one

The title compound was obtained by the same reaction and treatment as in Example 61 using tert-butyl (2S,4R)-4-[tert-butyl(dimethyl)silyl]oxy-2-[1-(naphthalen-1-ylmethyl)imidazol-2-yl]pyrrolidine-1-carboxylate described in Reference Example 62 and 2-(3-methoxy-1,2-oxazol-5-yl)-3-methylbutanoic acid. MS (ESI) m/z:475.3[M+H] +

### Example 78: Synthesis of 1-[(2S,4R)-4-hydroxy-2-[5-(4-methoxyphenyl)-1H-imidazol-2-yl]pyrrolidin-1-yl]-2-(3-methoxy-1,2-oxazol-5-yl)-3-methylbutan-1-one

The title compound was obtained by the same reaction and treatment as in Example 61 using 2-(3-methoxy-1,2-oxazol-5-yl)-3-methylbutanoic acid and tert-butyl-[(3R,5S)-5-[5-(4-methoxyphenyl)-1H-imidazol-2-yl]pyrrolidin-3-yl]oxy-dimethylsilane described in Reference Example 64. MS(ESI)m/z:441.1[M+H]+

### Example 79: Synthesis of 1-[(2S,4R)-4-hydroxy-2-[5-[[4-(trifluoromethyl)phenyl]methyl]-1H-imidazol-2-yl]pyrrolidin-1-yl]-2-(3-methoxy-1,2-oxazol-5-yl)-3-methylbutan-1-one

The title compound was obtained by the same reaction and treatment as in Example 61 using 2-(3-methoxy-1,2-oxazol-5-yl)-3-methylbutanoic acid and tert-butyl (2S,4R)-4-hydroxy-2-[5-[[4-(trifluoromethyl)phenyl]methyl]-1H-imidazol-2-yl]pyrrolidine-1-carboxylate described in Reference Example 67. MS(ESI)m/z:493.2[M+H]+

### Example 80: Synthesis of 3-[2-[(2S,4R)-4-hydroxy-1-[2-(3-methoxy-1,2-oxazol-5-yl)-3-methylbutanoyl]pyrrolidin-2-yl]-1H-imidazol-5-yl]-N-methylbenzamide

The title compound was obtained by the same reaction and treatment as in Example 61 using tert-butyl (2S,4R)-4-[tert-butyl(dimethyl)silyl]oxy-2-[5-[3-(methylcarbamoyl)phenyl]-1H-imidazol-2-yl]pyrrolidine-1-carboxylate described in Reference Example 68 and 2-(3-methoxy-1,2-oxazol-5-yl)-3-methylbutanoic acid. MS(ESI)m/z:468.2[M+H]+

### Example 81: Synthesis of N-[(2S)-1-[(2S,4R)-2-[5-[(4-bromophenyl)methyl]-1H-imidazol-2-yl]-4-hydroxypyrrolidin-1-yl]-3,3-dimethyl-1-oxobutan-2-yl]acetamide

tert-Butyl N-[(2S)-1-[(2S,4R)-2-[5-[(4-bromophenyl)methyl]-1H-imidazol-2-yl]-4-hydroxypyrrolidin-1-yl]-3,3-dimethyl-1-oxobutan-2-yl]carbamate (34 mg) described in Reference Example 70 was dissolved in 1,4-dioxane (3 mL), 4 M hydrochloric acid-dioxane (0.5 mL) was added at room temperature, and the mixture was stirred for 6 hr. After completion of the reaction, the reaction solution was concentrated under reduced pressure, and then azeotropically distilled with toluene. The obtained residue was dissolved in dichloromethane (5 mL), triethylamine (53 µL) and acetic anhydride (12 µL) were added, and the mixture was stirred at room temperature for 30 min. After completion of the reaction, 1 M sodium hydroxide aqueous solution was added, and the mixture was extracted with ethyl acetate, washed with saturated brine, and dried over anhydrous sodium sulfate. The organic layer was filtered, and the filtrate was concentrated under reduced pressure. The obtained residue was purified by silica gel column chromatography (ethyl acetate:methanol=90:10) to give the title compound (16 mg) as a colorless powder. MS(ESI)m/z:477.2/479.2[M+H]+

### Example 82: Synthesis of N-[(2S)-1-[(2S,4R)-2-[1-[2-(4-chlorophenyl)ethyl]imidazol-2-yl]-4-hydroxypyrrolidin-1-yl]-3,3-dimethyl-1-oxobutan-2-yl]acetamide

tert-Butyl (2S,4R)-4-[tert-butyl(dimethyl)silyl]oxy-2-[1-[2-(4-chlorophenyl)ethyl]imidazol-2-yl]pyrrolidine-1-carboxylate (52 mg) described in Reference Example 73 was dissolved in dichloromethane (2 mL), TFA (2 mL) was added, and the mixture was stirred at room temperature for 30 min. After completion of the reaction, the reaction solution was concentrated under reduced pressure, and then azeotropically distilled twice with toluene. The obtained residue was dissolved in DMF (3 mL), DIPEA (89 µL), (2S)-2-acetamido-3,3-dimethylbutanoic acid (29 mg), and HATU (51 mg) were added, and the mixture was stirred at room temperature for 30 min. The reaction mixture was diluted with ethyl acetate, and the organic layer was washed with water and saturated brine and dried over anhydrous sodium sulfate. The organic layer was filtered, and the filtrate was concentrated under reduced pressure. The obtained residue was dissolved in 1,4-dioxane (2 mL), 4 M hydrochloric acid-dioxane (2 mL) was added, and the mixture was stirred at room temperature for 2 hr. The reaction mixture was concentrated under reduced pressure, and the obtained residue was suspended in THF (3 mL). Triethylamine (71 µL) and acetic anhydride (29 µL) were added, and the mixture was stirred at room temperature for 30 min. After completion of the reaction, water and chloroform were added, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (26 mg) as a pale-yellow oil. MS(ESI)m/z:447.4/449.4[M+H]+

### Example 83: Synthesis of N-[(2S)-1-[(2S,4R)-2-[5-[(2-chlorophenyl)methyl]-1H-imidazol-2-yl]-4-hydroxypyrrolidin-1-yl]-3,3-dimethyl-1-oxobutan-2-yl]acetamide

The title compound was obtained by the same reaction and treatment as in Example 81 using tert-butyl N-[(2S)-1-[(2S,4R)-2-[5-[(2-chlorophenyl)methyl]-1H-imidazol-2-yl]-4-hydroxypyrrolidin-1-yl]-3,3-dimethyl-1-oxobutan-2-yl]carbamate described in Reference Example 76.
MS(ESI)m/z:433.4/435.4[M+H]+

### Example 84: Synthesis of N-[(2S)-1-[(2S,4R)-4-hydroxy-2-[5-[2-(trifluoromethyl)phenyl]-1H-imidazol-2-yl]pyrrolidin-1-yl]-3,3-dimethyl-1-oxobutan-2-yl]acetamide

The title compound was obtained by the same reaction and treatment as in Example 81 using tert-butyl N-[(2S)-1-[(2S,4R)-4-hydroxy-2-[5-[2-(trifluoromethyl)phenyl]-1H-imidazol-2-yl]pyrrolidin-1-yl]-3,3-dimethyl-1-oxobutan-2-yl]carbamate described in Reference Example 78. MS(ESI)m/z:453.2[M+H]+

### Example 85: Synthesis of N-[(2S)-1-[(2S,4R)-4-hydroxy-2-[5-[[2-(trifluoromethyl)phenyl]methyl]-1H-imidazol-2-yl]pyrrolidin-1-yl]-3,3-dimethyl-1-oxobutan-2-yl]acetamide

The title compound was obtained by the same reaction and treatment as in Example 81 using tert-butyl N-[(2S)-1-[(2S,4R)-4-hydroxy-2-[5-[[2-(trifluoromethyl)phenyl]methyl]-1H-imidazol-2-yl]pyrrolidin-1-yl]-3,3-dimethyl-1-oxobutan-2-yl]carbamate described in Reference Example 83. MS(ESI)m/z:465.2[M-H]-

### Example 86: Synthesis of N-[(2S)-1-[(2S,4R)-4-hydroxy-2-[5-[[3-(trifluoromethyl)phenyl]methyl]-1H-imidazol-2-yl]pyrrolidin-1-yl]-3,3-dimethyl-1-oxobutan-2-yl]acetamide

The title compound was obtained by the same reaction and treatment as in Example 81 using tert-butyl N-[(2S)-1-[(2S,4R)-4-hydroxy-2-[5-[[3-(trifluoromethyl)phenyl]methyl]-1H-imidazol-2-yl]pyrrolidin-1-yl]-3,3-dimethyl-1-oxobutan-2-yl]carbamate described in Reference Example 84. MS(ESI)m/z:467.3[M+H]+

### Example 87: Synthesis of N-[(2S)-1-[(2S,4R)-4-hydroxy-2-[1-[[4-(4-methyl-1,3-thiazol-5-yl)phenyl]methyl]imidazol-2-yl]pyrrolidin-1-yl]-3,3-dimethyl-1-oxobutan-2-yl]acetamide

The title compound was obtained by the same reaction and treatment as in Example 81 using tert-butyl N-[(2S)-1-[(2S,4R)-4-hydroxy-2-[1-[[4-(4-methyl-1,3-thiazol-5-yl)phenyl]methyl]imidazol-2-yl]pyrrolidin-1-yl]-3,3-dimethyl-1-oxobutan-2-yl]carbamate described in Reference Example 87. MS(ESI)m/z:496.5[M+H]+

### Example 88: Synthesis of N-[(2S)-1-[(2S,4R)-2-[5-(4-bromophenyl)-1H-imidazol-2-yl]-4-hydroxypyrrolidin-1-yl]-3,3-dimethyl-1-oxobutan-2-yl]acetamide

The title compound was obtained by the same reaction and treatment as in Example 81 using tert-butyl N-[(2S)-1-[(2S,4R)-2-[5-(4-bromophenyl)-1H-imidazol-2-yl]-4-hydroxypyrrolidin-1-yl]-3,3-dimethyl-1-oxobutan-2-yl]carbamate described in Reference Example 88. MS(ESI)m/z:463.3/465.4[M+H]+

### Example 89: Synthesis of N-[(2S)-1-[(2S,4R)-4-hydroxy-2-[5-[4-(4-methyl-1,3-thiazol-5-yl)phenyl]-1H-imidazol-2-yl]pyrrolidin-1-yl]-3,3-dimethyl-1-oxobutan-2-yl]acetamide

The title compound was obtained by the same reaction and treatment as in Example 81 using tert-butyl N-[(2S)-1-[(2S,4R)-4-hydroxy-2-[5-[4-(4-methyl-1,3-thiazol-5-yl)phenyl]-1H-imidazol-2-yl]pyrrolidin-1-yl]-3,3-dimethyl-1-oxobutan-2-yl]carbamate described in Reference Example 89. MS(ESI)m/z:482.4[M+H]+

### Example 90: Synthesis of N-[(2S)-1-[(2S,4R)-2-[5-[(3-bromophenyl)methyl]-1H-imidazol-2-yl]-4-hydroxypyrrolidin-1-yl]-3,3-dimethyl-1-oxobutan-2-yl]acetamide

The title compound was obtained by the same reaction and treatment as in Example 81 using tert-butyl N-[(2S)-1-[(2S,4R)-2-[5-[(3-bromophenyl)methyl]-1H-imidazol-2-yl]-4-hydroxypyrrolidin-1-yl]-3,3-dimethyl-1-oxobutan-2-yl]carbamate described in Reference Example 92.
MS(ESI)m/z:477.3/479.3[M+H]+

### Example 91: Synthesis of N-[(2S)-1-[(2S,4R)-4-hydroxy-2-[5-[[3-(4-methyl-1,3-thiazol-5-yl)phenyl]methyl]-1H-imidazol-2-yl]pyrrolidin-1-yl]-3,3-dimethyl-1-oxobutan-2-yl]acetamide

The title compound was obtained by the same reaction and treatment as in Example 81 using tert-butyl N-[(1S)-1-[(2S,4R)-4-hydroxy-2-[5-[[3-(4-methylthiazol-5-yl)phenyl]methyl]-1H-imidazol-2-yl]pyrrolidine-1-carbonyl]-2,2-dimethylpropyl]carbamate described in Reference Example 94. MS(ESI)m/z:496.4[M+H]+

### Example 92: Synthesis of N-[(2S)-1-[(2S,4R)-4-hydroxy-2-[5-[[4-(trifluoromethyl)phenyl]methyl]-1H-imidazol-2-yl]pyrrolidin-1-yl]-3,3-dimethyl-1-oxobutan-2-yl]acetamide

The title compound was obtained by the same reaction and treatment as in Example 81 using tert-butyl N-[(2S)-1-[(2S,4R)-4-hydroxy-2-[5-[[4-(trifluoromethyl)phenyl]methyl]-1H-imidazol-2-yl]pyrrolidin-1-yl]-3,3-dimethyl-1-oxobutan-2-yl]carbamate described in Reference Example 95. MS(ESI)m/z:467.3[M+H]+

### Example 93: Synthesis of N-[(1S)-1-[(2S,4R)-4-hydroxy-2-[5-[[4-(4-methyl-1,3-thiazol-5-yl)phenyl]methyl]-1H-imidazol-2-yl]pyrrolidin-1-yl]-3,3-dimethyl-1-oxobutan-2-yl]acetamide

N-[(2S)-1-[(2S,4R)-2-[5-[(4-bromophenyl)methyl]-1H-imidazol-2-yl]-4-hydroxypyrrolidin-1-yl]-3,3-dimethyl-1-oxobutan-2-yl]acetamide (13 mg) described in Reference Example 96 was dissolved in 1,4-dioxane (2 mL) and water (0.5 mL), and 4-methyl-5-(4,4,5,5-tetramethyl-1,3,2-dioxaborolan-2-yl)thiazole (9.2 mg), and sodium carbonate (8 mg) were added at room temperature. After deaeration, a 1,1'-(diphenylphosphino)ferrocene-palladium(II) dichloromethane complex (2 mg) was added, and the mixture was stirred under nitrogen atmosphere at 80°C for 1 hr. After completion of the reaction, anhydrous sodium sulfate was added. The mixture was filtered, and the filtrate was concentrated under reduced pressure. The obtained residue was purified by preparative HPLC (10 mM ammonium carbonate aqueous solution-acetonitrile) to give the title compound (5 mg) as a pale-brown powder. MS(ESI)m/z:496.4[M+H]+

### Example 94: Synthesis of N-[(2S)-1-[(2S,4R)-4-hydroxy-2-[1-[2-[4-(4-methyl-1,3-thiazol-5-yl)phenyl]ethyl]imidazol-2-yl]pyrrolidin-1-yl]-3,3-dimethyl-1-oxobutan-2-yl]acetamide

The title compound was obtained by the same reaction and treatment as in Example 94 using N-[(2S)-1-[(2S,4R)-2-[1-[2-(4-chlorophenyl)ethyl]imidazol-2-yl]-4-hydroxypyrrolidin-1-yl]-3,3-dimethyl-1-oxobutan-2-yl]acetamide described in Reference Example 97 and 4-methyl-5-(4,4,5,5-tetramethyl-1,3,2-dioxaborolan-2-yl)thiazole. MS(ESI)m/z:510.3[M+H]+

### Example 95: Synthesis of 2-[(2S,4R)-4-hydroxy-1-[2-(3-methoxy-1,2-oxazol-5-yl)-3-methylbutanoyl]pyrrolidin-2-yl]-N-methyl-1-[[3-(4-methyl-1,3-thiazol-5-yl)phenyl]methyl]-N-(pyridin-4-ylmethyl)imidazole-4-carboxamide

Methyl 2-[(2S,4R)-4-[tert-butyl(dimethyl)silyl]oxy-1-[(2-methylpropan-2-yl)oxycarbonyl]pyrrolidin-2-yl]-1-[[3-(4-methyl-1,3-thiazol-5-yl)phenyl]methyl]imidazole-4-carboxylate (83 mg) described in Reference Example 99 was dissolved in methanol (1 mL), THF (1 mL) and 1 M sodium hydroxide aqueous solution (1 mL) were added and the mixture was stirred at 50°C for 17 hr. After completion of the reaction, the reaction solution was concentrated under reduced pressure. THF and 1 M hydrochloric acid (1 mL) were added, and the mixture was concentrated again under reduced pressure. The obtained residue was dissolved in dichloromethane (1 mL), diisopropyl ethylamine (70 µL), and HATU (67 mg), N-methyl-N-(4-pyridylmethyl)amine (20 µL) were added, and the mixture was stirred at room temperature for 1.5 hr. After completion of the reaction, water and chloroform were added, and the organic layer was separated by a Phase-separator (registered trade mark), and concentrated under reduced pressure. The obtained residue was dissolved in 1,4-dioxane (0.7 mL), 4 M hydrochloric acid-dioxane (0.3 mL) was added at room temperature, and the mixture was stirred for 2 hr. After completion of the reaction, the reaction solution was concentrated under reduced pressure. The obtained residue was dissolved in dichloromethane (1 mL), 2-(3-methoxy-1,2-oxazol-5-yl)-3-methylbutanoic acid (33 mg), HATU (67 mg), and DIPEA (70 µL) were added, and the mixture was stirred at room temperature for 17 hr. After completion of the reaction, water and chloroform were added, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (20 mg) as a pale-yellow powder. MS(ESI)m/z:670.5[M+H]+

### Example 96: Synthesis of 2-[2-[(2S,4R)-4-hydroxy-1-[2-(3-methoxy-1,2-oxazol-5-yl)-3-methylbutanoyl]pyrrolidin-2-yl]imidazol-1-yl]-N-phenylacetamide

The title compound was obtained by the same reaction and treatment as in Example 95 using tert-butyl (2S,4R)-4-[tert-butyl(dimethyl)silyl]oxy-2-[1-(2-methoxy-2-oxoethyl)imidazol-2-yl]pyrrolidine-1-carboxylate described in Reference Example 100, 2-(3-methoxy-1,2-oxazol-5-yl)-3-methylbutanoic acid, and aniline instead of N-methyl-N-(4-pyridylmethyl)amine. MS (ESI)m/z:468.3[M+H] +

### Example 97: Synthesis of 4-[2-[(2S,4R)-4-hydroxy-1-[2-(3-methoxy-1,2-oxazol-5-yl)-3-methylbutanoyl]pyrrolidin-2-yl]-1H-imidazol-5-yl]-N-methylbenzamide

4-[2-[(2S,4R)-4-[tert-butyl(dimethyl)silyl]oxy-1-[(2-methylpropan-2-yl)oxycarbonyl]pyrrolidin-2-yl]-1H-imidazol-5-yl]benzoic acid (25 mg) described in Reference Example 101 was dissolved in N,N-dimethylformamide (2 mL), triethylamine (21 µL), methylamine hydrochloride (4 mg), and HATU (23 mg) were added and the mixture was stirred at room temperature for 1 hr. After completion of the reaction, water and chloroform were added, and the organic layer was separated by a Phase-separator (registered trade mark), and concentrated under reduced pressure. The obtained residue was dissolved in 1,4-dioxane (1 mL), 4 M hydrochloric acid-dioxane (1 mL) was added at room temperature, and the mixture was stirred for 1 hr. After completion of the reaction, the reaction solution was concentrated under reduced pressure. The obtained residue was dissolved in N,N-dimethylformamide (2 mL), triethylamine (0.2 mL), 2-(3-methoxy-1,2-oxazol-5-yl)-3-methylbutanoic acid (11 mg), and HATU (23 mg) were added and the mixture was stirred at room temperature for 18 hr. After completion of the reaction, water was added, and the mixture was extracted with ethyl acetate and concentrated under reduced pressure. The obtained residue was purified by preparative HPLC (10 mM ammonium carbonate aqueous solution-acetonitrile) to give the title compound (0.40 mg) as a pale-yellow oil.
MS (ESI) m/z:468.2[M+H] +

### Example 98: Synthesis of N-[(2S)-1-[(2S,4R)-4-hydroxy-2-[4-(phenylmethoxymethyl)-1H-imidazol-2-yl]pyrrolidin-1-yl]-3,3-dimethyl-1-oxobutan-2-yl]acetamide

4 M Hydrochloric acid-dioxane (1 mL) was added to tert-butyl N-[(2S)-1-[(2S,4R)-4-hydroxy-2-[1-[(4-methoxyphenyl)methyl]-4-(phenylmethoxymethyl)imidazol-2-yl]pyrrolidin-1-yl]-3,3-dimethyl-1-oxobutan-2-yl]carbamate (14 mg) described in Reference Example 105 at room temperature, and the mixture was stirred for 1 hr. After completion of the reaction, the reaction solution was concentrated under reduced pressure. The obtained residue was dissolved in dichloromethane (0.5 mL), acetic anhydride (3 µL) and triethylamine (15 µL) were added and the mixture was stirred at room temperature for 2 hr. After completion of the reaction, potassium carbonate (10 mg) and methanol (200 µL) were added, and the mixture was stirred at room temperature for 30 min. Water and chloroform were added, and the organic layer was separated by a Phase-separator (registered trade mark), and concentrated under reduced pressure. The obtained residue was dissolved in acetonitrile (1 mL), water (0.3 mL) and ammonium hexanitratocerate(IV) (92 mg) were added, and the mixture was stirred at room temperature for 3.5 hr. After completion of the reaction, water and chloroform were added, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (1.1 mg) as a colorless oil. MS(ESI)m/z:429.3[M+H]+

### Example 99: Synthesis of 2-[(2S,4R)-4-hydroxy-1-[2-(3-methoxyisoxazol-5-yl)-3-methylbutanoyl]pyrrolidin-2-yl]-N-isopropyl-N-(3-phenoxypropyl)-1H-imidazole-4-carboxamide

3-Phenoxypropan-1-amine (2 g), acetone (20 mL), and molecular sieve (5 g) were combined and the mixture was stirred at room temperature for 2 days. The reaction solution was filtered, washed with methanol, and the filtrate was concentrated under reduced pressure. The concentrated residue was dissolved in methanol (30 mL), sodium borohydride (250 mg) was added at room temperature, and the mixture was stirred as it was. The reaction solution was concentrated under reduced pressure, chloroform and water were added, and the mixture was extracted with chloroform. The organic layer was washed with water and saturated brine, dried over sodium sulfate and filtered. The filtrate was concentrated under reduced pressure. To the concentrated residue was added 30%hydrochloric acid-ethanol (1 mL), and the mixture was filtered and recrystallized from ethanol. 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (25 mg) described in Reference Example 8 was dissolved in DMF (1 mL), HATU (38 mg), the earlier precipitate (29 mg), and DIPEA (40 µL) were added at room temperature, and the mixture was stirred at room temperature for 16 hr. To the reaction mixture were added chloroform and water, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (17 mg) as a white powder.
MS(ESI)m/z:554.3[M+H]+

### Example 100: Synthesis of 1-[(2S,4R)-4-hydroxy-2-[1-[(1R)-1-(1-naphthyl)ethyl]imidazol-2-yl]pyrrolidin-1-yl]-2-(3-methoxyisoxazol-5-yl)-3-methylbutan-1-one

tert-Butyl (2S,4R)-4-[tert-butyl(dimethyl)silyl]oxy-2-[1-[(1R)-1-(1-naphthyl)ethyl]imidazol-2-yl]pyrrolidine-1-carboxylate (152 mg) described in Reference Example 118 was dissolved in methanol (1460 µL), 4 M hydrochloric acid-dioxane (730 µL) was added at room temperature and the mixture was stirred overnight. After completion of the reaction, the reaction solution was concentrated under reduced pressure, purified by preparative HPLC (0.05% trifluoroacetic acid aqueous solution-0.05% trifluoroacetic acid acetonitrile solution), and the solvent was evaporated. To the obtained residue were added, at room temperature, Et3N (74 µL), and a solution of 2-(3-methoxy-1,2-oxazol-5-yl)-3-methylbutanoic acid (46 mg), HOBt (36 mg), and WSC·HCl (51 mg) in DMF (1.8 mL), and the mixture was stirred for 6 hr. After completion of the reaction, water (100 µL) was added and the mixture was diluted with DMSO, and purified by preparative HPLC (0.05% trifluoroacetic acid aqueous solution-0.05% trifluoroacetic acid acetonitrile solution) to give the title compound (69 mg) as a white powder. MS(ESI)m/z:489.5[M+H]+

### Example 101: Synthesis of 2-[(2S,4R)-4-hydroxy-1-[2-(3-methoxyisoxazol-5-yl)-3-methylbutanoyl]pyrrolidin-2-yl]-N,5-dimethyl-N-[[4-(4-methylthiazol-5-yl)phenyl]methyl]-1H-imidazole-4-carboxamide

tert-Butyl (2S,4R)-4-hydroxy-2-[4-methyl-5-[methyl-[[4-(4-methylthiazol-5-yl)phenyl]methyl]carbamoyl]-1H-imidazol-2-yl]pyrrolidine-1-carboxylate (14 mg) described in Reference Example 117 was dissolved in methanol (547 µL), 4 M hydrochloric acid-dioxane (68 µL) was added at room temperature and the mixture was stirred for 1 day. Furthermore, 4 M hydrochloric acid-dioxane (68 µL) was added and the mixture was stirred for 1 day. After completion of the reaction, the reaction solution was concentrated under reduced pressure, and azeotropically distilled with toluene. Separately, to a solution of 2-(3-methoxy-1,2-oxazol-5-yl)-3-methylbutanoic acid (7.0 mg) and HOBt (5.5 mg) in DMF (0.27 mL) was added WSC·HCl (7.8 mg) at room temperature, and the mixture was stirred for 20 min. This solution was added to a mixture of the earlier concentrated residue and triethylamine (11 µL) at room temperature, and the mixture was stirred for 6 hr. After completion of the reaction, water (100 µL) was added and the mixture was diluted with DMSO, and purified by preparative HPLC (10 mM ammonium carbonate aqueous solution-acetonitrile) to give the title compound (13 mg) as a white powder. MS(ESI)m/z:593.5[M+H]+

### Example 102: Synthesis of 1-[(2S,4R)-4-hydroxy-2-[1-[[2-[4-(trifluoromethyl)phenyl]-4-pyridyl]methyl]imidazol-2-yl]pyrrolidin-1-yl]-2-(3-methoxyisoxazol-5-yl)-3-methylbutan-1-one

tert-Butyl (2S,4R)-4-[tert-butyl(dimethyl)silyl]oxy-2-[1-[[2-[4-(trifluoromethyl)phenyl]-4-pyridyl]methyl]imidazol-2-yl]pyrrolidine-1-carboxylate (80 mg) described in Reference Example 119 was dissolved in methanol (660 µL), 4 M hydrochloric acid-dioxane (165 µL) was added at room temperature and the mixture was stirred for 2 days. After completion of the reaction, the reaction solution was concentrated under reduced pressure and azeotropically distilled with toluene. Separately, to a solution of 2-(3-methoxy-1,2-oxazol-5-yl)-3-methylbutanoic acid (34 mg) and HOBt (27 mg) in DMF (1.3 mL) was added WSC·HCl (38 mg) at room temperature, and the mixture was stirred for 20 min. This solution was added to a mixture of the earlier concentrated residue and triethylamine (55 µL) at room temperature, and the mixture was stirred for 6 hr. After completion of the reaction water (100 µL) was added and the mixture was diluted with DMSO, and purified by preparative HPLC (10 mM ammonium carbonate aqueous solution-acetonitrile) to give the title compound (53 mg) as a white powder. MS(ESI)m/z:570.5[M+H]+

### Example 103: Synthesis of N-cyclopropyl-2-[(2S,4R)-4-hydroxy-1-[2-(3-methoxyisoxazol-5-yl)-3-methylbutanoyl]pyrrolidin-2-yl]-N-[[4-(4-methylthiazol-5-yl)phenyl]methyl]-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (25 mg) described in Reference Example 8 was dissolved in DMF (0.66 mL), HATU (38 mg) was added at room temperature and the mixture was stirred for 30 min. Then, N-[[4-(4-methylthiazol-5-yl)phenyl]methyl]cyclopropaneamine (20 mg) described in Reference Example 134 and DIPEA (0.045 mL) were added, and the mixture was stirred at room temperature for 2 hr. To the reaction mixture were added chloroform and water, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (30 mg) as a white powder.
MS(ESI)m/z:605.4[M+H]+

### Example 104: Synthesis of 2-[(2S,4R)-4-hydroxy-1-[2-(3-methoxyisoxazol-5-yl)-3-methylbutanoyl]pyrrolidin-2-yl]-N-[[4-(4-methylthiazol-5-yl)phenyl]methyl]-N-tetrahydrofuran-3-yl-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (25 mg) described in Reference Example 8 was dissolved in DMF (0.66 mL), HATU (38 mg) was added at room temperature and the mixture was stirred for 30 min. Then, N-[[4-(4-methylthiazol-5-yl)phenyl]methyl]tetrahydrofuran-3-amine (22 mg) described in Reference Example 135 and DIPEA (46 µL) were added, and the mixture was stirred at room temperature for 3 hr. To the reaction mixture were added chloroform and water, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (24 mg) as a white powder.
MS (ESI)m/z:635.4[M+H] +

### Example 105: Synthesis of 2-[(2S,4R)-4-hydroxy-1-[2-(3-methoxyisoxazol-5-yl)-3-methylbutanoyl]pyrrolidin-2-yl]-N-[[4-(4-methylthiazol-5-yl)phenyl]methyl]-N-tetrahydropyran-4-yl-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (13 mg) described in Reference Example 8 was dissolved in DMF (0.34 mL), HATU (20 mg) was added at room temperature and the mixture was stirred for 30 min. Then, N-[[4-(4-methylthiazol-5-yl)phenyl]methyl]tetrahydropyran-4-amine (10 mg) described in Reference Example 136 and DIPEA (24 µL) were added, and the mixture was stirred at room temperature for about 5 hr. To the reaction mixture were added chloroform and water, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (15 mg) as a white powder.
MS (ESI) m/z:649.4[M+H] +

### Example 106: Synthesis of N-cyclobutyl-2-[(2S,4R)-4-hydroxy-1-[2-(3-methoxyisoxazol-5-yl)-3-methylbutanoyl]pyrrolidin-2-yl]-N-[[4-(4-methylthiazol-5-yl)phenyl]methyl]-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (25 mg) described in Reference Example 8 was dissolved in DMF (0.66 mL), HATU (38 mg) was added at room temperature and the mixture was stirred for 30 min. Then, N-[[4-(4-methylthiazol-5-yl)phenyl]methyl]cyclobutanamine (18 mg) described in Reference Example 137 and DIPEA (46 µL) were added, and the mixture was stirred at room temperature for 2 hr. To the reaction mixture were added chloroform and water, and the organic layer was separated by a Phase-separator (registered trade mark), and concentrated under reduced pressure. The obtained residue was purified by preparative HPLC (10 mM ammonium carbonate aqueous solution-acetonitrile) and then the obtained residue was purified by silica gel column chromatography (ethyl acetate:methanol=100:0 - 85:15) to give the title compound (24 mg) as a white powder.
MS(ESI)m/z:619.4[M+H]+

### Example 107: Synthesis of 1-[(2S,4R)-4-hydroxy-2-[4-(3-phenoxypyrrolidine-1-carbonyl)-1H-imidazol-2-yl]pyrrolidin-1-yl]-2-(3-methoxyisoxazol-5-yl)-3-methylbutan-1-one

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 (20 mg), 3-phenoxypyrrolidine hydrochloride (11 mg), HATU (30 mg), DIPEA (36 µL), and DMF (0.60 mL) were mixed at room temperature and stirred for 4 hr. To the reaction mixture were added chloroform and water, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (17 mg) as a white powder. MS(ESI)m/z:524.3[M+H]+

### Example 108: Synthesis of 1-[(2S,4R)-4-hydroxy-2-[4-(3-phenylazetidine -1-carbonyl)-1H-imidazol-2-yl]pyrrolidin-1-yl]-2-(3-methoxyisoxazol-5-yl)-3-methylbutan-1-one

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (20 mg) described in Reference Example 8 was dissolved in DMF (0.6 mL), HATU (30 mg), 3-phenylazetidine (7.7 mg), and DIPEA (36 µL) were added at room temperature, and the mixture was stirred for about 4 hr. After completion of the reaction, chloroform and water were added, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (3.8 mg) as a white powder. MS(ESI)m/z:494.3[M+H]+

### Example 109: Synthesis of N-cyclohexyl-2-[(2S,4R)-4-hydroxy-1-[2-(3-methoxyisoxazol-5-yl)-3-methylbutanoyl]pyrrolidin-2-yl]-N-[[4-(4-methylthiazol-5-yl)phenyl]methyl]-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (25 mg) described in Reference Example 8 was dissolved in DMF (0.70 mL), HATU (38 mg) was added at room temperature and the mixture was stirred for 30 min. Then, N-[[4-(4-methylthiazol-5-yl)phenyl]methyl]cyclohexanamine (19 mg) described in Reference Example 139 and DIPEA (0.045 mL) were added, and the mixture was stirred at room temperature for 2 hr. To the reaction mixture were added chloroform and water, and the organic layer was separated by a Phase-separator (registered trade mark), and concentrated under reduced pressure. The obtained residue was purified by preparative HPLC (10 mM ammonium carbonate aqueous solution-acetonitrile) and the obtained residue was purified by silica gel column chromatography (ethyl acetate:methanol=100:0 - 85:15) to give the title compound (12 mg) as a white powder.
MS(ESI)m/z:647.4[M+H]+

### Example 110: Synthesis of N-cyclopentyl-2-[(2S,4R)-4-hydroxy-1-[2-(3-methoxyisoxazol-5-yl)-3-methylbutanoyl]pyrrolidin-2-yl]-N-[[4-(4-methylthiazol-5-yl)phenyl]methyl]-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (25 mg) described in Reference Example 8 was dissolved in DMF (0.70 mL), HATU (38 mg) was added at room temperature and the mixture was stirred for 1 hr. Then, N-[[4-(4-methylthiazol-5-yl)phenyl]methyl]cyclopentanamine (19 mg) described in Reference Example 138 and DIPEA (46 µL) were added, and the mixture was stirred at room temperature for 2 hr. To the reaction mixture were added chloroform and water, and the organic layer was separated by a Phase-separator (registered trade mark), and concentrated under reduced pressure. The obtained residue was purified by preparative HPLC (10 mM ammonium carbonate aqueous solution-acetonitrile), and the crude product was purified by preparative HPLC (0.05% trifluoroacetic acid aqueous solution-0.05% trifluoroacetic acid acetonitrile solution) to give the title compound (26 mg) as a white powder. MS(ESI)m/z:633.5[M+H]+

### Example 111: Synthesis of 1-[(2S,4R)-4-hydroxy-2-[4-(3-phenylpyrrolidine-1-carbonyl)-1H-imidazol-2-yl]pyrrolidin-1-yl]-2-(3-methoxyisoxazol-5-yl)-3-methylbutan-1-one

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (20 mg) described in Reference Example 8 was dissolved in DMF (0.60 mL), HATU (30 mg), 3-phenylpyrrolidine (8.6 mg), and DIPEA (36 µL) were added at room temperature, and the mixture was stirred at room temperature for about 4 hr. To the reaction mixture were added chloroform and water, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (11 mg) as a white powder.
MS(ESI)m/z:508.2[M+H]+

### Example 112: Synthesis of 1-[(2S,4R)-4-hydroxy-2-(1H-imidazol-2-yl)pyrrolidin-1-yl]-2-(3-methoxyisoxazol-5-yl)-3-methylbutan-1-one

tert-Butyl (2S,4R)-4-[tert-butyl(dimethyl)silyl]oxy-2-(1H-imidazol-2-yl)pyrrolidine-1-carboxylate (51 mg) described in Reference Example 21 was dissolved in 1,4-dioxane (800 µL), 4 M hydrochloric acid-dioxane (200 µL) was added at room temperature and the mixture was stirred for 20 hr. After completion of the reaction, the reaction solution was concentrated under reduced pressure. To the obtained residue were added 2-(3-methoxy-1,2-oxazol-5-yl)-3-methylbutanoic acid (43 mg), DIPEA (100 µL), HATU (68 mg), and DCM (1 mL) at room temperature and the mixture was stirred for 2.5 hr. After completion of the reaction, the reaction mixture was diluted with DMSO, and purified by preparative HPLC (10 mM ammonium carbonate aqueous solution-acetonitrile) to give the title compound (23 mg) as a white powder. MS(ESI)m/z:335.4[M+H]+

### Example 113: Synthesis of 1-[(2S,4R)-2-[1-[[5-(4-fluorophenyl)-3-pyridyl]methyl]imidazol-2-yl]-4-hydroxypyrrolidin-1-yl]-2-(3-methoxyisoxazol-5-yl)-3-methylbutan-1-one

The title compound was obtained by the same reaction and treatment as in Example 33 using tert-butyl (2S,4R)-4-[tert-butyl(dimethyl)silyl]oxy-2-[1-[[5-(4-fluorophenyl)-3-pyridyl]methyl]imidazol-2-yl]pyrrolidine-1-carboxylate described in Reference Example 121. MS(ESI)m/z:520.2[M+H]+

### Example 114: Synthesis of 2-[(2S,4R)-1-[(2S)-2-acetamido-3,3-dimethylbutanoyl]-4-hydroxypyrrolidin-2-yl]-N-(cyclopropylmethyl)-N-(4-phenylbutyl)-1H-imidazole-4-carboxamide

The title compound was obtained by the same reaction and treatment as in Example 81 using tert-butyl N-[(1S)-1-[(2S,4R)-2-[4-[cyclopropylmethyl(4-phenylbutyl)carbamoyl]-1H-imidazol-2-yl]-4-hydroxypyrrolidine-1-carbonyl]-2,2-dimethylpropyl]carbamate described in Reference Example 146. MS(ESI)m/z:538.6[M+H]+

### Example 115: Synthesis of 1-[(2S,4R)-4-hydroxy-2-[1-[2-[4-(4-methylthiazol-5-yl)phenyl]ethyl]imidazol-2-yl]pyrrolidin-1-yl]-2-(3-methoxyisoxazol-5-yl)-3-methylbutan-1-one

tert-Butyl (2S,4R)-4-[tert-butyl(dimethyl)silyl]oxy-2-[1-[2-[4-(4-methylthiazol-5-yl)phenyl]ethyl]imidazol-2-yl]pyrrolidine-1-carboxylate (21 mg) described in Reference Example 124 was dissolved in 1,4-dioxane (800 µL), 4 M hydrochloric acid-dioxane (200 µL) was added at room temperature and the mixture was stirred for 17 hr. After completion of the reaction, the reaction solution was concentrated under reduced pressure. To the obtained residue were added, at room temperature, 2-(3-methoxy-1,2-oxazol-5-yl)-3-methylbutanoic acid (19 mg), DIPEA (50 µL), HATU (32 mg), and DMF (1 mL), and the mixture was stirred for 2.5 hr. After completion of the reaction, the reaction mixture was diluted with DMSO, and purified by preparative HPLC (10 mM ammonium carbonate aqueous solution-acetonitrile) to give the title compound (16 mg) as a white powder. MS(ESI)m/z:536.5[M+H]+

### Example 116: Synthesis of 1-[(2S,4R)-2-(1H-benzimidazol-2-yl)-4-hydroxypyrrolidin-1-yl]-2-(3-methoxyisoxazol-5-yl)-3-methylbutan-1-one

The title compound was obtained by the same reaction and treatment as in Example 61 using tert-butyl (2S,4R)-2-(1H-benzimidazol-2-yl)-4-hydroxypyrrolidine-1-carboxylate described in Reference Example 123, and 2-(3-methoxy-1,2-oxazol-5-yl)-3-methylbutanoic acid. MS(ESI)m/z:385.4[M+H]+

### Example 117: Synthesis of 1-[(2S,4R)-4-hydroxy-2-[1-[(3-phenyl-1,2,4-oxadiazol-5-yl)methyl]imidazol-2-yl]pyrrolidin-1-yl]-2-(3-methoxyisoxazol-5-yl)-3-methylbutan-1-one

The title compound was obtained by the same reaction and treatment as in Example 60 using tert-butyl (2S,4R)-4-[tert-butyl(dimethyl)silyl]oxy-2-[1-[(3-phenyl-1,2,4-oxadiazol-5-yl)methyl]imidazol-2-yl]pyrrolidine-1-carboxylate described in Reference Example 125, and 2-(3-methoxy-1,2-oxazol-5-yl)-3-methylbutanoic acid. MS(ESI)m/z:493.1[M+H]+

### Example 118: Synthesis of 1-[(2S,4R)-2-[1-[(3,5-dimethyl-1-phenylpyrazol-4-yl)methyl]imidazol-2-yl]-4-hydroxypyrrolidin-1-yl]-2-(3-methoxyisoxazol-5-yl)-3-methylbutan-1-one

tert-Butyl (2S,4R)-4-[tert-butyl(dimethyl)silyl]oxy-2-[1-[(3,5-dimethyl-1-phenylpyrazol-4-yl)methyl]imidazol-2-yl]pyrrolidine-1-carboxylate (25 mg) described in Reference Example 126 was dissolved in 1,4-dioxane (800 µL), 4 M hydrochloric acid-dioxane (200 µL) was added at room temperature and the mixture was stirred for 20 hr. After completion of the reaction, the reaction solution was concentrated under reduced pressure. To the obtained residue were added, at room temperature, 2-(3-methoxy-1,2-oxazol-5-yl)-3-methylbutanoic acid (20 mg), DIPEA (50 µL), HATU (25 mg), and DMF (1 mL), and the mixture was stirred for 2.5 hr. After completion of the reaction, the reaction mixture was diluted with DMSO, and purified by preparative HPLC (10 mM ammonium carbonate aqueous solution-acetonitrile) to give the title compound (12 mg) as a white powder. MS(ESI)m/z:519.2[M+H]+

### Example 119: Synthesis of 2-[(2S,4R)-4-hydroxy-1-[2-(3-methoxyisoxazol-5-yl)-3-methylbutanoyl]pyrrolidin-2-yl]-N,N-dimethyl-1H-imidazole-4-carboxamide

The title compound was obtained by the same reaction and treatment as in Example 2 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8, and dimethylamine. MS(ESI)m/z:406.4[M+H]+

### Example 120: Synthesis of N-[(1S)-1-[(2S,4R)-4-hydroxy-2-[1-[[3-(4-methylthiazol-5-yl)phenyl]methyl]imidazol-2-yl]pyrrolidine-1-carbonyl]-2,2-dimethylpropyl]acetamide

To (2S)-2-amino-1-[(2S,4R)-4-hydroxy-2-[1-[[3-(4-methylthiazol-5-yl)phenyl]methyl]imidazol-2-yl]pyrrolidin-1-yl]-3,3-dimethylbutan-1-one hydrochloride (20 mg) described in Reference Example 128 were added DCM (1 mL), Et3N (21 µL), and acetic anhydride (4.3 µL) at room temperature and the mixture was stirred for 30 min. The reaction was discontinued by adding saturated aqueous sodium hydrogen carbonate and the reaction mixture was extracted with chloroform. The organic layer was separated by a Phase-separator (registered trade mark), and concentrated under reduced pressure. The obtained residue was purified by preparative HPLC (10 mM ammonium carbonate aqueous solution-acetonitrile) to give the title compound (11 mg) as a white powder. MS(ESI)m/z:496.1[M+H]+

### Example 121: Synthesis of N-[(1S)-1-[(2S,4R)-4-hydroxy-2-[1-[2-[3-(4-methylthiazol-5-yl)phenyl]ethyl]imidazol-2-yl]pyrrolidine-1-carbonyl]-2,2-dimethylpropyl]acetamide

DCM (1 mL), Et3N (21 µL), and acetic anhydride (4.5 µL) were added to (2S)-2-amino-1-[(2S,4R)-4-hydroxy-2-[1-[2-[3-(4-methylthiazol-5-yl)phenyl]ethyl]imidazol-2-yl]pyrrolidin-1-yl]-3,3-dimethylbutan-1-one hydrochloride (20 mg) described in Reference Example 130 at room temperature and the mixture was stirred for 30 min. The reaction was discontinued by adding saturated aqueous sodium hydrogen carbonate, and the reaction mixture was extracted with chloroform. The organic layer was separated by a Phase-separator (registered trade mark), and concentrated under reduced pressure. The obtained residue was purified by preparative HPLC (10 mM ammonium carbonate aqueous solution-acetonitrile) to give the title compound (11 mg) as a white powder. MS(ESI)m/z:510.2[M+H]+

### Example 122: Synthesis of N-(2-acetamidoethyl)-2-[(2S,4R)-1-[(2S)-2-[(1-fluorocyclopropanecarbonyl)amino]-3,3-dimethylbutanoyl]-4-hydroxypyrrolidin-2-yl]-N-(4-phenylbutyl)-1H-imidazole-4-carboxamide

N-(2-aminoethyl)-2-[(2S,4R)-1-[(2S)-2-[(1-fluorocyclopropanecarbonyl)amino]-3,3-dimethylbutanoyl]-4-hydroxypyrrolidin-2-yl]-N-(4-phenylbutyl)-1H-imidazole-4-carboxamide hydrochloride (20 mg) described in Reference Example 143 was dissolved in DCM (0.60 mL), Et3N (16 µL) and acetic anhydride (3 µL) were added at room temperature and the mixture was stirred for 2 hr. The reaction was quenched by adding sodium bicarbonate water, to the reaction mixture were added chloroform and water, and the organic layer was separated by a Phase-separator (registered trade mark), and concentrated under reduced pressure. The obtained residue was purified by preparative HPLC (10 mM ammonium carbonate aqueous solution-acetonitrile) to give the title compound (4.5 mg) as a colorless viscous substance. MS(ESI)m/z:613.3[M+H]+

### Example 123: Synthesis of N-[(1S)-1-[(2S,4R)-2-[4-[[cyclopropylmethyl(4-phenylbutyl)amino]methyl]-1H-imidazol-2-yl]-4-hydroxypyrrolidine-1-carbonyl]-2,2-dimethylpropyl]-1-fluoro-cyclopropanecarboxamide

The title compound was obtained by the same reaction and treatment as in Example 60 using tert-butyl N-[(1S)-1-[(2S,4R)-4-[tert-butyl(dimethyl)silyl]oxy-2-[4-[[cyclopropylmethyl(4-phenylbutyl)amino]methyl]-1H-imidazol-2-yl]pyrrolidine-1-carbonyl]-2,2-dimethyl-propyl]carbamate described in Reference Example 145, 1-fluorocyclopropanecarboxylic acid, and methanol instead of 1,4-dioxane. MS(ESI)m/z:568.4[M+H]+

### Example 124: Synthesis of N-[2-[acetyl(methyl)amino]ethyl]-2-[(2S,4R)-1-[(2S)-2-[(1-fluorocyclopropanecarbonyl)amino]-3,3-dimethylbutanoyl]-4-hydroxypyrrolidin-2-yl]-N-(4-phenylbutyl)-1H-imidazole-4-carboxamide

2-[(2S,4R)-1-[(2S)-2-[(1-fluorocyclopropanecarbonyl)amino]-3,3-dimethylbutanoyl]-4-hydroxypyrrolidin-2-yl]-1H-imidazole-4-carboxylic acid (30 mg) described in Reference Example 133, and tert-butyl N-methyl-N-[2-(4-phenylbutylamino)ethyl]carbamate (28 mg) described in Reference Example 144 were dissolved in DMF (0.80 mL), DIPEA (52 µL) and HATU (37 mg) were added at room temperature and the mixture was stirred for 2 hr. Then, diisopropyl ethylamine (26 µL), HATU (20 mg), and tert-butyl N-methyl-N-[2-(4-phenylbutylamino)ethyl]carbamate (14 mg) were added and the mixture was stirred for 20 hr. The reaction mixture was diluted with DMSO and purified by preparative HPLC (10 mM ammonium carbonate aqueous solution-acetonitrile). The residue was dissolved in 1,4-dioxane (0.88 mL), 4 M hydrochloric acid-dioxane (0.11 mL) was added at room temperature and the mixture was stirred for 2 hr. 4 M Hydrochloric acid-dioxane (0.22 mL) was added and the mixture was stirred for 20 hr. After completion of the reaction, the reaction solution was concentrated under reduced pressure and azeotropically distilled 3 times with toluene. The obtained residue was dissolved in dichloromethane (0.7 mL), acetic anhydride (8 µL) and triethylamine (19 µL) were added and the mixture was stirred at room temperature for 1 hr. To the reaction mixture was added sodium bicarbonate water and the mixture was extracted with chloroform. The organic layer was separated and dried over anhydrous sodium sulfate. The organic layer was filtered, and the filtrate was concentrated under reduced pressure. The obtained residue was purified by preparative HPLC (10 mM ammonium carbonate aqueous solution-acetonitrile) to give the title compound (10 mg) as a white powder. MS (ES I) m/z: 627.4 [M+H]+

### Example 125: Synthesis of N-[(1S)-1-[(2S,4R)-2-[5-[2-(3-chlorophenyl)ethyl]-1H-imidazol-2-yl]-4-hydroxypyrrolidine-1-carbonyl]-2,2-dimethylpropyl]acetamide

tert-Butyl N-[(1S)-1-[(2S,4R)-2-[5-[2-(3-chlorophenyl)ethyl]-1H-imidazol-2-yl]-4-hydroxypyrrolidine-1-carbonyl]-2,2-dimethylpropyl]carbamate (74 mg) described in Reference Example 113 was dissolved in 1,4-dioxane (3 mL), 4 M hydrochloric acid-dioxane (1 mL) was added at room temperature and the mixture was stirred for 6 hr. After completion of the reaction, the reaction solution was concentrated under reduced pressure and azeotropically distilled with toluene. To the obtained residue were added, at room temperature, DCM (4 mL), Et3N (82 µL), and acetic anhydride (20.7 µL) and the mixture was stirred for 2 hr. After completion of the reaction, the reaction was discontinued by adding 1 M sodium hydroxide aqueous solution, and the mixture was extracted with ethyl acetate. The organic layer was washed with saturated brine, and dried over anhydrous sodium sulfate. The organic layer was filtered, and the filtrate was concentrated under reduced pressure. The obtained residue was purified by silica gel column chromatography (ethyl acetate:methanol=100:0 - 90:10) to give the title compound (56 mg) as a pale-yellow powder. MS(ESI)m/z:447.2/449.1[M+H]+

### Example 126: Synthesis of N-[(1S)-1-[(2S,4R)-2-[5-[2-(4-chlorophenyl)ethyl]-1H-imidazol-2-yl]-4-hydroxypyrrolidine-1-carbonyl]-2,2-dimethylpropyl]acetamide

The title compound was obtained by the same reaction and treatment as in Example 81 using tert-butyl N-[(1S)-1-[(2S,4R)-2-[5-[2-(4-chlorophenyl)ethyl]-1H-imidazol-2-yl]-4-hydroxypyrrolidine-1-carbonyl]-2,2-dimethyl-propyl]carbamate described in Reference Example 109.
MS(ESI)m/z:447.4/449.4[M+H]+

### Example 127: Synthesis of 1-[(2S,4R)-4-hydroxy-2-[1-(2-naphthylmethyl)imidazol-2-yl]pyrrolidin-1-yl]-2-(3-methoxyisoxazol-5-yl)-3-methylbutan-1-one

2-(3-Methoxy-1,2-oxazol-5-yl)-3-methylbutanoic acid (6.3 mg), HOBt (4.7 mg), and WSC·HCl (6.8 mg) were dissolved in DMF (77 µL), and the mixture was stirred at room temperature for 30 min. The reaction mixture was added to a solution of (3R,5S)-5-[1-(2-naphthylmethyl)imidazol-2-yl]pyrrolidin-3-ol (8.4 mg) described in Reference Example 151 and Et3N (12 µL) in DMF (200 µL), and the mixture was stirred at room temperature for 5 hr. The reaction was discontinued by adding water (50 µL), and the mixture was purified by preparative HPLC (10 mM ammonium carbonate aqueous solution-acetonitrile) to give the title compound (10 mg) as a white powder. MS(ESI)m/z:475.1[M+H]+

### Example 128: Synthesis of (2S)-1-[(2S,4R)-4-hydroxy-2-(1H-imidazol-2-yl)pyrrolidin-1-yl]-3-methyl-2-[4-(3-methylisoxazol-5-yl)triazol-1-yl]butan-1-one

(2S)-3-methyl-2-[4-(3-methylisoxazol-5-yl)triazol-1-yl]butanoic acid (32 mg) described in Reference Example 153 and the (3R,5S)-5-(1H-imidazol-2-yl)pyrrolidin-3-ol hydrochloride (23 mg) described in Reference Example 122 were dissolved in DMF (1 mL), HATU (60 mg) and DIPEA (100 µL) were added at room temperature and the mixture was stirred for 1.5 hr. After completion of the reaction, chloroform and water were added, and the organic layer was separated by a Phase-separator (registered trade mark) and concentrated under reduced pressure. The obtained residue was purified by preparative HPLC (10 mM ammonium carbonate aqueous solution-acetonitrile) to give the title compound (18 mg) as a pale-yellow powder. MS (ESI)m/z:386.1[M+H] +

### Example 129: Synthesis of N-[(1S)-1-[(2S,4R)-2-[4-[[cyclopropanecarbonyl(4-phenylbutyl)amino]methyl]-1H-imidazol-2-yl]-4-hydroxypyrrolidine-1-carbonyl]-2,2-dimethyl-propyl]-1-fluoro-cyclopropanecarboxamide

tert-Butyl N-[(1S)-1-[(2S,4R)-4-[tert-butyl(dimethyl)silyl]oxy-2-[4-[[cyclopropanecarbonyl(4-phenylbutyl)amino]methyl]-1H-imidazol-2-yl]pyrrolidine-1-carbonyl]-2,2-dimethyl-propyl]carbamate (65 mg) described in Reference Example 156 was dissolved in methanol (0.9 mL), 2 M hydrochloric acid-methanol (456 µL) was added and the mixture was stirred for 18 hr. The reaction solution was concentrated under reduced pressure, and the obtained residue was added to 2 M hydrochloric acid-methanol (456 µL) at room temperature and the mixture was stirred for 5 hr. After completion of the reaction, the reaction solution was concentrated under reduced pressure. To the obtained residue were added, at room temperature, 1-fluorocyclopropanecarboxylic acid (4.7 mg), HATU (17 mg), DIPEA (26 µL), and DMF (0.30 mL), and the mixture was stirred for 15 hr. HATU (17 mg) and DIPEA (26 µL) were added and the mixture was stirred for 3 hr. After completion of the reaction, the reaction mixture was diluted with DMSO, and purified by preparative HPLC (10 mM ammonium carbonate aqueous solution-acetonitrile) to give the title compound (7.0 mg) as a colorless powder. MS(ESI)m/z:582.3[M+H]+

### Example 130: Synthesis of N-[(1S)-1-[(2S,4R)-2-[4-[[cyclopropylmethyl(4-phenylbutanoyl)amino]methyl]-1H-imidazol-2-yl]-4-hydroxypyrrolidine-1-carbonyl]-2,2-dimethylpropyl]-1-fluorocyclopropanecarboxamide

tert-Butyl N-[(1S)-1-[(2S,4R)-4-[tert-butyl(dimethyl)silyl]oxy-2-[4-[[cyclopropylmethyl(4-phenylbutanoyl)amino]methyl]-1H-imidazol-2-yl]pyrrolidine-1-carbonyl]-2,2-dimethyl-propyl]carbamate (50 mg) described in Reference Example 157 was dissolved in 2 M hydrochloric acid-methanol (886 µL) at room temperature, and the mixture was stirred for 15 hr. After completion of the reaction, the reaction solution was concentrated under reduced pressure. To the obtained residue were added, at room temperature, 1-fluorocyclopropanecarboxylic acid (3.5 mg), HATU (14 mg), DIPEA (21 µL), and DMF (0.24 mL), and the mixture was stirred for 3 hr was stirred. After completion of the reaction, the reaction mixture was diluted with DMSO, and purified by preparative HPLC (10 mM ammonium carbonate aqueous solution-acetonitrile) to give the title compound (8.8 mg) as a colorless powder. MS(ESI)m/z:582.3[M+H]+

### Example 131: Synthesis of N-cyclopropyl-2-[(2S,4R)-4-hydroxy-1-[2-(3-methoxyisoxazol-5-yl)-3-methylbutanoyl]pyrrolidin-2-yl]-N-(5-phenylpentyl)-1H-imidazole-4-carboxamide

DMF (0.70 mL) was added to 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (20 mg) described in Reference Example 8, N-(5-phenylpentyl)cyclopropanamine (11 mg) described in Reference Example 161, DIPEA (30 µL), and HATU (30 mg) were added, and the mixture was stirred at room temperature overnight. N-(5-phenylpentyl)cyclopropanamine (11 mg) described in Reference Example 161 and HATU (15 mg) were added, and the mixture was stirred for about 1 hr. To the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (18 mg) as a white powder. MS(ESI)m/z:564.4[M+H]+

### Example 132: Synthesis of N-(2-benzyloxyethyl)-N-cyclopropyl-2-[(2S,4R)-4-hydroxy-1-[2-(3-methoxyisoxazol-5-yl)-3-methylbutanoyl]pyrrolidin-2-yl]-1H-imidazole-4-carboxamide

DMF (0.60 mL) was added to 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (20 mg) described in Reference Example 8, N-(2-benzyloxyethyl)cyclopropaneamine (12 mg) described in Reference Example 163, HATU (30 mg), and DIPEA (0.027 mL) were added, and the mixture was stirred at room temperature for about 3 hr. To the reaction mixture was added water and the mixture was extracted with ethyl acetate. The organic layer was washed with water and saturated brine, dried over sodium sulfate and filtered. The filtrate was concentrated under reduced pressure, and purified by NH silica gel column chromatography (ethyl acetate:methanol=100:0 - 90:10) to give the title compound (15 mg) as a white powder. MS(ESI)m/z:552.5[M+H]+

### Example 133: Synthesis of N-cyclopropyl-2-[(2S,4R)-4-hydroxy-1-[2-(3-methoxyisoxazol-5-yl)-3-methylbutanoyl]pyrrolidin-2-yl]-N-(4-phenylbutyl)-1H-imidazole-4-carboxamide

DMF (0.80 mL) was added to 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid (16 mg) described in Reference Example 8, N-(4-phenylbutyl)cyclopropanamine (20 mg) described in Reference Example 160, DIPEA (27 µL), and HATU (30 mg) were added and the mixture was stirred at room temperature for about 4 hr. To the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (4.6 mg) as a white powder. MS (ESI)m/z:550.4 [M+H]+

### Example 134: Synthesis of 1-fluoro-N-[(1S)-1-[(2S,4R)-4-hydroxy-2-[1-methyl-4-(4-phenylpiperidine-1-carbonyl)imidazol-2-yl]pyrrolidine-1-carbonyl]-2,2-dimethylpropyl]cyclopropanecarboxamide

tert-Butyl N-[(1S)-1-[(2S,4R)-4-hydroxy-2-[1-methyl-5-(4-phenylpiperidine-1-carbonyl)imidazol-2-yl]pyrrolidine-1-carbonyl]-2,2-dimethyl-propyl]carbamate (52 mg) described in Reference Example 159 was dissolved in 1,4-dioxane (0.46 mL), 4 M hydrochloric acid-dioxane (687 µL) was added at room temperature and the mixture was stirred for 6 hr. After completion of the reaction, the reaction solution was concentrated under reduced pressure. To the obtained residue were added 1-fluorocyclopropanecarboxylic acid (3.4 mg), HATU (12 mg), DIPEA (19 µL), and DMF (0.44 mL) at room temperature, and the mixture was stirred for 15 hr. After completion of the reaction, the reaction mixture was diluted with DMSO, and purified by preparative HPLC (10 mM ammonium carbonate aqueous solution-acetonitrile) to give the title compound (9.5 mg) as a colorless powder. MS(ESI)m/z:554.3[M+H]+

### Example 135: Synthesis of (2S,3S)-2-(4-ethoxytriazol-1-yl)-1-[(2S,4R)-4-hydroxy-2-(1H-imidazol-2-yl)pyrrolidin-1-yl]-3-methylpentan-1-one

(2S,3S)-2-(4-ethoxytriazol-1-yl)-3-methylpentanoic acid (75 mg) described in Reference Example 155, (3R,5S)-5-(1H-imidazol-2-yl)pyrrolidin-3-ol hydrochloride (25 mg) described in Reference Example 122, and HATU (100 mg) were dissolved in DMF (1 mL), DIPEA (200 µL) was added, and the mixture was stirred at room temperature for 1 hr. After completion of the reaction, to the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (12 mg) as a pale-brown powder. MS(ESI)m/z:363.3[M+H]+

### Example 136: Synthesis of N-[(1S,2S)-1-[(2S,4R)-4-hydroxy-2-(1H-imidazol-2-yl)pyrrolidine-1-carbonyl]-2-methylbutyl]-2-(3-methylisoxazol-5-yl)acetamide

tert-Butyl N-[(1S,2S)-1-[(2S,4R)-4-hydroxy-2-(1H-imidazol-2-yl)pyrrolidine-1-carbonyl]-2-methylbutyl]carbamate (41 mg) described in Reference Example 162 was dissolved in 1,4-dioxane (1.2 mL), 4 M hydrochloric acid-dioxane (0.9 mL) was added at room temperature and the mixture was stirred overnight. After completion of the reaction, the reaction solution was concentrated under reduced pressure, and azeotropically distilled with toluene. To the obtained residue were added, at room temperature, DMF (0.70 mL), DIPEA (0.038 mL), 2-(3-methylisoxazol-5-yl)acetic acid (10 mg), and HATU (32 mg) and the mixture was stirred for 2 hr. To the reaction mixture were added chloroform and water, and the organic layer was separated by a Phase-separator (registered trade mark), concentrated under reduced pressure, and purified by preparative HPLC (10 mM ammonium carbonate aqueous solution-acetonitrile) to give the title compound (3.9 mg) as a white powder. MS(ESI)m/z:390.1[M+H]+

### Example 137: Synthesis of 1-[(2S,4R)-2-[4-(7-bromo-3,4-dihydro-1H-isoquinoline -2-carbonyl)-1H-imidazol-2-yl]-4-hydroxypyrrolidin-1-yl]-2-(3-methoxyisoxazol-5-yl)-3-methylbutan-1-one

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 6-bromo-1,2,3,4-tetrahydroisoquinoline.
MS(ESI)m/z:572.0/573.9[M+H]+

### Example 138: Synthesis of 1-[(2S,4R)-2-[4-(6-bromo-3,4-dihydro-1H-isoquinoline -2-carbonyl)-1H-imidazol-2-yl]-4-hydroxypyrrolidin-1-yl]-2-(3-methoxyisoxazol-5-yl)-3-methylbutan-1-one

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 7-bromo-1,2,3,4-tetrahydroisoquinoline.
MS(ESI)m/z:572.0/573.9[M+H]+

### Example 139: Synthesis of 1-[(2S,4R)-4-hydroxy-2-[4-[4-(4-pyridyl)piperidine-1-carbonyl]-1H-imidazol-2-yl]pyrrolidin-1-yl]-2-(3-methoxyisoxazol-5-yl)-3-methylbutan-1-one

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 1-phenylpiperazine. MS(ESI)m/z:523.1[M+H]+

### Example 140: Synthesis of 1-[(2S,4R)-4-hydroxy-2-[4-(4-phenylpiperazine-1-carbonyl)-1H-imidazol-2-yl]pyrrolidin-1-yl]-2-(3-methoxyisoxazol-5-yl)-3-methylbutan-1-one

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 4-(4-piperidyl)pyridine. MS(ESI)m/z:523.1[M+H]+

### Example 141: Synthesis of 1-[(2S,4R)-2-[4-(4-benzyloxypiperidine-1-carbonyl)-1H-imidazol-2-yl]-4-hydroxypyrrolidin-1-yl]-2-(3-methoxyisoxazol-5-yl)-3-methylbutan-1-one

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 4-benzyloxypiperidine. MS(ESI)m/z:552.2[M+H]+

### Example 142: Synthesis of 1-[(2S,4R)-2-[4-(3-benzyloxypiperidine-1-carbonyl)-1H-imidazol-2-yl]-4-hydroxypyrrolidin-1-yl]-2-(3-methoxyisoxazol-5-yl)-3-methylbutan-1-one

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 (23 mg), WSC·HCl (20 mg), and HOAt (15 mg) were dissolved in DMF (1.0 mL), 3-benzyloxypiperidine (30 mg) was added and the mixture was stirred at room temperature overnight. MeOH (1 mL) was added and the mixture was purified by preparative HPLC (10 mM ammonium carbonate aqueous solution-acetonitrile) to give the title compound (20 mg) as a white viscous substance.
MS(ESI)m/z:552.2[M+H]+

### Example 143: Synthesis of 1-[(2S,4R)-4-hydroxy-2-[4-[4-(6-methyl-3-pyridyl)piperidine-1-carbonyl]-1H-imidazol-2-yl]pyrrolidin-1-yl]-2-(3-methoxyisoxazol-5-yl)-3-methylbutan-1-one

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 (23 mg), WSC·HCl (20 mg), and HOAt (15 mg) were dissolved in DMF (1.0 mL), 2-methyl-5-(4-piperidyl)pyridine (30 mg) was added and the mixture was stirred at room temperature overnight. MeOH (1 mL) was added and the mixture was purified by preparative HPLC (10 mM ammonium carbonate aqueous solution-acetonitrile) to give the title compound (17 mg) as a pale-yellow viscous substance.
MS(ESI)m/z:537.2[M+H]+

### Example 144: Synthesis of 1-[(2S,4R)-4-hydroxy-2-[4-[4-(5-methyl-2-pyridyl)piperidine-1-carbonyl]-1H-imidazol-2-yl]pyrrolidin-1-yl]-2-(3-methoxyisoxazol-5-yl)-3-methylbutan-1-one

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 5-methyl-2-(4-piperidyl)pyridine. MS(ESI)m/z:537.2[M+H]+

### Example 145: Synthesis of 1-[(2S,4R)-2-[4-[4-[(2-fluorophenyl)methyl]piperazine-1-carbonyl]-1H-imidazol-2-yl]-4-hydroxypyrrolidin-1-yl]-2-(3-methoxyisoxazol-5-yl)-3-methylbutan-1-one

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 1-[(2-fluorophenyl)methyl]piperazine.
MS (ESI)m/z:555.2[M+H] +

### Example 146: Synthesis of 1-[(2S,4R)-4-hydroxy-2-[4-(2-phenylmorpholine-4-carbonyl)-1H-imidazol-2-yl]pyrrolidin-1-yl]-2-(3-methoxyisoxazol-5-yl)-3-methylbutan-1-one

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 (26 mg), HATU (40 mg), and DIPEA (40 µL) were dissolved in DMF (1.0 mL), 2-phenylmorpholine (30 mg) was added and the mixture was stirred at room temperature overnight. MeOH (1 mL) was added and the mixture was purified by preparative HPLC (10 mM ammonium carbonate aqueous solution-acetonitrile) to give the title compound (21 mg) as a white viscous substance.
MS(ESI)m/z:524.1[M+H]+

### Example 147: Synthesis of 1-[(2S,4R)-4-hydroxy-2-(4-methylthiazol-2-yl)pyrrolidin-1-yl]-2-(3-methoxyisoxazol-5-yl)-3-methylbutan-1-one

DMF (0.60 mL) was added to 2-(3-methoxy-1,2-oxazol-5-yl)-3-methylbutanoic acid (13 mg), DIPEA (0.028 mL), (3R,5S)-5-(4-methylthiazol-2-yl)pyrrolidin-3-ol (10 mg) described in Reference Example 164, and HATU (31 mg) were added, and the mixture was stirred at room temperature for 1 hr. To the reaction mixture was added water and the mixture was extracted with ethyl acetate. The organic layer was washed with water and saturated brine, dried over sodium sulfate and filtered. The filtrate was concentrated under reduced pressure and purified by NH silica gel column chromatography (ethyl acetate:methanol=100:0 - 80:20) to give the title compound (5.7 mg) as a colorless oil. MS(ESI)m/z:366.0[M+H]+

### Example 148: Synthesis of 1-[2-[(2S,4R)-4-hydroxy-1-[2-(3-methoxyisoxazol-5-yl)-3-methylbutanoyl]pyrrolidin-2-yl]-1H-imidazole-4-carbonyl]-N-phenylpiperidine-3-carboxamide

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and N-phenylpiperidine-3-carboxamide. MS(ESI)m/z:565.4[M+H]+

### Example 149: Synthesis of N-[1-[2-[(2S,4R)-4-hydroxy-1-[2-(3-methoxyisoxazol-5-yl)-3-methylbutanoyl]pyrrolidin-2-yl]-1H-imidazole-4-carbonyl]pyrrolidin-3-yl]-N-methylbenzamide

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and N-methyl-N-pyrrolidin-3-yl -benzamide hydrochloride. MS(ESI)m/z:565.4[M+H]+

### Example 150: Synthesis of 1-[(2S,4R)-2-[4-(4-anilinopiperidine-1-carbonyl)-1H-imidazol-2-yl]-4-hydroxypyrrolidin-1-yl]-2-(3-methoxyisoxazol-5-yl)-3-methylbutan-1-one

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and N-phenylpiperidine-4-amine dihydrochloride. MS(ESI)m/z:537.5[M+H]+

### Example 151: Synthesis of N-ethyl-2-[(2S,4R)-4-hydroxy-1-[2-(3-methoxyisoxazol-5-yl)-3-methylbutanoyl]pyrrolidin-2-yl]-N-[[4-(methylcarbamoyl)phenyl]methyl]-1H-imidazole-4-carboxamide

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 4-(ethylaminomethyl)-N-methyl-benzamide hydrochloride described in Reference Example 165. MS(ESI)m/z:553.4[M+H]+

### Example 152: Synthesis of N-[[4-(dimethylcarbamoyl)phenyl]methyl]-N-ethyl-2-[(2S,4R)-4-hydroxy-1-[2-(3-methoxyisoxazol-5-yl)-3-methylbutanoyl]pyrrolidin-2-yl]-1H-imidazole-4-carboxamide

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 4-(ethylaminomethyl)-N,N-dimethylbenzamide hydrochloride. MS(ESI)m/z:567.5[M+H]+

### Example 153: Synthesis of 2-[(2S,4R)-4-hydroxy-1-[2-(3-methoxyisoxazol-5-yl)-3-methylbutanoyl]pyrrolidin-2-yl]-N-methyl-N-[[4-(2-methyl-4-pyridyl)phenyl]methyl]-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 (20 mg), WSC·HCl (15 mg), and HOAt (11 mg) were dissolved in DMF (1.0 mL), N-methyl-1-[4-(2-methyl-4-pyridyl)phenyl]methanamine dihydrochloride (40 mg) described in Reference Example 170 and DIPEA (0.10 mL) were added and the mixture was stirred at room temperature overnight. MeOH (1 mL) was added and the mixture was purified by preparative HPLC (10 mM ammonium carbonate aqueous solution-acetonitrile) to give the title compound (17 mg) as a white viscous substance. MS(ESI)m/z:573.5[M+H]+

### Example 154: Synthesis of 2-[(2S,4R)-4-hydroxy-1-[2-(3-methoxyisoxazol-5-yl)-3-methylbutanoyl]pyrrolidin-2-yl]-N-methyl-N-[[4-(6-methyl-3-pyridyl)phenyl]methyl]-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 (20 mg), WSC·HCl (15 mg), and HOAt (11 mg) were dissolved in DMF (1.0 mL), N-methyl-1-[4-(6-methyl-3-pyridyl)phenyl]methanamine dihydrochloride (35 mg) described in Reference Example 172 and DIPEA (0.10 mL) were added and the mixture was stirred at room temperature overnight. MeOH (1 mL) was added and the mixture was purified by preparative HPLC (10 mM ammonium carbonate aqueous solution-acetonitrile) to give the title compound (20 mg) as a white viscous substance. MS(ESI)m/z:573.4[M+H]+

### Example 155: Synthesis of N-[[1-[(2-fluorophenyl)methyl]-4-piperidyl]methyl]-2-[(2S,4R)-4-hydroxy-1-[2-(3-methoxyisoxazol-5-yl)-3-methylbutanoyl]pyrrolidin-2-yl]-N-methyl-1H-imidazole-4-carboxamide

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 1-[1-[(2-fluorophenyl)methyl]-4-piperidyl]-N-methylmethanamine dihydrochloride described in Reference Example 173. MS(ESI)m/z:597.5[M+H]+

### Example 156: Synthesis of 2-[(2S,4R)-4-hydroxy-1-[2-(3-methoxyisoxazol-5-yl)-3-methylbutanoyl]pyrrolidin-2-yl]-N-methyl-N-[(1-phenyl-4-piperidyl)methyl]-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 (20 mg), WSC·HCl (15 mg), and HOAt (11 mg) were dissolved in DMF (1.0 mL), N-methyl-1-(1-phenyl-4-piperidyl)methanamine dihydrochloride (40 mg) described in Reference Example 167 and DIPEA (0.10 mL) were added and the mixture was stirred at room temperature overnight. MeOH (1 mL) was added and the mixture was purified by preparative HPLC (10 mM ammonium carbonate aqueous solution-acetonitrile) to give the title compound (16 mg) as a white viscous substance. MS(ESI)m/z:553.4[M+H]+

### Example 157: Synthesis of 1-benzyl-4-[2-[(2S,4R)-4-hydroxy-1-[2-(3-methoxyisoxazol-5-yl)-3-methylbutanoyl]pyrrolidin-2-yl]-1H-imidazole-4-carbonyl]piperazin-2-one

The title compound was obtained by the same reaction and treatment as in Example 156 using 2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 and 1-benzylpiperazin-2-one. MS(ESI)m/z:551.4[M+H]+

### Example 158: Synthesis of N-(2-anilinoethyl)-2-[(2S,4R)-4-hydroxy-1-[2-(3-methoxyisoxazol-5-yl)-3-methylbutanoyl]pyrrolidin-2-yl]-N-methyl-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 (26 mg), HATU (40 mg), and DIPEA (40 µL) were dissolved in DMF (1.0 mL), N-methyl-N'-phenylethane-1,2-diamine dihydrochloride (30 mg) and the mixture was stirred at room temperature overnight. MeOH (1 mL) was added and the mixture was purified by preparative HPLC (10 mM ammonium carbonate aqueous solution-acetonitrile) to give the title compound (8 mg) as a colorless oil.
MS(ESI)m/z:511.1[M+H]+

### Example 159: Synthesis of N-[(1R)-2-(benzylamino)-1-methyl-2-oxo-ethyl]-2-[(2S,4R)-4-hydroxy-1-[2-(3-methoxyisoxazol-5-yl)-3-methylbutanoyl]pyrrolidin-2-yl]-N-methyl-1H-imidazole-4-carboxamide

2-[(2S,4R)-4-hydroxy-1-[2-(3-methoxy-1,2-oxazol-5-yl)-3-methylbutanoyl]pyrrolidin-2-yl]-1H-imidazole-4-carboxylic acid described in Reference Example 8 (26 mg), HATU (40 mg), and DIPEA (40 µL) were dissolved in DMF (1.0 mL), (2R)-N-benzyl-2-(methylamino)propanamide hydrochloride (30 mg) was added and the mixture was stirred at room temperature overnight. MeOH (1 mL) was added and the mixture was purified by preparative HPLC (10 mM ammonium carbonate aqueous solution-acetonitrile) to give the title compound (5.5 mg) as a colorless oil. MS(ESI)m/z:553.2[M+H]+

### Example 160: Synthesis of 1-[(2S,4R)-4-hydroxy-2-(1H-imidazol-2-yl)pyrrolidin-1-yl]-3-methyl-2-[4-(3-methylisoxazol-5-yl)pyrazol-1-yl]butan-1-one

To a solution of ethyl 3-methyl-2-[4-(3-methylisoxazol-5-yl)pyrazol-1-yl]butanoate (16 mg) described in Reference Example 177 in methanol (2 mL) was added 1 M sodium hydroxide aqueous solution (0.17 mL), and the mixture was stirred at 50°C for 2 hr. The mixture was neutralized with 1 M hydrochloric acid and concentrated under reduced pressure. To the obtained residue were added (3R,5S)-5-(1H-imidazol-2-yl)pyrrolidin-3-ol hydrochloride (13 mg) described in Reference Example 122, DMF (2 mL), and DIPEA (50 µL), and the mixture was stirred at room temperature for 2 hr. After completion of the reaction, water was added, and the mixture was extracted with ethyl acetate. The organic layer was washed with water, concentrated under reduced pressure, and the residue was purified by silica gel column chromatography (chloroform -methanol=100:0 - 90:10) to give the title compound (8.3 mg) as a white powder. MS(ESI)m/z:385.1[M+H]+

### Example 161: Synthesis of 1-[(2S,4R)-4-hydroxy-2-(1H-imidazol-2-yl)pyrrolidin-1-yl]-3-methyl-2-(5-propyl-1,2,4-oxadiazol-3-yl)butan-1-one

The title compound was obtained by the same reaction and treatment as in Example 44 using (3R,5S)-5-(1H-imidazol-2-yl)pyrrolidin-3-ol hydrochloride described in Reference Example 122 and 3-methyl-2-(5-propyl-1,2,4-oxadiazol-3-yl)butanoic acid described in Reference Example 178. MS(ESI)m/z:348.1[M+H]+

### Example 162: Synthesis of 1-[(2S,4R)-4-hydroxy-2-(1H-imidazol-2-yl)pyrrolidin-1-yl]-3-methyl-2-(5-propyl-1,3,4-oxadiazol-2-yl)butan-1-one

The title compound was obtained by the same reaction and treatment as in Example 44 using (3R,5S)-5-(1H-imidazol-2-yl)pyrrolidin-3-ol hydrochloride described in Reference Example 122 and 3-methyl-2-(5-propyl-1,3,4-oxadiazol-2-yl)butanoic acid described in Reference Example 179. MS(ESI)m/z:348.1[M+H]+

### [Table 2-1]

**Table 2: Structural formulas of Example compounds**

| Ex. No. | structural formula | Ex. No. | structural formula |
|---|---|---|---|
| 1 | 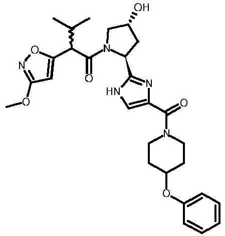 | 2 | 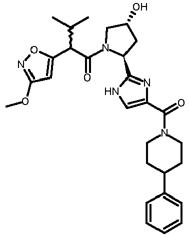 |
| 3 | 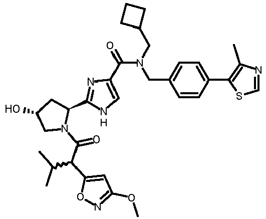 | 4 | 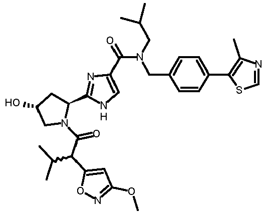 |
| 5 | 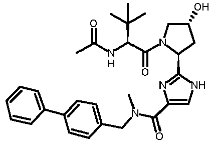 | 6 | 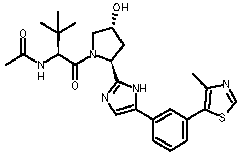 |
| 7 | 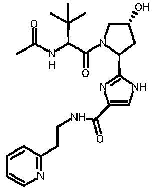 | 8 | 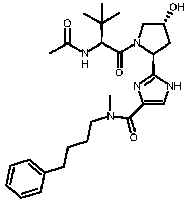 |
| 9 | 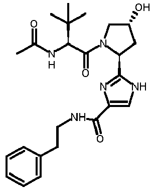 | 10 | 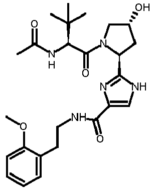 |
| 11 | 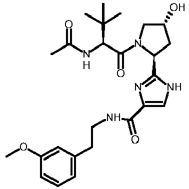 | 12 | 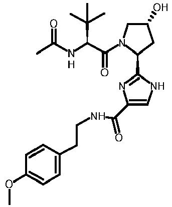 |

**[Table 2-2]**

| Ex. No. | structural formula | Ex. No. | structural formula |
|---|---|---|---|
| 13 | 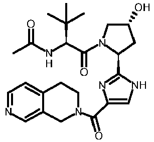 | 14 | 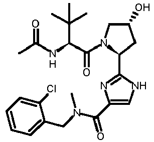 |
| 15 | 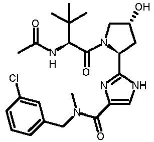 | 16 | 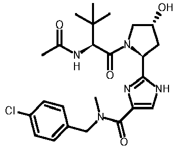 |
| 17 | 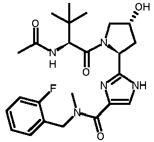 | 18 | 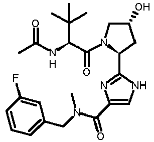 |
| 19 | 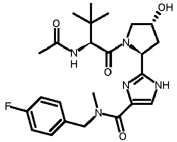 | 20 | 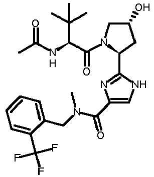 |
| 21 | 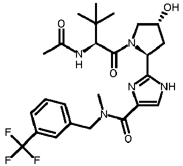 | 22 | 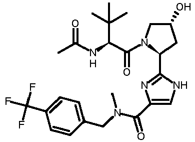 |
| 23 | 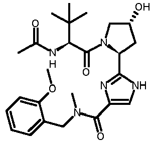 | 24 | 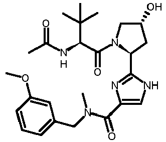 |
| 25 | 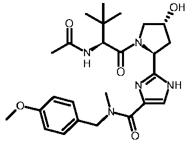 | 26 | 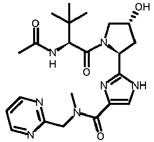 |

**[Table 2-3]**

| Ex. No. | structural formula | Ex. No. | structural formula |
|---|---|---|---|
| 27 | 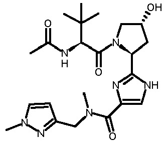 | 28 | 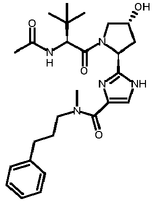 |
| 29 | 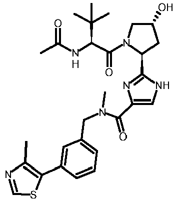 | 30 | 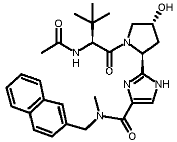 |
| 31 | 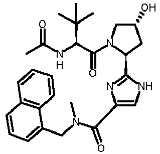 | 32 | 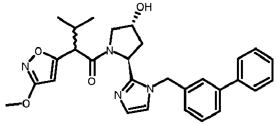 |
| 33 | 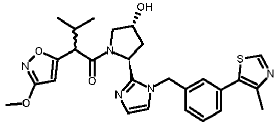 | 34 | 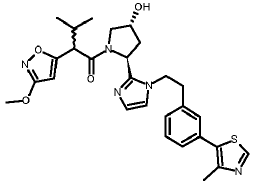 |
| 35 | 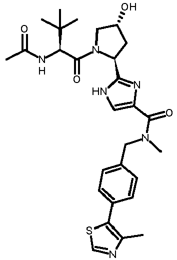 | 36 | 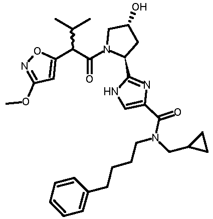 |

**[Table 2-4]**

| Ex. No. | structural formula | Ex. No. | structural formula |
|---|---|---|---|
| 37 | 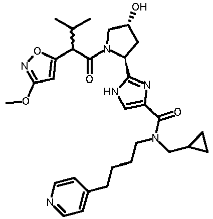 | 38 | 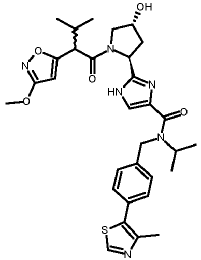 |
| 39 | 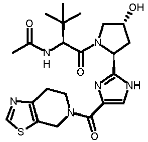 | 40 | 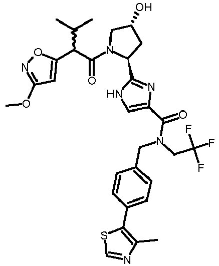 |
| 41 | 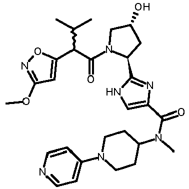 | 42 | 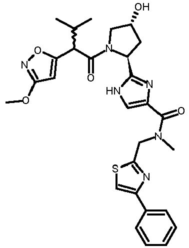 |
| 43 | 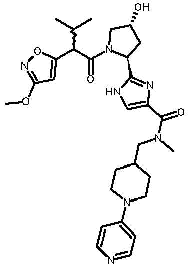 | 44 | 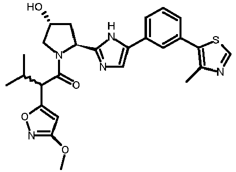 |
| 45 | 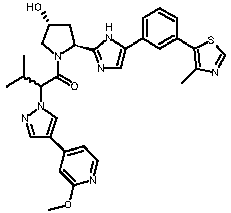 | 46 | 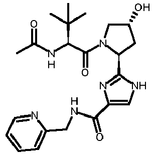 |

**[Table 2-5]**

| Ex. No. | structural formula | Ex. No. | structural formula |
|---|---|---|---|
| 47 | 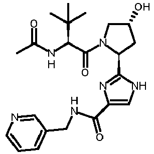 | 48 | 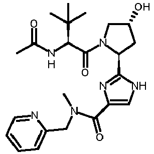 |
| 49 | 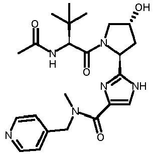 | 50 | 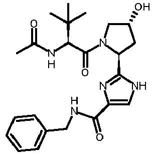 |
| 51 | 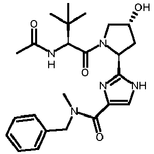 | 52 | 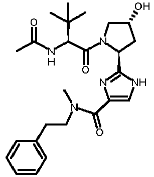 |
| 53 | 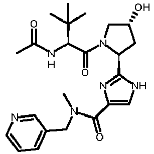 | 54 | 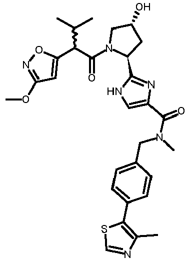 |
| 55 | 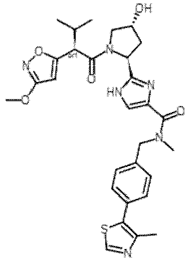 | 56 | 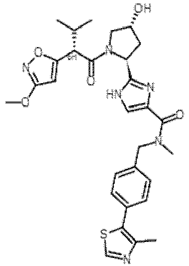 |

**[Table 2-6]**

| Ex. No. | structural formula | Ex. No. | structural formula |
|---|---|---|---|
| 57 | 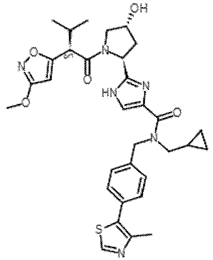 | 58 | 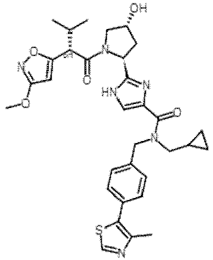 |
| 59 | 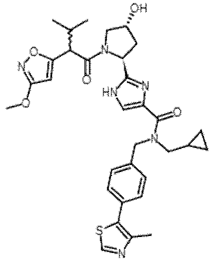 | 60 | 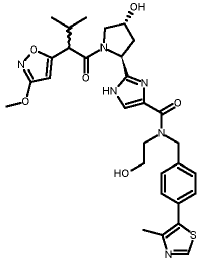 |
| 61 | 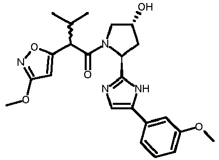 | 62 | 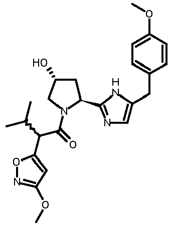 |
| 63 | 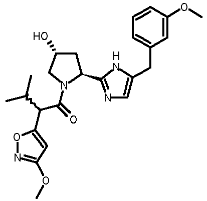 | 64 | 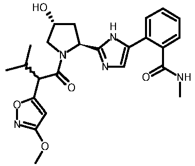 |
| 65 | 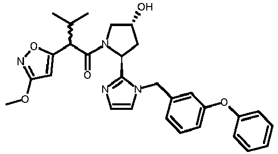 | 66 | 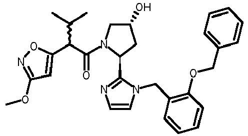 |
| 67 | 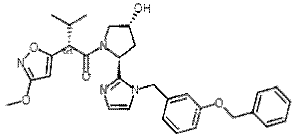 | 68 | 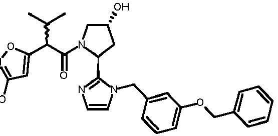 N J |

**[Table 2-7]**

| Ex. No. | structural formula | Ex. No. | structural formula |
|---|---|---|---|
| 69 | 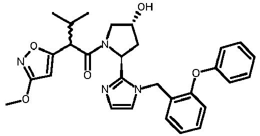 | 70 | 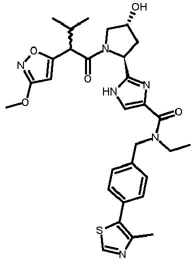 |
| 71 | 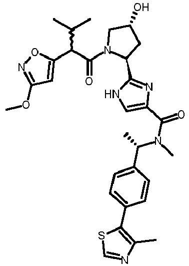 | 72 | 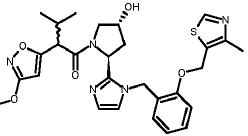 |
| 73 | 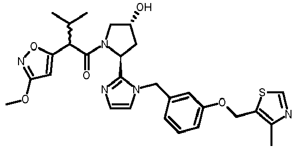 | 74 | 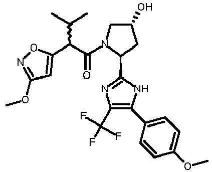 |
| 75 | 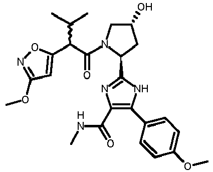 | 76 | 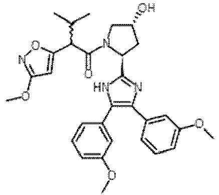 |
| 77 | 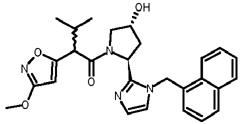 | 78 | 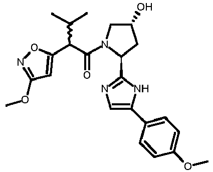 |

**[Table 2-8]**

| Ex. No. | structural formula | Ex. No. | structural formula |
|---|---|---|---|
| 79 | 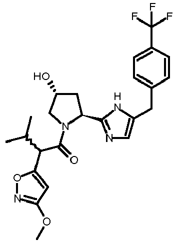 | 80 | 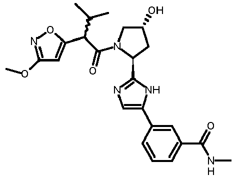 |
| 81 | 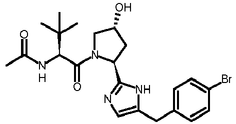 | 82 | 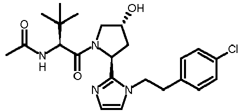 |
| 83 | 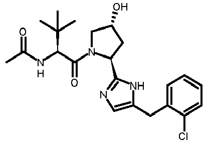 | 84 | 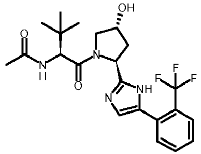 |
| 85 | 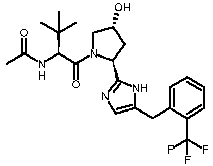 | 86 | 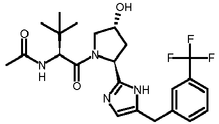 |
| 87 | 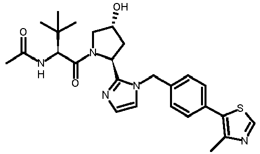 | 88 | 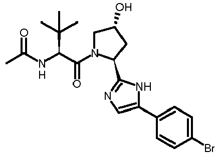 |
| 89 | 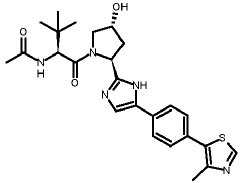 | 90 | 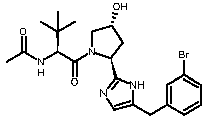 |
| 91 | 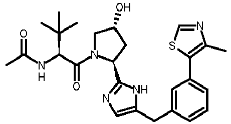 | 92 | 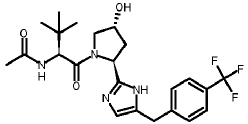 |

**[Table 2-9]**

| Ex. No. | structural formula | Ex. No. | structural formula |
|---|---|---|---|
| 93 | 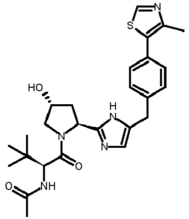 | 94 | 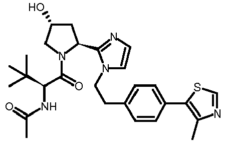 |
| 95 | 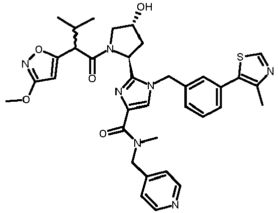 | 96 | 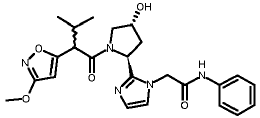 |
| 97 | 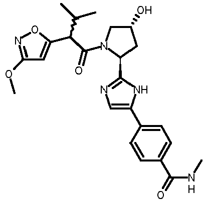 | 98 | 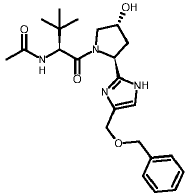 |
| 99 | 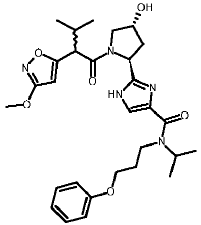 | 100 | 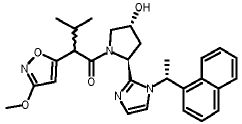 |
| 101 | 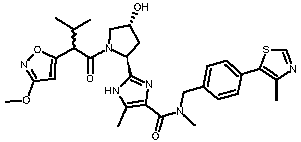 | 102 | 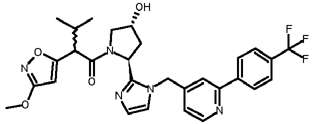 |
| 103 | 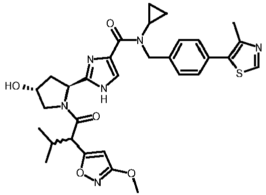 | 104 | 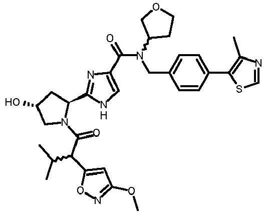 |

**[Table 2-10]**

| Ex. No. | structural formula | Ex. No. | structural formula |
|---|---|---|---|
| 105 | 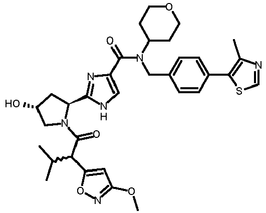 | 106 | 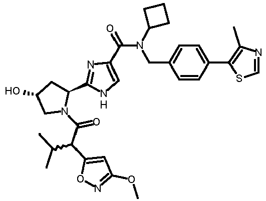 |
| 107 | 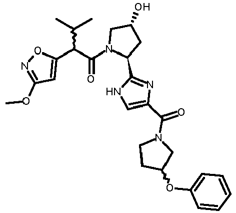 | 108 | 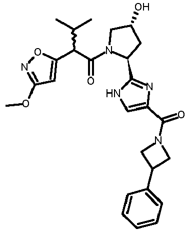 |
| 109 | 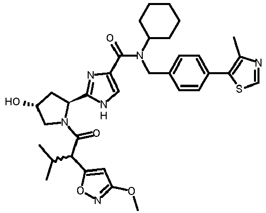 | 110 | 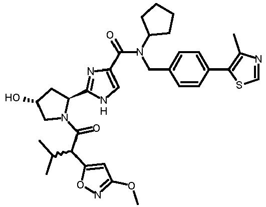 |
| 111 | 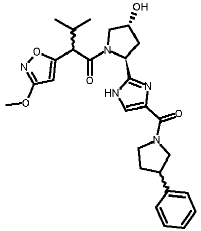 | 112 | 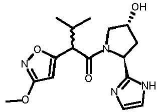 |
| 113 | 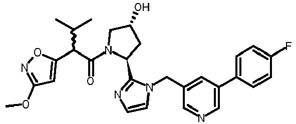 | 114 | 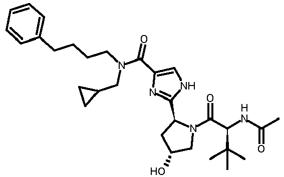 |

**[Table 2-11]**

| Ex. No. | structural formula | Ex. No. | structural formula |
|---|---|---|---|
| 115 | 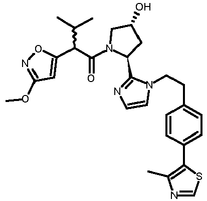 | 116 | 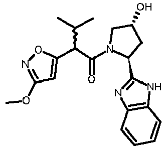 |
| 117 | 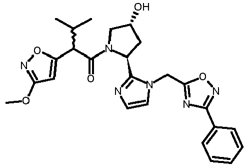 | 118 | 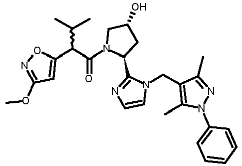 |
| 119 | 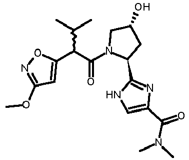 | 120 | 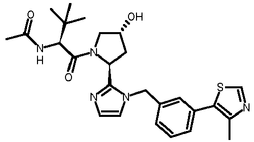 |
| 121 | 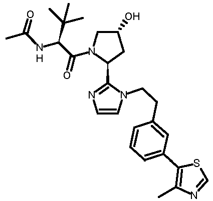 | 122 | 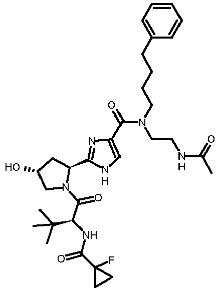 |
| 123 | 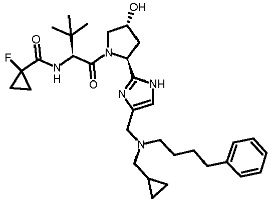 | 124 | 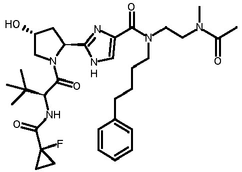 |
| 125 | 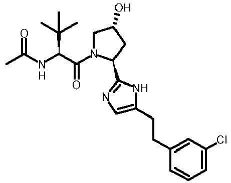 | 126 | 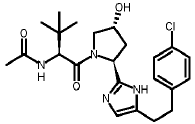 |

**[Table 2-12]**

| Ex. No. | structural formula | Ex. No. | structural formula |
|---|---|---|---|
| 127 | 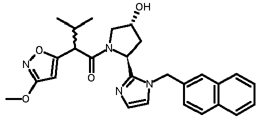 | 128 | 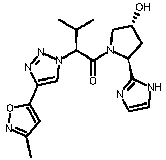 |
| 129 | 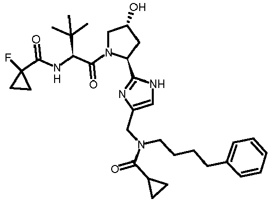 | 130 | 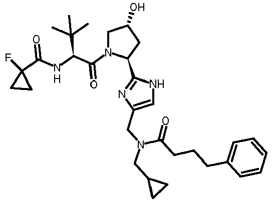 |
| 131 | 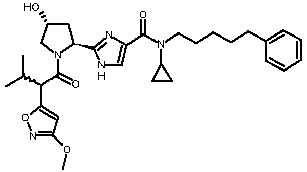 | 132 | 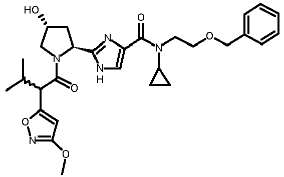 |
| 133 | 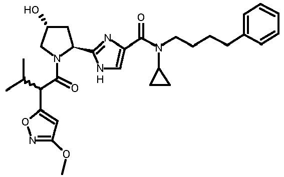 | 134 | 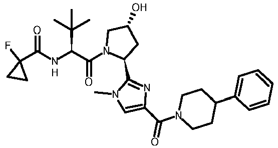 |
| 135 | 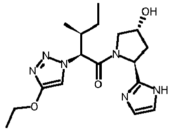 | 136 | 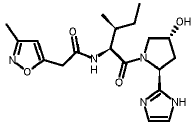 |
| 137 | 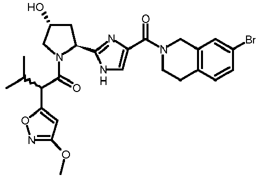 | 138 | 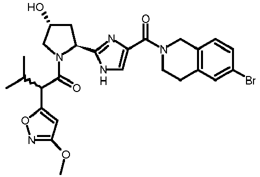 |
| 139 | 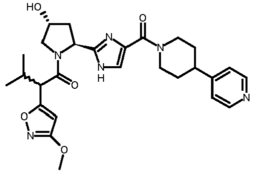 | 140 | 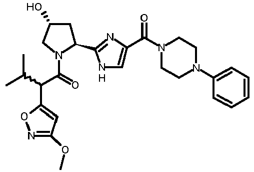 |

**[Table 2-13]**

| Ex. No. | structural formula | Ex. No. | structural formula |
|---|---|---|---|
| 141 | 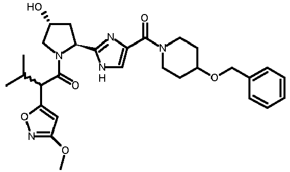 | 142 | 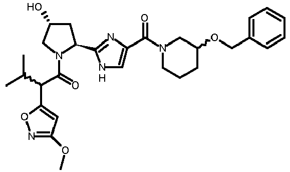 |
| 143 | 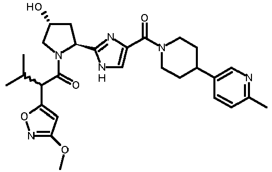 | 144 | 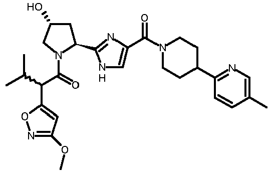 |
| 145 | 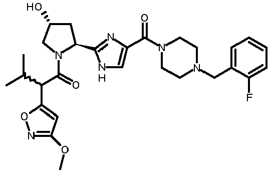 | 146 | 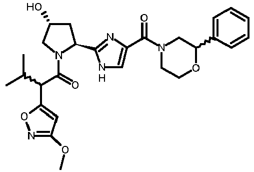 |
| 147 | 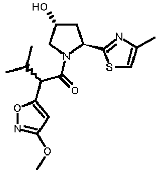 | 148 | 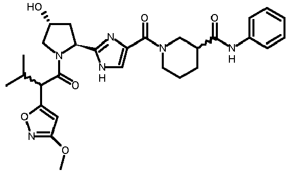 |
| 149 | 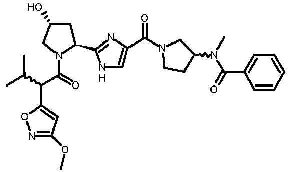 | 150 | 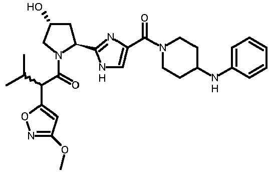 |
| 151 | 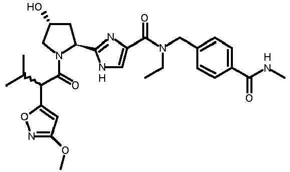 | 152 | 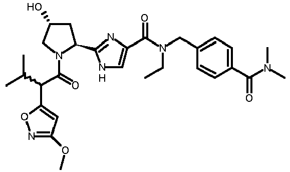 |
| 153 | 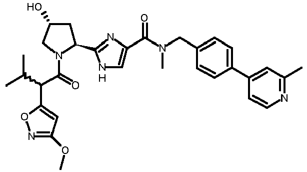 | 154 | 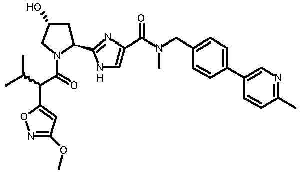 |

**[Table 2-14]**

| Ex. No. | structural formula | Ex. No. | structural formula |
|---|---|---|---|
| 155 | 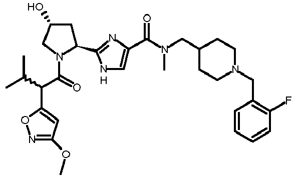 | 156 | 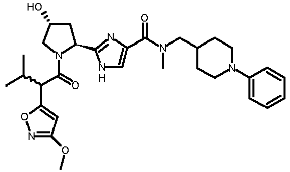 |
| 157 | 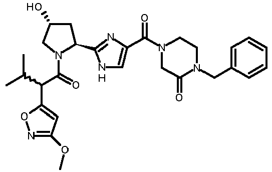 | 158 | 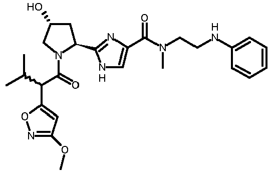 |
| 159 | 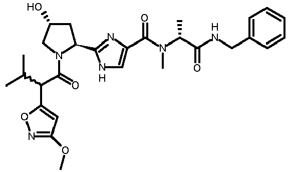 | 160 | 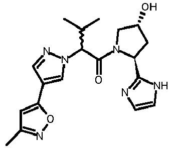 |
| 161 | 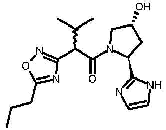 | 162 | 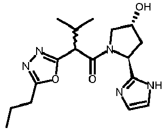 |

Example 163: Synthesis of N-(cyclopropylmethyl)-2-[(2S,4R)-4-hydroxy-1-[(2S)-2-[[2-[2-[2-[2-[4-[(6-isopropoxy-1,3-dimethyl-2-oxo-benzimidazol-5-yl)carbamoyl]-3-methoxy-phenoxy]ethoxy]ethoxy]ethoxy]acetoyl]amino]-3,3-dimethylbutanoyl]pyrrolidin-2-yl]-N-(4-phenylbutyl)-1H-imidazole-4-carboxamide

To a solution of Reference Example compound 181 (40 mg) in DMF (1 mL) were added N-methylmorpholine (0.027 mL), HOBt (13 mg), and EDCI (24 mg) and the mixture was stirred at room temperature for 0.5 hr. Reference Example 182 (50 mg) was added and the mixture was further stirred for 0.5 hr. After completion of the reaction, water was added, and the mixture was extracted with ethyl acetate. The organic layer was dried over anhydrous sodium sulfate, filtered, and concentrated under reduced pressure. The obtained residue was purified by preparative HPLC (water (0.1%TFA)-acetonitrile) to give the title compound (76 mg) as a yellow solid.
MS(ESI)m/z:1053.8[M+H]⁺

### Example 164: Synthesis of N-[5-[4-[2-[[2-[2-[[(1S)-1-[(2S,4R)-2-[4-[cyclopropylmethyl(4-phenylbutyl)carbamoyl]-1H-imidazol-2-yl]-4-hydroxypyrrolidine-1-carbonyl]-2,2-dimethylpropyl]amino]-2-oxo-ethoxy]acetoyl]amino]ethoxy]phenyl]-4-fluoro-2-[(3R*,5S*)-3,4,5-trimethylpiperazin-1-yl]phenyl]-6-oxo-4-(trifluoromethyl)-1H-pyridine-3-carboxamide

### (164-1): 2-[2-[[(1S)-1-[(2S,4R)-2-[4-[cyclopropylmethyl(4-phenylbutyl)carbamoyl]-1H-imidazol-2-yl]-4-hydroxypyrrolidine-1-carbonyl]-2,2-dimethyl-propyl]amino]-2-oxo-ethoxy]acetic acid (Example compound 164-1)

To a mixture of Reference Example compound 181 (42 mg), DMF (2.0 mL), DIPEA (0.29 mL), and 2-(2-tertbutoxy -2-oxo-ethoxy) acetic acid (17 mg) was added HATU (33 mg) under ice-cooling and the mixture was stirred at room temperature for 1 hr. To the reaction mixture was added water, and the mixture was extracted with ethyl acetate, dried over anhydrous sodium sulfate, and the solvent was evaporated under reduced pressure. The obtained residue was purified by silica gel column chromatography to give the title compound (39 mg) as a colorless oil. MS(ESI)m/z:612.5[M+H]⁺

### (164-2):N-[5-[4-[2-[[2-[2-[[(1S)-1-[(2S,4R)-2-[4-[cyclopropylmethyl(4-phenylbutyl)carbamoyl]-1H-imidazol-2-yl]-4-hydroxypyrrolidine-1-carbonyl]-2,2-dimethylpropyl]amino]-2-oxo-ethoxy]acetoyl]amino]ethoxy]phenyl]-4-fluoro-2-[(3R*,5S*)-3,4,5-trimethylpiperazin-1-yl]phenyl]-6-oxo-4-(trifluoromethyl)-1H-pyridine-3-carboxamide (Example 164)

To a solution of Example compound 164-1 (39 mg), DMF (1.5 mL), DIPEA (0.145 mL), and Reference Example compound 180 (54 mg) was added HATU (33 mg) under ice-cooling, and the mixture was stirred at room temperature for 3 hr. To the reaction mixture was added water and the mixture was extracted with chloroform. The organic layer was dried over anhydrous sodium sulfate, filtered, and the solvent was evaporated under reduced pressure. To a mixture of the obtained residue and chloroform (2 mL) was added TFA (1.0 mL) under ice-cooling and the mixture was stirred for 3 hr. The reaction mixture was diluted with chloroform and toluene, the solvent was evaporated under reduced pressure. The obtained residue was purified by preparative HPLC (10 mM ammonium carbonate aqueous solution-acetonitrile) to give the title compound (6 mg) as a pale-yellow solid.
MS(ESI)m/z:1155.7[M+H]⁺

### Example 165: Synthesis of N-[2-[4-[2-fluoro-5-[[6-oxo-4-(trifluoromethyl)-1H-pyridine-3-carbonyl]amino]-4-[(3R*,5S*)-3, 4, 5-trimethylpiperazin-1-yl]phenyl]phenoxy]ethyl]-N'-[(1S)-1-[(2S,4R)-4-hydroxy-2-[5-[3-(4-methylthiazol-5-yl)phenyl]-1H-imidazol-2-yl]pyrrolidine-1-carbonyl]-2,2-dimethylpropyl]pentanediamide

To a solution of Reference Example compound 183 (25 mg) in ethanol (2 mL) was added 10%Pd/C (100 mg), and the mixture was stirred under hydrogen atmosphere at 60°C for 8 hr Insoluble material in the reaction mixture was filtered off through celite, and the filtrate was concentrated under reduced pressure. The obtained residue was purified by preparative HPLC (10 mM ammonium carbonate aqueous solution-acetonitrile) to give a colorless oil (8 mg). Using the obtained oil, the title compound (14 mg) was obtained as a pale-yellow solid by the same reaction and treatment as in Example 164 (164-2). MS(ESI)m/z:1097.4[M+H]⁺

**[Table 2-15]**

| Ex. No. | structural formula | Ex. No. | structural formula |
|---|---|---|---|
| 163 | 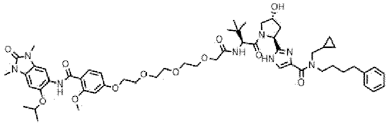 | 164 | 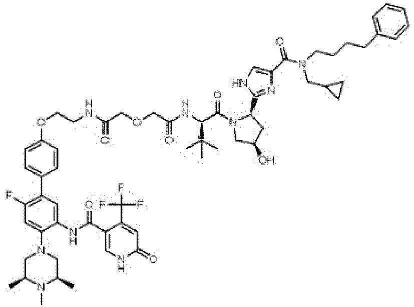 |
| 165 | 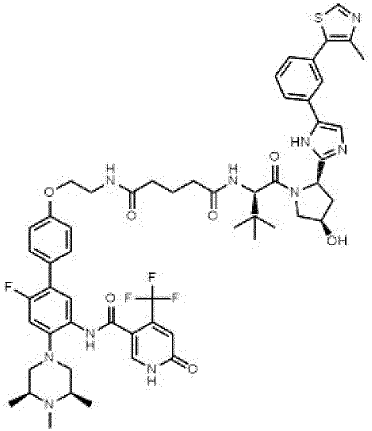 | | |

### [Experimental Example]

### Experimental Example 1: Measurement of intermolecular interactions using surface plasmon resonance (SPR)

As the device, Biacore T200 (manufactured by GE Healthcare) was used. VHL was obtained by co-expressing Elongin B and Elongin C with a Twin-Strep-tag added to the N-terminus. As the sensor chip, Sensor chip CM5 (manufactured by GE Healthcare) was used. First, Strep-Tactin XT (manufactured by GE Healthcare) was immobilized on the sensor chip by amine coupling. Thereafter, VHL was immobilized using the affinity of Strep-Tactin XT and Twin-Strep-tag, and VHL was further immobilized on the sensor chip by amine coupling.

As the running buffer, a mixed solution of 20 mM HEPES pH 7.5, 150 mM NaCl, 1 mM TCEP, 0.005% Tween 20, and 2% DMSO was used. Solutions of various compound concentrations of the test compounds (compounds described in Examples 1 to 162 above) were flowed on the sensor chip with VHL immobilized thereon at a flow rate of 30 uL/min under 25°C conditions, and changes in mass on the sensor chip were observed. The Contact time and the Dissociation time were determined according to the properties of the compounds. For many compounds, 60 seconds and 120 seconds were respectively used. The observed data was analyzed using Biacore T200 Evaluation software Version 2.0 (manufactured by GE Healthcare) and the Kd value for VHL of each compound was calculated.

The test results of this Experiment are shown in Table 3 below. In the Table, "***" indicates a Kd value of less than 1 µM, "**" indicates a Kd value of not less than 1 µM and less than 10 µM, and "*" indicates a Kd value of not less than 10 µM and less than 50 µM.

### [Table 3]

**Table 3: Kd value of each Example compound**

| Example No. | Kd | Example No. | Kd |
|---|---|---|---|
| Example 1 | ** | Example 6 | ** |
| Example 14 | ** | Example 15 | ** |
| Example 16 | ** | Example 17 | ** |
| Example 18 | ** | Example 19 | ** |
| Example 20 | ** | Example 21 | ** |
| Example 22 | ** | Example 23 | ** |
| Example 24 | ** | Example 25 | ** |
| Example 29 | ** | Example 30 | ** |
| Example 31 | ** | Example 35 | ** |
| Example 40 | ** | Example 44 | *** |
| Example 48 | ** | Example 49 | ** |
| Example 51 | ** | Example 53 | ** |
| Example 54 | *** | Example 61 | ** |
| Example 62 | ** | Example 63 | ** |
| Example 64 | ** | Example 65 | * |
| Example 66 | * | Example 69 | * |
| Example 74 | ** | Example 75 | ** |
| Example 76 | ** | Example 78 | ** |
| Example 79 | ** | Example 80 | ** |
| Example 81 | ** | Example 84 | ** |
| Example 89 | ** | Example 90 | ** |
| Example 91 | ** | Example 93 | ** |
| Example 96 | * | Example 97 | ** |
| Example 102 | * | Example 107 | *** |
| Example 108 | *** | Example 111 | *** |
| Example 112 | ** | Example 113 | * |
| Example 139 | ** | Example 140 | ** |
| Example 141 | ** | Example 144 | ** |
| Example 147 | * | Example 148 | ** |
| Example 150 | ** | Example 153 | *** |
| Example 154 | *** | Example 155 | ** |
| Example 158 | *** | Example 159 | *** |
| Example 160 | * | Example 161 | * |
| Example 162 | * | | |

### Experimental Example 2: Protein degradation-inducing action in cancer cells

Using Example compounds 164 and 165, the degradation-inducing action on WDR5 protein in cancer cells was evaluated using LNCaP cell line which is a cell line derived from human prostate cancer. As a control, GAPDH was used. The test results are shown in Fig. 1.

Cell culture LNCaP cell line was seeded in an evaluation plate in DMEM culture medium containing 10% bovine serum (FBS). A test compound was dissolved in DMSO, a dilution series was prepared, mixed with a culture medium, added to an evaluation plate, and cultured for one day and one night to expose the cells to the compound. After the above-mentioned culture, the cells were collected and lysed using a cell lysing solution (RIPA buffer) containing a protease inhibitor (Halt Protease Inhibitor Cock tailt). This cell lysate was dissolved in an electrophoresis buffer (NuPAGE LDS Sample Buffer, manufactured by ThermoFischer) added with a reducing agent (NuPAGE Sample Reducing Agent, manufactured by ThermoFischer). After a high temperature treatment (95°C, 5 min) of the lysate, NuPAGE electrophoresis was performed. Proteins in the NuPAGE gel were transferred to a PVDF membrane by using a semi-dry transfer device (iBlot2 system, manufactured by ThermoFischer). After the transfer, Western blotting was performed using iBind Western Systems (manufactured by ThermoFischer). In practice, after blocking with iBind Solution adjusted to 1X, the membrane was placed in the iBind Western Device, primary antibody (antihuman WDR5 protein rabbit antibody, manufactured by Cell Signaling) solution, secondary antibody (anti-rabbit WDR5 protein labeled antibody, manufactured by Cell Signaling), and 1XiBind Solution were added to a designated place and allowed to stand at room temperature for 2 hr 30 min. The membrane after practice was washed with ultrapure water (water purified with a MilliQ device, manufactured by Merck Millipore), an antibody-dependent chemiluminescence was performed with a coloring solution for HRP (Immobilon Western Chemiluminescent Substrate, manufactured by Merck Millipore), and a band corresponding to WDR5 protein on the membrane was detected using an image analyzer (Amersham Imager 600, manufactured by Amersham).

### Experimental Example 3: Hibit assay

Using Example compound 163, a Hibit assay was performed. The test results are shown in Fig. 2.

A BRPF1 protein-expressing plasmid vector added with an 11-amino acid peptide tag Hibit was introduced into the cells by using an introduction reagent such as Lipofectamine and the like. Thereafter, a test compound was diluted with a culture medium and added. After culturing for a while, the amount of Hibit-added protein was quantified by measuring the luminescent intensity with a plate reader or the like using Nano-Glo HiBiT Lytic Detection System.

### [Industrial Applicability]

The present invention provides a target protein degradation-inducing compound that is a bifunctional compound having a portion that binds to Von-Hippel-Lindau, which is a substrate recognition protein of a ubiquitin ligase complex constituted of low-molecular-weight compounds, at one end, and a portion that is capable of binding or binds to a target protein at the other end.

This application is based on a patent application No. 2020-065410 filed in Japan (filing date: March 31, 2020), the contents of which are incorporated in full herein.

## Claims

1. A compound represented by the following structural formula (I): 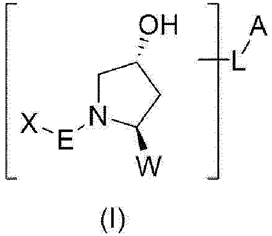
wherein L is bonded to either W or X;
E is a bond, -CO-, -SO- or -SO₂-;
X is an optionally substituted alkyl group,
an optionally substituted cycloalkyl group,
an optionally substituted aryl group,
an optionally substituted heterocyclic group, or
an optionally substituted heteroaryl group;
W is an optionally substituted aryl group,
an optionally substituted fused heterocyclic group containing 1 to 6 same or different atoms selected from a nitrogen atom, an oxygen atom, and a sulfur atom and having 6 to 10 ring-constituting atoms,
an optionally substituted heteroaryl group containing 1 to 6 same or different atoms selected from a nitrogen atom, an oxygen atom, and a sulfur atom and having 3 to 10 ring-constituting atoms,
an alkyl group optionally substituted by a group selected from a halogen atom, a hydroxy group, an alkoxy group, and a heterocyclic group,
a cyano group, or a hydrogen atom;
L is a bond or a chemical linker; and
A is a target-directed ligand,
or a pharmacologically acceptable salt thereof.

2. The compound according to claim 1, wherein W is an optionally substituted fused heterocyclic group containing 1 to 6 same or different atoms selected from a nitrogen atom, an oxygen atom, and a sulfur atom and having 6 to 10 ring-constituting atoms,
or a pharmacologically acceptable salt thereof.

3. The compound according to claim 1 or 2, wherein W is a group represented by the formula: 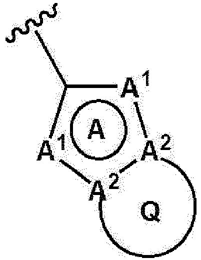 wherein
group A is an optionally substituted heteroaryl group having five ring-constituting atoms, two A¹ are each independently a group or atom selected from CR^{Z1}, N, NR^{Z2}, O, and S,
two A² are each independently C or N; and
ring Q is
an aromatic hydrocarbocycle,
an aromatic heterocycle optionally containing 1 to 6 same or
different atoms selected from a nitrogen atom, an oxygen atom,
and a sulfur atom,
a non-aromatic hydrocarbocycle, or
a heterocycle optionally containing 1 to 6 same or different atoms selected from a nitrogen atom, an oxygen atom, and a sulfur atom,
wherein the ring has 5 to 7 ring-constituting atoms and is optionally substituted at a substitutable position by a group selected from
a halogen atom,
a hydroxy group,
a cyano group,
a hydroxycarbonyl group,
an oxo group,
a thioxo group,
an optionally substituted alkyl group,
an optionally substituted cycloalkyl group,
an optionally substituted alkoxy group,
an optionally substituted cycloalkyloxy group,
-CD-N(R^{7a})(R^{7b}),
-N(R^{7a})(R^{7b}),
-N (R^{7c})-CO-R^{7d}, and
-CO-R^{7e},
(in each of the above-mentioned formulas,
R^{Z1} and R^{Z2} are each independently a hydrogen atom; a halogen atom; a hydroxy group; a cyano group; a hydroxycarbonyl group; an optionally substituted alkyl group; an optionally substituted cycloalkyl group; an optionally substituted alkoxy group; an optionally substituted alkoxycarbonyl group; - CO-N(R^{7a})(R^{7b}); -N(R^{7a})(R^{7b}); -N (R^{7c})-CO-R^{7d}; an aryl group; or an optionally substituted heteroaryl group containing 1 to 3 same or different atoms selected from a nitrogen atom, an oxygen atom, and a sulfur atom and having 5 or 6 ring-constituting atoms (provided that R^{Z2} is not a halogen atom; hydroxy group; cyano group; hydroxycarbonyl group; -N (R^{7a})(R^{7b}), or - N (R^{7c})-CO-R^{7d});
R^{7a} and R^{7b} are each independently a hydrogen atom; or an alkyl group optionally substituted by a group selected from a halogen atom, a hydroxy group, an alkoxy group, and a cyano group, or
R^{7a} and R^{7b} are optionally bonded to each other to form, together with the adjacent nitrogen atom, a heterocycle optionally substituted by a group selected from a halogen atom, a hydroxy group, an alkoxy group, and a cyano group;
R^{7c} and R^{7d} are each independently a hydrogen atom; or an alkyl group optionally substituted by a group selected from a halogen atom, a hydroxy group, an alkoxy group, and a cyano group; and
R^{7e} is an optionally substituted alkyl group, an optionally substituted cycloalkyl group, an optionally substituted aryl group, or an optionally substituted heteroaryl group),
or a pharmacologically acceptable salt thereof.

4. The compound according to any one of claims 1 to 3, wherein W is a group represented by the formula: 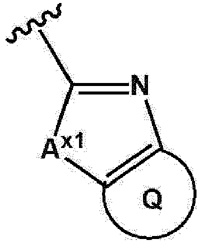 wherein
A^{x1} is a group or atom selected from -NR^{Z2}-, -O-, and -S-,
ring Q is a non-aromatic hydrocarbocycle having 5 or 6 ring-constituting atoms; or
a heterocycle optionally containing 1 to 4 same or different atoms selected from a nitrogen atom, an oxygen atom, and a sulfur atom and having 5 or 6 ring-constituting atoms, wherein the ring is optionally substituted at a substitutable position by a group selected from a halogen atom, an alkyl group having 1 to 6 carbon atoms, and -CO-R^{7e},
wherein R^{z2} and R^{7e} are as defined above,
or a pharmacologically acceptable salt thereof.

5. The compound according to claim 1, wherein W is an optionally substituted heteroaryl group containing 1 to 6 same or different atoms selected from a nitrogen atom, an oxygen atom, and a sulfur atom and having 3 to 10 ring-constituting atoms, or a pharmacologically acceptable salt thereof.

6. The compound according to claim 1 or 5, wherein W is a group represented by the formula: 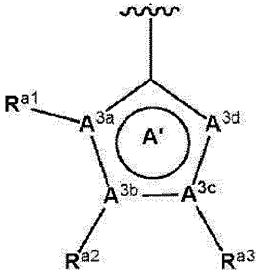 wherein
group A' is a heteroaryl group having 5 ring-constituting atoms, A^{3a}, A^{3b}, A^{3c}, and A^{3d} are each independently, an atom selected from a nitrogen atom, an oxygen atom, a carbon atom, and a sulfur atom, when A^{3a}, A^{3b}, and A^{3c} have a nitrogen atom or a carbon atom, they respectively have R^{a1}, R^{a2}, and R^{a3},
wherein R^{a1} is
(1) a hydrogen atom,
(2) an optionally substituted alkyl group,
(3) an optionally substituted cycloalkyl group,
(4) an optionally substituted aryl group,
(5) an optionally substituted heteroaryl group, or
(6) an optionally substituted heterocyclic group, and
R^{a2} and R^{a3} are each independently
(1) a hydrogen atom,
(2) -CN,
(3) a halogen atom,
(4) an optionally substituted alkyl group,
(5) an optionally substituted cycloalkyl group,
(6) an optionally substituted aryl group,
(7) an optionally substituted heteroaryl group,
(8) an optionally substituted heterocyclic group,
(9) -V^{a3a}-(optionally substituted alkyl),
(10) -V^{a3a}-(optionally substituted cycloalkyl),
(11) -V^{a3a}-(optionally substituted aryl),
(12) -V^{a3a}-(optionally substituted heteroaryl), or
(13) -V^{a3a}-(optionally substituted heterocyclic group)
(in the above-mentioned formulas, V^{a3a} is
1) -CO-,
2) -NR^{Va3}-
wherein R^{Va3} is
(a) a hydrogen atom,
(b) an optionally substituted alkyl group having 1 to 6 carbon atoms, or
(c) an optionally substituted cycloalkyl group having 3 to 6 carbon atoms,
3) -O-,
4) -S-,
5) -SO-, or
6) -SO₂-, or
(14) -V^{a3b}-NR^{Na3}R^{Na3'}
wherein V^{a3b} is
1) -CO-
2) -SO-, or
3) -SO₂-, and
R^{Na3} and R^{Na3}' are each independently
1) a hydrogen atom,
2) an optionally substituted alkyl group,
3) an optionally substituted cycloalkyl group,
4) an optionally substituted aryl group,
5) an optionally substituted heteroaryl group, or
6) an optionally substituted heterocyclic group,
or a pharmacologically acceptable salt thereof.

7. The compound according to claim 6, wherein the group A' is an imidazolyl group, a pyrazolyl group, a thiazolyl group, an isothiazolyl group, a thiadiazolyl group, an isothiadiazolyl group, an oxazolyl group, an isoxazolyl group, an oxadiazolyl group, a triazolyl group, or a tetrazolyl group, or a pharmacologically acceptable salt thereof.

8. The compound according to claim 7, wherein the group A' is a group selected from an imidazolyl group, a thiazolyl group, and an oxazolyl group, or a pharmacologically acceptable salt thereof.

9. The compound according to any one of claims 1, 5 to 8, wherein
W is a group represented by the formula: 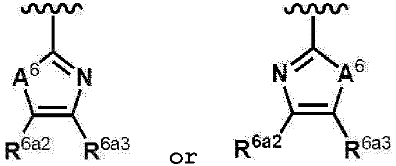 wherein
A⁶ is -O-, -S-, or -NR^{6a1}-
wherein R^{6a1} is
(1) a hydrogen atom,
(2) an optionally substituted alkyl group,
(3) an optionally substituted cycloalkyl group,
(4) an optionally substituted aryl group,
(5) an optionally substituted heteroaryl group, or
(6) an optionally substituted heterocyclic group;
R6a2 is
(1) a hydrogen atom,
(2) CN,
(3) a halogen atom,
(4) an optionally substituted alkyl group having 1 to 4 carbon atoms,
(5) an optionally substituted cyclopropyl group,
(6) an optionally substituted oxetanyl group,
(7) an optionally substituted azetidinyl group,
(8) an optionally substituted aryl group having 6 to 10 carbon atoms, or
(9) -CO-NR^{a2N6}R^{a2N6'}
wherein R^{a2N6} and R^{a2N6'} are each independently
1) a hydrogen atom,
2) an optionally substituted alkyl group having 1 to 4 carbon atoms,
3) an optionally substituted cyclopropyl group,
4) an optionally substituted oxetanyl group, or
5) an optionally substituted azetidinyl group; and
R^{6a3} is
(1) a hydrogen atom,
(2) -CN,
(3) a halogen atom,
(4) an optionally substituted alkyl group,
(5) an optionally substituted cycloalkyl group,
(6) an optionally substituted aryl group,
(7) an optionally substituted heteroaryl group,
(8) an optionally substituted heterocyclic group,
(9) -CO-(optionally substituted heterocyclic group), or
(10) -CD-NR^{a3N6}R^{a3N6'}
wherein R^{a3N6} and R^{a3N6'} are each independently
1) a hydrogen atom,
2) an optionally substituted alkyl group,
3) an optionally substituted cycloalkyl group,
4) an optionally substituted aryl group,
5) an optionally substituted heteroaryl group, or
6) an optionally substituted heterocyclic group,
or a pharmacologically acceptable salt thereof.

10. The compound according to claim 9, wherein
R^{6a1} is a hydrogen atom, and
R^{6a3} is a hydrogen atom or an alkyl group having 1 to 6 carbon atoms,
or a pharmacologically acceptable salt thereof.

11. The compound according to claim 9, wherein R^{6a1} is a hydrogen atom, and
R6a3 is
(1) -Y^{6a}-W^{6a}
wherein
Y^{6a} is
1) a bond,
2) -(CR^{Y1}R^{Y1'})_{ny6a}-A^{Y6a}-(CR^{Y2}R^{Y2'})_{ny6b}-
wherein
R^{Y1}, R^{Y1'}, R^{Y2}, and R^{Y2'} are each independently
(a) a hydrogen atom,
(b) a halogen atom,
(c) -O-(optionally substituted alkyl having 1 to 6 carbon atoms),
(d) -O-(optionally substituted cycloalkyl having 3 to 6 carbon atoms),
(e) an optionally substituted alkyl group having 1 to 6 carbon atoms, or
(f) an optionally substituted cycloalkyl group having 3 to 6 carbon atoms, or
(g) R^{Y1} and R^{Y1'}, and R^{Y2} and R^{Y2'}, are each independently optionally bonded to each other to form, together with the adjacent carbon atom, an optionally substituted cycloalkyl group having 3 to 10 carbon atoms, or an optionally substituted heterocycle containing 1 to 6 same or different atoms selected from a nitrogen atom, an oxygen atom, and a sulfur atom and having 3 to 10 ring-constituting atoms,
ny6a is an integer of 1 to 6,
A^{Y6a} is
(a) a bond,
(b) -CO-,
(c) -O-,
(d) -SO-,
(e) -SO₂-,
(f) -NR^{NY6a}-,
wherein R^{NY6a} is
(i) a hydrogen atom,
(ii) an optionally substituted alkyl group having 1 to 6 carbon atoms,
(iii) an optionally substituted cycloalkyl group having 3 to 6 carbon atoms,
(iv) an optionally substituted heterocyclic group having 3 to 6 ring-constituting atoms,
(v) -CO-(optionally substituted alkyl having 1 to 6 carbon atoms), or
(vi) -CO-(optionally substituted cycloalkyl having 3 to 6 carbon atoms), or
(g) -V^{Y6b}-NR^{NY6b}- or -NR^{NY6b}-V^{Y6b}-
wherein V^{Y6b} is
(i) -CO-,
(ii) -SO-, or
(iii) -SO₂-,
R^{NY6b} is
(i) a hydrogen atom,
(ii) an optionally substituted alkyl group having 1 to 6 carbon atoms,
(iii) an optionally substituted cycloalkyl group having 3 to 6 carbon atoms, or
(iv) an optionally substituted heterocyclic group having 3 to 6 ring-constituting atoms,
ny6b is an integer of 0 to 6, and
W^{6a} is
1) a hydrogen atom,
2) a halogen atom,
3) an optionally substituted cycloalkyl group having 3 to 10 carbon atoms,
4) an optionally substituted aryl group having 6 to 10 carbon atoms,
5) an optionally substituted heteroaryl group containing 1 to 6 same or different atoms selected from a nitrogen atom, an oxygen atom, and a sulfur atom and having 5 to 10 ring-constituting atoms, or
6) an optionally substituted heterocyclic group containing 1 to 6 same or different atoms selected from a nitrogen atom, an oxygen atom, and a sulfur atom and having 3 to 10 ring-constituting atoms,
or a pharmacologically acceptable salt thereof.

12. The compound according to claim 11, wherein
W^{6a} is
-W^{6a'}-(CR^{Y3}R^{Y3'})_{ny6c}-A^{Y6b}-(CR^{Y4}R^{Y4'})_{ny6d}-W^{6b}
wherein
W^{6a'} is
(1) an optionally substituted cycloalkyl divalent group having 3 to 10 carbon atoms,
(2) an optionally substituted aryl divalent group having 6 to 10 carbon atoms,
(3) an optionally substituted heteroaryl divalent group containing 1 to 6 same or different atoms selected from a nitrogen atom, an oxygen atom, and a sulfur atom and having 5 to 10 ring-constituting atoms, or
(4) an optionally substituted heterocyclic divalent group containing 1 to 6 same or different atoms selected from a nitrogen atom, an oxygen atom, and a sulfur atom and having 3 to 10 ring-constituting atoms,
R^{Y3}, R^{Y3'}, R^{Y4}, and R^{Y4'} are each independently
(1) a hydrogen atom,
(2) a halogen atom,
(3) -O-(optionally substituted alkyl having 1 to 6 carbon atoms),
(4) -O-(optionally substituted cycloalkyl having 3 to 6 carbon atoms),
(5) an optionally substituted alkyl group having 1 to 6 carbon atoms,
(6) an optionally substituted cycloalkyl group having 3 to 6 carbon atoms, or
(7) R^{Y3} and R^{Y3'}, and R^{Y4} and R^{Y4'}, are each independently optionally bonded to each other to form, together with the adjacent carbon atom, an optionally substituted cycloalkyl group having 3 to 10 carbon atoms, or an optionally substituted heterocycle containing 1 to 6 same or different atoms selected from a nitrogen atom, an oxygen atom, and a sulfur atom and having 3 to 10 ring-constituting atoms,
A^{Y6b} is
(1) a bond,
(2) -O-,
(3) -SO-,
(4) -SO₂-,
(5) -NR^{NY6c}-,
wherein R^{NY6c} is
1) a hydrogen atom,
2) an optionally substituted alkyl group having 1 to 6 carbon atoms,
3) an optionally substituted cycloalkyl group having 3 to 6 carbon atoms,
4) an optionally substituted heterocyclic group having 3 to 6 carbon atoms,
5) -CO-(optionally substituted alkyl having 1 to 6 carbon atoms), or
6) -CO-(optionally substituted cycloalkyl having 3 to 6 carbon atoms), or .(6) -V^{Y6d}-NR^{NY6d}- or -NR^{NY6d}-V^{Y6d}-
wherein V^{Y6d} is
(a) -CO-,
(b) -SO-, or
(c) -SO₂-, and
R^{NY6d} is
1) a hydrogen atom,
2) an optionally substituted alkyl group having 1 to 6 carbon atoms,
3) an optionally substituted cycloalkyl group having 3 to 6 carbon atoms, or
4) an optionally substituted heterocyclic group having 3 to 6 carbon atoms,
ny6c and ny6d are each independently an integer of 0 to 6,
W^{6b} is
(1) a hydrogen atom,
(2) a halogen atom,
(3) an optionally substituted aryl group having 6 to 10 carbon atoms,
(4) an optionally substituted heteroaryl group containing 1 to 6 same or different atoms selected from a nitrogen atom, an oxygen atom, and a sulfur atom and having 5 to 10 ring-constituting atoms,
(5) an optionally substituted cycloalkyl group having 3 to 10 carbon atoms,
(6) an optionally substituted heterocyclic group containing 1 to 6 same or different atoms selected from a nitrogen atom, an oxygen atom, and a sulfur atom and having 3 to 10 ring-constituting atoms,
(7) -CO-(optionally substituted heterocyclic group containing 1 to 6 same or different atoms selected from a nitrogen atom, an oxygen atom, and a sulfur atom and having 3 to 10 ring-constituting atoms),
(8) -CONR^{NW6}R^{NW6'}
wherein R^{NW6} and R^{NW6'} are each independently
1) a hydrogen atom,
2) an optionally substituted alkyl group having 1 to 6 carbon atoms, or
3) an optionally substituted cycloalkyl group having 3 to 6 carbon atoms, or
(9) -NR^{NW6"}R^{NW6‴}
wherein R^{NW6"} and R^{NW6‴} are each independently
1) a hydrogen atom,
2) an optionally substituted alkyl group having 1 to 6 carbon atoms,
3) an optionally substituted cycloalkyl group having 3 to 6 carbon atoms,
4) an optionally substituted heterocyclic group having 3 to 6 carbon atoms,
5) -CO-(optionally substituted alkyl having 1 to 6 carbon atoms), or
6) -CO-(optionally substituted cycloalkyl having 3 to 6 carbon atoms),
or a pharmacologically acceptable salt thereof.

13. The compound according to claim 12, wherein
ny6c and ny6d are both 0, and
A^{Y6b} is a bond, or a pharmacologically acceptable salt thereof.

14. The compound according to claim 9, wherein
R6a1 is
(1) a hydrogen atom, or
(2) an optionally substituted alkyl group,
R^{6a2} is
(1) a hydrogen atom,
(2) CN,
(3) a halogen atom,
(4) an optionally substituted alkyl group having 1 to 4 carbon atoms,
(5) an optionally substituted cyclopropyl group,
(6) an optionally substituted oxetanyl group,
(7) an optionally substituted azetidinyl group, or
(8) an optionally substituted aryl group having 6 to 10 carbon atoms,
R^{6a3} is a group represented by the formula:
-CD-R^{9a}-W^{9a}-Y^{9b}-W^{9b}
wherein
R^{9a} is
(1) a group represented by the formula: 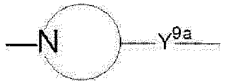 wherein 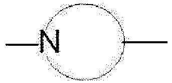 is
a heterocyclic group containing a nitrogen atom, optionally further containing 1 to 5 same or different atoms selected from a nitrogen atom, an oxygen atom, and a sulfur atom, and having 3 to 10 ring-constituting atoms, which is optionally further substituted in addition to Y^{9a}, and
Y^{9a} is
1) -(CR^{Y5}R^{Y5'})_{ny9a}-A^{Y9a}-(CR^{Y6}R^{Y6'})_{ny9b}-
wherein R^{Y5}, R^{Y5'}, R^{Y6}, and R^{Y6'} are each independently
(a) a hydrogen atom,
(b) a halogen atom,
(c) -O-(optionally substituted alkyl having 1 to 6 carbon atoms),
(d) -O-(optionally substituted cycloalkyl having 3 to 6 carbon atoms),
(e) an optionally substituted alkyl group having 1 to 6 carbon atoms,
(f) an optionally substituted cycloalkyl group having 3 to 6 carbon atoms, or
(g) R^{Y5} and R^{Y5'}, and R^{Y6} and R^{Y6'}, are each independently optionally bonded to each other to form, together with the adjacent carbon atom, an optionally substituted cycloalkyl group having 3 to 10 carbon atoms, or an optionally substituted heterocycle containing 1 to 6 same or different atoms selected from a nitrogen atom, an oxygen atom, and a sulfur atom and having 3 to 10 ring-constituting atoms;
ny9a and ny9b are each independently an integer of 0 to 6;
A^{Y9a} is
(a) a bond,
(b) -O-,
(c) -SO-,
(d) -SO₂-,
(e) -NR^{NY9c}-
wherein R^{NY9c} is
(i) a hydrogen atom,
(ii) an optionally substituted alkyl group having 1 to 6 carbon atoms,
(iii) an optionally substituted cycloalkyl group having 3 to 6 carbon atoms,
(iv) an optionally substituted heterocyclic group having 3 to 6 ring-constituting atoms,
(v) -CO-(optionally substituted alkyl having 1 to 6 carbon atoms), or
(vi) -CO-(optionally substituted cycloalkyl having 3 to 6 carbon atoms),
(f) -V^{Y9d}-NR^{NY9d}- or -NR^{NY9d}-V^{Y9d}-
wherein V^{Y9d} is
(i) -CO-,
(ii) -SO-, or
(iii) -SO₂-, and
R^{NY9d} is
(i) a hydrogen atom,
(ii) an optionally substituted alkyl group having 1 to 6 carbon atoms,
(iii) an optionally substituted cycloalkyl group having 3 to 6 carbon atoms, or
(iv) an optionally substituted heterocyclic group having 3 to 6 ring-constituting atoms, or
(2) a group represented by the formula: -NR^{N9}-, -NR^{N9}- (CR^{Y7}R^{Y7'})ₙ₉-,
-NR^{N9}-(CR^{Y7}R^{Y7'})ₙ₉-V⁹-(CR^{Y8}R^{Y8'})ₘ₉-,
-NR^{N9}-(CR^{Y7}R^{Y7'})ₙ₉-V⁹-NR^{N9'}-(CR^{Y8}R^{Y8'})ₘ₉-,
-NR^{N9}-(CR^{Y7}R^{Y7'})ₒ₉-NR^{N9'}-V⁹-(CR^{Y8}R^{Y8'})ₘ₉-,
-NR^{N9}-(CR^{Y7}R^{Y7'})ₒ₉-NR^{N9"}-(CR^{Y8}R^{Y8'})ₘ₉-, -NR^{N9}-(CR^{Y7}R^{Y7'})ₒ₉-O-(CR^{Y8}R^{Y8'})ₘ₉-,
or
-NR^{N9}-(CR^{Y7}R^{Y7'})ₒ₉-S-(CR^{Y8}R^{Y8'})ₘ₉-
in the above-mentioned formulas,
R^{N9} is
1) a hydrogen atom,
2) -(CR^{N9a}R^{N9a'})_{nN9a}-Y^{N9}-W^{N9}
wherein R^{N9a} and R^{N9a'} are each independently
(a) a hydrogen atom,
(b) a halogen atom,
(c) -O-(optionally substituted alkyl having 1 to 6 carbon atoms),
(d) -O-(optionally substituted cycloalkyl having 3 to 6 carbon atoms),
(e) an optionally substituted alkyl group having 1 to 6 carbon atoms,
(f) an optionally substituted cycloalkyl group having 3 to 6 carbon atoms, or
(g) R^{N9a} and R^{N9a'} are optionally bonded to each other to form, together with the adjacent carbon atom, an optionally substituted cycloalkyl group having 3 to 10 carbon atoms, or
(h) an optionally substituted heterocyclic group containing 1 to 6 same or different atoms selected from a nitrogen atom, an oxygen atom, and a sulfur atom and having 3 to 10 ring-constituting atoms;
nN9a is an integer of 1 to 6;
Y^{N9} is
(a) a bond-,
(b) -O-,
(c) -SO-,
(d) -SO₂-,
(e) -NR^{N9b}-
wherein R^{N9b} is
(i) a hydrogen atom,
(ii) an optionally substituted alkyl group having 1 to 6 carbon atoms,
(iii) an optionally substituted cycloalkyl group having 3 to 6 carbon atoms,
(iv) an optionally substituted heterocyclic group having 3 to 6 ring-constituting atoms,
(v) -CO-(optionally substituted alkyl having 1 to 6 carbon atoms), or
(vi) -CO-(optionally substituted cycloalkyl having 3 to 6 carbon atoms, or
(f) -V^{N9c}-NR^{N9c}- or -NR^{N9c}-V^{N9c}-
wherein V^{N9c} is
(i) -CO-,
(ii) -SO-, or
(iii) -SO₂-,
RN9c is
(i) a hydrogen atom,
(ii) an optionally substituted alkyl group having 1 to 6 carbon atoms, or
(iii) an optionally substituted cycloalkyl group having 3 to 6 carbon atoms,
W^{N9} is
(a) a hydrogen atom,
(b) an optionally substituted alkyl group having 1 to 6 carbon atoms,
(c) an optionally substituted cycloalkyl group having 3 to 10 carbon atoms,
(d) an optionally substituted aryl group having 6 to 10 carbon atoms,
(e) an optionally substituted heterocyclic group containing 1 to 6 same or different atoms selected from a nitrogen atom, an oxygen atom, and a sulfur atom and having 3 to 10 ring-constituting atoms, or
(f) a halogen atom;
R^{Y7}, R^{Y7'} , R^{Y8}, and R^{Y8'} are each independently
1) a hydrogen atom,
2) a halogen atom,
3) -O-(optionally substituted alkyl having 1 to 6 carbon atoms),
4) -O-(optionally substituted cycloalkyl having 3 to 6 carbon atoms),
5) an optionally substituted alkyl group having 1 to 6 carbon atoms,
6) an optionally substituted cycloalkyl group having 3 to 6 carbon atoms, or
7) R^{Y7} and R^{Y7'}, and R^{Y8} and R^{Y8'}, are each independently optionally bonded to each other to form, together with the adjacent carbon atom, an optionally substituted cycloalkyl group having 3 to 10 carbon atoms, or an optionally substituted heterocycle containing 1 to 6 same or different atoms selected from a nitrogen atom, an oxygen atom, and a sulfur atom and having 3 to 10 ring-constituting atoms;
n9 is an integer of 1 to 6;
V⁹ is
1) -CO-,
2) -SO-,
3) -SO₂-, or
4) an optionally substituted heterocyclic divalent group having 3 to 6 ring-constituting atoms;
m9 is an integer of 0 to 6;
R^{N9'} is
1) a hydrogen atom,
2) an optionally substituted alkyl group having 1 to 6 carbon atoms,
3) an optionally substituted cycloalkyl group having 3 to 6 carbon atoms, or
4) an optionally substituted heterocyclic group having 3 to 6 ring-constituting atoms;
o9 is an integer of 2 to 6;
R^{N9"} is
1) a hydrogen atom,
2) an optionally substituted alkyl group having 1 to 6 carbon atoms,
3) an optionally substituted cycloalkyl group having 3 to 6 carbon atoms,
4) an optionally substituted heterocyclic group having 3 to 6 carbon atoms,
5) -CO-(optionally substituted alkyl having 1 to 6 carbon atoms), or
6) -CO-(optionally substituted cycloalkyl having 3 to 6 carbon atoms);
W^{9a} is
(1) an optionally substituted alkylene group having 1 to 6 carbon atoms,
(2) an optionally substituted aryl divalent group having 6 to 10 carbon atoms,
(3) an optionally substituted heteroaryl divalent group containing 1 to 6 same or different atoms selected from a nitrogen atom, an oxygen atom, and a sulfur atom and having 5 to 10 ring-constituting atoms,
(4) an optionally substituted cycloalkyl divalent group having 3 to 10 carbon atoms, or
(5) an optionally substituted heterocyclic divalent group containing 1 to 6 same or different atoms selected from a nitrogen atom, an oxygen atom, and a sulfur atom and having 3 to 10 ring-constituting atoms,
Y^{9b} is a group represented by
(1) -(CR^{Y9}R^{Y9'})ny9c-A^{Y9b}-(CR^{Y10}R^{Y10'}) ny9d-
wherein R^{Y9}, R^{Y9'}, R^{Y10}, and R^{Y10'} are each independently
1) a hydrogen atom,
2) a halogen atom,
3) -O-(optionally substituted alkyl having 1 to 6 carbon atoms),
4) -O-(optionally substituted cycloalkyl having 3 to 6 carbon atoms),
5) an optionally substituted alkyl having 1 to 6 carbon atoms, or
6) an optionally substituted cycloalkyl group having 3 to 6 carbon atoms, or
7) R^{Y9} and R^{Y9'}, and R^{Y10} and R^{Y10'}, are each independently optionally bonded to each other to form, together with the adjacent carbon atom, an optionally substituted cycloalkyl group having 3 to 10 carbon atoms, or an optionally substituted heterocyclic group containing 1 to 6 same or different atoms selected from a nitrogen atom, an oxygen atom, and a sulfur atom and having 3 to 10 ring-constituting atoms,
ny9c and ny9d are each independently an integer of 0 to 6,
A^{Y9b} is
1) a bond,
2) -O-,
3) -SO-,
4) -SO₂-,
5) -NR^{NY9e}-,
wherein R^{NY9e} is
(a) a hydrogen atom,
(b) an optionally substituted alkyl group having 1 to 6 carbon atoms,
(c) an optionally substituted cycloalkyl group having 3 to 6 carbon atoms,
(d) an optionally substituted heterocyclic group having 3 to 6 ring-constituting atoms,
(e) -CO-(optionally substituted alkyl having 1 to 6 carbon atoms),
(f) -CO-(optionally substituted cycloalkyl having 3 to 6 carbon atoms), or
6) -V^{Y9f}-NR^{NY9f}- or -NR^{NY9f}-V^{Y9f}-
wherein V^{Y9f} is
(i) -CO-,
(ii) -SO-, or
(iii) -SO₂-,
R^{NY9f} is
(i) a hydrogen atom,
(ii) an optionally substituted alkyl group having 1 to 6 carbon atoms,
(iii) an optionally substituted cycloalkyl group having 3 to 6 carbon atoms, or
(iv) an optionally substituted heterocyclic group having 3 to 6 ring-constituting atoms,
W^{9b} is
(1) a hydrogen atom,
(2) a halogen atom,
(3) an optionally substituted aryl group having 6 to 10 carbon atoms,
(4) an optionally substituted heteroaryl group containing 1 to 6 same or different atoms selected from a nitrogen atom, an oxygen atom, and a sulfur atom and having 5 to 10 ring-constituting atoms,
(5) an optionally substituted cycloalkyl group having 3 to 10 carbon atoms,
(6) an optionally substituted heterocyclic group containing 1 to 6 same or different atoms selected from a nitrogen atom, an oxygen atom, and a sulfur atom and having 3 to 10 ring-constituting atoms, or
(7) -CONR^{NW9}R^{NW9'}
wherein R^{NW9} and R^{NW9'} are each independently
1) a hydrogen atom,
2) an optionally substituted alkyl group having 1 to 6 carbon atoms, or
3) an optionally substituted cycloalkyl group having 3 to 6 carbon atoms, or
4) -NR^{NW9"}R^{NW9‴}
wherein R^{NM9"} and R^{NW9‴} are each independently
(a) a hydrogen atom,
(b) an optionally substituted alkyl group having 1 to 6 carbon atoms,
(c) an optionally substituted cycloalkyl group having 3 to 6 carbon atoms,
(d) an optionally substituted heterocyclic group having 3 to 6 ring-constituting atoms,
(e) -CO-(optionally substituted alkyl having 1 to 6 carbon atoms), or
(f) -CO-(optionally substituted cycloalkyl having 3 to 6 carbon atoms),
or a pharmacologically acceptable salt thereof.

15. The compound according to claim 14, wherein
R^{9a} is a group represented by the formula:
(1) -NR^{N9}-,
(2) -NR^{N9}-(CR^{Y7}R^{Y7'})ₙ₉-,
(3) -NR^{N9}-(CR^{Y7}R^{Y7'})ₙ₉-V⁹-(CR^{Y8}R^{Y8'})ₘ₉-,
(4) -NR^{N9}-(CR^{Y7}R^{Y7'})ₙ₉-V⁹-NR^{N9'}-(CR^{Y8}R^{Y8'})ₘ₉-,
(5) -NR^{N9}-(CR^{Y7}R^{Y7'})ₒ₉-NR^{N9'}-V⁹-(CR^{Y8}R^{Y8'})ₘ₉-,
(6) -NR^{N9}-(CR^{Y7}R^{Y7'})ₒ₉-NR^{N9"}-(CR^{Y8}R^{Y8'})ₘ₉-,
(7) -NR^{N9}-(CR^{Y7}R^{Y7'})ₒ₉-O-(CR^{Y8}R^{Y8'})ₘ₉- or
(8) -NR^{N9}-(CR^{Y7}R^{Y7'})ₒ₉-S-(CR^{Y8}R^{Y8'})ₘ₉-
(in the above-mentioned formulas, each symbol is as defined above),
or a pharmacologically acceptable salt thereof.

16. The compound according to claim 15, wherein
W^{9a} is
(1) an optionally substituted alkylene group having 1 to 6 carbon atoms,
(2) an optionally substituted aryl divalent group having 6 to 10 carbon atoms,
(3) an optionally substituted heteroaryl divalent group containing 1 to 6 same or different atoms selected from a nitrogen atom, an oxygen atom, and a sulfur atom and having 5 to 10 ring-constituting atoms, or
(4) an optionally substituted heterocyclic divalent group containing 1 to 4 nitrogen atoms, and having 5 or 6 ring-constituting atoms,
or a pharmacologically acceptable salt thereof.

17. The compound according to claim 16, wherein
ny9c and ny9d are both 0, and A^{Y9b} is a bond,
or a pharmacologically acceptable salt thereof.

18. The compound according to claim 16 or 17, wherein
W^{9a} is
(1) an optionally substituted alkylene group having 1 to 6 carbon atoms,
(2) an optionally substituted phenylene group,
(3) an optionally substituted pyridine-diyl group,
(4) an optionally substituted thiazole-diyl group, or
(5) an optionally substituted piperidine-diyl group,
or a pharmacologically acceptable salt thereof.

19. The compound according to claim 14, wherein
R^{9a} is a group represented by the formula: 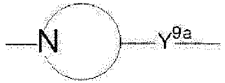 wherein 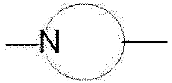
and Y^{9a} are as defined above,
or a pharmacologically acceptable salt thereof.

20. The compound according to claim 19, wherein
ny9c and ny9d are both 0, and A^{Y9b} is a bond,
or a pharmacologically acceptable salt thereof.

21. The compound according to claim 19 or 20, wherein
W^{9b} is a hydrogen atom,
or a pharmacologically acceptable salt thereof.

22. The compound according to any one of claims 14, 19 to 21, wherein
a group represented by the formula: 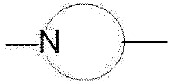
is an azetidinyl group, a pyrrolidinyl group, a piperidyl group, a piperazinyl group, a morpholinyl group, a tetrahydronaphthyridinyl group, a tetrahydrothiazolopyridyl group, or a tetrahydroisoquinolyl group, each of which is optionally further substituted by an oxo group or a halogen atom in addition to Y^{9a},
or a pharmacologically acceptable salt thereof.

23. The compound according to claim 9, wherein
A⁶ is -NR^{6a1}-,
R6a1 is
(1) -Y^{10a}-W^{10a}-Y^{10b}-W^{10b}
wherein Y^{10a} is
1) a bond,
2)
- (CR^{Y11}R^{Y11'})ₙ₁₀-,
3)
- (CR^{Y11}R^{Y11'})ₙ₁₀-V¹⁰-(CR^{Y12}R^{Y12'})ₘ₁₀-,
4)
- (CR^{Y11}R^{Y11'})ₙ₁₀-V¹⁰-NR^{N10'}-(CR^{Y12}R^{Y12'})ₘ₁₀-,
5)
- (CR^{Y11}R^{Y11'})ₒ₁₀-NR^{N10'}-V¹⁰-(CR^{Y12}R^{Y12'})ₘ₁₀-,
6)
- (CR^{Y11}R^{Y11'})ₒ₁₀-NR^{N10"}- (CR^{Y12}R^{Y12'})ₘ₁₀-,
7)
- (CR^{Y11}R^{Y11'})ₒ₁₀-O-(CR^{Y12}R^{Y12'})ₘ₁₀-,
or
8)
- (CR^{Y11}R^{Y11'})ₒ₁₀-S-(CR^{Y12}R^{Y12'})ₘ₁₀-
wherein R^{Y11}, R^{Y11'}, R^{Y12}, and R^{Y12'} are each independently
(a) a hydrogen atom,
(b) a halogen atom,
(c) -O-(optionally substituted alkyl having 1 to 6 carbon atoms),
(d) -O-(optionally substituted cycloalkyl having 3 to 6 carbon atoms),
(e) an optionally substituted alkyl group having 1 to 6 carbon atoms,
(f) an optionally substituted cycloalkyl group having 3 to 6 carbon atoms, or
(g) R^{Y11} and R^{Y11'}, and R^{Y12} and R^{Y12'}, are each independently optionally bonded to form an optionally substituted cycloalkyl having 3 to 10 carbon atoms, or an optionally substituted heterocycle containing 1 to 6 atoms selected from a nitrogen atom, an oxygen atom, and a sulfur atom and having 3 to 10 ring-constituting atoms,
n10 is an integer of 1 to 6,
V¹⁰ is
(a) -CO-,
(b) -SO-, or
(c) -SO₂-,
m10 is an integer of 0 to 6,
R^{N10'} is
(a) a hydrogen atom,
(b) an optionally substituted alkyl group having 1 to 6 carbon atoms,
(c) an optionally substituted cycloalkyl group having 3 to 6 carbon atoms, or
(d) an optionally substituted heterocyclic group having 3 to 6 ring-constituting atoms,
o10 is an integer of 2 to 6,
R^{N10"} is
(a) a hydrogen atom,
(b) an optionally substituted alkyl group having 1 to 6 carbon atoms,
(c) an optionally substituted cycloalkyl group having 3 to 6 carbon atoms,
(d) an optionally substituted heterocyclic group having 3 to 6 carbon atoms,
(e) -CO-(optionally substituted alkyl having 1 to 6 carbon atoms), or
(f) -CO-(optionally substituted cycloalkyl having 3 to 6 carbon atoms),
W^{10a} is
1) an optionally substituted alkylene group having 1 to 6 carbon atoms,
2) an optionally substituted aryl divalent group having 6 to 10 carbon atoms,
3) an optionally substituted heteroaryl divalent group containing 1 to 6 atoms selected from a nitrogen atom, an oxygen atom, and a sulfur atom and having 5 to 10 ring-constituting atoms,
4) an optionally substituted cycloalkyl divalent group having 3 to 10 carbon atoms, or
5) an optionally substituted heterocycle divalent group containing 1 to 6 atoms selected from a nitrogen atom, an oxygen atom, and a sulfur atom and having 3 to 10 ring-constituting atoms,
Y^{10b} is
1) - (CR^{Y13}R^{Y13'})_{ny10c}-A^{Y10b}-(CR^{Y14}R^{Y14'})_{ny10d}-
wherein R^{Y13}, R^{Y13'}, R^{Y14}, and R^{Y14'} are each independently
(a) a hydrogen atom,
(b) a halogen atom,
(c) -O-(optionally substituted alkyl having 1 to 6 carbon atoms),
(d) -O-(optionally substituted cycloalkyl having 3 to 6 carbon atoms),
(e) an optionally substituted alkyl group having 1 to 6 carbon atoms,
(f) an optionally substituted cycloalkyl group having 3 to 6 carbon atoms, or
(g) R^{Y13} and R^{Y13'}, and R^{Y14} and R^{Y14'}, are each independently optionally bonded to each other to form, together with the adjacent carbon atom, an optionally substituted monocyclic or condensed cycloalkane having 3 to 10 carbon atoms, or an optionally substituted heterocycle containing 1 to 6 same or different atoms selected from a nitrogen atom, an oxygen atom,
and a sulfur atom and having 3 to 10 ring-constituting atoms,
ny10c and ny10d are each independently an integer of 0 to 6,
A^{Y10b} is
(a) a bond,
(b) -O-,
(c) -SO-,
(d) -SO₂-,
(e) -NR^{NY10e}-
wherein R^{NY10e} is
(i) a hydrogen atom,
(ii) an optionally substituted alkyl group having 1 to 6 carbon atoms,
(iii) an optionally substituted cycloalkyl group having 3 to 6 carbon atoms,
(iv) an optionally substituted heterocyclic group having 3 to 6 carbon atoms,
(v) -CO-(optionally substituted alkyl having 1 to 6 carbon atoms),
(vi) -CO-(optionally substituted cycloalkyl having 3 to 6 carbon atoms), or
(f) -V^{Y10f}-NR^{NY10f}- or NR^{NY10f}-V^{Y10f}-
wherein V^{Y10f} is
-CO-,
-SO- or
-SO₂-,
R^{NY10f} is
a hydrogen atom,
an optionally substituted alkyl group having 1 to 6 carbon atoms,
an optionally substituted cycloalkyl group having 3 to 6 carbon atoms, or
an optionally substituted heterocyclic group having 3 to 6 carbon atoms,
W^{10b} is
1) a hydrogen atom,
2) a halogen atom,
3) an optionally substituted aryl group having 6 to 10 carbon atoms,
4) an optionally substituted heteroaryl group containing 1 to 6 same or different atoms selected from a nitrogen atom, an oxygen atom, and a sulfur atom and having 5 to 10 ring-constituting atoms,
5) an optionally substituted monocyclic or condensed cycloalkyl having 3 to 10 carbon atoms,
6) an optionally substituted heterocyclic group containing 1 to 6 atoms selected from a nitrogen atom, an oxygen atom, and a sulfur atom and having 3 to 10 ring-constituting atoms,
7) -CONR^{NW10}R^{NW10'}
wherein R^{NW10} and R^{NW10'} may be the same or different and are each independently
(a) a hydrogen atom,
(b) an optionally substituted alkyl group having 1 to 6 carbon atoms, or
(c) an optionally substituted cycloalkyl group having 3 to 6 carbon atoms, or
8) -NR^{NW10"}R^{NW10‴}
wherein R^{NW10"} and R^{NW10‴} may be the same or different and are each independently
(a) a hydrogen atom,
(b) substituted or unsubstituted alkyl having 1 to 6 carbon atoms,
(c) an optionally substituted cycloalkyl having 3 to 6 carbon atoms,
(d) an optionally substituted heterocyclic group having 3 to 6 ring-constituting atoms,
(e) -CO-(optionally substituted alkyl having 1 to 6 carbon atoms), or
(f) -CO-(optionally substituted cycloalkyl having 3 to 6 carbon atoms),
or a pharmacologically acceptable salt thereof.

24. The compound according to claim 23, wherein
Y^{10a} is
(1) a bond,
(2)
- (CR^{Y11}R^{Y11'})ₙ₁₀-
or
(3)
-(CR^{Y11}R^{Y11'})ₒ₁₀-O-(CR^{Y12}R^{Y12'})ₘ₁₀-
wherein each symbol is as defined above,
or a pharmacologically acceptable salt thereof.

25. The compound according to claim 23 or 24, wherein
W^{10a} is
(1) an optionally substituted aryl divalent group having 6 to 10 carbon atoms,
(2) an optionally substituted heteroaryl divalent group containing 1 to 6 same or different atoms selected from a nitrogen atom, an oxygen atom, and a sulfur atom and having 5 to 10 ring-constituting atoms,
(3) an optionally substituted heterocyclic divalent group containing 1 to 6 same or different atoms selected from a nitrogen atom, an oxygen atom, and a sulfur atom and having 3 to 10 ring-constituting atoms, or
(4) an optionally substituted alkylene group having 1 to 6 carbon atoms,
or a pharmacologically acceptable salt thereof.

26. The compound according to any one of claims 23 to 25, wherein
ny10c and ny10d are both 0, and
A^{Y10b} is a bond,
or a pharmacologically acceptable salt thereof.

27. The compound according to any one of claims 23 to 26, wherein
W^{10a} is
(1) a phenylene group,
(2) a pyridine-diyl group,
(3) an optionally substituted monocyclic heterocyclic divalent group containing 1 or 2 same or different atoms selected from a nitrogen atom, an oxygen atom, and a sulfur atom, having 4 to 7 ring-constituting atoms, or
(4) an optionally substituted alkylene group having 1 to 6 carbon atoms,
or a pharmacologically acceptable salt thereof.

28. The compound according to any one of claims 23 to 27, wherein
W^{10b} is
(1) an optionally substituted aryl group having 6 to 10 carbon atoms, or
(2) an optionally substituted heteroaryl containing 1 to 6 atoms selected from a nitrogen atom, an oxygen atom, and a sulfur atom, and having 5 to 10 ring-constituting atoms,
or a pharmacologically acceptable salt thereof.

29. The compound according to any one of claims 1 to 28, wherein
E is
(1) a bond
(2) -CO-,
(3) -SO-, or
(4) -SO₂-,
X is
(1) an optionally substituted alkyl group having 1 to 6 carbon atoms,
(2) an optionally substituted cycloalkyl group having 3 to 10 carbon atoms,
(3) an optionally substituted aryl group having 6 to 10 carbon atoms,
(4) an optionally substituted heterocyclic group containing 1 to 6 same or different atoms selected from a nitrogen atom, an oxygen atom, and a sulfur atom and having 3 to 10 ring-constituting atoms, or
(5) an optionally substituted heteroaryl group containing 1 to 6 same or different atoms selected from a nitrogen atom, an oxygen atom, and a sulfur atom and having 5 to 10 ring-constituting atoms,
or a pharmacologically acceptable salt thereof.

30. The compound according to any one of claims 1 to 29, wherein
E is -CO-,
or a pharmacologically acceptable salt thereof.

31. The compound according to any one of claims 1 to 31, wherein
X is
-CHR^{m}-X¹-X²
wherein
R^{m} is
(1) an optionally substituted alkyl group having 1 to 10 carbon atoms,
(2) an optionally substituted aryl group,
(3) an optionally substituted heteroaryl group,
(4) an optionally substituted heterocyclic group, or
(5) an optionally substituted cycloalkyl group having 3 to 10 carbon atoms,
X¹ is
(1) a bond,
(2) an optionally substituted aryl divalent group having 6 to 10 carbon atoms,
(3) an optionally substituted heteroaryl divalent group containing 1 to 6 same or different atoms selected from a nitrogen atom, an oxygen atom, and a sulfur atom and having 5 to 10 ring-constituting atoms,
(4) an optionally substituted cycloalkyl divalent group having 3 to 10 carbon atoms,
(5) an optionally substituted heterocyclic divalent group containing 1 to 6 same or different atoms selected from a nitrogen atom, an oxygen atom, and a sulfur atom and having 3 to 10 ring-constituting atoms, or
(6) -NR^{1X}-
wherein
R^{1X} is
1) a hydrogen atom,
2) an optionally substituted alkyl group having 1 to 6 carbon atoms, or
3) an optionally substituted cycloalkyl group having 3 to 6 carbon atoms,
X² is
(1) a hydrogen atom,
(2) an optionally substituted alkyl group,
(3) an optionally substituted aryl group having 6 to 10 carbon atoms,
(4) optionally substituted heteroaryl group containing 1 to 6 same or different atoms selected from a nitrogen atom, an oxygen atom, and a sulfur atom and having 5 to 10 ring-constituting atoms,
(5) an optionally substituted cycloalkyl group having 3 to 10 carbon atoms,
(6) optionally substituted heterocyclic group containing 1 to 6 same or different atoms selected from a nitrogen atom, an oxygen atom, and a sulfur atom and having 3 to 10 ring-constituting atoms,
(7) -CO-,
(8) -CO-(optionally substituted alkyl),
(9) -CO-(optionally substituted aryl having 6 to 10 carbon atoms),
(10) -CO-(optionally substituted heteroaryl containing 1 to 6 same or different atoms selected from a nitrogen atom, an oxygen atom, and a sulfur atom and having 5 to 10 ring-constituting atoms),
(11) -CO-(optionally substituted cycloalkyl having 3 to 10 carbon atoms),
(12) -CO-(optionally substituted heterocyclic group containing 1 to 6 same or different atoms selected from a nitrogen atom, an oxygen atom, and a sulfur atom and having 3 to 10 ring-constituting atoms),
(13) -CO-(optionally substituted alkylene having 1 to 6 carbon atoms)-(O- optionally substituted alkylene having 1 to 6 carbon atoms)ᵣ-
wherein r is an integer of 1 to 6,
(14) -CO-(optionally substituted alkylene having 1 to 6 carbon atoms)-O-(optionally substituted alkylene having 1 to 6 carbon atoms)-CONH-(optionally substituted alkylene having 1 to 6 carbon atoms)-,
(15) -CO-(optionally substituted alkylene having 1 to 6 carbon atoms)-CONH-(optionally substituted alkylene having 1 to 6 carbon atoms)-, or
(16) -OR^{2X} or -NR^{2X}R^{2X'}
wherein
R^{2X} and R^{2X'} are each independently
1) a hydrogen atom,
2) an optionally substituted alkyl group having 1 to 6 carbon atoms, or
3) an optionally substituted cycloalkyl group having 3 to 6 carbon atoms,
(provided when X¹ is NR^{1x}, then X² is not -OR^{2X} or -NR^{2X}R^{2X'},
or a pharmacologically acceptable salt thereof.

32. The compound according to claim 31, wherein
X¹ is
-NR^{1X}-
wherein R^{1X} is as defined above,
or a pharmacologically acceptable salt thereof.

33. The compound according to claim 32, wherein
X² is
(1) -CO-(optionally substituted alkyl having 1 to 6 carbon atoms),
(2) -CO-(optionally substituted cycloalkyl having 3 to 6 carbon atoms),
(3) -CO-(optionally substituted alkylene having 1 to 6 carbon atoms)-(O- optionally substituted alkylene having 1 to 6 carbon atoms)ᵣ-
wherein r is an integer of 1 to 6,
(4) -CO-(optionally substituted alkylene having 1 to 6 carbon atoms)-O-(optionally substituted alkylene having 1 to 6 carbon atoms)-CONH-(optionally substituted alkylene having 1 to 6 carbon atoms)-, or
(5) -CO-(optionally substituted alkylene having 1 to 6 carbon atoms)-CONH-(optionally substituted alkylene having 1 to 6 carbon atoms)-,
or a pharmacologically acceptable salt thereof.

34. The compound according to claim 31, wherein
X¹ is
(1) an optionally substituted aryl divalent group having 6 to 10 carbon atoms, or
(2) an optionally substituted heteroaryl divalent group containing 1 to 6 same or different atoms selected from a nitrogen atom, an oxygen atom, and a sulfur atom and having 5 to 10 ring-constituting atoms,
or a pharmacologically acceptable salt thereof.

35. The compound according to claim 34, wherein
X¹ is
(1) an optionally substituted heteroaryl divalent group containing 1 to 4 same or different atoms selected from a nitrogen atom, an oxygen atom, and a sulfur atom, and having 5 or 6 ring-constituting atoms,
or a pharmacologically acceptable salt thereof.

36. The compound according to claim 34, wherein
X¹ is
(1) an optionally substituted heteroaryl divalent group containing 1 to 4 same or different atoms selected from a nitrogen atom, an oxygen atom, and a sulfur atom, and having 5 ring-constituting atoms,
or a pharmacologically acceptable salt thereof.

37. The compound according to any one of claims 1 to 36, wherein
L is
- (Lₐ)q-
wherein
q is an integer of 1 to 100, and
Lₐ in the number of q are each independently
(1) a bond,
(2) CR^{L1}R^{L1'},
(3) O,
(4) S,
(5) CO,
(6) SO,
(7) SO₂,
(8) NR^{L1},
(9) CONR^{L1}
(10) NR^{L1}CO,
(11) NR^{L1}CONR^{L1'},
(12) SONR^{L1},
(13) NR^{L1}SO,
(14) SO₂NR^{L1},
(15) NR^{L1}SO₂,
(16) NR^{L1}SO₂NR^{L1'}
(17) CR^{L1}=CR^{L1'},
(18) C≡C,
(19) SiR^{L1}R^{L1'},
(20) P(O)R^{L1},
(21) P(O)OR^{L1},
(22) NR^{L1}C(=NCN)NR^{L1'},
(23) -NR^{L1}C(=NCN) -,
(24) an optionally substituted cycloalkyl divalent group,
(25) an optionally substituted heterocyclic divalent group,
(26) an optionally substituted aryl divalent group, or
(27) an optionally substituted heteroaryl divalent group
(in the above-mentioned formulas, R^{L1} and R^{L1'} are each independently a group selected from
1) a hydrogen atom
2) a halogen atom,
3)
-CN,
4)
-NO₂,
5)
-SF₅,
6)
-CO₂H,
7)
-N(R^{L2}R^{L2'}),
8)
-A^{L2}R^{L2}
9) an optionally substituted alkyl group,
10) an optionally substituted cycloalkyl group,
11) an optionally substituted heterocyclic group,
12) an optionally substituted aryl group,
13) an optionally substituted heteroaryl group,
14)
-SO₂R^{L2},
15)
-P(O)(OR^{L2})OR^{L2'},
16)
-C≡CR^{L2},
17)
-C(R^{L2})=C(R^{L2'}R^{L2"}),
18)
-COR^{L2},
19)
-CON(R^{L2}R^{L2'}),
20)
-SO₂N(R^{L2}R^{L2'}),
21)
-N(R^{L2})CON(R^{L2'}R^{L2"}),
and
22)
-N(R^{L2})SO₂N(R^{L2'}R^{L2"})
(in the above-mentioned formulas, A^{L2} is an oxygen atom or a sulfur atom,
R^{L2}, R^{L2'}, and R^{L2"} are each independently
a) a hydrogen atom,
b) an optionally substituted C₁-C₈ alkyl group, or
c) an optionally substituted C₃-C₈ cycloalkyl group, or a pharmacologically acceptable salt thereof.

38. The compound according to claim 37, wherein
q is an integer of 1 to 50,
or a pharmacologically acceptable salt thereof.

39. The compound according to claim 37 or 38, wherein
L is -L₁-L₂-L₃-
wherein
L₁ and L₃ are each independently
(1) a bond,
(2) CR^{L1} R^{L1'},
(3) O,
(4) S,
(5) SO,
(6) SO₂,
(7) NR^{L1},
(8) SO₂NR^{L1},
(9) NR^{L1}SO₂
(10) SONR^{L1},
(11) NR^{L1}SO,
(12) CONR^{L1},
(13) NR^{L1}CO,
(14) NR^{L1}CONR^{L1'},
(15) NR^{L1}SO₂NR^{L1'}, or
(16) CO, and
L₂ is
(CH₂)ₚ₁ₐ-O-(CH₂-CH₂-O)ₚ₂ₐ-(CH₂)ₚ₃ₐ
wherein
p1a and p3a are each an integer of 0 to 10 when an atom that directly binds of the adjacent L₁ or L₃ is a carbon atom or bond, and an integer of 2 to 10 in other cases, and
p2a is an integer of 0 to 10,
or a pharmacologically acceptable salt thereof.

40. The compound according to claim 37 or 38, wherein
L is -L_{b1}-L_{b2}-L_{b3}-
wherein
L_{b1} and L_{b3} are each independently
(1) a bond,
(2) CR^{L1}R^{L1'},
(3) O,
(4) S,
(5) SO,
(6) SO₂,
(7) NR^{L1},
(8) SO₂NR^{L1},
(9) NR^{L1}SO₂,
(10) SONR^{L1},
(11) NR^{L1}SO,
(12) CONR^{L1},
(13) NR^{L1}CO,
(14) NR^{L1}CONR^{L1'},
(15) NR^{L1}SO₂NR^{L1'}, or
(16) CO, and
L_{b2} is
(1) a bond,
(2)
(CH₂)₁₋₁₀,
(3)
(CH₂)₀₋₆-O-(CH₂)₀₋₆,
(4)
(CH₂)₀₋₆-CONH-(CH₂)₀₋₆,
(5)
(CH₂)₀₋₆-NHCO-(CH₂)₀₋₆,
(6)
(CH₂)₀₋₆-NH-(CH₂)₀₋₆,
(7)
(CH₂)₀₋₆-NHSO₂-(CH₂)₀₋₆, or
(8)
(CH₂)₀₋₆-SO₂NH-(CH₂)₀₋₆,
wherein R^{L1} and R^{L1'} are as defined above,
or a pharmacologically acceptable salt thereof.

41. The compound according to any one of claims 1 to 40, wherein
A is a group having a moiety capable of binding to a target protein or a moiety that binds to the target protein, or a pharmacologically acceptable salt thereof.

42. The compound according to any one of claims 1 to 41, wherein
the target protein to which A binds is a protein having a biological function selected from the group consisting of structure, regulation, hormone, enzyme, gene regulation, immunity, contraction, storage, transport, and signal transduction, or a pharmacologically acceptable salt thereof.

43. The compound according to any one of claims 1 to 41, wherein
the target protein to which A binds is selected from the group consisting of structural protein, receptor, enzyme, cell surface protein, and proteins related to integrated cellular function, including proteins involved in catalytic activity, aromatase activity, motor activity, helicase activity, metabolic process (anabolism and catabolism), antioxidant activity, proteolysis, biosynthesis, kinase activity, oxidoreductase activity, transferase activity, hydrolase activity, lyase activity, isomerase activity, ligase activity, enzyme regulatory factor activity, signal transducer activity, structural molecular activity, binding activity (protein, lipid carbohydrates), receptor activity, cellular motility, membrane fusion, intercellular signal transduction, control of biological processes, development, cell differentiation, stimulus response, cell adhesion, cell death, transport (protein transporter activity, nuclear transport, transporter activity, channel transporter activity, carrier activity, permease activity, secretion activity, electron transporter activity), pathogenesis, virus outer shell, chaperon regulatory factor activity, nucleic acid binding activity, transcriptional regulator activity, epigenetics control, aggregation, extracellular organization, biogenetic activity, or translation regulatory factor activity, or a pharmacologically acceptable salt thereof.

44. The compound according to any one of claims 1 to 41, wherein
the target protein to which A binds is selected from the group consisting of proteins related to cancer related protein, autoimmune disease related protein, inflammatory disease related protein, neurodegenerative disease related protein, muscular disease related protein, sensory system disease related protein, circulatory disease related protein, metabolic disease related protein, and genetic disease related protein, or a pharmacologically acceptable salt thereof.

45. A medicament comprising the compound according to claim 1 or a pharmacologically acceptable salt thereof as an active ingredient.

46. The medicament according to claim 45 which is a prophylactic or therapeutic agent for diseases caused by dysregulation of protein activity.

47. A pharmaceutical composition comprising the compound according to claim 1 or a pharmacologically acceptable salt thereof as an active ingredient together with a pharmaceutically acceptable carrier.

48. Use of the compound according to claim 1 or a pharmacologically acceptable salt thereof for the production of a prophylactic or therapeutic agent for a disease caused by dysregulation of protein activity.
